# EUROPEAN PATENT SPECIFICATION

(11) **EP 1 912 494 B1**
(45) Date of publication and mention of the grant of the patent: **17.10.2012**
(21) Application number: 06776794.7
(22) Date of filing: 11.08.2006
(51) Int. Cl.: A01K 67/027, C12N 15/85, C07K 14/47, C12N 15/12, C12N 9/12, C12N 15/54

(54) **Isolation of T-complex distorters and applications thereof**
Isolation der T-Komplex-Distorter sowie ihre Anwendungen
Isolement des générateurs de distorsion du complexe T et applications correspondantes

(30) Priority: 12.08.2005 EP 05017651
(43) Date of publication of application: 23.04.2008
(73) Proprietor: Max-Planck-Gesellschaft zur Förderung der Wissenschaften e.V., 80539 München (DE)
(72) Inventor: HERRMANN, Bernhard, 14163 Berlin (DE); BAUER, Hermann, 14195 Berlin (DE)
(74) Representative: Vossius & Partner
(86) International application number: PCT/EP2006/007977
(87) International publication number: WO 2007/020026

(56) References cited:
- WO-A-00/40607
- WO-A-99/25815
- PIAO Y ET AL: "Construction of long-transcript enriched cDNA libraries from submicrogram amounts of total RNAs by a universal PCR amplification method." GENOME RESEARCH. SEP 2001, vol. 11, no. 9, September 2001 (2001-09), pages 1553-1558, XP002373477 ISSN: 1088-9051 & DATABASE GENBANK NCBI; 20 September 2004 (2004-09-20), PIAO ET AL.: "Construction of long-transcript enriched cDNA libraries from submicrogram amounts of total RNAs by a universal PCR amplification method" XP002373587 Database accession no. NM_145968.1 cited in the application
- PASTERIS N G ET AL: "Isolation, characterization, and mapping of the mouse and human Fgd2 genes, faciogenital dysplasia (FGD1; Aarskog syndrome) gene homologues." GENOMICS. 15 AUG 1999, vol. 60, no. 1, 15 August 1999 (1999-08-15), pages 57-66, XP004444836 ISSN: 0888-7543
- SCHIMENTI JOHN C ET AL: "Mutations in Serac1 or Synj2 cause proximal t haplotype-mediated male mouse sterility but not transmission ratio distortion." PROCEEDINGS OF THE NATIONAL ACADEMY OF SCIENCES OF THE UNITED STATES OF AMERICA. 1 MAR 2005, vol. 102, no. 9, 1 March 2005 (2005-03-01), pages 3342-3347, XP002373479 ISSN: 0027-8424 cited in the application
- HERRMANN B G ET AL: "A protein kinase encoded by the t complex responder gene causes non-mendelian inheritance." NATURE. 11 NOV 1999, vol. 402, no. 6758, 11 November 1999 (1999-11-11), pages 141-146, XP002373480 ISSN: 0028-0836 cited in the application
- LYON MARY F: "Transmission ratio distortion in mice." ANNUAL REVIEW OF GENETICS. 2003, vol. 37, 2003, pages 393-408, XP002373584 ISSN: 0066-4197 cited in the application
- MARTIN-DELEON PATRICIA A ET AL: "Spam1-associated transmission ratio distortion in mice: Elucidating the mechanism" REPRODUCTIVE BIOLOGY AND ENDOCRINOLOGY, vol. 3, 10 August 2005 (2005-08-10), pages 32-43, XP002373481 ISSN: 1477-7827
- BAUER HERMANN ET AL: "The t complex-encoded GTPase-activating protein Tagap1 acts as a transmission ratio distorter in mice" NATURE GENETICS, vol. 37, no. 9, September 2005 (2005-09), pages 969-973, XP002372066 ISSN: 1061-4036

## Description

The present invention relates to a method for producing a transgenic non human male mammal , as defined in the appended claims, wherein the transgene(s) confer(s) a change in the transmission ratio of (a) genetic trait(s) to the offspring of said non human male mammal to a non-Mendelian ratio, said method comprising introducing (a) a first nucleic acid molecule encoding an expression product as defined with a Responder function into a chromosome of a non-human germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s); and (b) at least one second nucleic acid molecule encoding an expression product with a Distorter function into (a) chromosome(s) of a non-human germ cell, (fertilized) egg cell, embryonic cell or a cell derived therefrom, of the same species as the transgenic male to be prepared, wherein said expression product with a Distorter function is one of the defined to factors involved in G protein signaling, wherein said first nucleic acid molecule encoding an expression product with Responder function and said at least one second nucleic acid molecule encoding an expression product with a Distorter function are introduced into the same or different chromosomes.

Furthermore, the invention relates to a method for producing a transgenic non human male mammal , as defined in the appended claims, wherein the transgene(s) confer(s) a change in the transmission ratio of (a) genetic trait(s) to the offspring of said non human male mammal to a non-Mendelian ratio, said method comprising introducing (a) a first nucleic acid molecule encoding an expression product as defined with a Responder function into a chromosome of a non-human germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s); and (b) at least one second nucleic acid molecule encoding an expression product directed against the Distorter function into (a) chromosome(s) of a non-human germ cell, (fertilized) egg cell, embryonic cell or a cell derived therefrom, of the same species as the transgenic male to be prepared, wherein said Distorter function is an expression product which is encoded by a nucleic acid molecule encoding an expression product with a Distorter function, wherein said expression product with a Distorter function is, one of the defined factors involved in G protein signaling; and/or (c) a second nucleic acid molecule for inactivation of the Distorter function by homologous recombination, wherein said second nucleic acid molecule for inactivation of the Distorter function by homologous recombination is at least partially identical to said nucleic acid molecule encoding an expression product with a Distorter function, wherein said first nucleic acid molecule encoding an expression product with a Responder function and said at least one second nucleic acid molecule encoding an expression product directed against the Distorter function and/or said second nucleic acid molecule for inactivation of the Distorter function by homologous recombination are introduced into the same or different chromosomes, thereby partially or completely inactivating the Distorter function.

In this specification, a number of documents including manufacturers' manuals are cited.

The mouse t-complex, a region of approximately 12 cM genetic distance on the proximal part of chromosome 17, contains several loci acting in concert to produce a phenomenon called transmission ratio distortion (TRD). The latter designation indicates the fact that the so-called t-haplotype form of this chromosomal region has a selective advantage over the wild type form in that it is transmitted to the offspring at non-Mendelian ratios of up to 99%. This transmission at non-Mendelian ratio is achieved by the concerted action of at least five loci, the *t complex Distorters* Tcd 1 a and Tcd1b (D1a, D1b), Tcd2 (D2) and Tcd3 (D3), and the *t complex responder, Tcr* (R^{t})(Lyon 1984, Lyon et al 2000). More Distorters have been postulated (Silver and Remis 1987).

According to Lyon's model (Lyon 1986) which formally explains the genetic interactions of these loci, D1, D2 and D3 act strongly and harmfully on the wild type allele of the Responder and weakly on the t form of the Responder (R^{t}), leading to distortion in favor of R^{t}. R^{t} might protect sperm carrying it from this harmful action of the Distorters. The Distorters act in trans while the Responder acts in cis. This means that the chromosome, which contains R^{t} is transmitted at non-Mendelian ratio to the offspring. If D2 or all the Distorters are present, the chromosome containing R^{t} is transmitted at a frequency of more than 50% up to 99% to the offspring. If no Distorter or only D1 or D3 are present, however, the chromosome containing R^{t} is transmitted at less than 50% to the offspring (as low as 12%, "low" phenotype). The Distorters are only transmitted at ratios over 50% if they are tightly linked to R^{t}. The trans-acting and cis-acting properties of the Distorters and the Responder, respectively, have been demonstrated by the transmission ratio properties of so-called partial t-haplotypes, which carry only a subset of the above named loci.

Genetic mapping of molecular markers on partial t-haplotypes allowed a rough localization of D1a, D1b, D2, D3 and R^{t} to subregions of the T/t-complex and relative to these molecular markers (Lyon 1984); (Fox, Martin et al. 1985); (Herrmann, Bucan et al. 1986); (Silver and Remis 1987); (Bullard, Ticknor et al. 1992); (Lyon et al 2000). Only one locus, R^{t} could be mapped fairly precisely to a region of appr. 200 kb, the so-called T66B region (renamed later Leh66B ; (Fox, Martin et al. 1985); (Schimenti, Vold et al. 1987); (Nadeau, Varnum et al. 1989); (Rosen, Bullard et al. 1990); (Bullard, Ticknor et al. 1992)). The genomic region T66B has been cloned molecularly and analyzed. A partial restriction map covering approximately 145kb of it has been published ((Schimenti, Vold et al. 1987); (Rosen, Bullard et al. 1990); (Bullard, Ticknor et al. 1992)).

An extensive and careful search of this region for genes expressed during spermatogenesis led to the identification of a fusion gene expressed during spermiogenesis, the haploid phase of sperm development. Molecular and genetic analyses showed that the fusion gene encoding a mutant form of a novel protein kinase, Smok, represents Tcr (Herrmann, Koschorz et al. 1999). Transgene analyses demonstrated that Smok^{Tcr}, in combination with Tcd loci, distorts the transmission ratio of itself and preferably closely linked genetic traits. Co-segregation of a transgene construct encoding Smok^{Tcr} with the Y-chromosome resulted in sex ratio distortion (Herrmann, Koschorz et al. 1999).

t complex Distorters could only be mapped very roughly to large chromosomal subregions of several megabase each in size due to suppression of meiotic recombination between the t-haplotype and the wild type chromosome. Rare recombinants have occurred between these chromosomes allowing separation of the different loci, but molecular access to the Distorter loci is extremely difficult. Several attempts to isolate t-Distorters have been reported, though none of the candidates has been verified by genetic means (for review see (Schimenti 2000); (Lyon 2003), Systematic approaches using deletion mapping in the Tcd1 region and candidate gene isolation also has failed to identify a Distorter at the molecular level (Planchart, You et al. 2000); (Lyon, Schimenti et al. 2000).

In Schimenti et al. (2005) a BAC encoding several genes was used for testing of its potential to rescue a sterility phenotype in transgenic animals. Two genes encoded on the BAC were disclosed as candidates for expressing rescuing activity, among them Synj2. An additional test for distorter activity encoded on the BAC was negative, thus none of the genes was linked to the transmission distortion phenomenon. The fact that Synj is related to G-protein signalling is merely fortuitous, and the data excluded a relation of any gene encoded on the BAC to transmission ratio distortion. Thus, the authors were able to relate two genes of different nature to male fertility rather than to isolate a candidate distorter.

The combined teachings of the prior art thus did not provide any clue how the genetic elements responsible for the Distorter phenotype might be identified and, hence, did not disclose any means of applicability related to said genetic entity. A preferred goal would be the targeted transmission ratio distortion using, as a basis, the molecular entity of the Distorter(s). The technical problem underlying the present invention therefore was to overcome these long standing prior art difficulties and to provide such means.

The solution to said technical problem is achieved by providing the embodiments characterized in the claims

The present invention relates to a method for producing a transgenic non human male mammal as defined in the appended claims, wherein the transgene(s) confer(s) a change in the transmission ratio of (a) genetic trait(s) to the offspring of said non human male mammal to a non-Mendelian ratio, said method comprising introducing (a) a first nucleic acid molecule encoding the defined expression product with a Responder function into a chromosome of a non-human germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s); and (b) at least one second nucleic acid molecule encoding an expression product with a Distorter function into (a) chromosome(s) of a non-human germ cell, (fertilized) egg cell, embryonic cell or a cell derived therefrom, of the same species as the transgenic male to be prepared, wherein said expression product with a Distorter function is one of the defined factors involved in G protein signaling, wherein said first nucleic acid molecule encoding an expression product with Responder function and said at least one second nucleic acid molecule encoding an expression product with a Distorter function are introduced into the same or different chromosomes.

Furthermore, the invention relates to a method for producing a transgenic non human male mammal as defined in the appended claim, wherein the transgene(s) confer(s) a change in the transmission ratio of (a) genetic trait(s) to the offspring of said non human male mammal to a non-Mendelian ratio, said method comprising introducing (a) a first nucleic acid molecule encoding the defined expression product with a Responder function into a chromosome of a non-human germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s); and (b) at least one second nucleic acid molecule encoding an expression product directed against the Distorter function into (a) chromosome(s) of a non-human germ cell, (fertilized) egg cell, embryonic cell or a cell derived therefrom, of the same species as the transgenic male to be prepared, wherein said Distorter function is an expression product which is encoded by a nucleic acid molecule encoding an expression product with a Distorter function, wherein said expression product with a Distorter function is one of the defined factors involved in G protein signaling; and/or (c) a second nucleic acid molecule for inactivation of the Distorter function by homologous recombination, wherein said second nucleic acid molecule for inactivation of the Distorter function by homologous recombination is at least partially identical to said nucleic acid molecule encoding an expression product with a Distorter function, wherein said first nucleic acid molecule encoding an expression product with a Responder function and said at least one second nucleic acid molecule encoding an expression product directed against the Distorter function and/or said second nucleic acid molecule for inactivation of the Distorter function by homologous recombination are introduced into the same or different chromosomes, thereby partially or completely inactivating the Distorter function.

The term "genetic trait" relates to a heritable feature or characteristic of an organism encoded by (a) nucleic acid molecule(s) contained in its genome, comprising naturally occurring characteristics as well as (a) feature(s) encoded by (a) nucleic acid molecule(s) engineered in vitro, which has/have been introduced into its genome.

The term "confer a change in the transmission ratio of a genetic trait(s) to the offspring of said non human male animal, preferably mammal, fish, bird or insect to a non-Mendelian ratio" as used in accordance with the present invention refers to changing the transmission ratio of (a) genetic trait(s) from the parents to their offspring to ratios markedly deviating from the expected Mendelian ratio of 50% (equal transmission). "Markedly deviating" in connection with the present invention means that the ratios might be less than 50 percent, preferably less than 40%, more preferably less than 30%, even more preferably less than 20% and most preferably less than 10% (reduced transmission) or might be at least 60%, more preferably at least 70 %, even more preferably at least 80% and most preferably at least 90 % (enhanced transmission).

The term "said chromosome containing said genetic trait(s)" as used in connection with the present invention means that the genetic trait is part of the chromosome and will then be transmitted together with said chromosome through the germline.

The term "said chromosome conferring said genetic trait(s)" as used in connection with the present invention means that the entire chromosome is to be considered as the genetic trait therefore the transmission ratio of said genetic trait is also changed when the entire chromosome will be transmitted through the germline.

The term "Responder function" as used in connection with the present invention refers to the unique property of a Responder to distort the transmission ratio of itself and (a) closely linked genetic trait(s) to non-Mendelian ratios, i.e. a marked deviation from 50%.

The term "Distorter function" as used in connection with the present invention refers to the potency of (a) Distorter(s) to enhance or reduce the transmission ratio of a Responder and (a) closely linked genetic trait(s), wherein the transmission ratio of the Responder and (a) closely linked genetic trait(s) markedly deviates from the Mendelian ratio.

The term "nucleic acid molecule encoding an expression product with Distorter function/with Responder function" relates to nucleic acid molecules wherein the deduction of the amino acid sequence of the nucleic acid molecules used in connection with the method of the present invention allows the conclusion that the (poly)peptide is the expression product that contributes to the Distorter/Responder function. However, it is not excluded that the mRNA contributes to or triggers said Distorter/Responder function. Also, it is envisaged in accordance with the present invention that the expression level, stage of expression during spermatogenesis or the copy number of said nucleic acid molecule results in or contributes to the Distorter/Responder function. Therefore, in a preferred embodiment of the nucleic acid molecule used in connection with the method of the invention said expression product is an RNA or a (poly)peptide.

The term "(poly)peptide" as used in the present invention describes a group of molecules which comprise the group of peptides, as well as the group of polypeptides. The group of peptides is consisting of molecules with up to 30 amino acids, the group of polypeptides is consisting of molecules with more than 30 amino acids. Furthermore, the term "protein" as used in connection with the present invention is to be considered identical with the term "(poly)peptide".

The terms "Distorter" or "Responder' therefore as used in connection with the present invention are as defined in the appended claims. Preferably, the terms refer to the (poly)peptide with Distorter or Responder function encoded by the corresponding nucleic acid molecules. Also, as mentioned above, said term might refer to the corresponding mRNAs or the nucleic acid molecule encoding the (poly)peptide with Distorter or Responder function.

Preferably, the nucleic acid molecule encoding a Distorter function is selected from the group consisting of SEQ ID NOs 1 and 2 (mouse wildtype Tagap1); SEQ ID NOs 3 to 6 (mouse Tagap^{t1}; Tagap^{t2}; Tagap^{t3}; Tagap^{t4} (Tcd1a)); SEQ ID NOs 7 and 8 (homop sapiens and Rattus Tagap); SEQ ID NOs 9 to 11 (mouse wildtype Fgd2 transcript variants); SEQ ID NOs 12 and 55 (mouse Fgd2^{t6/w5;} (Tcd2, transcript variants 1 and 1a)); SEQ ID NOs 13 and 14 (mouse and human Tiam2 (Tcd 1b)); SEQ ID NOs 31 to 33 (Bos taurus, Canis familiaris and Gallus gallus Tagap1); SEQ ID NOs 56 to 60 (Danio rerio, Macacca mulatta, Monodelphis domestica, Xenopus tropicalis and Pan troglodytes Tagap1), SEQ ID NOs 34 to 38 (mouse Fgd2 ^{t6/w5} (Tcd2; transcript variant 2); Bos Taurus, Canis familiaris and Rattus Fgd2; Rattus Fgd2 (splice variant)); SEQ ID NOs 61 to 66 (Macacca mulatto (3 transcript variants), Monodelphis domestica, Pan troglodytes and Homo sapiens Fgd2); SEQ ID NOs 47 to 50 (Bos taurus, Gallus gallus, Rattus and Canis familiaris Tiam2) and SEQ ID NOs 67 to 72 (Macacca mulatto (2 transcript variants), Monodelphis domestica (3 transcript variants) and Pan troglodytes Tiam2).

It is also preferred that the nucleic acid molecule encoding a Responder function is as shown in SEQ ID NO 15 or 16 (Smok^{Tcr}, Tcr, R^{t}).

The term "factors involved in G protein signaling" as used herein refers to any factor and preferably any protein that is a part of a G protein signaling cascade and in particular to members of the GTPase superfamily. For example, said members comprise trimeric G proteins and monomeric GTPases, and (poly)peptides triggering, controlling, modifying (a) signal pathway(s) involving small GTPases or regulated by (a) signal pathway(s) involving small GTPases.

The term "homologous recombination" refers to gene targeting of a nuclear gene locus of interest by integration of a nucleic acid molecule construct containing (a) genomic fragment(s) of said gene thereby altering the DNA sequence of said nuclear gene locus. It is preferred that by introducing said nucleic acid molecule construct into the nuclear gene locus the gene activity of the Distorter is down-regulated or abolished.

Within the meaning of the present invention, the term "directed against the Distorter function" means that the nucleic acid molecule or the expression product of said nucleic acid molecule directed against the Distorter reduces or interferes with the activity of the expression product(s).

The term "thereby partially or completely inactivating the Distorter function" as used in connection with the present invention refers to interfering with the gene activity of the Distorter by destruction of the mRNA, inhibition of translation of the mRNA, inhibition of the protein or enzymatic activity, or by other mechanisms allowing down-regulation or abolishment of the gene or protein activity of the Distorter. For example, in any of the above interferences partial inactivation means inactivation of at least 50%, preferably of at least 60%, more preferably of at least 70%, even more preferably of at least 80%, even more preferably of at least 90%, even more preferably of at least 95% and most preferably 100% (complete inactivation) A number of methods and assays which are known to the person skilled in the art allowing measuring down-regulation of transcript or protein levels, inhibition of protein translation or down-regulation of protein activity (Sambrook J. 1989). The skilled person can devise an assay wherein for example the amount of Distorter transcripts in cells containing said Distorter and expressing a nucleic acid molecule directed against said Distorter is compared to cells containing said Distorter but not said nucleic acid molecule directed against said Distorter. Likewise, the protein expression level of cells expressing said Distorter can be compared to cells expressing in addition a nucleic acid molecule allowing down-regulation or abolishment of the gene or protein activity of the Distorter for instance by western blot analysis using an antibody binding to the protein product of said Distorter. The protein activity of the expression product of a Distorter allele which has been altered in vitro in order to interfere with the protein activity of said wild type Distorter product can be compared to the activity of said wild type Distorter protein using in vitro activity assays such as for example those known in the art devised for assaying the activity of GAP or GEF proteins on target GTPases, such as for example, the one shown herein below, the method comprising expression of the Distorter protein in vitro or in bacterial cells. Expression products derived from dominant negative alleles can be assayed in mixing experiments in the presence of the wild type protein for its ability to interfere with the activity of the wild type protein. Furthermore, the activity of constitutively active proteins can be compared to the activity of the wild type protein comprising relating the activity of either protein to the protein amounts used in the assay.

The term "partially identical", as used herein, means in a first alternative that the genomic fragments used for integrating the nucleic acid molecule construct by homologous recombination are completely identical with the target nucleic acid molecule encoding an expression product with Distorter function. In this alternative the overall construct is partially identical because the target nucleic acid molecule encoding an expression product with Distorter function is not identical with the sequence in the construct used for the inactivation. Alternatively, even said genomic fragments may not be completely identical with the target nucleic acid molecule encoding an expression product with Distorter function but are sufficiently identical to allow recombination.

As outlined above and in other terms, the invention solves the recited technical problem by providing a reproducible method for changing the transmission ratio of genetic traits in non-human mammals . In particular, as mentioned above, prior art methods failed to identify a Distorter which, however, is needed for carrying out the method of the present invention.

Several genes with respect to their role as a distorter were examined. This included analyses to determine the genomic position, expression analyses to compare the t-haplotype versus the wild type and detailed sequence analyses. From these experiments, only a subset of these genes turned out to be promising and these selected candidates were further analyzed functionally by establishing transgenic and knock out mouse lines.

In the prior art, all t-Distorter candidate genes which have been reported had been identified by the criteria that they were a) located within the t-complex region and b) play a role in sperm specific functions or are primarily expressed in sperm cells, such as Tctex1, Tctex2, Tcte2 and Tcp11 (Fraser and Dudley, 1999). This obvious assumption that t-Distorters are likely involved in sperm specific functions was used as an aid for preselection of likely candidates from the hundreds of genes located in the t-complex region, but turned out to be false. Additionally, it was known in the prior art that transmission ratio distortion relates to sperm motility and that the Responder relates to a Smok kinase (Herrmann et al, 1999), from which a person skilled in the art might have derived that factors involved in calcium signaling or cAMP signaling might be involved in the Distorter phenotype. However, the prior art did not give any clue whatsoever that factors involved in G protein signaling might function as a Distorter. The method of the present invention therefore for the first time makes use of nucleic acid molecules encoding such factors which are involved in G protein signaling for the above-indicated purpose.

The method of the present invention is based on the fact that in mouse the t-haplotype chromosome is transmitted at non-Mendelian ratio (significantly higher or lower than 50%) to the offspring. This phenomenon involves Tcr (Responder) and several Tcd (Distorter) loci, wherein the Tcd loci in the t-haplotype, that is the mutant forms, enhance the transmission ratio of Tcr. In the wild-type form, on the other hand, the Tcd+ loci reduce the transmission ratio of Tcr (the "low" phenotype). In the prior art, Tcd loci could not be localized precisely by chromosomal mapping due to recombination suppression between the t-haplotype and the wild type chromosome. Thus the coarse localization of Tcd loci to regions of several megabases in size each prevented the identification of t-Distorters. Even deletion mapping of Tcd1 did not allow identification of this factor (Lyon et al 2000) ;(Schimenti et al 2000).

Thus, these prior art difficulties had to be overcome in order to solve the ignorance of the molecular nature of a t-Distorter. The present invention not only makes use of Distorters which are factors involved in G protein signaling, but on top of this solved the problem which was in the prior art that the isolation of a Distorter could not be achieved. In the course of isolating a nucleic acid molecule which encodes one of the Distorters which can be used in connection with the present invention, in particular Tagap1, the inventors isolated a specific fusion gene, which showed similarity to FGF receptor oncogene partner (Fop) and is highly expressed in testis of wild type mice, but not of mice carrying the t-haplotype, suggesting that it is related to transmission ratio distortion. However, the inventors could demonstrate by gene targeting and genetic testing that this fusion gene did not show any Distorter activity. Nevertheless, the inventors did not turn to another candidate, but continued the study of this locus. Southern blot analysis indicated that this gene or a part thereof was present in a second locus on the chromosome, but there was no indication that that second gene or gene fragment was expressed. Rapid amplification of cDNA end (RACE) technology was attempted to complete the 5'-region of the second gene transcript, but these experiments produced the 5'end sequence of the known fusion gene instead because this gene is highly expressed in testis. Only after several months of unsuccessful trials eventually a different 5'- end was identified, which then led to the isolation of Tagap1. The transcript of the missing locus, Tagap1, per se was only very weakly expressed in testis and did not hint to a Distorter function either. Only by completing the entire gene the inventors could recognize the missing (second) gene as a gene involved in G protein signalling. Gene targeting and genetic testing demonstrated that Tagap1 is able to alter the transmission ratio of a t-haplotype carrying Tcr. However, the targeted allele reduced the transmission ratio, in contrast to the teachings of the prior art, since Lyon had shown that a deletion of Tcd1 on the wild type chromosome enhanced the transmission ratio (Lyon 1992). Large efforts involving extensive genetic analyses and the production and analyses of transgenic lines had to be undertaken by the inventors to finally show that the t-loci of Tagap1 constitute a Distorter which enhances the transmission ratio of a t-haplotype and which must be different from the t-Distorter identified by Lyon by genetic means using the deletion chromosome T^{22H}.

From the above, the inventors hypothesized that further factors involved in G protein signaling might be involved in Distorter function as for example shown in Example 3 and could identify further factors in the cascade showing Distorter function. Hence, the present invention for the first time links G protein signalling to the phenomenon of transmission ratio distortion.

The method of the present invention comprises deriving an adult non-human mammal from said non human mammalian germ cell, fertilized egg cell, embryonic cell or cell derived therefrom containing the nucleic acid molecules as defined in the appended claims. The fertilized egg cell or an embryo into which said embryonic cell has been introduced (thereby forming a chimera), or the fertilized egg or zygote derived from introducing the nucleus of the cell containing the nucleic acid molecules for use in the present invention into an egg cell or zygote whose genome has been removed (thereby forming an embryo containing said the nucleic acid molecules as defined in the present invention in its genome), is/are transferred into a foster mother and the embryo let develop to term.

The offspring of the foster mother are then determined for the integration of the nucleic acid molecules as described in the present invention and the sex is determined by methods known to the person skilled in the art. Such methods comprise for example genotyping by PCR and further methods as also described below.

The male offspring can further be characterized by visual inspection of outer genitalia and also by detecting male specific markers. Said techniques are also known to the person skilled in the art.

All methods employed for deriving an adult animal are known in the prior art and further described, where applicable, in the specification of the preset invention.

The transgenic non-human male mammal produced by the method of the present invention can be prepared in at least two alternative ways. The nucleic acid molecules as defined in the present invention can be introduced into the same non-human germ cell, (fertilized) egg cell, embryonic cell or a cell derived therefrom and the adult organism let develop as described above. Alternatively, the nucleic acid molecules for use in the present invention can be introduced into different cells thereby producing two different adult organisms with the same methods as described above. The male and female of said two organisms are then crossed and the offspring is analyzed for the nucleic acid molecule as described in the present invention and the male is characterized as described above.

As mentioned, the techniques involved in animal breeding and animal crossing and the techniques involved in transgenesis are known to the person skilled in the art and, where applicable, are described in the present specification.

Alternatively, in the case that the investigator wishes to start at a different stage of accomplishing the invention the following alternative embodiments are set up.

According to one of these embodiments the invention relates to a method for producing a transgenic non human mammal as defined in the appended claims said method comprising introducing (a) a first nucleic acid molecule encoding the defined expression product with a Responder function into a chromosome of a non-human germ cell, (fertilized) egg cell, embryonic cell or a cell derived therefrom, of the same species as the transgenic animal to be prepared, said chromosome containing or conferring said genetic trait(s), thereby linking on said chromosome said Responder function to the genetic trait(s); and (b) at least one second nucleic acid molecule encoding an expression product with a Distorter function into (a) chromosome(s) of a non-human germ cell, (fertilized) egg cell, embryonic cell or a cell derived therefrom, of the same species as the transgenic female to be prepared, wherein said expression product with a Distorter function is one of the defined factors involved in G protein signaling, wherein said first nucleic acid molecule encoding an expression product with Responder function and said at least one second nucleic acid molecule encoding an expression product with a Distorter function are introduced into the same or different chromosomes.

Alternatively, the invention envisages a method for producing a transgenic non human mammal as defined in the appended claims said method comprising introducing (a) a first nucleic acid molecule encoding the defined expression product with a Responder function into a chromosome of a non-human germ cell, (fertilized) egg cell, embryonic cell or a cell derived therefrom, of the same species as the transgenic animal to be prepared, said chromosome containing or conferring said genetic trait(s), thereby linking on said chromosome said Responder function to the genetic trait(s); and (b) at least one second nucleic acid molecule encoding an expression product directed against the Distorter function into (a) chromosome(s) of a non-human germ cell, (fertilized) egg cell, embryonic cell or a cell derived therefrom, of the same species as the transgenic female to be prepared, wherein said Distorter function is an expression product which is encoded by a nucleic acid molecule encoding an expression product with a Distorter function, wherein said expression product with a Distorter function is one of the defined factors involved in G protein signaling; and/or (c) a second nucleic acid molecule for inactivation of the Distorter function by homologous recombination, wherein said second nucleic acid molecule for inactivation of the Distorter function by homologous recombination is at least partially identical to said nucleic acid molecule encoding an expression product with a Distorter function, wherein said first nucleic acid molecule encoding an expression product with a Responder function and said at least one second nucleic acid molecule encoding an expression product directed against the Distorter function and/or said second nucleic acid molecule for inactivation of the Distorter function by homologous recombination are introduced into the same or different chromosomes, thereby partially or completely inactivating the Distorter function.

The above mammal may be male or female. Methods for the production of transgenic animals wherein only step (b) is carried out are described. Again, said animals are mammals.

From these animals the male animal of the main embodiments (if the outcome is not a male anyway) can be generated.

Methods for the generation of transgenic mammal are well known in the art and are described (for example in (DePamphilis 1993), (Chapman, Lawson et al. 2005)).

Other methods comprise the use of retroviral, in particular lentiviral particles carrying constructs engineered in vitro for the infection of cells, preferably egg cells, zygotes or early embryos, the integration of recombinant DNA constructs into embryonic stem cells and production of stem cell/embryo chimera, or the generation of egg cells or sperm cells from embryonic stem cells having integrated the recombinant DNA construct, by differentiation of said cells in vitro (Lever et al., 2004); (Hubner et al., 2003); (Geijsen et al., 2004).

A further method comprises recombinase mediated cassette exchange (RMCE) whereby a construct which is flanked by non-identical target sites, such as IoxP and Iox2272 sites, recognized by a site specific recombinase, such as Cre is exchanged by homologous recombination mediated by the recombinase for a fragment which is contained in a chromosome and which is flanked by said sites (such as IoxP and Iox2272 in this example), thereby integrating the construct into said chromosome (Pirottin, Grobet et al. 2005).

The method of the invention may also relate to the cloning of transgenic animals. This includes the steps of introducing the nucleic acid molecule for use in the present invention, recombinant DNA molecule or vector comprising said nucleic acid molecule into the nucleus of a cell, preferably an embryonic cell, replacing the nucleus of an oocyte, a zygote or an early embryo with said nucleus comprising said nucleic acid molecule, recombinant DNA molecule or vector, transferring either said oocyte, zygote or early embryo into a foster mother or first in vitro or in vivo culturing said oocyte, zygote or early embryo and subsequently transferring the resulting embryo into a foster mother and allowing the embryo to develop to term; see, for example, (Wilmut, Schnieke et al. 1997).

A method for the production of a transgenic non-human animal, for example transgenic mouse, comprises introduction of a nucleic acid molecule or targeting vector into a germ cell, an embryonic cell, stem cell or an egg or a cell derived therefrom. Production of transgenic embryos and screening of those can be performed, e.g., as described (Joyner 1993). The DNA of the embryonal membranes of embryos can be analyzed using, e.g., Southern blots with an appropriate probe. A general method for making transgenic non-human animals is described in the art, see for example WO 94/24274. For making transgenic non-human organisms (which include homologously targeted non-human animals), embryonic stem cells (ES cells) are preferred. Murine ES cells, such as AB-1 line grown on mitotically inactive SNL76/7 cell feeder layers (McMahon and Bradley, Cell 62: 1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used for homologous gene targeting. Other suitable ES lines include, but are not limited to, the E14 line (Hooper et al., Nature 326: 292-295 (1987)), the D3 line (Doetschman et al., J. Embryol. Exp. Morph. 87: 27-45 (1985)), the CCE line (Robertson et al., Nature 323: 445-448 (1986)), the AK-7 line (Zhuang et al., Cell 77: 875-884 (1994) ). The success of generating a mouse line from ES cells bearing a specific targeted mutation depends on the pluripotency of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant nonhuman females and are born as chimeric mice. The resultant transgenic mice are chimeric for cells having either the recombinase or reporter loci and are backcrossed and screened for the presence of the correctly targeted transgene (s) by PCR or Southern blot analysis on tail biopsy DNA of offspring so as to identify transgenic mice heterozygous for either the recombinase or reporter Also described herein are

Methods for producing transgenic flies, such as Drosophila melanogaster , see for example US-A-4,670,388, Brand & Perrimon, Development (1993) 118: 401-415; and Phelps & Brand, Methods (April 1998) 14: 367-379.

In a preferred embodiment of the method of the present invention said mammal is selected from the group consisting of Mus, Rattus, Bos, Sus and Ovis.

It is more preferred that Mus is Mus musculus, Rattus is Rattus norvegicus, Bos is Bos taurus, Sus is Sus scrofa f. domestica.

In another preferred embodiment of the method of the present invention said genetic trait is sex.

In further preferred embodiment of the method of the present invention said chromosome is an X or Y chromosome Also described herein is Changing the transmission ratio in insects, which will have an important impact on the fight against insect pests. For example, by mixing a number of transgenic male Anopheles prepared as described with a naturally occurring Anopheles population, responsible for the spreading of malaria, the production of e.g. predominantly male offspring of the transgenic Anopheles is expected (see also explanation herein below). These male Anopheles will change the overall frequency of males in the population and thus lead to an overall mating problem in the Anopheles population which eventually will lead to an overall reduced amount of Anopheles. A corresponding strategy may be employed with, for example, locusts.

In a still further preferred embodiment of the method of the present invention said chromosome is an autosome.

In accordance with the present invention the factor involved in G protein signalling is a factor involved in Rho signalling.

The term "a factor involved in Rho signaling" as used herein refers to small G proteins of the Rho subfamily, as well as to molecules acting upstream or downstream of G proteins in terms of signal transduction. Such molecules comprise in particular members of the families of GEFs (guanine nucleotide exchange factors), which enhance the activity of small G proteins, GAPs (GTPase activating proteins) which act as negative regulators and GDIs (guanine nucleotide dissociation inhibitors) which also attenuate small G protein signaling (Schmidt and Hall, 2002); (Donovan et al., 2002); (DerMardirossian and Bokoch, 2005). Finally, the term refers to target molecules, influenced by small G proteins (Bishop and Hall, 2000).

In accordance with the present invention said first nucleic acid molecule encoding an expression product with Responder function is selected from the group consisting of (a) a nucleic acid molecule comprising or consisting of the nucleic acid molecule as shown in SEQ ID No: 15 or 16 or a fragment thereof; (b) a nucleic acid molecule being an allelic variant or a orthologue of the nucleic acid molecule of (a); ; and (c) a nucleic acid molecule which is related to the nucleic acid molecule of (a) or (b) by the degeneration of the genetic code, as defined in the appended claims.

In further preferred embodiment of the method of the present invention said (at least one) second nucleic acid molecule encoding an expression product with a Distorter function is/are selected from the group consisting of (a) a nucleic acid molecule comprising or consisting of the nucleic acid molecule as shown in any one of SEQ ID NOs: 1 to 14, 31 to 33, 34 to 38, 47 to 72 or a fragment thereof, (b) a nucleic acid molecule being an allelic variant or a orthologue of the nucleic acid molecules of (a); ; and (c) a nucleic acid molecule which is related to the nucleic acid molecule of (a) or (b) by the degeneration of the genetic code, as defined in the appended claims.

The term "an allelic variant " as used in connection with the present invention refers to different wild type forms and t-alleles of the nucleic acid molecules.

The nucleic acid molecules can be further manipulated *in vitro* in order to achieve an optimized transmission ratio distortion effect and/or to adapt it to the specific requirements of the' breeding scheme employed, thus further improving the selectability of genetic traits as further described below. A number of standard manipulations known in the field are taken into consideration, such as those resulting in the exchange of amino acids in the catalytic domain(s) which is the GAP domain in case of the GTPase activating proteins and the DH (Dbl-homology) domain in case of the guanine nucleotide exchange factors, overexpression or knock out mutagenesis of said nucleic acid molecules, construction of hypomorphic or hypermorphic (poly)peptides by mutagenesis, deletion or alteration of candidate modification sites on said (poly)peptide, deletion or alteration of binding sites for other (poly)peptides involved in the G protein signaling cascade (see for example (Dvorsky and Ahmadian, 2004)), synthesis of antisense RNA, siRNA, shRNA, N-terminal or C-terminal truncations, introduction of frame shifts, which alter part of the amino acid sequence of the protein, etc., resulting either in null, hypomorphic, constitutively active, antimorphic or dominant negative alleles. It is also envisaged that a distortion of the transmission ratio can be achieved with several, if not all, manipulated forms of the nucleic acid molecules described above. Thus, a manipulated allele affecting the transmission ratio most effectively will have to be identified empirically for each gene by employing activity assays in vitro and in cell culture systems such as NIH-3T3 cells and transgenic animal systems.

The term "orthologue" as used in connection with the present invention refers to genes present in different organisms and which have the same function.

The term "fragment" as used in connection with the method of the present invention relates to the fact that said fragment retains the Responder/Distorter function.

If fragments, allelic variants or orthologue of a specifically identified sequence conferring responder or Distorter function are preferred to throughout this specification, it is understood that these fragments etc. retain or essentially retain the Responder or Distorter function. "Essentially retain" means in accordance with these embodiments that at least 70% of the function are retained, preferably at least 80% such as at least 90%.

Also described herein are nucleic acid molecules hybridizing to a nucleic acid molecule complementary to the nucleic acid molecule of (a) or (b).

The term "hybridizing" as used herein preferably refers to "hybridizing under stringent conditions", and is well known to the skilled artisan and corresponds to conditions of high stringency. Appropriate stringent hybridization conditions for each nucleic acid sequence may be established by a person skilled in the art on well-known parameters such as temperature, composition of the nucleic acid molecules, salt conditions etc.; see, for example, (Sambrook J. 1989) (Hames 1985), see in particular the chapter "Hybridization Strategy" by Britten & Davidson, 3 to 15. Stringent hybridization conditions are, for example, conditions comprising overnight incubation at 42° C in a solution comprising: 50% formamide, 5x SSC (750 mM NaCl, 75 mM trisodium citrate), 50 mM sodium phosphate (pH 7.6), 5x Denhardt's solution, 10% dextran sulfate, and 20 micrograms/ml denatured, sheared salmon sperm DNA, followed by washing the filters in 0.1 x SSC at about 65°. Other stringent hybridization conditions are for example 0.2 x SSC (0.03 M NaCl, 0.003Msodium citrate, pH 7) at 65°C. In addition, to achieve even higher stringency, washes performed following stringent hybridization can be done at higher salt concentrations (e.g. 5X SSC). Note that variations in the above conditions may be accomplished through the inclusion and/or substitution of alternate blocking reagents used to suppress background in hybridization experiments. Typical blocking reagents include, but are not limited to, Denhardt's reagent, BLOTTO, heparin, denatured salmon sperm DNA, and commercially available proprietary formulations. The inclusion of specific blocking reagents may require modification of the hybridization conditions described above, due to problems with compatibility. Also contemplated are nucleic acid molecules encoding an interaction partner of a biomolecule, wherein the interaction partner is capable of modulating the activity said biomolecule and wherein the nucleic acid molecules hybridize to the nucleic acid molecule encoding the biomolecule at even lower stringency hybridization conditions. Changes in the stringency of hybridization and signal detection are, for example, accomplished through the manipulation of formamide concentration (lower percentages of formamide result in lowered stringency); salt conditions, or temperature. For example, lower stringency conditions include an overnight incubation at 37 degree C in a solution comprising 6X SSPE (20X SSPE = 3M NaCl; 0.2M NaH₂PO₄; 0.02M EDTA, pH 7.4), 0.5% SDS, 30% formamide, 100 µg/ml salmon sperm blocking DNA; followed by washes at 50 degree C with 1XSSPE, 0.1% SDS. In addition, to achieve even lower stringency, washes performed following stringent hybridization can be done at higher salt concentrations (e.g. 5X SSC). Variations in the above conditions may be accomplished through the inclusion and/or substitution of alternate blocking reagents used to suppress background in hybridization experiments. Typical blocking reagents include, but are not limited to, Denhardt's reagent, BLOTTO, heparin, denatured salmon sperm DNA, and commercially available proprietary formulations. The inclusion of specific blocking reagents may require modification of the hybridization conditions described above, due to problems with compatibility.

As shown above, it is preferred that the method of the present invention be carried out by using the nucleic acid molecules encoding a Distorter function described in SEQ ID NOs: 1 to 14, 31 to 33, 34 to 38, 47 to 72. These sequences relate to wildtype Tagap1 (SEQID NO: 1 and 2), the t-alleles of Tagap1, Tagap1^{t1 to t4} (SEQ ID NOs: 3 to 6), the homo sapiens Tagap (SEQ ID NO: 7), the Rattus Tagap (SEQ ID NO: 8), threetranscript variants of wildtype mouse Fgd2 (SEQ ID NOs: 9 to 11), three t-alleles of Fgd2 (SEQ ID NOs: 12, 34 and 55), the mouse and homo sapiens Tiam 2 (SEQ ID NOs 13 and 14), the Bos taurus, Canis familiaris and Gallus gallus Tagap (SEQ ID NOs 31 to 33), the Danio rerio, Macacca mulatta, Monodelphis domestica, Xenopus tropicalis and Pan troglodytes Tagap1 (SEQ ID NOs 56 to 60); Bos Taurus, Canis familiaris and Rattus Fgd2; Rattus Fgd2 (splice variant) (SEQ ID NOs 35 to 38), the Macacca mulatta (3 transcript variants), Monodelphis domestica and Pan troglodytes Fgd2 (SEQ ID NOs 61 to 66), Bos taurus, Gallus gallus, Rattus and Canis familiaris Tiam2 (SEQ ID NOs 47 to 50) and Macacca mulatta (2 transcript variants), Monodelphis domestica (3 transcript variants) and Pan troglodytes Tiam2 (SEQ ID NOs 67 to 72).

In another preferred embodiment of the method of the present invention said at least one second nucleic acid molecule encoding an expression product with a Distorter function is/are selected from the group consisting of (a) a nucleic acid molecule comprising or consisting of the nucleic acid molecule as shown in any one of SEQ ID NOs 1 to 12, 31 to 38 or 55 to 66 or a fragment thereof (b) a nucleic acid molecule being an allelic variant or a orthologue of the nucleic acid molecule of (a); and (c) a nucleic acid molecule which is related to the nucleic acid molecule of (a) or (b) by the degeneration of the genetic code, thereby enhancing said transmission ratio of said genetic trait(s), as defined in the appended claims. In a further preferred embodiment of the method of the present invention said at least one second nucleic acid molecule encoring an expression product with a Distorter function is/are selected from the group consisting of (a) a nucleic acid molecule comprising or consisting of the nucleic acid molecule as shown in of SEQ ID NO: 13 or 14, 47 to 50 or 67 to 72 or a fragment thereof; (b) a nucleic acid molecule being an allelic variant or a orthologue of the nucleic acid molecule of (a); and (c) a nucleic acid molecule which is related to the nucleic acid molecule of (a) or (b) by the degeneration of the genetic code, thereby reducing said transmission ratio of said genetic trait(s), as defined in the appended claims.

In another preferred embodiment of the method of the present invention said nucleic acid molecule encoding an expression product with a Distorter function is selected from the group consisting of (a) a nucleic acid molecule comprising or consisting of the nucleic acid molecule as shown in any one of SEQ ID NOs: 1 to 12, 31 to 38 or 55 to 66 or a fragment thereof; (b) a nucleic acid molecule being an allelic variant or a orthologue of the nucleic acid molecule of (a); ; and (c) a nucleic acid molecule which is related to the nucleic acid molecule of (a) or (b) by the degeneration of the genetic code, thereby reducing said transmission ratio of said genetic trait(s), as defined in the appended claims.

In still another preferred embodiment of the method of the present invention said nucleic acid molecule encoding an expression product with a Distorter function is selected from the group consisting of (a) a nucleic acid molecule comprising or consisting of the nucleic acid molecule as shown in SEQ ID NOs 13 or 14, 47 to 50 or 66 to 72 or a fragment thereof; (b) a nucleic acid molecule being an allelic variant or a orthologue of the nucleic acid molecule of (a); ; and (c) a nucleic acid molecule which is related to the nucleic acid molecule of (a) or (b) by the degeneration of the genetic code, thereby enhancing said transmission ratio of said genetic trait(s), as defined in the appended claims.

In a further preferred embodiment the method of the present invention further comprises crossing the transgenic non human male mammal obtained by the method of the present invention with a non human female mammal, and analyzing the offspring of said cross for transmission of said genetic trait(s).

The above preferred embodiments of the method of the present invention are of particular interest for the applicability of the present invention. The genetic trait(s) of interest can be transmitted to the offspring either at an enhanced or at a reduced ratio with respect to the Mendelian ratio. In particular it is envisaged to use the methods of the present invention in the field of farm animal breeding as a tool for manipulating the transmission ratio of genetic traits. The most interesting trait in this respect is sex. The method of the present invention making use of the Distorter now allows an enhancement of the effect of the Responder, for example it will be possible to obtain strong selection for or against sperm carrying the Y chromosome. It is therefore envisaged that a transgene construct expressing the nucleic acid molecule encoding the Responder function and expressing at least one other nucleic acid molecule encoding (a) Distorter function(s) and/or products directed against the Distorter function be integrated on the Y-chromosome of the farm animal species. In one embodiment of the present invention action of the Distorter(s) would impair the sperm cells carrying the Responder, which would result in a preferential or exclusive transmission of the X-chromosome and thus generation of female offspring. In another embodiment of the present invention the action of the Distorter(s) and/or the product(s) directed against the Distorter function would impair all sperm cells, while the sperm cells carrying and expressing the Responder would be rescued. In that latter embodiment the Y chromosome would be preferentially or exclusively transmitted to the offspring resulting in the production of male offspring. Likewise, the construct expressing the Responder function and the construct(s) expressing at least one Distorter function and/or product(s) directed against the Distorter function could be integrated on the X chromosome and allow the generation of males preferentially or exclusively transmitting the Y chromosome or, in the latter example, wherein a high transmission ratio of the Responder construct is achieved, the X chromosome. It will depend on the design of the construct(s) (as taught above) expressing the Distorter function and/or product(s) directed against the Distorter function whether enhanced transmission or reduced transmission of the chromosome carrying the Responder construct will be achieved. For example, in the present invention it could be shown that inactivation of wild type Tagap1 and inactivation of wild type Fgd2 both resulted in a reduced transmission of Tcr, while the respective t-Distorters Tagap1^{Tcd1a} and Fgd2^{Tcd2} enhance the transmission of Tcr. In contrast, it is envisaged that loss of Tiam2 function enhances the transmission of Tcr, while a hypermorphic allele is envisaged to reduce the transmission ratio of Tcr. Thus, the teachings of the t-Distorters and of the mutations in the wild type gene provided by the present invention provide the knowledge how Distorter alleles need to be engineered to achieve enhancement or reduction of the transmission ratio of the chromosome carrying the Responder construct and the genetic trait(s) linked to it. Importantly, Distorters act additively or synergistically, thus the combinatorial use of several nucleic acids encoding Distorter function(s) and/or products directed against the Distorter function(s) is preferred in order to achieve an optimal effect. For example, it is envisaged that the combination of nucleic acid molecules encoding products directed against the function of Tagap1 and of Fgd2 and of a construct overexpressing Tiam2 achieves a strong effect with respect to selection against sperm carrying and expressing the Responder construct, since all three expression products singly should reduce the transmission ratio of the Responder.

It is furthermore envisaged that the constructs expressing the Distorter(s) and/or the constructs expressing an expression product directed against the Distorter function and/or the constructs for inactivation of the Distorter function can be integrated independently of the Responder construct on the same or on different chromosomes. Such a tool for preselection of sex in farm animals is most desirable for Bos taurus, a species for which specialized strains for milk or meat production have been derived. Female offspring is needed for milk production whereas for meat production male offspring is preferred in this species. In most other farm animal species female offspring is most desired. Thus, preselection of sex is of general importance in farm animal breeding.

Although it is possible without undue burden to identify mutated or wild-type Distorters in animals other than the mouse on the basis of the genetic structure of the Distorter that is provided in accordance with the present invention, it is envisaged that the mouse t-complex Distorter may find applications, for example in breeding, also when introduced into other animals. Specific applications of the Distorter function are addressed herein below.

It is furthermore envisaged that generally the set of genes, Responder and one or more distorters, used to create a transgenic non-human mammal is chosen independently of the target animal species and can be applied in any combination allowed by the included sequence listing. For example, it is imaginable that the mouse responder is combined with one or more distorters of one or more different species to be transferred to an animal which belongs to none of said species.

For example, it is imaginable that the mouse responder is combined with one or more distorters of one or more different species to be transferred to a non-human mammal which belongs to none of said species. A more specific example would include the mouse responder gene combined with e. g. Macacca distorter genes to be transduced into Bos taurus or e. g. one Macacca distorter and a different Canis distorter to be transduced into Bos taurus.

One or more distorters may be chosen from the sequences corresponding to the targeted animal. For example, the distorters belong to the species Bos taurus and are used to create a transgenic animal belonging to the same species.

In accordance with the present invention said expression product directed against the Distorter function is an aptamer, a siRNA or shRNA or miRNA, a ribozyme, or an antisense nucleic acid molecule specifically hybridizing to said nucleic acid molecules encoding a Distorter (poly)peptides as used in connection with the present invention.

It is envisaged that shRNA (small hairpin RNA) molecules expressed from a construct integrated into the genome can be used for degradation of RNA molecules (known as RNA interference) transcribed from (a) endogenous gene(s) encoding (a) Distorter(s) thereby partially or completely down-regulating the function of said Distorter(s). Likewise vectors comprising nucleic acid molecules encoding a miRNA (microRNA) can be utilized for inhibition of translation of the RNA encoding said Distorter(s) (Kim 2005). Constructs expressing aptamers can be utilized to inhibit protein-protein interaction such as between a Distorter protein and another (poly)peptide of the Distorter/Responder signaling cascade in order to interfere with the propagation of the signal thereby inhibiting said signal pathway. The person skilled in the art is able to design shRNA or miRNA constructs on the basis of the sequence of the mRNA of the gene the function of which shall be down-regulated. The efficacy of the constructs can easily be tested in cellular systems. Aptamers able to inhibit protein-protein interaction can be selected in vitro or in cellular system such as the yeast the method comprising assaying inhibition of protein-protein interaction as measured in the yeast two-hybrid assay (Schmidt, Diriong et a). 2002); (Kurtz, Esposito et al. 2003); (Cassiday and Maher 2003).

Also described herein is an antibody as "antibody fragment or derivative thereof" which relates to single chain antibodies, or fragments thereof, synthetic antibodies, antibody fragments, such as Fab, a F(ab₂)', Fv fragments, single domain antibodies etc., or a chemically, modified derivative of any of these. Derivatives include scFvs. Antibodies described herein or their corresponding immunoglobulin chain(s) can be further modified using conventional techniques known in the art, for example, by using amino acid deletion(s), insertion(s), substitution(s), addition(s), and/or recombination(s) and/or any other modification(s) (e.g. posttranslational and chemical modifications, such as glycosylation and phosphorylation) known in the art either alone or in combination. Methods for introducing such modifications in the DNA sequence underlying the amino acid sequence of an immunoglobulin chain are well known to the person skilled in the art; see, e.g., Sambrook et al.; Molecular Cloning: A Laboratory Manual; Cold Spring Harbor Laboratory Press, 2nd edition 1989 and 3rd edition 2001.

The term "antibody fragment or derivative thereof" particularly relates to (poly)peptide constructs comprising at least one CDR such as two, three and preferably all six CDRs of an antibody, e.g. in the scFv format. Framework regions of the antibody may also be replaced by unspecific non-antibody-related sequences.

Fragments or derivatives of the recited antibody molecules may also define (poly)peptides which are parts of the above antibody molecules and/or which are modified by chemical/biochemical or molecular biological methods. Corresponding methods are known in the art and described inter alia in laboratory manuals (see Sambrook et al., loc cit.; Gerhardt et al.; Methods for General and Molecular Bacteriology; ASM Press, 1994; Lefkovits; Immunology Methods Manual: The Comprehensive Sourcebook of Techniques; Academic Press, 1997; Golemis; Protein-Protein Interactions: A Molecular Cloning Manual; Cold Spring Harbor Laboratory Press, 2002; Antibodies, A Laboratory Manual, Ed Harlow and David Lane, Cold Spring Harbor Laboratory, 1988).

In a preferred embodiment of the method of the present invention said at least one second nucleic acid molecule encoding the expression product with a Distorter function is modified, thereby further reducing or further enhancing the Distorter function activity.

The term "further reducing or further enhancing the Distorter function activity" as used in connection with the method of the present invention refers to the fact that the method of the present invention can be further optimized by genetically manipulating the nucleic acid molecules encoding the Distorters. For example, dominant active or dominant negative alleles of (a) Distorter(s) may be designed and assayed in vitro for their ability to enhance or interfere with the activity of the wild type allele of said Distorter, followed by genetic testing in vivo in transgenic animals of the allele(s) which improve the activity of said Distorter or enhance down-regulation of said Distorter in vitro. Other alterations of the nucleic acid sequence resulting in changes of the polypeptide encoded by said Distorter gene may be introduced in vitro by exchanging nucleic acid molecules or by synthesizing genes or gene parts in vitro or by random mutagenesis, and (high-throughput) in vitro assays can be designed to measure the activity of the altered proteins or their ability to enhance or inhibit or interfere with (a) component(s) of the Distorter/Responder signal cascade.

In further preferred embodiment of the method of the present invention said first nucleic acid molecule encoding an expression product with a Responder function and said at least one second nucleic acid molecule encoding an expression product with Distorter function and/or said at least one second nucleic acid molecule encoding an expression product directed against the Distorter function and/or said second nucleic acid molecule for inactivation of the Distorter function by homologous recombination and a promoter controlling expression in spermatogenesis and/or spermiogenesis and/or a stop cassette are integrated in said X or Y chromosome or corresponding sex chromosome or in one of said autosomes in a reversible inactive state of expressibility. Preferably, said promoter is a heterologous promoter.

The term "reversibe inactive state of expressibility" as used in connection with the method of the present invention refers to the possibilty to keep the above nucleic acid molecules in an inactive state of expressibility which can by genetic means be activated, as further described below.

In particular, it is envisaged that the construct(s) expressing the Distorter(s) and/or products directed against the Distorter function(s) reduce the transmission of the nucleic acid molecule encoding a Responder function to such a low ratio that the transmission of the Responder construct is almost or completely excluded. This would mean that one important component of the functional principle of the present invention is not passed on to the next generation by natural breeding. To circumvent this problem it is envisaged to introduce a construct containing the nucleic acid molecule encoding the Responder in an inactive state, for instance by inserting a transcription stop cassette between the promoter controlling expression of the Responder gene and the nucleic acid encoding the Responder, comprising flanking the stop cassette by loxP sites in same orientation. This construct would be inactive with respect to expression of the Responder and thus could be transmitted at Mendelian ratio to the offspring. Males producing sperm allowing preselection of (a) trait(s) are envisaged to be produced by breeding the male or female carrying the inactive Responder construct to a female or male carrying a construct expressing Cre recombinase prior to spermiogenesis. Activation of the Responder construct would then occur by excision of the transcription Stop cassette due to the action of the Cre recombinase during embryonic development or in germ cells. Other combinations of recombinase and specific recognition sites for the recombinase, or the use of other nucleic acid molecules instead of a stop cassette, or the inverse orientation of the nucleic acid encoding the Responder flanked by sites for site specific recombinases in inverse orientation, are also envisaged ways to achieve a reversible inactive state of expressibility.

It is also envisaged to achieve transmission of (a) construct(s) which is not transmitted through sperm cells by propagation of said construct(s) in females. This is particularly useful when the construct(s) is/are integrated on the X chromosome and/or (an) autosome(s). It is furthermore envisaged that the construct(s) will only be activated in sperm cells, in particular if promoter(s) are used which activate transcription specifically during spermatogenesis and/or spermiogenesis. Thus, it is envisaged that selection against transmission of said construct(s) will be restricted to transmission through sperm cells, while transmission through the female germ cells occurs normally.

The use of constructs designed for selection against a genetic trait such as male sex is rendered in some rare cases difficult by the fact that the transgene construct may not or hardly be transmitted to the offspring of the carrier male animal. In such cases it is envisaged to use sperm cells at random or after preselection of cells carrying the transgene construct in order to significantly enhance the likelihood for the production of offspring carrying said transgene construct. Selection can be effected, e.g., by cell sorting.

It is also envisaged to make use of in vitro fertilization since it has been shown -that transmission ratio distortion in mouse does not occur during in vitro fertilization procedures; other methods are ICSI (intracellular sperm cell injection), (Horiuch, Emuta et al. 2002).

Furthermore, the present invention relates to a non human male or female mammal, wherein said non human male or female

mammal is transgenic for the nucleic acid molecule encoding an expression product with a Distorter function and/or the nucleic acid molecule encoding an expression product directed against the Distorter function and/or the nucleic acid molecule for inactivation of the Distorter function by homologous recombination as defined in the present invention and for the nucleic acid molecule encoding an expression product with a Responder function, as defined in the appended claims.

The present invention also relates to a pair of non human male and female, mamma , wherein at least one of the male and/or female is a transgenic non human mammal as defined in the appended claims.

Preferably, the nucleic acid molecule or part thereof encoding an expression product with a Responder function and/or the nucleic acid molecule or part thereof encoding an expression product with a Distorter function and/or the nucleic acid molecule or part thereof encoding an expression product directed against the Distorter function and/or the nucleic acid molecule or part thereof for inactivation of the Distorter function by homologous recombination as defined in the present invention is/are flanked by recombinase recognition sites.

It is also described that one of the pair has (only) the Responder stably integrated into the germline whereas the partner of the pair has (only) integrated the Distorter into the germline. Upon crossing the offspring will carry both the Responder and the Distorter in the germline. In this manner, male offspring may be selected that is described in accordance with the main embodiments of the invention.

It is further preferred that the above pair of non-human male and female mammal has further stably integrated into its genomic DNA a nucleic acid molecule encoding a site specific DNA recombinase.

In the specific cases with low or no transmission of the transgene construct designed for selection against a genetic trait it is envisaged to use constructs, which are in an inactive state and therefore not selected against under standard breeding conditions, but can be activated after expression of a site specific recombinase. Using this method the (inactive) transgene construct can be transmitted at Mendelian rates. After expression of a site specific recombinase (such as Cre) from an inducible construct or by breeding of the male or female carrying the Cre gene in an active state to the female or male carrying said inactive transgene construct offspring can be generated which carries said transgene construct in an active state allowing selection against the sperm cells carrying said transgene construct. The latter offspring could then be utilized for the production of animals, which do not carry the undesired genetic trait.

Several methods can be utilized to keep a transgene construct in an inactive state, the most common being the use of a transcription stop cassette and/or a reporter gene inserted between the promoter driving expression of the transgene construct and the open reading frame (ORF) of the gene kept inactive by this method. It is envisaged that the stop cassette and/or reporter is flanked by recognition sequences for the site specific recombinase in direct repeat orientation allowing deletion of the stop cassette and/or reporter upon recombination, and subsequent expression of the ORF made active by this recombination event.

It is more preferred that in the pair of non human male and female mammal of the present invention said DNA recombinase is Cre, wherein said recognition sites are loxP sites, or flp, wherein said recognition sites are FRT sites, or Φc31, wherein said recognition sites are att sites.

It is also more preferred that in the pair of transgenic non human male and female mammals of the present invention said DNA recombinase is controlled by regulatory elements that are active prior to spermiogenesis.

The present invention also relates to sperm obtainable from a male of the transgenic non-human of the present invention, as defined in the appended claims

The present invention further relates to the use of the sperm of the present invention for the production of offspring.

The present invention also relates to the use of the nucleic acid molecule encoding an expression product with a Distorter function as defined in the present invention, for the identification of chemicals or biological compounds able to trigger the (premature) activation or inhibition of the Responder/Distorter signalling cascadeas defined in the appended claims.

The term "Responder/Distorter signalling cascade" as used above refers to any G protein signalling cascade, wherein at least one of the G proteins or other proteins in the cascade confers Responder/Distorter function.

Such compounds could be applicable as potent contraceptiva since it is envisaged that the activation or inhibition (repression) of said signaling cascade may affect the motility of sperm, due to rapid exhaustion of their energy reserve, and/or by inhibiting sperm movement and/or by affecting the ability of sperm to fertilize ovulated eggs.

It is envisaged that the identification of said chemical or biological compounds could be achieved by standard screening technology using the activity of the wild type Distorter protein expressed in vitro or in cell culture cells as an assay. It is e.g. known that GTPase-activating proteins such as Tagap1 enhance the GTPase activity of target GTPases rendering them inactive, or that GEFs such as Fgd2 or Tiam2 exchange GDP for GTP in said GTPases rendering them active. Assay systems for the activity of GAPs and GEFs and GTPases and other proteins involved in G protein signaling are well known in the art (Balch 1995); (Der 2000) allowing an artisan to screen for compounds triggering or inhibiting said proteins in vitro or in cell culture systems. It is envisaged that the compounds are then tested for their effects on sperm motility in vitro and on their effect in preventing fertilization of egg cells by sperm in vivo.

The present invention further relates to the use of the nucleic acid molecule encoding an expression product with a Distorter function as defined in the present invention for the isolation of receptor molecules and/or other members of the Responder/Distorter signaling cascade to which said expression product may bind, as defined in the appended claims.

Furthermore, the nucleic acid molecule as defined in the method of the present invention or the expression product as defined in the method of the present invention can be used for the isolation of receptor molecules and/or other members of the Responder/Distorter signaling cascade to which said expression product which would be expected to be a (poly)peptide may bind. Said signal transducing molecules are envisaged to be preferably identified by immunoprecipitation of protein complexes involving the Distorter (poly)peptide and cloning of the corresponding genes encoding them, or by Two Hybrid Screening techniques in yeast employing standard technology. In particular, most preferably the Distorter gene or (poly)peptide may be used to isolate the membrane receptor of the signaling molecule which is envisaged to activate said Responder/Distorter signaling cascade. Said membrane receptor is envisaged to be most preferable as a target for the development of novel contraceptives.

The present invention also relates to a method for the detection of a nucleic acid molecule encoding an expression product with a Distorter function and/or a nucleic acid molecule encoding an expression product directed against the Distorter function and/or a nucleic acid molecule for inactivation of the Distorter function by homologous recombination as defined in the present invention in a non human male or female mammal as defined in the present invention comprising identifying said nucleic acid molecule encoding an expression product with a Distorter function and/or said nucleic acid molecule encoding an expression product directed against the Distorter function and/or said nucleic acid molecule for inactivation of the Distorter function by homologous recombination in said non human male or female mammal by polymerase chain reaction (PCR), gene (micro)array hybridization, single nucleotide polymorphism (SNP) analysis, and/or sequencing with primers hybridizing to said nucleic acid molecule.

In a further embodiment of the , method, the non human male or female mammal, the pair of non human male and female mammalian animals, the sperm or the use according to the present invention the nucleic acid molecule encoding an expression product with a Distorter function, wherein said expression product with a Distorter function is a factor involved in G protein signaling is selected from the group consisting of: (a) a nucleic acid molecule comprising or consisting of the nucleic acid molecule of any one of SEQ ID NOs: 3 to 6 and 12 or a fragment thereof; (b) a nucleic acid molecule being an allelic variant or an orthologue of the nucleic acid molecule of (a); (c) a nucleic acid molecule which hybridizes under stringent conditions to the nucleic acid molecule of (a), wherein said nucleic acid molecule encodes a polypeptide which has (i) at the position corresponding to position 49 of SEQ ID NO: 17 an I (ii) at the position corresponding to position 144 of SEQ ID NO: 17 an L (iii) at the position corresponding to position 323 of SEQ ID NO: 17 a T and (iv) which terminates after position 442; (d) a nucleic acid molecule which hybridizes under stringent conditions to the nucleic acid molecule of (a), wherein said nucleic acid molecule encodes a polypeptide which has(i) at the position corresponding to position 49 of SEQ ID NO: 17 an I;(ii) at the position corresponding to position 137 of SEQ ID NO: 17 an E;(iii) at the position corresponding to position 207 of SEQ ID NO: 17 an F;(iv) at the position corresponding to position 301 of SEQ ID NO: 17 an M;(v) at the position corresponding to position 323 of SEQ ID NO: 17 an T;(vi) at the position corresponding to position 332 of SEQ ID NO: 17 a D;(vii) at the position corresponding to position 407-413 of SEQ ID NO: 17 an internal deletion;(viii) at the position corresponding to position 440 of SEQ ID NO: 17 an M;(ix) at the position corresponding to position 471 of SEQ ID NO: 17 an L;(x) at the position corresponding to position 552 of SEQ ID NO: 17 an I;(xi) at the position corresponding to position 596 of SEQ ID NO: 17 a K;(xii) at the position corresponding to position 607 of SEQ ID NO: 17 an R;(xiii) at the position corresponding to position 610 of SEQ ID NO: 17 an S; and (xiv) at the position corresponding to position 703 of SEQ ID NO: 17 a V; (e) a nucleic acid molecule which hybridizes under stringent conditions to the nucleic acid molecule of (a), wherein said nucleic acid molecule encodes a polypeptide which has (i) at the position corresponding to position 49 of SEQ ID NO: 17 an I; (ii) at the position corresponding to position 54 of SEQ ID NO: 17 a G; (iii) at the position corresponding to position 137 of SEQ ID NO: 17 an E; (iv) at the position corresponding to position 173 of SEQ ID NO: 17 a G; (v) at the position corresponding to position 207 of SEQ ID NO: 17 an F; (vi) at the position corresponding to position 301 of SEQ ID NO: 17 an M; (vii) at the position corresponding to position 323 of SEQ ID NO: 17 a T; (viii) at the position corresponding to position 332 of SEQ ID NO: 17 a D; (ix) at the position corresponding to position 407-413 of SEQ ID NO: 17 an internal deletion; (x) at the position corresponding to position 440 of SEQ ID NO: 17 an M; (xi) at the position corresponding to position 471 of SEQ ID NO: 17 an L; (xii) at the position corresponding to position 508 of SEQ ID NO: 17 an S; (xiii) at the position corresponding to position 552 of SEQ ID NO: 17 an I; (xiv) at the position corresponding to position 596 of SEQ ID NO: 17 a K; (xv) at the position corresponding to position 607 of SEQ ID NO: 17 an R; (xvi) at the position corresponding to position 610 of SEQ ID NO: 17 an S; and (xvii) at the position corresponding to position 703 of SEQ ID NO: 17 a V; (f) a nucleic acid molecule which hybridizes under stringent conditions to the nucleic acid molecule of (a), wherein said nucleic acid molecule encodes a polypeptide which has (i) at the position corresponding to position 49 of SEQ ID NO: 17 an I; (ii) at the position corresponding to position 137 of SEQ ID NO: 17 an E; (iii) at the position corresponding to position 207 of SEQ ID NO: 17 an F; (iv) at the position corresponding to position 301 of SEQ ID NO: 17 an M; (v) at the position corresponding to position 323 of SEQ ID NO: 17 a T; (vi) at the position corresponding to position 332 of SEQ ID NO: 17 a D; (vii) at the position corresponding to position 407-413 of SEQ ID NO: 17 an internal deletion; (viii) at the position corresponding to position 440 of SEQ ID NO: 17 an M; (ix) at the position corresponding to position 471 of SEQ ID NO: 17 an L; (x) at the position corresponding to position 530 of SEQ ID NO: 17 an E; (xi) at the position corresponding to position 552 of SEQ ID NO: 17 an I; (xii) at the position corresponding to position 573 of SEQ ID NO: 17 an R; (xiii) at the position corresponding to position 596 of SEQ ID NO: 17 a K; (xiv) at the position corresponding to position 607 of SEQ ID NO: 17 an R; (xv) at the position corresponding to position 610 of SEQ ID NO: 17 an S; and (xvi) at the position corresponding to position 703 of SEQ ID NO: 17 a V, as defined in the appended claims.

The nucleic acid molecule as described in any case retains the Distorter function. It has preferably a minimal length of at least 200 or 300 nucleotides. Such a molecule may also be used for example as a specific probe for hybridization reactions and would comprise at least one of the mutations of any one of SEQ ID NOs: 3 to 6. It is however also preferred that the nucleic acid molecules of the invention be significantly larger such as at least 500 or 1000 nucleotides. The nucleic acid molecules or fragments thereof as described may be fused to flanking sequences. In any case, the nucleic acid molecules as described may have a length of up to 500 nucleotides, 1000 nucleotides, 2000 nucleotides, 5000 nucleotides, 10000 nucleotides and in particular cases even up to 100000 nucleotides. When integrated into larger genomic regions, the nucleotides may have chromosomal length.

Also described herein are oligonucleotides/primers of a length of at least 8 and up to preferably 50 nucleotides, that are part of the above identified sequences or hybridize to the complementary strand thereof wherein said oligonucleotides/primers contain the sequence of at least one codon coding for any of the above-identified specific amino acid positions (or a complementary sequence thereof).

In a preferred embodiment of the method, the non human male or female mammal, the pair of non human male and female mammalian animals, the sperm or the use according to the present invention , the nucleic acid molecule is a DNA molecule.

It is furthermore preferred that said expression product is an RNA or a (poly)peptide.

The deduction of the amino acid sequence from the nucleic acid sequence defined werein allows the conclusion that the polypeptide is the expression product that contributes to the Responder/Distorter phenotype. However, it is not excluded that the mRNA contributes to said Responder/Distorter phenotype. Also, it is envisaged in accordance with the present invention that in certain embodiments the expression level, stage of expression during spermatogenesis or the copy number of said gene results in or contributes to the Distorter phenotype. Therefore, in a preferred embodiment, said expression product is an RNA or a (poly)peptide.

In accordance with the present invention the nucleic acid molecule as defined above with a regulatory region being capable of controlling expression of said nucleic acid molecule, as defined in the appended claims is comprising in a recombinant DNA molecule.

It is further preferred that said regulatory region is a naturally occurring region or a genetically engineered derivative thereof.

In accordance with the present invention said regulatory region comprises or is a promoter.

Described also is a vector comprising the recombinant DNA molecule as described herein.

The vector may simply be used for propagation of the genetic elements comprised therein. Advantageously, it is an expression vector and/or a targeting vector. Expression vectors such as Pichia pastoris derived vectors or vectors derived from viruses such as CMV, SV-40, baculovirus or retroviruses, vaccinia virus, adeno-associated virus, herpes viruses, or bovine papilloma virus, may be used for delivery of the recombinant DNA molecule or vector of the invention into targeted cell population. Methods which are well known to those skilled in the art can be used to construct recombinant viral vectors; see, for example, the techniques described in Sambrook, loc. cit. and Ausubel, loc. cit. Alternatively, the recombinant DNA molecules and vectors of the invention can be reconstituted into liposomes for delivery to target cells.

The vector may comprise may a heterologous promoter.

Said heterologous promoter not naturally operatively linked with the nucleic acid contributing to the Distorter function may be used to determine a certain time point of the onset of Distorter expression. This time point may be the same or a different one that is set when the natural Distorter transcription unit is employed. For example, said heterologous promoter may also be active in the early or late haploid phase of spermatogenesis.

In accordance with the present invention, said heterologous promoter is controlling gene expression in spermatogenesis and/or in spermiogenesis.

It is even more preferred that said the heterologous promoter is the testis promoter of c-kit, ACE, Tcr or Smok.

Also described herein is a method for the identification of a nucleic acid molecule encoding an expression product with a Distorter function, comprising the steps of (a) isolating a nucleic acid molecule encoding a candidate expression product with a Distorter function from the mouse t-complex by means of genomic localization, wherein said nucleic acid molecule is involved in G protein signalling; and (b) testing the nucleic acid molecule isolated in step (a) for a change of the transmission ratio of the Responder or of a genetic trait linked to a Responder in an experimental non human animal, wherein when said transmission ratio is enhanced or reduced, said nucleic acid molecule isolated in (a) is a nucleic acid molecule encoding an expression product with Distorter function.

The above method is exemplified, inter alia, in Example 4 for Fgd2. The methods described in detail in Example 4 provide an ideal example how a Distorter can be identified and verified in vivo by genetic testing of a null allele.

The present invention relates in addition to a method for the identification of an expression product of a nucleic acid molecule encoding a Distorter as defined in the appended claims, comprising the steps of (a) isolating an expression product of a nucleic acid molecule encoding a candidate Distorter by means of protein-protein interaction with a known Distorter derived from the mouse t-complex; and (b) testing the nucleic acid molecule encoding said expression product isolated in (a) for change of the transmission ratio of the Responder or of a genetic trait linked to a Responder in an experimental non human animal, wherein when said transmission ratio is enhanced or reduced, said expression product isolated in (a) is an expression product with Distorter function.

It is preferred that in step (b) of the above identification methods, hypomorphic or hypermorphic alleles of said nucleic acid molecule are used for testing for change of the transmission ratio.

it is envisaged that (a) (poly)peptide(s) binding to a known Distorter (poly)peptide is/are identified by co-immunoprecipitation of protein complexes involving the Distorter (poly)peptide, or by affinity chromatography purification of a protein binding to said Distorter polypeptide or a part thereof, or by other methods allowing purification and analysis of protein complexes such as mass spectrometry, and subsequent cloning of the corresponding genes encoding the proteins binding to said Distorter (poly)peptide, or by Two Hybrid Screening techniques in yeast employing standard technology (Chien, Bartel et al. 1991).

Genetic testing in transgenic animals for the ability of the Distorter candidate identified by the methods described above to enhance or reduce the transmission ratio of the Responder can be performed using, for example, hypomorphic or amorphic or hypermorphic alleles of said Distorter candidate, which are constructed for example by introduction of (a) nucleic acid molecule(s) expressing a shRNA directed against said Distorter candidate, or by targeting the nuclear gene locus of said Distorter candidate thereby inactivating the gene function, or by introducing a construct expressing the wild type Distorter candidate thereby increasing the dosage of the expression products of said Distorter candidate.

The figures show:

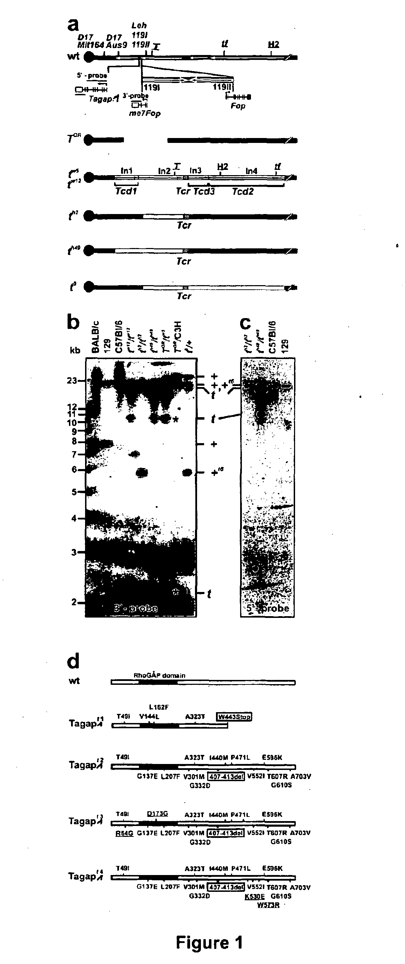

**Figure 1** *Tagap1* is a candidate for *Tcd1.* **a**, Schematic map of the *t*-complex on chromosome 17. The approximate positions of *Tagap1, Fop* and the fusion gene *me7Fop* are indicated, gene maps are expanded. The centromere is indicated by a filled circle, wild-type chromatin by filled bars, *t*-chromatin by open bars, inversions (In1-In4) by arrows. The molecular and genetic markers, and the structure of partial *t-*haplotypes have been described previously (Lyon 1984). The approximate extent of the deletion in *T^{OR}* is indicated by a gap, positions of *Tcd*s by brackets, *Tcr* by a hatched box. Maps are not to scale. **b**, Genomic mapping of *me7Fop* and *Tagap1* to the *Tcd1* region in *t*-haplotypes. The 3'-probe of *me7Fop* detects two wild-type bands, which are polymorphic in different strains and in *t⁶*, and three *t*-specific bands, which are absent from T*^{OR}* and not observed in *t⁶*, localizing both genes to the *Tcd1* interval. **c,** *Tagap1* maps to the *Tcd1* region and is amplified in *t*-haplotypes. A *Tagap1*-specific 5'-probe detects two polymorphic fragments in *t^{h49}*/*t^{h49}* DNA equalling four-fold the signal intensity of the wild-type band, as determined by quantification. **d**, *t*-haplotypes encode four different classes of *Tagap1* transcript. Schematic representation of representative cDNA clones isolated from testis of *t^{h49}*/*t^{h49}* mutant animals, in comparison to the wild-type gene. Mutations resulting in deletion of amino acid residues are boxed, mutations distinguishing products derived from different *Tagap1* loci are underlined.

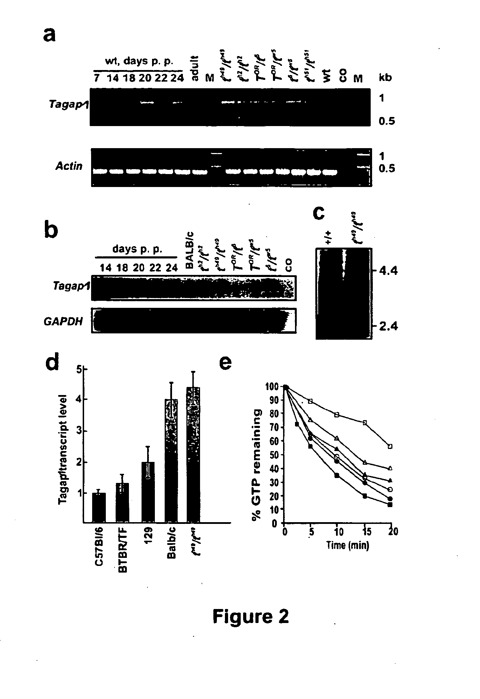

**Figure 2** Expression analysis and GAP-activity assays of *Tagap1.* **a**, *Tagap1* is already expressed at early stages of spermatogenesis. RT-PCR analysis of RNA isolated from various postnatal (p.p.) testes, and from testes of adult wild type mice or males carrying various *t*-haplotypes; Actin served as control. **b,** RNAse protection assay confirming the RT-PCR data. **c,** Northern blot analysis of 8 µg poly(A+)RNA hybridized with the *Tagap1*-specific 5'-probe. The *t*-specific mRNA migrates faster than the wild type. **d,** Analysis of *Tagap1* transcripts by quantitative PCR reveals up to four-fold higher levels of *Tagap1* transcript in *t^{h49}* as compared to wild type strains. **e,** Tagap1 enhances the GTPase activity of RhoA. Squares, RhoA; triangles, cdc42; circles, Rac1; open symbols, reaction carried out without Tagap1; filled symbols, with Tagap1. Abbr.: +, wild type; p.p., days post-partum.

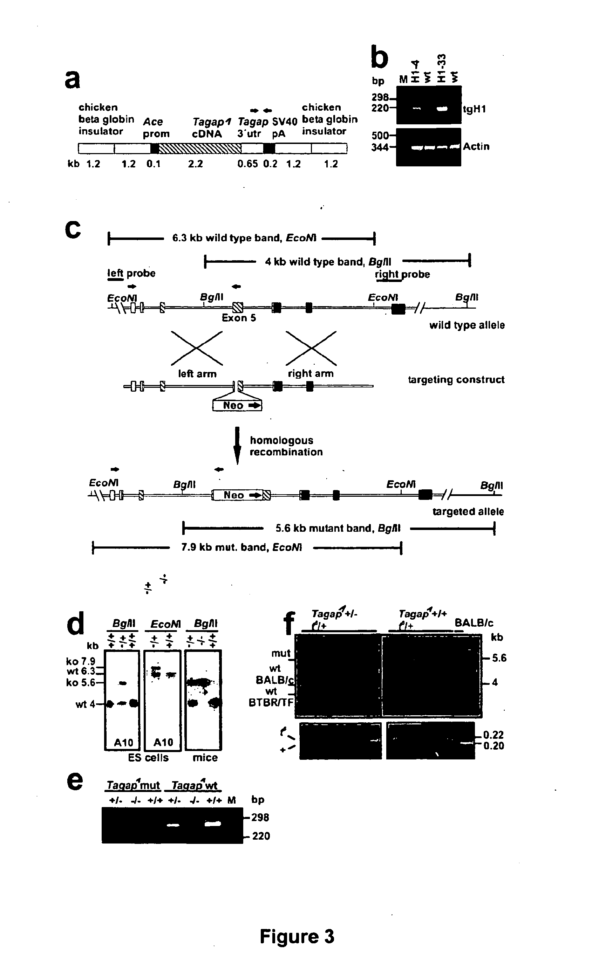

**Figure 3** Construction of gain- and loss-of-function alleles of *Tagap1.* **a,** Transgenic construct used for over-expression of wild type *Tagap1*. Black arrows indicate primers for genotyping. **b,** RT-PCR analysis of testis RNA verifying expression of the transgenic constructs. Actin served as positive control. Abbr.: H1-4, *Tg(Tagap1)H1-4Bgh;* H1-33, *Tg(Tagap1)H1-33Bgh.* **c,** Targeting of the *Tagap1* gene. Introns are depicted as double lines, exons as boxes; coding regions are hatched, GAP-domain encoding regions filled. A PGKneo selection cassette was integrated into exon 5. Primers used for expression analysis in **(e)** are indicated by black arrows. **d,** Identification of the targeted allele *Tagap1^{tm3Bgh}* in clone A10. Genomic Southern blot analysis identifies the predicted size *BgIII* and *EcoNI* fragments, detected with the right and left probe, respectively, in the ES-cell clone A10. Right panel: genotyping of a heterozygous and a homozygous mutant male, confirming germ line transmission of the mutant allele. **e,** Genotyping of males used for testing the effect of the *Tagap1* knock-out allele on the transmission ratio of the *t⁶*-haplotype. **f,** RT-PCR analysis of testis RNA from wild type (+/+), heterozygous (+/-) and homozygous (-/-) mutant animals with primers specific for the mutated (left part) and wild type allele (right part), demonstrating loss of the wild-type *Tagap1* transcript in -/- animals.

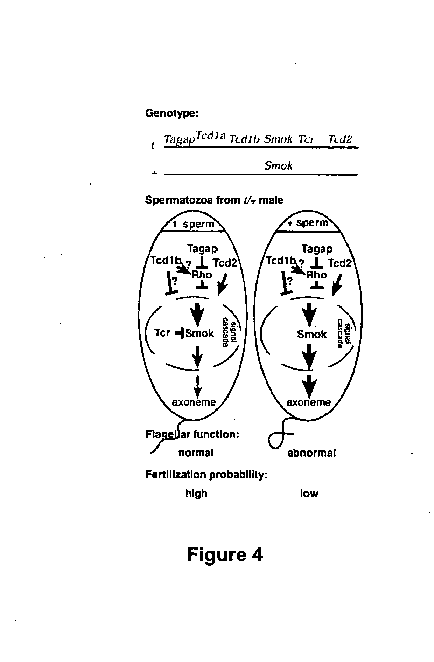

**Figure 4** Model of the role of *Tcds* and *Tcr* in transmission ratio distortion. *t-*haplotypes encode several *Tcds* (*Tcd1^{Tagap1}, Tcd1b, Tcd2* are indicated, wild type alleles not shown) and *Tcr* acting upstream of Smok kinase controlling flagellar behaviour. Tagap1 is a negative regulator of a Rho family member, which inhibits Smok. Tagap1^{Tcd1a} (Tagap1) enhances down-regulation of Rho, resulting in up-regulation of Smok. *Tcd1b* is a hypomorphic or amorphic allele of an activator of Rho or of an inhibitor of Smok, further enhancing Smok activity epistatically to Tagap1. Likewise, Tcd2 further promotes up-regulation of Smok. All sperm (*t* and +) produced by *t*/+ males are affected by Tcds, which act in *trans.* This negative effect of Tcds is counter-balanced by Tcr, which is restricted to *t*-sperm and thus rescues *t*-sperm only. This results in an advantage of *t*-sperm in fertilizing the eggs and promotes the transmission of the t-haplotype to the offspring. For details see text.

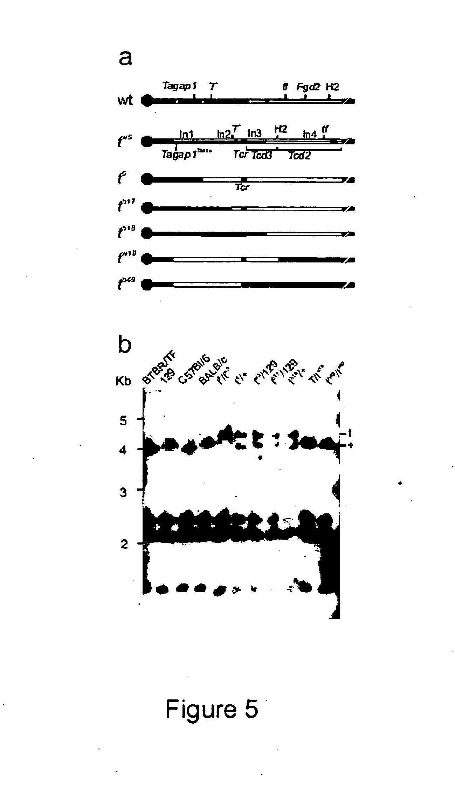

**Figure 5** *Fgd2* maps to the *Tcd2* region. **a,** Structure of a complete (*t^{w5}*) and various partial *t* haplotypes (*t^{x}*) used for mapping of *Fgd2.* The *Tcd2* region is defined as the segment of *t* chromatin, which is present in *t^{h18}* and exchanged for wild type chromatin in *t^{w18}.* The centromere is shown as filled circle at the left, wild type chromatin is symbolized by filled bars, *t* chromatin by open bars and inversions (In1 - In4) by arrows. Markers and *t* haplotypes have been described (Lyon 1984). wt, wild type. **b**, Southern blot analysis of genomic DNA digested with *Pst*I*,* using a full length *Fgd2* cDNA clone as probe, reveals a *t*-specific band of 4.5 kb, which occurs in *t* haplotypes carrying *Tcd2,* but not in *t^{w18}*, thus mapping *Fgd2* to the *Tcd2* region.

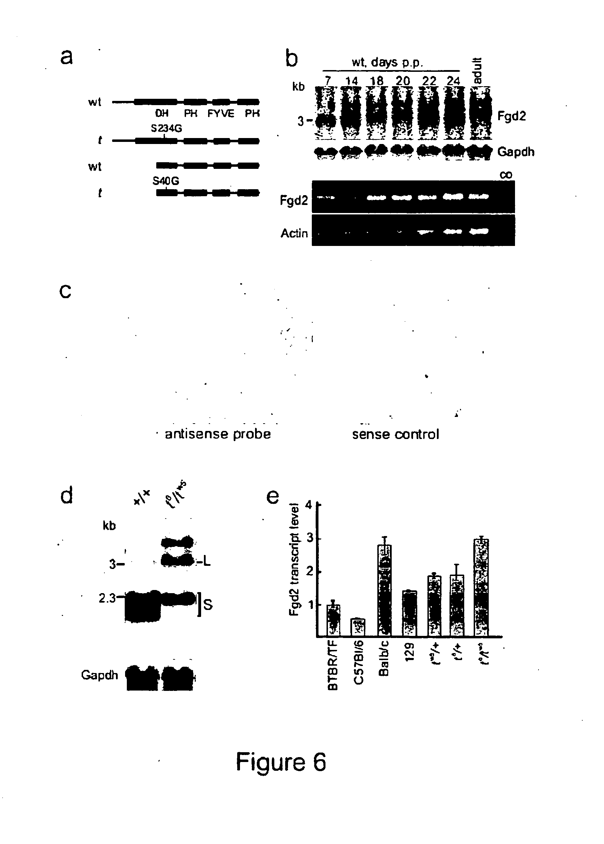

**Figure 6** *Fgd2* expression from the *t* haplotype allele is strongly enhanced as compared to wild type alleles. **a,** Domain structure of Fgd2 proteins encoded by the long and the short transcript variants derived from this gene. The mutation S40G in transcript variant 2 is equivalent to the S234G mutation in transcript variant 1. **b,** Temporal expression profile of the long *Fgd2* transcript in postnatal testes, representing the first cycle of spermatogenesis. Top panels: Northern blot, lower panels: RT-PCR-analysis. p.p., post partum. **c**, In situ hybridization of *Fgd2* antisense or sense control transcripts to testis cryo-sections showing expression of *Fgd2* in meiotic spermatocytes and round spermatids. **d,** Northern blot analysis of *Fgd2* expression in testis derived from wild type (+/+) and *t⁶*/*t^{w5}* males demonstrates strongly enhanced expression of the long (L) and simultaneous strongly reduced expression of the short (S) *Fgd2* transcript in *t* haplotypes. **e,** Quantitative RT-PCR analysis of the long *Fgd2* testis transcript in various strains and *t* haplotypes, demonstrating up to 6-fold higher expression of *Fgd2* in *t* haplotypes compared to wild type strains, which show considerable differences. +, wild type strain BTBR/TF; Gapdh, Gapdh loading control.

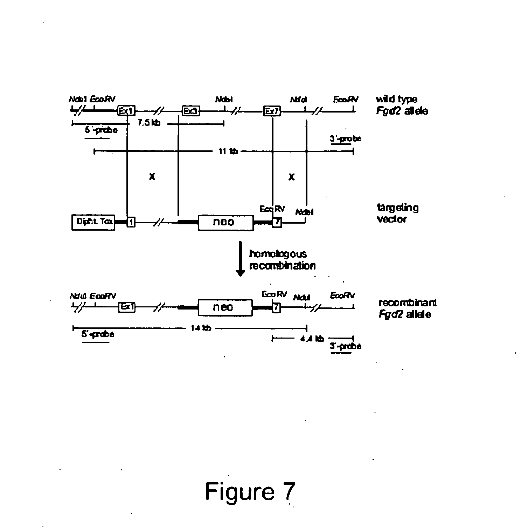

**Figure 7** Targeting of the mouse *Fgd2* gene by homologous recombination. The targeting vector was constructed by ligation of the left and right homology arm, both derived by PCR amplification of genomic DNA, to the vector pDT/pGKneoflox 3xpA (see Methods). Out of 132 clones analyzed, 1 displayed the expected RFLP with the 5'-probe (7.5 kb *Nde*I fragment for the wild type allele and 14 kb for the targeted allele) and 3'-probe (11 kb *EcoR*V versus 4.4 kb for the wild type allele and mutant allele respectively) demonstrating successful targeting of the genomic locus by homologous recombination.

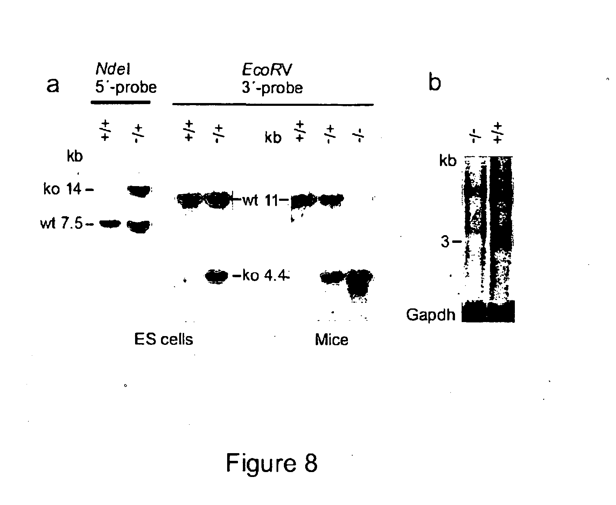

**Figure 8** Gene targeting of *Fgd2* by homologous recombination **a,** Southern blot analysis of DNA derived from targeted and control ES cells (left panel), and of mice (right panel) carrying the mutant allele (-), with the 5'- and 3'-probes. **b,** Northern blot analysis of testis RNA derived from wild type and mutant animals. The long *Fgd2* transcript is not detected in homozygous mutant animals. Gapdh, Gapdh loading control; ko, band derived from targeted allele; wt, wild type fragment; +, wild type locus; - , mutant locus.

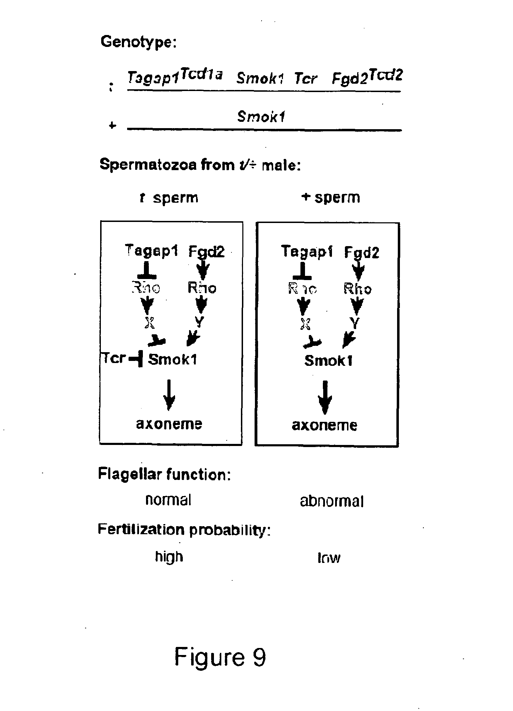

**Figure 9** Model of transmission ratio distortion. The *t* haplotype encodes several Distorters. Only two are shown for clarity, which are expressed in all sperm cells derived from a *t*/+ male and act on two opposing Rho signalling pathways regulating Smok1. Smok1 is thought to be involved in sperm motility control. *Tagap1^{Tcd1a}* and *Fgd2^{Tcd2}* represent hypermorphic alleles expressing strongly elevated gene activity as compared to the wild type. Enhanced down-regulation of the inhibitory pathway by Tagap1 and stronger up-regulation of the activating pathway by Fgd2 additively induce hyper-activation of Smok1 in all sperm, resulting in abnormal flagellar function and low fertilization probability. This harmful effect of the Distorters is rescued by the dominant-negative action of Tcr, which is restricted to *t* sperm, giving the latter an advantage in fertilizing the egg cells. Neither the Rho switch molecules nor their target effector proteins (X, Y) are known. Arrows symbolize activation, bars inhibition; green arrow, normal signaling; purple arrow, impaired signalling.

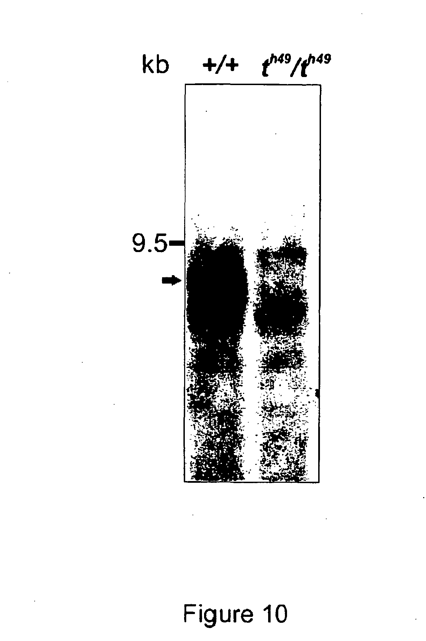

**Figure 10** Northern blot analysis of 8 µg poly(A+)RNA hybridized with a Tiam2 specific probe. The Tiam2 specific band, observed in wild type testis RNA is not detected in *t^{h49}*/*t^{h49}*, indicating, that the gene is not expressed in the *t*-haplotype.

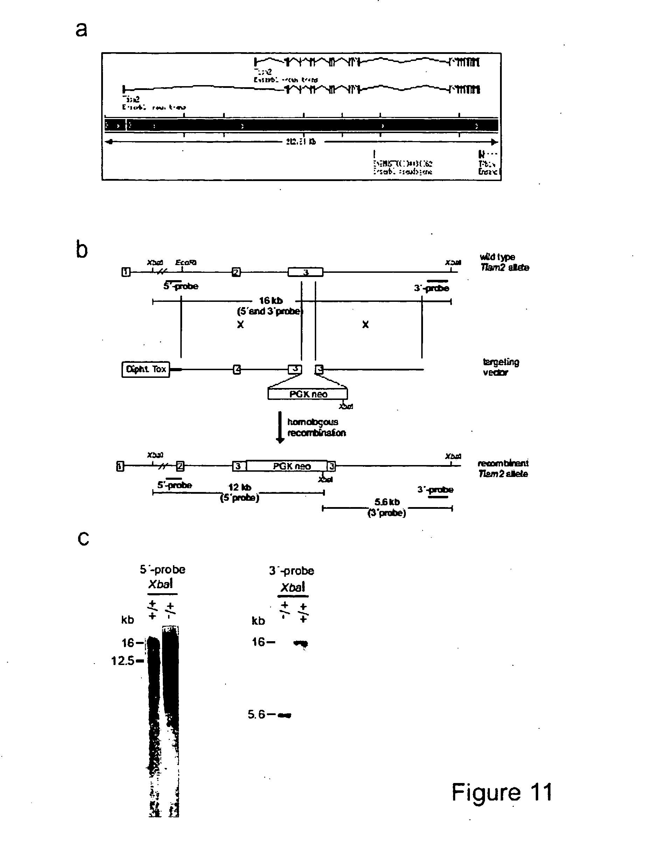

**Figure 11** Genomic Structure of Tiam2 and generation of a loss-of-function allele in embryonic stem cells. **a,** Transcripts annotated by the ensembl genome server (hftp://www.ensembl.org). **b,** Targeting of the Tiam2 gene. Introns are depicted as lines, exons as boxes. The targeting vector was constructed by ligation of the left and right homology arm, both derived by PCR amplification of genomic DNA, to the vector pDT/pGKnoflox 3xpA. The targeting event resulted in the integration of the PGK neo selection cassette into exon 3, thereby interrupting the open reading frame of the gene. **c,** Identification of the targeted Tiam2 allele. Genomic southern blot analyses identify *Xba*I fragments of the predicted size with the left (5'-) and right (3'-) probe respectively in ES cells.

The examples illustrate the invention.

### Material and Methods

### Mice and genetics

### Taqap1

For mapping of *Tagap1* on genomic DNA the following *t*-haplotypes were used: *t^{w12}*/*t^{w12} t^{h2}*/*t^{h2}*, *t^{h49}*/*t^{h49}*, *t^{h51}*/*t^{h51}, T^{OR}*/*t^{w5}, T^{OR}*/*^{t6}, t⁶*/*+.* The *t*-haplotypes *t^{w12}, t^{h49}*, *t^{h51}* and *t^{w5}* carry *Tcd1,* while *t⁶* and *t^{h2}*, which is derived from *t⁶*, lack *Tcd1* activity. Southern blot analysis of *Kpn*I digested genomic DNA was performed by standard procedures (Church and Gilbert 1984) using a fragment corresponding to position 942-3001 of the *Tagap1* cDNA as 3'-probe, and position 124-942 as 5'-probe. Genotyping of mice for the transmission of *t⁶* was done by PCR using primers for the marker *Hba-4ps*. Transgenic lines were generated in the inbred strain FVB/N by pronuclear injection of construct DNA using standard procedures. The *Tagap1^{tm3Bgh}* allele was generated in Balb/c ES-cells obtained by B. Ledermann (Basel Institute for Immunology) (Dinkel, Aicher et al. 1999). Also other ES cells known to the person skilled in the art, however, can be used. Transgenic and knock-out lines were back crossed several generations to the strain BTBR/TF-+*tfl*+*tf* before testing for Distorter activity.

### Fgd2

Fgd2 was mapped by Southern blot analysis of *Pst*I digested genomic DNA derived from various complete and partial *t* haplotypes obtained by rare recombination between wild type and *t* haplotype chromosomes (see Fig. 5a), using a cDNA of *Fgd2* as probe (position 170 - 2576 in Acc. Nr. AF017368, SEQ ID NO: 9). We derived an ES-cell line from the strain BTBR/TF-+tf/+tf and generated the targeted allele *Fgd2^{tm4Bgh}* by standard procedures (Fig. 8a, b). A heterozygous *Fgd2^{tm4Bgh}*/+ female was mated to a *t^{h49}*/*t^{h49}* male to generate male litter mates of the genotypes +/+; *t^{h49}*/+ and +*IFgd2^{tm4Bgh}*; *t^{h49}I+* for testing the effect of the targeted allele on the transmission ratio of the *t* haplotype (table 1). We genotyped mice for *t^{h49}* by Southern blot analysis of *Kpn*I (or alternatively *Bam*HI) digested genomic DNA using the 3'- fragment of Tagap1 as probe as described.

### Transcript analysis

### Tagap1

The 5'ends of *me7Fop* and *Tagap1* were obtained by 5'-RACE using the GeneRacer kit (Invitrogen). Standard reverse transcription was performed with 1 microgram of total RNA using AMV-RT (Promega). RNAse protection assays were done using standard procedures (Gilman 1997). The *Tagap1* probe was synthesized in vitro in the presence of [³²P] UTP from a fragment derived from the Tagap1 cDNA by PCR (sense 5'-GACTCCTAGGGTCAGAGTGTCATG-3', antisense 5'-TGGGCTCCACATCTGGGTCATT-3') cloned in pCRII TOPO (Invitrogen). The *GAPDH* control RNA was transcribed from the pTRI-GAPDH template (Ambion). Northern analysis was performed by standard techniques (Sambrook J. 1989) using 8 µg of single purified poly(A+)RNA using the Fast Track system (Invitrogen). Quantitative PCR analysis was carried out on an ABI PRISM 7900 HT SDS (Applied Biosystems) using the TaqMan probe 5'-ATCCTCTGCCTTAAAGGTCCTTCAACGGAA-3' (5' FAM and 3' TAMRA labeled) and primers sense 5'-CCAGACCCATCCAGGACATC-3' and antisense 5'-CTGGCAGCTTTCCTGAATATC-3'. As a reference, GAPDH expression was determined using the mouse GAPDH assay (Applied Biosystems).

### Fgd2

A plasmid cDNA library from testis RNA derived from a *t⁶*/*t^{w5}* male using the SuperScript plasmid cDNA cloning system (Life Technologies) was constructed and screened by colony filter hybridization using *Fgd2* derived cDNAs as probes. We also obtained cDNAs encompassing the full coding sequence from *t* haplotypes and wild type by RT-PCR. We sequenced clones from both sources and analyzed the results using the Lasergene DNA Star package. We isolated total RNA using Trizol (Invitrogen). For quantitative real time PCR we used an ABI PRISM 7900 HT SDS (Applied Biosystems). As a reference gene, we analyzed *Gapdh* expression with the mouse GAPDH assay (Applied Biosystems). We isolated polyA+ RNA using the Fast Track system (Invitrogen) and performed northern blot analysis using the Ambion GlyMAX Northern kit. In situ hybridization on 10 µm cryostat sections was essentially performed as described (Brent, Schweitzer et al. 2003). We produced riboprobes by in vitro transcription from the TOPO pCRII vector containing a *Fgd2* cDNA fragment obtained by PCR amplification. Digoxygenin-labeled probes were detected by phosphatase reaction of the substrate NBT/BCIP (Sigma).

### Gene targeting and transgene constructs

### Tagap1

The Tagap1 targeting vector was constructed by ligation of the left and right arm, both derived by PCR amplification of genomic DNA, to the PGK-neo cassette. Culture, electroporation, selection, isolation of ES-cell clones, DNA preparation in 96 well plates and Southern blot analysis were done according to standard procedures (Ramirez-Solis, Davis et al. 1993). The transgenic construct *Tg(Tagap1)H1Bgh* consists of the *Angiotensin Converting Enzyme (ACE)* testis promoter and transcription start (extending from -91 to +17 bp), driving expression in elongating spermatids (Morita, Murata et al. 1993), followed by the complete ORF of wild-type *Tagap1,* its 3'UTR and the SV40 polyadenylation signal derived from the vector pCS2+ (Rupp, Snider et al. 1994), replacing the *Tagap1* polyA-signal sequence. This transcription unit is flanked by tandem copies of the chicken-globin insulator (Chung, Whiteley et al. 1993). Transgenic animals were identified by PCR using the primers: sense 5'-AGGGCCCTTGGGGTCAGG-3', antisense 1 5'-CTGTCAGTCTCCATTCCAATGAAG-3' and antisense 2: 5'-CAGTTAGCTGGCAAATGCTGTC-3'. The wild-type band is 541 bp in length, the transgenic construct produces bands of 165 bp and 266 bp.

For gene targeting of the Fgd2 locus, a fragment containing the PGK-promoter driven neomycin resistance gene flanked by loxP sites was isolated from the vector pPGKneo FloxI (gift of Moises Mallo) by *EcoRVI EcoRI* digest and ligated into the *EcoRVI EcoRI* digested pDT Bluescript vector (provided by Achim Gossler), which contains the diphteria toxin-A chain coding sequence under the control of RNA polymerase II promoter. pDT/pGKneoflox 3xpA was digested with *Not*I, filled in, cut *Xba*I and ligated to the left homology region, which was obtained by PCR amplification of genomic DNA using primers s: 5'-ACTAGTCTGCTTCTGGGGTAACT -3' containing a *Spe*I site and as: 5'-ATAGGCCTGCTCCGTCT -3' followed by digestion with *Spe*I. The obtained construct was digested *EcoRVI Sal*I and the right homology region, obtained by PCR on genomic DNA with primers s (*EcoRV*): 5'-GATATCAAGAATCCCGCGGTACGAACTG -3' and as (*Sal*I): 5'-GTCGACGACAACGCCCGACATCATAGAG -3' and cut with *EcoR*V and *Sal*I was ligated into this vector. The resulting targeting vector was linearized by restriction digest with *Sal*I. Establishment of a BTBR/TF-ES cell line, culture, electroporation, selection, isolation of ES-cell clones, DNA- preparation in 96 well plates and Southern blot analysis was done according to standard procedures. The left probe (LP) and right probe (RP) for Southern blot analysis were generated by PCR with primers LPs 5'- ACAGGTCTCACGTAGCCGAATC -3', LPas 5'-CGGGTGAAGCAGGTCTACCACA -3' and RPs 5'-TGGATGCCGCTCAGTTGCTAAT -3',RPas 5'- TGAAACTCAGTGTGTAGACCAG - 3'respectively.

### Fgd2

We isolated the left (3.9 kb) and right (2.6 kb) homology regions by PCR- amplification of genomic DNA derived from the strain BTBR/TF-+tf/+tf. Using restriction sites included in the oligonucleotides we ligated the homology regions to either side of a PGK-promoter/neo resistance gene/triple-pA cassette inserted in a pBluescript vector containing the diphtheria toxin-A chain gene (kindly provided by Achim Gossler), which thereby flanked the left homology region. An *Eco*RV restriction site creating a RFLP for genotyping of the targeted allele was introduced by ligation of the right homology region to the selection cassette. We linearized the resulting targeting vector with *Sal*I. We established a BTBR/TF ES cell line, electroporated the targeting construct, selected and isolated ES cell clones, prepared DNA in 96-well plates, and performed Southern blot analysis according to standard procedures, using 5'- and 3'-probes obtained by PCR amplification of genomic DNA. The targeting event replaced exons 3 to 6 and part of exon 7 by the neo selection cassette, which also removed a genomic *Nde*I site, creating another RFLP for genotyping. We verified correct targeting of the locus and genotyped mice using *EcoR*V digested DNA hybridized with the 3'-probe, which detects an 11 kb fragment derived from the wild type and a 4.4 kb fragment derived from the targeted *Fgd2* allele. The 5'-probe detects a 7.5 kb fragment in *Nde*I digested DNA derived from the wild type and a 14 kb fragment derived from the mutant *Fgd2* allele. Out of 132 clones analyzed, 1 displayed the expected mutant fragments with both probes demonstrating successful targeting of the *Fgd2 locus* by homologous recombination.

Oligonucleotide sequences and PCR conditions for PCR experiments concerning the characterization of Fgd2 are listed in table 4.

### In vitro GAP assays

The catalytic domain of wild type Tagap1, small G proteins and the C-terminal polypeptide of Tagap1, the latter serving as negative control, were produced as GST-fusion proteins in *E. coli* BL21 using the pGEX vectors as described (Frangioni and Neel 1993) (Self and Hall 1995). For quantification of relative amount of proteins used, all preparations were adjusted relative to a BSA standard. GAP assays were performed in triplicate (G proteins at 6 nM; Tagap1: 15 nM) essentially as published (Self and Hall 1995).

### Example 1: Isolation of a candidate gene for Tcd1

We have used a positional cloning approach to identify a candidate for *Tcd1*, based on the following criteria: 1) The gene must be located in the genomic interval comprising *Tcd1*, 2) it must be expressed in testis, 3) show alterations in the *t-*haplotype form vs. the wild type, and 4) should encode a protein involved in signalling. The latter criterion was based on our proposal that *Tcds* encode components of signalling cascades acting upstream of Smok (Herrmann, Koschorz et al. 1999).

Since chromosomal rearrangements have a high potential of affecting gene function we started our search for *Tcd* candidates in the region *D17Leh119I*, which marks the end of a large inverted duplication in the wild type chromosome (Herrmann, Barlow et al. 1987). Genomic fragments, spanning the duplication breakpoint were hybridised to a cDNA library and a gene, designated *me7Fop,* showing similarity with *FGF receptor oncogene partner* (*Fop*) was identified (Fig. 1 a). Northern analysis showed that this gene is highly expressed in wild type testis, whereas no transcripts are detectable in testes from males carrying the *Tcd1* region in the *t*-haplotype form (e.g. *t^{h51}*, *t^{h49}* or complete *t*-haplotypes). A detailed analysis of the gene structure showed that the 5'-region of *me7Fop* is derived from *Fop*, while most of the coding region and the 3'-untranslated sequence come from an unrelated gene.

The part of *me7Fop,* which is not derived from *Fop* occurs in a second locus on the wild type chromosome. This is shown by genomic mapping using the 3'-region of *me7Fop* as probe (Fig. 1b). In wild type genomes two bands are detectable, whereas *t*-haplotypes produce three polymorphic *t*-specific bands. Both wild type fragments are missing in the deletion chromosome *T^{OR}* since genomic DNA from a *T^{OR}*/*t^{w5}* animal shows only the *t*-specific bands. This data maps both gene fragments to the proximal *t*-haplotype.

The analysis of the partial *t*-haplotype *t⁶* allows a more accurate assignment of both genes to the region harbouring *Tcd1*. The *t*-haplotype *t⁶* and its derivative *t^{h2}* have lost *Tcd1* activity by a recombination event in which the proximal portion of the *t*-haplotype has been exchanged for wild type DNA (Lyon 1984). Thus *Tcd1* is located in the region, which is wild type in *t⁶*. The Southern blot analysis revealed no *t*-specific band in *t⁶* or *t^{h2}*, but a wild type and a *t⁶*-specific band. Accordingly, both gene fragments map to the *Tcd1* region (Fig. 1a). We mutated *me7Fop* by gene targeting and analysed its possible role as Distorter of the transmission ratio. Distorter activity was not observed excluding *me7Fop* as candidate for *Tcd1.*

We extended the analysis to the gene from which *me7Fop* was derived. 5'-RACE protocols and database searches were utilized to obtain a complete cDNA clone. Sequence analysis showed that it encodes a protein of 714 amino acid residues involving a domain with high similarity to GTPase-activating proteins (GAP) for Rho small G proteins (Fig. 1d).

In the course of our studies this gene appeared in public databases as *T-cell activation Rho GTPase-activating protein* (*Tagap1,* accession number NM_145968). Genomic Southern blot analysis using a *Tagap1*-specific 5'-fragment of the cDNA as probe showed that wild type strains contain a single band, which is also present in *t⁶*/+ DNA. In contrast, genomic DNA derived from *t*-haplotypes showed two stronger polymorphic bands (Fig. 1c). This polymorphism confirms the mapping of *Tagap1* to the *Tcd1* region. Quantification of the wild-type and the *t*-specific signals revealed that the *Tcd1*-bearing *t*-haplotypes harbour four *Tagap1* loci, while the wild-type genome contains a single complete *Tagap1* gene. This result was confirmed by quantitative PCR on genomic DNA using *Tagap1* specific primers. Hybridisation with the 3'-probe, which detects *me7Fop* and *Tagap1,* and quantification of the bands revealed two-fold higher signal intensity in genomic DNA from *t^{h49}*/*t^{h49}* mice compared to *t^{h2}*/*t^{h2}* mice or wild type strains.

In accordance with multiple genomic copies of *Tagap1* on the *t*-haplotype, sequencing of *Tagap1* cDNAs derived from testis of *t^{h49}*/*t^{h49}* males revealed several types of transcripts with multiple non-silent nucleotide changes compared to the wild type sequence (Fig. 1d). The major cDNA class (51 out of 74 clones analysed), derived from *Tagap1^{t1}* contains a transition of G to A at codon 433, turning a tryptophane (TGG) into a premature stop codon (TGA). This mutation truncates the predicted protein, leaving the N-terminal RhoGAP domain intact. Two additional mutations (V144L, L162F) were found in the RhoGAP domain of Tagap1^{t1}, and two (T49I, A323T) outside of this region. None of these alterations were found in transcripts derived from wild-type strains, nor in the partial *t*-haplotypes lacking *Tcd1*, in *t⁶* and *t^{h2}*_{.}

The remaining three *t-*specific Tagap1 cDNAs do not contain the W443X mutation. Instead, a number of non-silent point mutations and a 21 base pair deletion 3' to the RhoGAP domain were detected in these clones (Fig. 1 d). Two alterations in the RhoGAP domain (G137E, L270F) are shared by all three genes, while *Tagap1^{t3}* differs from *Tagap1^{t2}* and *Tagap1^{t4}* by one additional mutation (D173G) in this domain. Aside from shared alterations, these cDNAs also show differences distinguishing them from each other, suggesting that they are derived from three distinct *Tagap1* genes, which arose by triplication of a single locus.

The combined genomic and cDNA sequence data demonstrate that *t-*haplotypes contain four *Tagap1* loci in the *Tcd1* region, while the wild type has two, one complete (*Tagap1*) and one altered gene (*me7Fop*), which has lost the GAP-domain due to a rearrangement. The fact that the *t-*alleles of *Tagap1* are altered with respect to the wild type is consistent with the criteria for a *Tcd* candidate.

RNA expression analysis by RT-PCR and RNAse protection assays showed that *Tagap1* is transcribed in the testis already at the earliest stage analysed, day 7 *post partum* (Fig. 2 a,b). Thus *Tagap1* is expressed already in diploid spermatocytes, which may be conducive to distribution of the gene products to all sperm cells, since spermatids develop in a syncytium. Northern blot analysis using poly(A+) RNA suggested low level transcription in this organ (Fig. 2c). In situ hybridisation analysis on testis sections using a *Tagap1* specific probe did not produce distinct signals.

The transcript detected in the *t-*haplotype shows a slightly faster migration compared to the wild type mRNA (Fig. 2c). The reason for this is unclear, as no major size differences (except for the 21-base deletion) were observed in any of the *t-*specific cDNA clones analysed. It is conceivable that poly-adenylation differences may account for the smaller transcript size. Shortening of the poly-A tail has been shown to accompany translational activation of some mRNAs during spermiogenesis (Kleene 1989). Whether or not the observed difference has a functional relevance, however, remains to be determined.

*Tagap1* transcripts were detected in all organs examined by RT-PCR analysis, and *Tagap1* ESTs have been reported in public databases from a large variety of tissues and organs, suggesting that the gene is ubiquitously expressed.

Quantitative RT-PCR showed that *t-*haplotypes express up to four fold higher levels of *Tagap1* transcripts than wild-type strains (Fig. 2d). This finding suggests that sperm derived from *t*/+ males on different wild type backgrounds may produce substantially different levels of Tagap1 protein. This may have a profound effect on the transmission ratio of the *t-*haplotype, consistent with earlier reports that the genetic background has an important impact in transmission ratio distortion (Gummere, McCormick et al. 1986).

Finally we examined the specificity of the GAP-domain of wild type Tagap1 towards three "classical" small GTPases, RhoA, cdc42 and Rac1, which are, among several others, expressed in testis (Wennerberg and Der 2004). G proteins act as molecular switches, which transmit a signal in their active, GTP-bound form, whereas they become inactive after GTP hydrolysis. GAPs enhance the intrinsic GTPase activity of small G proteins, promoting their inactive state. Our data show that the GTPase activity of RhoA was strongly enhanced by the GAP domain of Tagap1, whereas the other family members were only mildly (Cdc42) or hardly (Rac1) stimulated, identifying RhoA as a possible in vivo target of Tagap1 (Fig. 2e).

### Example 2: Tagap1 distorts the transmission ratio of t-haplotypes

Since the genetic and molecular data suggested that the *t-*loci of *Tagap1* might cause a gain-of-function phenotype, we tested whether over-expression of wild-type *Tagap1* in elongating spermatids, from a transgene construct controlled by the testis-specific ACE promoter, would alter the transmission ratio of the partial *t-haplotype t⁶* lacking *Tcd1* (Fig. 1a, 3a) (Howard, Balogh et al. 1993). Two independent transgenic lines harbouring the construct and expressing the transcript were generated, crossed into males carrying *t⁶* and analysed (Fig. 3b, Table 1). In both lines, a significant increase of the transmission of the *t⁶*-haplotype to the offspring was observed, as compared to non-transgenic litter mates (combined data: 88%, vs. 80% *t⁶* offspring, p<0.01). Thus, a dosage increase of wild type *Tagap1* in testis phenocopied a *t-complex Distorter,* consistent with the idea that the *t*-loci of *Tagap1* encode *Tcd1* activity.

To create a loss-of-function allele of *Tagap1,* we disrupted the gene by inserting a selection cassette into exon 5, resulting in premature termination of the transcript (Fig. 3c,d,f). Accordingly, the translation product is predicted to be truncated upstream of the RhoGAP domain. This allele, termed *Tagap^{tm3Bgh}* (in accordance with standard nomenclature) was bred in *trans* to the partial *t-*haplotype *t⁶*. Litter mates carrying either the wild type allele or *Tagap^{tm3Bgh}* in trans to *t⁶* were tested for transmission ratio distortion (Fig. 3e, Table1). Complementary to the transgenic experiments, the transmission ratio of *t⁶* from *Tagap^{tm3Bgh}l+; t⁶*/*+* males was strongly reduced compared to the ratio obtained from *Tagap1* +/+; *t⁶*/*+* litter mates (69% vs. 84% *t⁶* offspring; Table 1). Statistical analysis demonstrated that the difference is highly significant (p < 0.001). Thus, the loss of function experiment confirmed the results of the gain-of-function experiment, directly demonstrating a role of *Tagap1* in transmission ratio distortion.

Taken together, the genetic and molecular data strongly suggest that the *t-*haplotype loci of *Tagap1* represent *Tcd1.* From the transgenic gain-of-function phenotype one would conclude that these loci act as dominant gain-of-function mutation. Consistent with this conclusion is the fact the *Tagap1* gene is amplified in *t-*haplotypes and that the four loci together express up to fourfold more transcript in testis than wild type strains. The inactivation (in terms of GAP activity) of one of the originally two *Tagap1* genes by a chromosomal rearrangement in the wild type may have had a selective advantage over the progenitor chromosome since it decreased the overall Tagap1 activity in *t*/+ heterozygotes, possibly "defending" the wild type chromosome better against the disadvantageous hyperactivity caused by the *t-*specific *Tagap1* loci.

It has been shown that a large deletion of the wild type chromosome, *T^{22H},* phenocopies *Tcd1,* which led to the suggestion that *Tcd1* represents a hypomorphic or amorphic allele (Lyon 1992). In contrast, we demonstrated that a hypermorphic allele of *Tagap1* phenocopies a *t-complex Distorter* located in the *Tcd1* region. This discrepancy could be reconciled by recent data suggesting the existence of two separate loci, *Tcd1a* and *Tcd1b,* which are both lacking from *T^{22H}* (ref. (Lyon, Schimenti et al. 2000)). Moreover, the distortion of the *t⁶* transmission ratio caused by the gain- and loss-of-function alleles of *Tagap1* is considerably lower than that expected for *Tcd1* encoded by the partial *t-*haplotype *t^{h51}* (8-15% for *Tagap1* vs. >27% for *t^{h51} ;* ref. (Lyon 1984)). Though some of this difference may be accounted for by variation in the genetic background or by a stronger effect of the *Tagap1* loci in the *t-*haplotype, our data support the identification of two Tcd1 loci.

We suggest that the *t-Tagap1* loci, in accordance with their map position on the chromosome, encode *Tcd1a,* and should be named *Tagap1^{Tcd1a}* (according to nomenclature rules). The contribution of each of the four loci and the exact mechanism by which they produce a hypermorph remains to be explored in detail.

In accordance with the finding of two *Tcd1* loci and the data shown here, we predict that the second locus, *Tcd1b*, represents a hypomorphic or amorphic allele of a gene acting upstream of or epistatically to the G protein controlled by *Tagap1.*

### Example 3: Model of the role of Tagap1 in transmission ratio distortion

Whereas the applicant does not wish to be bound by any theory, the following model of the role of Tagap1 is envisaged.

We have previously proposed that the *Tcds* act upstream of *Smok* (Herrmann, Koschorz et al. 1999). According to our model, Smok activity is enhanced through the action of *t-*Distorters in all spermatozoa derived from *t*/+ males resulting in abnormal flagellar function. This negative effect of the *t-*Distorters is counterbalanced by Tcr, which is restricted to cells expressing the gene, thus rescuing *t-*sperm, while +-sperm remain dysfunctional. This would then lead to an advantage of the *t-*sperm in fertilising the eggs.

The findings presented here allow us to refine this model (Fig. 4): Tagap1 down-regulates a member of the Rho subfamily of small G proteins, which acts as negative regulator of Smok kinases. Tagap1^{Tcd1a} enhances down-regulation of this Rho GTPase, which leads to indirect up-regulation of Smok. We predict that the wild type allele of *Tcd1b* encodes either an activator of the Rho protein controlled by Tagap1, or an indirect inhibitor of Smok acting through another factor. If the *t-*haplotype allele *Tcd1b* represents a hypomorphic or amorphic allele, as suggested, this mutation would cause a reduction of the Tcd1b protein level indirectly resulting in up-regulation of Smok. Tcd1b would thus act additively or synergistically with and epistatically to *Tagap1^{Tcd1a}.* Tcd2 would further enhance this effect. Only *Tcr* expressing spermatozoa would be rescued from the motility defect caused by *Tcds*, resulting in preferential fertilisation of the eggs by *t-*sperm. Of course, at this point of analysis, other models are conceivable.

Tagap1 for the first time links Rho signalling to transmission ratio distortion. Previous reports have provided evidence for a role of Rho-GTPases in sperm motility, and the Rho binding protein Rhophilin and its interaction partner Ropporin are found in the flagellum(Hinsch, Habermann et al. 1993); (Nakamura, Fujita et al. 1999); (Fujita, Nakamura et al. 2000). Rhophilin is localised on the outer surface of the outer dense fibers of the sperm tail, directly opposing Ropporin, which is localised at the inner surface of the fibrous sheath (Fujita, Nakamura et al. 2000).

Rho-GTPases are well known for their essential role in cell motility and chemotaxis, which has been extensively studied in human neutrophils, fibroblasts and in the slime mould *Dictyostelium discoideum* (Van Haastert and Devreotes 2004). In these cell types Rho-GTPases control repeated extension of pseudopodia at the leading edge in response to a shallow gradient of signalling molecules, enabling the cell to move towards the stimulus. Whether these analogous functions of Rho-GTPases in cell motility in neutrophils and in sperm motility are part of a common mechanism, despite the fact that the former involves actin and myosin fibers while the latter involves microtubuli remains to be explored.

The identification of a *t-complex-Distorter* provides access to understanding the molecular principles of transmission ratio distortion and promotes the investigation of the role of Rho signalling in sperm motility.

### Example 4: Isolation of a candidate gene for Tcd2

The identification of Tagap1 as a distorter inspired us to search also for candidates of *Tcd2* in the distal *t* haplotype region. We identified several genes encoding signalling molecules in this region, and analysed them with respect to our criteria for *Tcd* candidates outlined in Example 1. One candidate gene fulfilling these criteria was investigated in detail. It encodes Fgd2, a G-nucleotide exchange factor (GEF) for Rho small G proteins (Pasteris et Gorsky, 1999). GEFs promote the active state of small G-proteins by catalyzing the exchange of GDP for GTP (Schmidt and Hall, 2002). According to its map position in the ENSEMBL genome database, *Fgd2* should be located within the fourth inversion of the *t* complex, which contains *Tcd2.* We confirmed that *Fgd2* indeed is located in the *Tcd2* region by mapping it to partial *t* haplotypes, previously used to define the location of *Tcd2* (Fig. 5a, b) (Lyon, 1984). *Fgd2* encodes a protein with a N-terminal Dbl-homology (GEF) domain in tandem with a PH-domain, a FYVE domain and an additional C-terminal PH domain (Fig. 6a). The *t* form of the deduced Fgd2 protein differs from the published wild type sequence in a single amino acid residue. Serine 234 in the GEF domain was replaced in the *t* form by a glycine residue (S234G). *Fgd2* is expressed in a number of organs (Pasteris and Gorsky, 1999). Expression in the testis is already detected at seven days post partum, corresponding to early meiotic stages of spermatogenesis, as revealed by northern- and RT-PCR analyses (Fig. 6b). In situ hybridization analysis of testis sections confirmed this result and furthermore showed that *Fgd2* transcripts can be detected up to the round spermatid stage (Fig. 6c). This expression pattern may facilitate the distribution of Fgd2 products to all sperm cells, a prerequisite for a *Distorter.* Early expression during spermatogenesis was previously also shown for the *Distorter Tagap₁^{Tcd1a}* (see Example 1).

*Fgd2* also expresses a shorter (approximately 2.3 kb) transcript of variable size in wild type strains, from a promoter located within the gene (Fig. 6d). The shorter transcript encodes a N-terminally truncated protein lacking a substantial part of its DH domain (Fig. 6a). Since the GEF domain of this protein most likely is not functional, its role remains obscure. Northern blot analysis of testis RNA derived from wild type and *t⁶lt^{w5}* compound heterozygous mice showed that *t* haplotype mice express much higher levels of the long *Fgd2* RNA than wild type mice (Fig. 6d). This observation was confirmed by qRT-PCR (Fig. 6e). We found that *t⁶*/*t^{w5}* males express up to 6-fold higher levels of Fgd2 RNA in the testis than wild type strains, which show various levels of transcripts. A similar result was recently obtained in the analysis of the *Distorter Tagap1^{Tcd1a},* which was shown to represent a hypermorph. In Example 1 the high levels of *Tagap1^{Tcd1a}* transcripts were shown to be caused at least in part by amplification of the *Tagap1* gene. This mechanism does not hold true for *Fgd2.* Instead, it seems that in *t⁶*/*t^{w5}* testis the level of the large *Fgd2* transcript is highly increased on the expense of the smaller transcript, which is strongly reduced compared to wild type. *Tagap1,* as *Fgd2,* also shows various levels of RNA expression in different wild type strains. These findings are in line with the observation of different penetrance of the TRD phenotype on various wild type strains (Gummere et al., 1986).

### Example 5: Fgd2 is a candidate for Tcd2

The identification of Tagap1 as Distorter of the transmission ratio of t-haplotypes for the first time demonstrated an important role of small GTPases (preferentially of the Rho type) in transmission ratio distortion. This finding led us to suggest that other proteins involved in G protein signalling might also play a role in TRD. Therefore, the genomic region comprising the *t-*haplotype was searched with bioinformatics tools for genes encoding proteins of this group. Several were identified, among them *Fgd2,* encoding for a protein containing a Dbl homology domain and belonging to the GEF (guanine nucleotide exchange factor) family of proteins. *Fgd2* is located in the distal portion of the *t-*haplotype, the In4 region. *Fgd2* cDNA fragments were isolated by RT-PCR from testis of a male carrying the *t-*haplotypes *t⁶*/*t^{w5}.* Sequence analysis demonstrated a number of mutations in the *t-*specific transcript with respect to the wild type transcript suggesting *Fgd2* as candidate for Tcd2. Only one amino acid mutation S234G was consistently found in the t-haplotypes.

On the basis of these data, involvement in G protein signalling, location in the *t-*haplotype region, expression in testis and modification of the coding region in the *t-*allele, Fgd2 was genetically analysed with respect to enhancement or reduction of the transmission ratio of the Tcr carrying t-haplotype *t^{h49}*, which lacks Tcd2. We engineered a targeted (loss-of-function) allele of *Fgd2, Fgd2^{tm4Bgh},* in which the Dbl homology domain was inactivated, in embryonic stem cells as described (Fig. 7, 8a, b). This allele was crossed into animals carrying *t^{h49}*. Males heterozygous for *Fgd2^{tm4Bgh}* and for *t^{h49}* were tested for the transmission ratio of *t^{h49}* to the offspring. Litter mates heterozygous for *t^{h49}* and wild type at the *Fgd2* locus served as control. The breeding results demonstrate a significant reduction of the transmission ratio of t^{h49} from males carrying *Fgd2^{tm4Bgh}* as compared to litter mates of genotype *t^{h49}*/+;+/+. This result as shown in Table 2 (replaced by a more recent table including the data of former table 2 supplemented by additional data; the overall result is not altered) clearly demonstrates that *Fgd2* is involved in transmission ratio distortion and a candidate for Tcd2.

Thus the teachings of Tagap1 led us to the discovery of another Distorter, Fgd2.

### Example 6: Model for Fgd2 in transmission ratio distortion

The identification of *Tcd2* as hypermorphic allele of the GEF encoding gene *Fgd2* allows refining of our model of the molecular basis of transmission ratio distortion (Fig. 9). In Example 2 it was shown that over-expression of Tagap1, a GTPase activating protein and inhibitor of Rho small G proteins, increases the transmission rate of the *t* haplotype, while a loss-of-function allele has the opposite effect. It was shown that Fgd2, a GEF and activator of Rho GTPases acts in the same manner, that is a dosage increase enhances while a reduction of its activity lowers the *t* transmission rate. Thus, both Distorters act in parallel, while having opposing effects on their respective target Rho proteins. From these data we conclude that Fgd2 and Tagap1 must regulate different Rho targets. Therefore there are two signalling cascades exerting opposing effects on Smok1. One pathway, revealed by Tagap1, inhibits, the other, identified by Fgd2, activates Smok1. The hypermorph *Tagap1^{Tcd1a}* reduces inhibition, while *Fgd2^{Tcd2}* enhances activation of Smok1. In this manner both Distorter signalling cascades additively hyper-activate Smok1, followed by impairment of motility parameters in all sperm. Tcr is able to rescue this harmful effect of the Distorters, thus restoring normal flagellar function. Since the effect of Tcr is restricted to *t* sperm, the latter are able to out-compete the impaired wild type sperm in the race for eggs.

### Example 7: Tiam2 is a candidate for Tcd1b

Another candidate for a *t-*Distorter was identified with bioinformatics tools in the Tcd1 subregion of the *t-*haplotype. This region contains another member of the family of Dbl homology domain proteins, *Tiam2.* Thus, this gene also belongs to the GEF family of proteins involved in G protein signalling. Primary characterization of the *Tiam2* transcripts in *t-*haplotypes failed to identifiy a *t-*specific transcript in testis using the sensitive RT-PCR technology, suggesting that the *t*-allele of *Tiam2* is not transcribed in testis. This finding was confirmed by Northern analysis, which failed to identify a transcript of the expected size in RNA derived from the testis of a *t^{h49}*/*t^{h49}*male, whereas a strong band was detected in testis RNA of a wild type control male (Fig. 10). Thus, the *t-*haplotype appears to carry a loss-of-function allele of *Tiam2,* strongly suggesting *Tiam2* as candidate for a second Distorter in the Tcd1 region. According to our model of TRD (see example 3), *Tiam2* is a candidate for Tcd1b.

Based on these results, we decided to functionally analyze Tiam2 with respect to its role in TRD using the same strategies as for Tagap1. For a loss-of function analysis, we engineered a mutant allele of Tiam2 in mouse embryonic stem cells (Fig. 11 b). We constructed a targeting vector, which, after homologous recombination with the wild type locus results in an insertion of the PGKneo selection cassette in exon 3, thereby inactivating the gene (Fig. 11 b). Out of approximately 750 clones analyzed, 2 were shown by southern blot analysis to have undergone the desired homologous recombination event. In *Xba*l digested genomic DNA of these clones, the 5'-probe detects a 16 kb fragment in wild type and a 12.5 kb probe in correctly targeted clones. Southern blot analysis using the 3'-probe detected a 16 kb band derived from the wild type and a 5.6 kb band originating from the mutant allele (Fig. 11c).

The correctly targeted clones were injected into blastocysts and chimerae were obtained. These chimerae are mated with mice heterozygous for the *t-*haplotype *t⁶* (*t⁶*/+). In the next generation, the transmission rate of *t⁶* from male offspring with the genotype *t⁶*/+; +/+ will be compared with the *t⁶* transmission rate of littermates heterozygous for the Tiam2 mutant allele *(t⁶*/*+;* +/-). A statistically significant difference of the transmission ratio between these two groups demonstrates that Tiam2 is a distorter of the transmission ratio.

In addition, as for Tagap1, we also will analyze Tiam2 function by transgenic overexpression of a wild type Tiam2 allele. We have isolated cDNAs from the Tiam2 gene, which are used to clone a transgenic construct. This construct consists of a testis specific ACE promoter (Howard et al., 1993) controlling expression of the full length Tiam2 open reading frame, and the rabbit beta-globin polyadenylation sequence. The transgenic construct will be injected into pronuclei of C57BL/6 derived oocytes to produce transgenic lines, which will be tested for expression of the transgenic construct in testis. Transgenic animals will be crossed to animals heterozygous for *t⁶ (t⁶*/*+).* Male littermates of the genotype *t⁶*/+; +/+ and *t⁶*/+; *Tg(Tiam2)H2Bghl0* will be tested for the transmission rate of *t⁶*. A statistically significant difference of the transmission ratio between these two groups of transgenic and non-transgenic littermates demonstrates that Tiam2 is a distorter of the transmission ratio.

### References

Balch, W. E., Der, C.J., Hall, A. (editors) (1995). "Small GTPases and Their Regulators." Methods in Enzymology 256**.**
Brent, A.E., Schweitzer, R. & Tabin, C.J. (2003) A somitic compartment of tendon progenitors. Cell 113, 235-48.
Bishop, A. L. and A. Hall (2000). "Rho GTPases and their effector proteins." Biochem J 348 Pt 2: 241-55.
Bullard, D. C., C. Ticknor, et al. (1992). "Functional analysis of a t complex responder locus transgene in mice." Mamm Genome 3(10): 579-87.
Cassiday, L. A. and L. J. Maher, 3rd (2003). "Yeast genetic selections to optimize RNA decoys for transcription factor NF-kappa B." Proc Natl Acad Sci U S A 100(7): 3930-5.
Chapman, S. C., A. Lawson, et al. (2005). "Ubiquitous GFP expression in transgenic chickens using a lentiviral vector." Development 132(5): 935-40.
Chien, C. T., P. L. Bartel, et al. (1991). "The two-hybrid system: a method to identify and clone genes for proteins that interact with a protein of interest." Proc Natl Acad Sci U S A 88(21): 9578-82.
Chung, J. H., M. Whiteley, et al. (1993). "A 5' element of the chicken beta-globin domain serves as an insulator in human erythroid cells and protects against position effect in Drosophila." Cell 74(3): 505-14.
Church, G. M. and W. Gilbert (1984). "Genomic sequencing." Proc Natl Acad Sci U S A 81(7): 1991-5.
DePamphilis, e. W. P. M. M. L. (1993). "Guide to techniques in mouse development." Methods in Enzymology, Academic Press NY, 1993. Vol. 225**:**
Der, C. J., Hall, A. (2000). "Regulators and effectors of small GT Pases." Methods in Enzymology. Vol.325**.**
DerMardirossian, C. and G. M. Bokoch (2005). "GDIs: central regulatory molecules in Rho GTPase activation." Trends Cell Biol 15(7): 356-63.
Dinkel, A., W. K. Aicher, et al. (1999). "Efficient generation of transgenic BALB/c mice using BALB/c embryonic stem cells." J Immunol Methods 223(2): 255-60.
Donovan, S., K. M. Shannon, et al. (2002). "GTPase activating proteins: critical regulators of intracellular signaling." Biochim Biophys Acta 1602(1): 23-45.
Dvorsky, R. and M. R. Ahmadian (2004). "Always look on the bright site of Rho: structural implications for a conserved intermolecular interface." EMBO Rep 5(12): 1130-6. Eddy, E. M., Toshimori, K. et al. (2003). Fibrous sheath of mammalian spermatozoa. FEBS Lett 334, 32-6.
Fox, H. S., G. R. Martin, et al. (1985). "Molecular probes define different regions of the mouse t complex." Cell 40(1): 63-9.
Frangioni, J. V. and B. G. Neel (1993). "Solubilization and purification of enzymatically active glutathione S-transferase (pGEX) fusion proteins." Anal Biochem 210(1): 179-87.
Fraser, L. R. and K. Dudley (1999). "New insights into the t-complex and control of sperm function." Bioessays 21(4): 304-12.
Fujita, A., K. Nakamura, et al. (2000). "Ropporin, a sperm-specific binding protein of rhophilin, that is localized in the fibrous sheath of sperm flagella." J Cell Sci 113 (Pt 1): 103-12.
Geijsen, N., M. Horoschak, et al. (2004). "Derivation of embryonic germ cells and male gametes from embryonic stem cells." Nature 427(6970): 148-54.
Gilman, M. (1997). Ribonuclease Protection Assay, John Wiley & Sons, Inc., New York.
Gummere, G. R., P. J. McCormick, et al. (1986). "The influence of genetic background and the homologous chromosome 17 on t-haplotype transmission ratio distortion in mice." Genetics 114(1): 235-45.
Hames, S. J. H. a. B. D. (1985). "Nucleic acid hybridization, a practical approach"." IRL Press, Oxford.
Herrmann, B., M. Bucan, et al. (1986). "Genetic analysis of the proximal portion of the mouse t complex: evidence for a second inversion within t haplotypes." Cell 44(3): 469-76.
Herrmann, B. G., D. P. Barlow, et al. (1987). "A large inverted duplication allows homologous recombination between chromosomes heterozygous for the proximal t complex inversion." Cell 48(5): 813-25.
Herrmann, B. G., B. Koschorz, et al. (1999). "A protein kinase encoded by the t complex responder gene causes non-mendelian inheritance." Nature 402(6758): 141-6.
Hinsch, K. D., B. Habermann, et al. (1993). "ADP-ribosylation of Rho proteins inhibits sperm motility." FEBS Lett 334(1): 32-6.
Horiuch, T., C. Emuta, et al. (2002). "Birth of normal calves after intracytoplasmic sperm injection of bovine oocytes: a methodological approach." Theriogenology 57(3): 1013-24.
Howard, T., R. Balogh, et al. (1993). "Sperm-specific expression of angiotensin-converting enzyme (ACE) is mediated by a 91-base-pair promoter containing a CRE-like element." Mol Cell Biol 13(1): 18-27.
Hubner, K., G. Fuhrmann, et al. (2003). "Derivation of oocytes from mouse embryonic stem cells." Science 300(5623): 1251-6.
Joyner, A. L. E. (1993). "Gene Targeting, A Practical Approach." Oxford University Press. Kim, V. N. (2005). "MicroRNA biogenesis: coordinated cropping and dicing." Nat Rev Mol Cell Biol 6(5): 376-85.
Kleene, K. C. (1989). "Poly(A) shortening accompanies the activation of translation of five mRNAs during spermiogenesis in the mouse." Development 106(2): 367-73.
Kurtz, S. E., K. Esposito, et al. (2003). "Inhibition of an activated Ras protein with genetically selected peptide aptamers." Biotechnol Bioeng 82(1): 38-46.
Lever, A. M., P. M. Strappe, et al. (2004). "Lentiviral vectors." J Biomed Sci 11(4): 439-49.
Lyon, M. F. (1984). "Transmission ratio distortion in mouse t-haplotypes is due to multiple distorter genes acting on a responder locus." Cell 37(2): 621-8.
Lyon, M. F. (1986). "Male sterility of the mouse t-complex is due to homozygosity of the distorter genes." Cell 44(2): 357-63.
Lyon, M. F. (1992). "Deletion of mouse t-complex distorter-1 produces an effect like that of the t-form of the distorter." Genet Res 59(1): 27-33.
Lyon, M. F. (2003). "Transmission ratio distortion in mice." Annu Rev Genet 37: 393-408.
Lyon, M. F., J. C. Schimenti, et al. (2000). "Narrowing the critical regions for mouse t complex transmission ratio distortion factors by use of deletions." Genetics 155(2): 793-801.
Morita, T., K. Murata, et al. (1993). "Mouse t haplotype-specific double insertion of B2 repetitive sequences in the Tcp-1 intron 7." Mamm Genome 4(1): 58-9.
Nadeau, J. H., D. Varnum, et al. (1989). "Genetic evidence for two t complex tail interaction (tct) loci in t haplotypes." Genetics 122(4): 895-903.
Nakamura, K., A. Fujita, et al. (1999). "Rhophilin, a small GTPase Rho-binding protein, is abundantly expressed in the mouse testis and localized in the principal piece of the sperm tail." FEBS Lett 445(1): 9-13.
Pasteris, N. G and Gorsky, J. L. (1999). Isolation, characterization, and mapping of the mouse and human Fgd2 genes, faciogenital dysplasia (FDG1; Aarskog syndrome) gene homologues. Genomics 60, 57-66.
Pirottin, D., L. Grobet, et al. (2005). "Transgenic engineering of male-specific muscular hypertrophy." Proc Natl Acad Sci U S A 102(18): 6413-8.
Planchart, A., Y. You, et al. (2000). "Physical mapping of male fertility and meiotic drive quantitative trait loci in the mouse t complex using chromosome deficiencies." Genetics 155(2): 803-12.
Ramirez-Solis, R., A. C. Davis, et al. (1993). "Gene targeting in embryonic stem cells." Methods Enzymol 225: 855-78.
Rosen, L. L., D. C. Bullard, et al. (1990). "Molecular cloning of the t complex responder genetic locus." Genomics 8(1): 134-40.
Rupp, R. A., L. Snider, et al. (1994). "Xenopus embryos regulate the nuclear localization of XMyoD." Genes Dev 8(11): 1311-23.
Sambrook J., F., E.F., Maniatis, T. (1989). Molecular Cloning, A Laboratory Manual, Cold Spring Harbor Laboratory Press.
Schimenti, J., Reynolds, J. L. & Planchart (2005). Mutations in Serac1 or Synj2 cause proximal t haplotype-mediated male mouse sterility but not transmission ratio distortion. Proc Natl Acad Sci U S A 102, 3342-7.
Schimenti, J. (2000). "Segregation distortion of mouse t haplotypes the molecular basis emerges." Trends Genet 16(6): 240-3.
Schimenti, J., L. Vold, et al. (1987). "An unstable family of large DNA elements in the center of the mouse t complex." J Mol Biol 194(4): 583-94.
Schmidt, A. and A. Hall (2002). "Guanine nucleotide exchange factors for Rho GTPases: turning on the switch." Genes Dev 16(13): 1587-609.
Schmidt, S., S. Diriong, et al. (2002). "Identification of the first Rho-GEF inhibitor, TRIPalpha, which targets the RhoA-specific GEF domain of Trio." FEBS Lett 523(1-3): 35-42.
Self, A. J. and A. Hall (1995). "Measurement of intrinsic nucleotide exchange and GTP hydrolysis rates." Methods Enzymol 256: 67-76.
Self, A. J. and A. Hall (1995). "Purification of recombinant Rho/Rac/G25K from Escherichia coli." Methods Enzymol 256: 3-10.
Silver, L. M. and D. Remis (1987). "Five of the nine genetically defined regions of mouse t haplotypes are involved in transmission ratio distortion." Genet Res 49(1): 51-6.
Van Haastert, P. J. and P. N. Devreotes (2004). "Chemotaxis: signalling the way forward." Nat Rev Mol Cell Biol 5(8): 626-34.
Wennerberg, K. and C. J. Der (2004). "Rho-family GTPases: it's not only Rac and Rho (and I like it)." J Cell Sci 117(Pt 8): 1301-12.
Wilmut, I., A. E. Schnieke, et al. (1997). "Viable offspring derived from fetal and adult mammalian cells." Nature 385(6619): 810-3.
Willison, K. & Ashworth A. Mammalian spermatogenic gene expression. Trends Genet. 3, 351 - 355 (1987).

**Table 1**

| | | | | Offspring | | | |
|---|---|---|---|---|---|---|---|
| Genotype of male | Number of males | *t⁶* | wt | total | % *t⁶* | χ² | P |
| *TgH1-33*/*0; t⁶l+* | 5 | 218 | 39 | 257 | 85 | 7.08 | 0.01 |
| *t⁶*/+ | 5 | 125 | 43 | 168 | 74 | | |
| *TgH1-4*/*0; t⁶*/+. | 7 | 190 | 19 | 209 | 91 | 5.17 | 0.025 |
| *t⁶*/+ | 5 | 200 | 39 | 239 | 84 | | |

| Combined data: | | | | | | | |
|---|---|---|---|---|---|---|---|
| *Tg*/0; *t⁶*/+ | 12 | 408 | 58 | 466 | 88 | 9.57 | 0.01 |
| *t⁶*/+ | 10 | 325 | 82 | 407 | 80 | | |
| *Tagap1^{tm3Bgh}*/*+; t⁶*/+ | 9 | 245 | 109 | 354 | 69 | 21.09 | 0.001 |
| *Tagap1* +/+; *t⁶*/+ | 9 | 292 | 56 | 348 | 84 | | |

### Abbr.: TgH1-33, Tg(Tagap1)H1-33Bgh; TgH1-4, Tg(Tagap1)H1-4Bgh, wt, wild type.

### Table 1 Transmission ratio of t⁶ from males lacking or over-expressing Tagap1.

For overexpression experiments, two independent transgenic lines (TgH1-33 and tgH1-4) were established, the expression of the transgenic construct was verified and both lines were bred to the partial t-haplotype *t⁶*. The transmission ratio *of t⁶* was compared between transgenic and non-transgenic animals. Both lines significantly increase the transmission ratio of *t⁶*.

An inactivated *Tagap1* allele (loss-of-function) produced by gene targeting results in the complementary effect on the transmission ratio of the *t-*haplotype (lower part). The transmission ratio of the t-haplotype by *t⁶*/+ animals is strongly reduced by heterozygozity for *Tagap1^{tm3Bgh}.*

**Table 2:**

| | | | | Offspring | | | |
|---|---|---|---|---|---|---|---|
| Genotype of male | Number of males | t^{h49} | wt | total | % t^{h49} | χ² | P |
| *Fgd2* +/+; *t^{h49}*/+ | 7 | 150 | 169 | 319 | 47 | 8.44 | 0.01 |
| *Fgd2^{tm4Bgh}*/+; *t^{h49}*/+ | 7 | 105 | 191 | 296 | 35 | | |

### Table 2: Transmission ratio of t^{h49} from males lacking Fgd2.

The transmission ratio of the t-haplotype by *t^{h49}* animals is decreased by a loss-of-function allele of Fgd2.

**Table 3:**

| **Overview of the Sequences disclosed in the Sequence listing** | |
|---|---|
| Coding Sequences | Organism/gene/allele, isoform |
| 1 | Tagap1 mouse wildtype FVB/N |
| 2 | Tagap1 mouse wildtype BALB/c |
| 3 | Tagap1 t1 mouse |
| 4 | Tagap1 t2 mouse |
| 5 | Tagap1 t3 mouse |
| 6 | Taga1 t4 mouse |
| 7 | Tagap homo sapiens |
| 8 | Tagap Rattus |
| 9 | Fgd2 mouse transcript variant 3 |
| 10 | Fgd2 mouse transcript variant 1 |
| 11 | Fgd2 mouse transcript variant 2 |
| 12 | Fgd2 mouse transcript variant 1 t⁶/t^{w5} |
| 13 | Tiam2 mouse wildtype |
| 14 | Tiam2 homo sapiens |
| 15 | Tcr, short 5'utr mus musculus |
| 16 | Tcr, long 5'utr mus musculus |
| 31 | Tagap1 Bos taurus |
| 32 | Tagap1 Canis familiaris |
| 33 | Tagap1 Gallus gallus |
| 34 | Fgd2 mouse transcript variant 2 t⁶/t^{W5} |
| 35 | Fgd2 Bos taurus |
| 36 | Fgd2 Canis familiaris |
| 37 | Fgd2 Rattus norvegicus |
| 38 | Fgd2 (splice variant) Rattus norvegicus |
| 47 | Tiam2 Bos taurus |
| 48 | Tiam2 Gallus gallus |
| 49 | Tiam2 Rattus norvegicus |
| 50 | Tiam2 Canis familiaris |
| 55 | Fgd2 mouse transcript variant 1a t⁶/t^{w5} |
| 56 | Tagap1 Danio rerio |
| 57 | Tagap1 Macacca mulatta |
| 58 | Tagap1 Monodelphis domestica |
| 59 | Tagap1 Xenopus tropicalis |
| 60 | Taga1 Pan troglodytes |
| 61 | Fgd2 Macacca mulatta transcript variant 1 |
| 62 | Fgd2 Macacca mulatta transcript variant 2 |
| 63 | Fgd2 Macacca mulatta transcript variant 3 |
| 64 | Fgd2 Monodelphis domestica |
| 65 | Fgd2 Pan troglodytes |
| 66 | Fgd2 Homo sapiens |
| 67 | Tiam2 Macacca mulatta transcript variant 1 |
| 68 | Tiam2 Macacca mulatta transcript variant 2 |
| 69 | Tiam2 Monodelphis domestica transcript variant 1 |
| 70 | Tiam2 Monodelphis domestica transcript variant 2 |
| 71 | Tiam2 Monodelphis domestica transcript variant 3 |
| 72 | Tiam2 Pan troglodytes (fragment)* |

| **Deduced Protein sequences** | |
|---|---|
| 17 | Tagap1 mouse wildtype FVB/N |
| 18 | Tagap1 mouse wildtype BALB/c |
| 19 | Tagap1 t1 mouse |
| 20 | Tagap1 t2 mouse |
| 21 | Tagap1 t3 mouse |
| 22 | Tagap1 t4 mouse |
| 23 | Tagap homo sapiens |
| 24 | Tagap Rattus |
| 25 | FGD2 mouse transcript variant 3 |
| 26 | FGD2 mouse transcript variant 1 |
| 27 | FGD2 mouse transcript variant 2 |
| 28 | FGD2 mouse transcript variant 1 t⁶/t^{w5}) |
| 29 | Tiam2 mouse wildtype |
| 30 | Tiam2 homo sapiens |
| 39 | Tagap1 Bos taurus |
| 40 | Tagap1 Canis familiaris |
| 41 | Tagap1 Gallus gallus |
| 42 | Fgd2 mouse transcript variant 2 t⁶/t^{w5} |
| 43 | Fgd2 Bos taurus |
| 44 | Fgd2 Canis familiaris |
| 45 | Fgd2 Rattus norvegicus |
| 46 | Fgd2 (splice variant) Rattus norvegicus |
| 51 | Tiam2 Bos taurus |
| 52 | Tiam2 Gallus gallus |
| 53 | Tiam 2 Rattus norvegicus |
| 54 | Tiam2 Canis familiaris |
| 73 | Fgd2 mouse transcript variant 1a t⁶/tw⁵ |
| 74 | Tagap1 Danio rerio |
| 75 | Tagap1 Macacca mulatta |
| 76 | Tagap1 Monodelphis domestica |
| 77 | Tagap1 Xenopus tropicalis |
| 78 | Taga1 Pan troglodytes |
| 79 | Fgd2 Macacca mulatta transcript variant 1 |
| 80 | Fgd2 Macacca mulatta transcript variant 2 |
| 81 | Fgd2 Macacca mulatta transcript variant 3 |
| 82 | Fgd2 Monodelphis domestica |
| 83 | Fgd2 Pan troglodytes |
| 84 | Fgd2 Homo sapiens |
| 85 | Tiam2 Macacca mulatta transcript variant 1 |
| 86 | Tiam2 Macacca mulatta transcript variant 2 |
| 87 | Tiam2 Monodelphis domestica transcript variant 1 |
| 88 | Tiam2 Monodelphis domestica transcript variant 2 |
| 89 | Tiam2 Monodelphis domestica transcript variant 3 |
| 90 | Tiam2 Pan troglodytes (fragment)* |

| | |
|---|---|
| *With the methods disclosed in the invention it is not an undue burden for the skilled person to identify and isolate the complete Tiam2 distorter gene. | |

**Table 4 Oligonucleotide primer sequences and PCR conditions**

| **Experiment** | **Primer sequence** | **Number of cycles/ annealing temp.** | **Product size** |
|---|---|---|---|
| **Quantitative RT-PCR, long transcript specific (****Fig 6e****)** | s: 5'- TGAAGCTCATTTTCTCCAACATCT -3' | 40 cycles/ 60 °C | 71 bp |
| | as: 5'- CTGCAGCTCGGGAAGGAA -3' | | |
| | Probe: (5'- FAM, 3'- TAMRA-labeled) 5'- CTCCATCTATCGTTTCCACGCCCAGTT -3' | | |
| **RT-PCR, long transcript specific (****Fig 6b****)** | s: 5'- GGCTGTGGTAGACCTGCTTC -3' | 35 cycles/ 58 °C | 461 bp |
| | as: 5'- AGACGGAGCAGGCCTAT -3' | | |
| **RT-PCR, beta Actin control (****Fig 6b****)** | s: 5'- TGGAATCCTGTGGCATCCATGAAA -3' | 25 cycles/ 58 °C | 349 bp in NM_007393 |
| | as: 5'- TAAAACGCAGCTCAGTAACAGTCCG -3' | | |
| **PCR-amplification of template for in vitro transcription of probe for in situ hybridization (****Fig 6c****)** | s: 5'- AGAATCCCGCGGTACGA -3' | 35 cycles/ 58 °C | 395 bp |
| | as: 5'- GTCAGCTCCCGCACCT -3' | | |
| **PCR amplification of coding region of long Fgd2 transcript** | s: 5'- GGCTAGCAGGATGGAGCGA -3' | 38 cycles/ 66 °C | 2407 bp |
| | as: 5'- GTGCTCTCAGGTTCTTGTGTAG -3' | | |
| **PCR amplification of probe for northern hybridization (****Fig 6d****)** | s: 5'- GGCTTGCGGCTATGTAG -3' | 38 cycles/ 66 °C | 949 bp |
| | as: 5'- GTGCTCTCAGGTTCTTGTGTAG -3' | | |
| **PCR amplification of long transcript specific probe for northern hybridization (****Fig 6b****)** | s: 5'- GGCTAGCAGGATGGAGCGA -3' | 35 cycles/ 57°C | 361 bp |
| | as: 5'- AGACGGAGCAGGCCTAT -3' | | |
| **PCR amplification of left arm of targeting construct *(SpeI* site in forward primer) (****Fig 7****) *** | f: 5'- CTGCTTCTGGGGTAACT -3' | 35 cycles/ 65 °C | 3914 bp |
| | r: 5'- ATAGGCCTGCTCCGTCT -3' | | |
| **PCR amplification of right arm of targeting construct *(EcoRV* site in forward-, *Sal*I site in reverse primer) (****Fig 7****)** | f: 5'- AAGAATCCCGCGGTACGAACTG -3' | 35 cycles/ 65 °C | 2589 bp |
| | r: 5'- GACAACGCCCGACATCATAGAG -3' | | |
| **PCR amplification of 5'-probe for detection of targeted allele (****Fig 7****)** | f: 5'- ACAGGTCTCACGTAGCCGAATC -3' | 35 cycles/ 56 °C | 642 bp |
| | r: 5'- CGGGTGAAGCAGGTCTACCACA -3' | | |
| **PCR amplification of 3'-probe for detection of targeted allele (****Fig 7****)** | f: 5'- TGGATGCCGCTCAGTTGCTAAT -3' | 35 cycles/ 56 °C | 652 bp |
| | r: 5'- TGAAACTCAGTGTGTAGACCAG -3' | | |

| | | | |
|---|---|---|---|
| * A 180 bp deletion at the 3'-end of the left arm occurred during construction of the targeting vector s, sense primer; as, antisense primer, f, forward primer, r, reverse primer | | | |

## Claims

1. A method for producing a transgenic non human, mammalian male animal, wherein the transgene(s) confer(s) a change in the transmission ratio of (a) genetic trait(s) to the offspring of said non human, mammalian male animal to a non-Mendelian ratio, said method comprising introducing
(a) a first nucleic acid molecule encoding an expression product with a Responder function into a chromosome of a non-human mammalian germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s), wherein said first nucleic acid molecule encoding an expression product with Responder function is selected from the group consisting of
(aa) a nucleic acid molecule comprising or consisting of the nucleic acid molecule as shown in SEQ ID NO: 15 or 16 or a fragment thereof, wherein the expression product encoded by the fragment retains at least 70% of the responder function;
(ab) a nucleic acid molecule being an allelic variant or orthologue of the nucleic acid molecule of (aa), wherein the expression product encoded by the allelic variant or orthologue retains at least 70% of the responder function; and
(ac) a nucleic acid molecule which is related to the nucleic acid molecule of (aa) or (ab) by the degeneration of the genetic code; and
(b) at least one second nucleic acid molecule encoding an expression product with a Distorter function into (a) chromosome(s) of a non-human mammalian germ cell, (fertilized) egg cell, embryonic cell or a cell derived of the same species as the transgenic male to be prepared, wherein said expression product with a Distorter function is selected from the group consisting of Tagap1, Tiam2 and Fgd2,
wherein said first nucleic acid molecule encoding an expression product with Responder function and said at least one second nucleic acid molecule encoding an expression product with a Distorter function are introduced into the same or different chromosomes and wherein transcription of the nucleic acid molecules is activated by use of a promoter that activates transcription specifically during spermatogenesis and/or spermiogenesis.

2. A method for producing a transgenic non human, mammalian male animal, wherein the transgene(s) confer(s) a change in the transmission ratio of (a) genetic trait(s) to the offspring of said non human, mammalian male animal to a non-Mendelian ratio, said method comprising introducing
(a) a first nucleic acid molecule encoding an expression product with a Responder function into a chromosome of a non-human, mammalian germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s), wherein said first nucleic acid molecule encoding an expression product with Responder function is selected from the group consisting of
(aa) a nucleic acid molecule comprising or consisting of the nucleic acid molecule as shown in SEQ ID NO: 15 or 16 or a fragment thereof, wherein the expression product encoded by the fragment retains at least 70% of the responder function;
(ab) a nucleic acid molecule being an allelic variant or orthologue of the nucleic acid molecule of (aa), wherein the expression product encoded by the allelic variant or orthologue retains at least 70% of the responder function; and
(ac) a nucleic acid molecule which is related to the nucleic acid molecule of (aa) or (ab) by the degeneration of the genetic code; and
(b) at least one second nucleic acid molecule encoding an expression product directed against the Distorter function into (a) chromosome(s) of a non-human, mammalian germ cell, (fertilized) egg cell, embryonic cell or a cell derived therefrom, of the same species as the transgenic male to be prepared, wherein said Distorter function is an expression product which is encoded by a nucleic acid molecule encoding an expression product with a Distorter function, wherein said expression product with a Distorter function is selected from the group consisting of Tagap1, Tiam2 and Fgd2, wherein said expression product directed against the Distorter function is an aptamer, a siRNA or shRNA or miRNA, a ribozyme, or an antisense nucleic acid molecule specifically hybridizing under stringent conditions to said nucleic acid molecules encoding a Distorter selected from the group consisting of Tagap1, Tiam2 and Fgd2; and/or
(c) a second nucleic acid molecule for inactivation of the Distorter function by homologous recombination, wherein said second nucleic acid molecule for inactivation of the Distorter function by homologous recombination is at least partially identical to said nucleic acid molecule encoding an expression product with a Distorter function and wherein said expression product with a Distorter function is selected from the group consisting of Tagap1, Tiam2 and Fgd2,
wherein said first nucleic acid molecule encoding an expression product with a Responder
function and said at least one second nucleic acid molecule encoding an expression product directed against the Distorter function and/or said second nucleic acid molecule for inactivation of the Distorter function by homologous recombination are introduced into the same or different chromosomes, and wherein transcription of the nucleic acid molecules is activated by use of a promoter that activates transcription specifically during spermatogenesis and/or spermiogenesis; thereby inactivating the Distorter function completely or to at least 50%.

3. The method of claim 1 or 2, wherein said mammal is selected from the group consisting of Mus, Rattus, Bos, Sus and Ovis.

4. The method of any one of claims 1 to 3, wherein said genetic trait is sex.

5. The method of any one of claims 1 to 4, wherein said chromosome is an X or Y chromosome.

6. The method of any one of claims 1 to 4, wherein said chromosome is an autosome.

7. The method of any one of claims 1 to 6, wherein said (at least one) second nucleic acid molecule encoding an expression product with a Distorter function is/are selected from the group consisting of
(a) a nucleic acid molecule comprising or consisting of the nucleic acid molecule as shown in any one of SEQ ID NOs:1 to 14, 31 to 38, 47 to 50 or 55 to 72 or a fragment thereof, wherein the fragment retains at least 70% of the distorter function;
(b) a nucleic acid molecule being an allelic variant or orthologue of the nucleic acid molecules of (a), wherein the allelic variant or orthologue retains at least 70% of the distorter function;
and
(c) a nucleic acid molecule which is related to the nucleic acid molecule of (a) or (b) by the degeneration of the genetic code.

8. The method of any one of claims 1 and 3 to 6, wherein said at least one second nucleic acid molecule encoding an expression product with a Distorter function is/are selected from the group consisting of
(a) a nucleic acid molecule comprising or consisting of the nucleic acid molecule as shown in any one of SEQ ID NOs: 1 to 12, 31 to 38 or 55 to 66 or a fragment thereof, wherein the fragment retains at least 70% of the distorter function;
(b) a nucleic acid molecule being an allelic variant or orthologue of the nucleic acid molecule of (a), wherein the allelic variant or orthologue retains at least 70% of the distorter function;
and
(c) a nucleic acid molecule which is related to the nucleic acid molecule of (a) or (b) by the degeneration of the genetic code,
thereby enhancing said transmission ratio of said genetic trait(s).

9. The method of any one of claims 1 and 3 to 6, wherein said at least one second nucleic acid molecule encoding an expression product with a Distorter function is/are selected from the group consisting of
(a) a nucleic acid molecule comprising or consisting of the nucleic acid molecule as shown in SEQ ID NO: 13 or 14, 47 to 50 or 67 to 72 or a fragment thereof, wherein the fragment retains at least 70% of the distorter function;
(b) a nucleic acid molecule being an allelic variant or orthologue of the nucleic acid molecule of (a), wherein the allelic variant or orthologue retains at least 70% of the distorter function;
and
(c) a nucleic acid molecule which is related to the nucleic acid molecule of (a) or (b) by the degeneration of the genetic code,
thereby reducing said transmission ratio of said genetic trait(s).

10. The method of any one of claims 2 to 6, wherein said nucleic acid molecule encoding an expression product with a Distorter function is selected from the group consisting of
(a) a nucleic acid molecule comprising or consisting of the nucleic acid molecule as shown in any one of SEQ ID NOs 1 to 12, 31 to 38 or 55 to 66 or a fragment thereof, wherein the fragment retains at least 70% of the distorter function;
(b) a nucleic acid molecule being an allelic variant or orthologue of the nucleic acid molecule of (a), wherein the allelic variant or orthologue retains at least 70% of the distorter function;
and
(c) a nucleic acid molecule which is related to the nucleic acid molecule of (a) or (b) by the degeneration of the genetic code,
thereby reducing said transmission ratio of said genetic trait(s).

11. The method of any one of claims 2 to 6, wherein said nucleic acid molecule encoding an expression product with a Distorter function is selected from the group consisting of
(a) a nucleic acid molecule comprising or consisting of the nucleic acid molecule as shown in SEQ ID NO 13 or 14, 47 to 50 or 67 to 72 or a fragment thereof, wherein the fragment retains at least 70% of the distorter function;
(b) a nucleic acid molecule being an allelic variant or orthologue of the nucleic acid molecule of (a), wherein the allelic variant or orthologue retains at least 70% of the distorter function;
and
(c) a nucleic acid molecule which is related to the nucleic acid molecule of (a) or (b) by the degeneration of the genetic code,
thereby enhancing said transmission ratio of said genetic trait(s).

12. The method of any one of claims 1 to 11, further comprising crossing the transgenic non human male mammal obtained by the method of anyone of claims 1 to 11 with a non human female mammal and analyzing the offspring of said cross for transmission of said genetic trait(s).

13. The method of any one of claims 1 to 12, wherein said at least one second nucleic acid molecule encoding the expression product with a Distorter function is modified, thereby further reducing or further enhancing the Distorter function activity.

14. The method of any one of claims 5 to 13, wherein said first nucleic acid molecule encoding an expression product with a Responder function and said at least one second nucleic acid molecule encoding an expression product with Distorter function and/or said at least one second nucleic acid molecule encoding an expression product directed against the Distorter function and/or said second nucleic acid molecule for inactivation of the Distorter function by homologous recombination and a promoter controlling expression in spermatogenesis and/or spermiogenesis and/or a stop cassette are integrated in said X or Y chromosome or in one of said autosomes in a reversible inactive state of expressibility.

15. A non human male or female mammal, wherein said non human male or female mammal is transgenic for the nucleic acid molecule encoding an expression product with a Responder function and the nucleic acid molecule encoding an expression product with a Distorter function and/or the nucleic acid molecule encoding an expression product directed against the Distorter function and/or the nucleic acid molecule for inactivation of the Distorter function by homologous recombination as said nucleic acid molecules being as defined in any one of claims 1 to 14 and wherein said mammal is selected from the group consisting of Rattus, Bos, Sus and Ovis.

16. A non human male or female mammal, wherein said non human male or female mammal is transgenic for the nucleic acid molecule encoding an expression product with a Responder function and the nucleic acid molecule encoding an expression product with a Distorter function and/or the nucleic acid molecule encoding an expression product directed against the Distorter function and/or the nucleic acid molecule for inactivation of the Distorter function by homologous recombination as said nucleic acid molecules being as defined in any one of claims 1 to 5 and 7 to 14, wherein said mammal is Mus and wherein said nucleic acid molecules are in an X or Y chromosome.

17. The non human male or female mammal of claim 16, wherein said first nucleic acid molecule encoding an expression product with Responder function and said at least one second nucleic acid molecule encoding an expression product with a Distorter function and/or said at least one second nucleic acid molecule encoding an expression product directed against the Distorter function and/or said second nucleic acid molecule for inactivation of the Distorter function by homologous recombination are introduced into the same chromosome.

18. A pair of non human male and female mammalian animals, wherein at least one of the male and/or female is a transgenic non human mammal as defined in claim 15 to 17.

19. The pair of non-human male and female mammalian animals of claim 18, wherein the nucleic acid molecule or part thereof encoding an expression product with a Responder function and/or the nucleic acid molecule or part thereof encoding an expression product with a Distorter function and/or the nucleic acid molecule or part thereof encoding an expression product directed against the Distorter function and/or the nucleic acid molecule or part thereof for inactivation of the Distorter function by homologous recombination all said nucleic acid molecules being as defined in any one of claims 1 to 14, is/are flanked by recombinase recognition sites.

20. The pair of non-human male and female mammalian animals of claim 18 or 19 having further stably integrated into its genomic DNA a nucleic acid molecule encoding a site specific DNA recombinase.

21. The pair of non human male and female mammalian animals of claim 20, wherein said DNA recombinase is Cre, wherein said recognition sites are loxP sites, or flp, wherein said recognition sites are FRT sites, or Φc31, wherein said recognition sites are att sites.

22. The pair of non human male and female mammalian animals of claim 20 or 21, wherein said DNA recombinase is controlled by regulatory elements that are active prior to spermiogenesis.

23. Sperm obtainable from a male of the transgenic non-human, mammalian animal as defined in claims 15, 17 or claims 18-22 when referring back to claims 15 or 17, wherein the sperm comprises the nucleic acid molecule encoding an expression product with a Responder function and the nucleic acid molecule encoding an expression product with a Distorter function and/or the nucleic acid molecule encoding an expression product directed against the Distorter function and/or the nucleic acid molecule for inactivation of the Distorter function by homologous recombination all said nucleic acid molecules being as defined in any one of claims 1 to 14.

24. Use of the sperm of claim 23 for the production of offspring.

25. Use of the nucleic acid molecule encoding an expression product with a Distorter function as defined in any one of claims 1 to 14, for the identification of chemicals or biological compounds able to trigger the (premature) activation or inhibition of the Responder/Distorter signalling cascade, wherein the effectiveness of these compounds is tested via their effect on sperm motility in vitro.

26. Use of the nucleic acid molecule encoding an expression product with a Distorter function as defined in any one of claims 1 to 14 for the isolation of receptor molecules of the Responder/Distorter signaling cascade and/or other members of the Responder/Distorter signaling cascade to which said expression product may bind.

27. A method for the detection of the nucleic acid molecule encoding an expression product with a Distorter function and/or the nucleic acid molecule encoding an expression product directed against the Distorter function and/or the nucleic acid molecule for inactivation of the Distorter function by homologous recombination All Said Nucleic Acid Molecules Being as defined in any one of claims 1 to 14, in the non human male or female mammal as defined in claim 15 to 17, comprising identifying said nucleic acid molecule in said non human male or female mammal by polymerase chain reaction (PCR), gene (micro)array hybridization, single nucleotide polymorphism (SNP) analysis, and/or sequencing with primers hybridizing to said nucleic acid molecule.

28. The method of any one of claims 1 to 14 or 27, or the non human male or female mammal of claim 15 to 17, or the pair of non human male and female mammalian animals of any one of claims 18 to 22, or the sperm of claim 23, or the use according to claim 25 or 26, wherein the nucleic acid molecule encoding an expression product with a Distorter function, wherein said expression product with a Distorter function is a factor involved in G protein signaling, is selected from the group consisting of:
(a) a nucleic acid molecule comprising or consisting of the nucleic acid molecule of any one of SEQ ID NOs: 3 to 6 and 12 or a fragment thereof, wherein the expression product encoded by the fragment retains At least 70% of the distorter function;
(b) a nucleic acid molecule being an allelic variant or an orthologue of the nucleic acid molecule of (a), wherein the expression product encoded by the allelic variant or orthologue retains At least 70% of the distorter function;
(c) a nucleic acid molecule which hybridizes under stringent conditions to the nucleic acid molecule of (a) Referring to SEQ ID Nos 3 to 6, wherein said nucleic acid molecule encodes a polypeptide which has
(i) at the position corresponding to position 49 of SEQ ID NO: 17 an I
(ii) at the position corresponding to position 144 of SEQ ID NO: 17 an L
(iii) at the position corresponding to position 323 of SEQ ID NO: 17 a T and
(iv) which terminates after position 442;
(d) a nucleic acid molecule which hybridizes under stringent conditions to the nucleic acid molecule of (a) Referring to SEQ ID Nos 3 to 6, wherein said nucleic acid molecule encodes a polypeptide which has
(i) at the position corresponding to position 49 of SEQ ID NO: 17 an I;
(ii) at the position corresponding to position 137 of SEQ ID NO: 17 an E;
(iii) at the position corresponding to position 207 of SEQ ID NO: 17 an F;
(iv) at the position corresponding to position 301 of SEQ ID NO: 17 an M;
(v) at the position corresponding to position 323 of SEQ ID NO: 17 an T;
(vi) at the position corresponding to position 332 of SEQ ID NO: 17 a D;
(vii) at the position corresponding to position 407-413 of SEQ ID NO: 17 an internal deletion;
(viii) at the position corresponding to position 440 of SEQ ID NO: 17 an M;
(ix) at the position corresponding to position 471 of SEQ ID NO: 17 an L;
(x) at the position corresponding to position 552 of SEQ ID NO: 17 an I;
(xi) at the position corresponding to position 596 of SEQ ID NO: 17 a K;
(xii) at the position corresponding to position 607 of SEQ ID NO: 17 an R;
(xiii) at the position corresponding to position 610 of SEQ ID NO: 17 an S; and
(xiv) at the position corresponding to position 703 of SEQ ID NO: 17 a V;
(e) a nucleic acid molecule which hybridizes under stringent conditions to the nucleic acid molecule of (a) Referring to SEQ ID Nos 3 to 6, wherein said nucleic acid molecule encodes a polypeptide which has
(i) at the position corresponding to position 49 of SEQ ID NO: 17 an I;
(ii) at the position corresponding to position 54 of SEQ ID NO: 17 a G;
(iii) at the position corresponding to position 137 of SEQ ID NO: 17 an E;
(iv) at the position corresponding to position 173 of SEQ ID NO: 17 a G;
(v) at the position corresponding to position 207 of SEQ ID NO: 17 an F;
(vi) at the position corresponding to position 301 of SEQ ID NO: 17 an M;
(vii) at the position corresponding to position 323 of SEQ ID NO: 17 a T;
(viii) at the position corresponding to position 332 of SEQ ID NO: 17 a D;
(ix) at the position corresponding to position 407-413 of SEQ ID NO: 17 an internal deletion;
(x) at the position corresponding to position 440 of SEQ ID NO: 17 an M;
(xi) at the position corresponding to position 471 of SEQ ID NO: 17 an L;
(xii) at the position corresponding to position 508 of SEQ ID NO: 17 an S;
(xiii) at the position corresponding to position 552 of SEQ ID NO: 17 an I;
(xiv) at the position corresponding to position 596 of SEQ ID NO: 17 a K;
(xv) at the position corresponding to position 607 of SEQ ID NO: 17 an R;
(xvi) at the position corresponding to position 610 of SEQ ID NO: 17 an S; and
(xvii) at the position corresponding to position 703 of SEQ ID NO: 17 a V; and
(f) a nucleic acid molecule which hybridizes under stringent conditions to the nucleic acid molecule of (a) Referring to SEQ ID Nos 3 to 6, wherein said nucleic acid molecule encodes a polypeptide which has
(i) at the position corresponding to position 49 of SEQ ID NO: 17 an I;
(ii) at the position corresponding to position 137 of SEQ ID NO: 17 an E;
(iii) at the position corresponding to position 207 of SEQ ID NO: 17 an F;
(iv) at the position corresponding to position 301 of SEQ ID NO: 17 an M;
(v) at the position corresponding to position 323 of SEQ ID NO: 17 a T;
(vi) at the position corresponding to position 332 of SEQ ID NO: 17 a D;
(vii) at the position corresponding to position 407-413 of SEQ ID NO: 17 an internal deletion;
(viii) at the position corresponding to position 440 of SEQ ID NO: 17 an M;
(ix) at the position corresponding to position 471 of SEQ ID NO: 17 an L;
(x) at the position corresponding to position 530 of SEQ ID NO: 17 an E;
(xi) at the position corresponding to position 552 of SEQ ID NO: 17 an I;
(xii) at the position corresponding to position 573 of SEQ ID NO: 17 an R;
(xiii) at the position corresponding to position 596 of SEQ ID NO: 17 a K;
(xiv) at the position corresponding to position 607 of SEQ ID NO: 17 an R;
(xv) at the position corresponding to position 610 of SEQ ID NO: 17 an S; and
(xvi) at the position corresponding to position 703 of SEQ ID NO: 17 a V

29. The method, or the non human male or female mammal, or the pair of non human male and female mammalian animals, or the sperm, or the use according to claim 28, wherein the nucleic acid molecule is a DNA molecule.

30. The method, or the non human male or female mammal, or the pair of non human male and female mammalian animals, or the sperm, or the use according to claim 28 or 29, wherein said expression product is an RNA or a (poly)peptide.

31. The method, or the non human male or female mammal, or the pair of non human male and female mammalian animals, or the sperm, or the use according to claim 28 to 30, wherein the heterologous promoter is the testis promoter of c-kit, ACE, Tcr or Smok.

32. A method for the identification of an expression product of a nucleic acid molecule encoding a Distorter, comprising the steps of
(a) isolating an expression product of a nucleic acid molecule encoding a candidate Distorter by means of protein-protein interaction with a known Distorter derived from the mouse t-complex, wherein said known Distorter is selected from the group consisting of Tagap1, Tiam2 and Fgd2; and
(b) testing the nucleic acid molecule encoding said expression product isolated in (a) for change of the transmission ratio of the Responder as defined in claim 1 or of a genetic trait linked to said Responder in an experimental non human animal, wherein when said transmission ratio is enhanced or reduced, said expression product isolated in (a) is an expression product with Distorter function.

33. The method of claim 32, wherein in step (b) hypomorphic or hypermorphic alleles of said nucleic acid molecule are used for testing for change of the transmission ratio.

## Patentansprüche

1. Verfahren zur Herstellung eines transgenen, nicht-menschlichen, männlichen Säugetiers,
wobei das/die Transgen(e) eine Veränderung in der Vererbungsrate von (einem) genetischen Merkmal(en) auf die Nachkommen des nicht-menschlichen, männlichen Säugetiers zu einem nicht-Mendelschen Verhältnis bewirkt/bewirken, wobei das Verfahren das Einbringen umfasst von
(a) einem ersten Nukleinsäuremolekül, das ein Expressionsprodukt mit einer Responder-Funktion kodiert, in ein Chromosom einer nicht-menschlichen Säugetier-Keimzelle, -(befruchteten) Eizelle, -Embryonalzelle oder einer davon abgeleiteten Zelle, der selben Art wie das herzustellende transgene männliche Tier, wobei das Chromosom das/die genetische(n) Merkmal(e) enthält oder diese vermittelt, wodurch auf dem Chromosom die Responder-Funktion mit dem/den genetischen Merkmal(en) verknüpft wird, wobei das erste Nukleinsäuremolekül, das ein Expressionsprodukt mit Responder-Funktion kodiert, ausgewählt ist aus der Gruppe bestehend aus
(aa) einem Nukleinsäuremolekül umfassend oder bestehend aus dem Nukleinsäuremolekül wie in SEQ ID NO:15 oder 16 dargestellt, oder einem Fragment davon, wobei das Expressionsprodukt, das von dem Fragment kodiert wird, mindestens 70% der Responder-Funktion behält;
(ab) einem Nukleinsäuremolekül, das eine allelische Variante oder ein Ortholog des Nukleinsäuremoleküls nach (aa) ist, wobei das Expressionsprodukt, das von der allelischen Variante oder dem Ortholog kodiert wird, mindestens 70% der Responder-Funktion behält; und
(ac) einem Nukleinsäuremolekül, das durch die Degeneration des genetischen Codes mit dem Nukleinsäuremolekül nach (aa) oder (ab) verwandt ist; und
(b) mindestens einem zweiten Nukleinsäuremolekül, das ein Expressionsprodukt mit einer Distorter-Funktion kodiert, in (ein) Chromosom(en) einer nicht-menschlichen Säugetier-Keimzelle, -(befruchteten) Eizelle, -Embryonalzelle oder einer davon abgeleiteten Zelle der selben Art wie das herzustellende transgene männliche Tier, wobei das Expressionsprodukt mit einer Distorter-Funktion ausgewählt ist aus der Gruppe bestehend aus Tagap1, Tiam2 und Fgd2,
wobei das erste Nukleinsäuremolekül, das ein Expressionsprodukt mit Responder-Funktion kodiert und das mindestens eine zweite Nukleinsäuremolekül, das ein Expressionsprodukt mit einer Distorter-Funktion kodiert, in das selbe oder verschiedene Chromosom(en) eingebracht werden und wobei die Transkription der Nukleinsäuremoleküle durch die Verwendung eines Promoters aktiviert wird, der die Transkription spezifisch während der Spermatogenese und/oder der Spermiogenese aktiviert.

2. Verfahren zur Herstellung eines transgenen, nicht-menschlichen, männlichen Säugetiers,
wobei das/die Transgen(e) eine Veränderung in der Vererbungsrate von (einem) genetischen Merkmal(en) auf die Nachkommen des nicht-menschlichen, männlichen Säugetiers zu einem nicht-Mendelschen Verhältnis bewirkt/bewirken, wobei das Verfahren das Einbringen umfasst von
(a) einem ersten Nukleinsäuremolekül, das ein Expressionsprodukt mit einer Responder-Funktion kodiert, in ein Chromosom einer nicht-menschlichen Säugetier-Keimzelle, -(befruchteten) Eizelle, -Embryonalzelle oder einer davon abgeleiteten Zelle, der selben Art wie das herzustellende transgene männliche Tier, wobei das Chromosom das/die genetische(n) Merkmal(e) enthält oder diese vermittelt, wodurch auf dem Chromosom die Responder-Funktion mit dem/den genetischen Merkmal(en) verknüpft wird, wobei das erste Nukleinsäuremolekül, das ein Expressionsprodukt mit Responder-Funktion kodiert, ausgewählt ist aus der Gruppe bestehend aus
(aa) einem Nukleinsäuremolekül umfassend oder bestehend aus dem Nukleinsäuremolekül wie in SEQ ID NO:15 oder 16 dargestellt, oder einem Fragment davon, wobei das Expressionsprodukt, das von dem Fragment kodiert wird, mindestens 70% der Responder-Funktion behält;
(ab) einem Nukleinsäuremolekül, das eine allelische Variante oder ein Ortholog des Nukleinsäuremoleküls nach (aa) ist, wobei das Expressionsprodukt, das von der allelische Variante oder dem Ortholog kodiert wird, mindestens 70% der Responder-Funktion behält; und
(ac) einem Nukleinsäuremolekül, das durch die Degeneration des genetischen Codes mit dem Nukleinsäuremolekül nach (aa) oder (ab) verwandt ist; und
(b) mindestens einem zweiten Nukleinsäuremolekül, das ein Expressionsprodukt kodiert, das gegen die Distorter-Funktion gerichtet ist, in (ein) Chromosom(en) einer nicht-menschlichen Säugetier-Keimzelle, -(befruchteten) Eizelle, -Embryonalzelle oder einer davon abgeleiteten Zelle, der selben Art wie das herzustellende transgene männliche Tier, wobei die Distorter-Funktion ein Expressionsprodukt ist, das durch ein Nukleinsäuremolekül kodiert ist, das ein Expressionsprodukt mit einer Distorter-Funktion kodiert, wobei das Expressionsprodukt mit einer Distorter-Funktion ausgewählt ist aus der Gruppe bestehend aus Tagap1, Tiam2 und Fgd2, wobei das Expressionsprodukt, das gegen die Distorter-Funktion gerichtet ist, ein Aptamer, eine siRNA oder shRNA oder miRNA, ein Ribozym, oder ein antisense Nukleinsäuremolekül ist, das/die spezifisch unter stringenten Bedingungen an die Nukleinsäuremoleküle hybridisieren, die einen Distorter ausgewählt aus der Gruppe bestehend aus Tagap1, Tiam2 und Fgd2 kodieren; und/oder
(c) einem zweiten Nukleinsäuremolekül für die Inaktivierung der Distorter-Funktion durch homologe Rekombination, wobei das zweite Nukleinsäuremolekül für die Inaktivierung der Distorter-Funktion durch homologe Rekombination mindestens teilweise identisch zu dem Nukleinsäuremolekül ist, das ein Expressionsprodukt mit Distorter-Funktion kodiert und wobei das Expressionsprodukt mit einer Distorter-Funktion ausgewählt ist aus der Gruppe bestehend aus Tagap1, Tiam2 und Fgd2,
wobei das erste Nukleinsäuremolekül, das ein Expressionsprodukt mit einer Responder-Funktion kodiert und das mindestens eine zweite Nukleinsäuremolekül, das ein Expressionsprodukt kodiert, das gegen die Distorter-Funktion gerichtet ist, und/oder das zweite Nukleinsäuremolekül für die Inaktivierung der Distorter-Funktion durch homologe Rekombination, in das selbe oder verschiedene Chromosom(en) eingebracht werden und wobei die Transkription der Nukleinsäuremoleküle durch die Verwendung eines Promoters aktiviert wird, der die Transkription spezifisch während der Spermatogenese und/oder der Spermiogenese aktiviert; wodurch die Distorter-Funktion vollständig oder zu mindestens 50% inaktiviert wird.

3. Verfahren nach Anspruch 1 oder 2, wobei das Säugetier ausgewählt ist aus der Gruppe bestehend aus Mus, Rattus, Bos, Sus und Ovis.

4. Verfahren nach einem der Ansprüche 1 bis 3, wobei das genetische Merkmal das Geschlecht ist.

5. Verfahren nach einem der Ansprüche 1 bis 4, wobei das Chromosom ein X oder Y Chromosom ist.

6. Verfahren nach einem der Ansprüche 1 bis 4, wobei das Chromosom ein Autosom ist.

7. Verfahren nach einem der Ansprüche 1 bis 6, wobei das (mindestens eine) zweite Nukleinsäuremolekül, das ein Expressionsprodukt mit einer Distorter-Funktion kodiert, ausgewählt ist aus der Gruppe bestehend aus
(a) einem Nukleinsäuremolekül umfassend oder bestehend aus dem Nukleinsäuremolekül wie in einer der SEQ ID NOs: 1 bis 14, 31 bis 38, 47 bis 50 oder 55 bis 72 dargestellt, oder einem Fragment davon, wobei das Fragment mindestens 70% der Distorter-Funktion behält;
(b) einem Nukleinsäuremolekül, das eine allelische Variante oder ein Ortholog des Nukleinsäuremoleküls nach (a) ist, wobei die allelische Variante oder das Ortholog mindestens 70% der Distorter-Funktion behält;
und
(c) einem Nukleinsäuremolekül, das durch die Degeneration des genetischen Codes mit dem Nukleinsäuremolekül nach (a) oder (b) verwandt ist.

8. Verfahren nach einem der Ansprüche 1 und 3 bis 6, wobei das mindestens eine zweite Nukleinsäuremolekül, das ein Expressionsprodukt mit einer Distorter-Funktion kodiert, ausgewählt ist/sind aus der Gruppe bestehend aus
(a) einem Nukleinsäuremolekül umfassend oder bestehend aus dem Nukleinsäuremolekül wie in einer der SEQ ID NOs: 1 bis 12, 31 bis 38 oder 55 bis 66 dargestellt, oder einem Fragment davon, wobei das Fragment mindestens 70% der Distorter-Funktion behält;
(b) einem Nukleinsäuremolekül, das eine allelische Variante oder ein Ortholog des Nukleinsäuremoleküls nach (a) ist, wobei die allelische Variante oder das Ortholog mindestens 70% der Distorter-Funktion behält;
und
(c) einem Nukleinsäuremolekül, das durch die Degeneration des genetischen Codes mit dem Nukleinsäuremolekül nach (a) oder (b) verwandt ist,
wodurch die Vererbungsrate des/der genetischen Merkmale(s) erhöht wird.

9. Verfahren nach einem der Ansprüche 1 und 3 bis 6, wobei das mindestens eine zweite Nukleinsäuremolekül, das ein Expressionsprodukt mit einer Distorter-Funktion kodiert, ausgewählt ist/sind aus der Gruppe bestehend aus
(a) einem Nukleinsäuremolekül umfassend oder bestehend aus dem Nukleinsäuremolekül wie in einer der SEQ ID NOs: 13 oder 14, 47 bis 50 oder 67 bis 72 dargestellt, oder einem Fragment davon, wobei das Fragment mindestens 70% der Distorter-Funktion behält;
(b) einem Nukleinsäuremolekül, das eine allelische Variante oder ein Ortholog des Nukleinsäuremoleküls nach (a) ist, wobei die allelische Variante oder das Ortholog mindestens 70% der Distorter-Funktion behält;
und
(c) einem Nukleinsäuremolekül, das durch die Degeneration des genetischen Codes mit dem Nukleinsäuremolekül nach (a) oder (b) verwandt ist,
wodurch die Vererbungsrate des/der genetischen Merkmale(s) vermindert wird.

10. Verfahren nach einem der Ansprüche 2 bis 6, wobei das Nukleinsäuremolekül, das ein Expressionsprodukt mit einer Distorter-Funktion kodiert, ausgewählt ist aus der Gruppe bestehend aus
(a) einem Nukleinsäuremolekül umfassend oder bestehend aus dem Nukleinsäuremolekül wie in einer der SEQ ID NOs: 1 bis 12, 31 bis 38 oder 55 bis 66 dargestellt, oder einem Fragment davon, wobei das Fragment mindestens 70% der Distorter-Funktion behält;
(b) einem Nukleinsäuremolekül, das eine allelische Variante oder ein Ortholog des Nukleinsäuremoleküls nach (a) ist, wobei die allelische Variante oder das Ortholog mindestens 70% der Distorter-Funktion behält;
und
(c) einem Nukleinsäuremolekül, das durch die Degeneration des genetischen Codes mit dem Nukleinsäuremolekül nach (a) oder (b) verwandt ist,
wodurch die Vererbungsrate des/der genetischen Merkmale(s) vermindert wird.

11. Verfahren nach einem der Ansprüche 2 bis 6, wobei das Nukleinsäuremolekül, das ein Expressionsprodukt mit einer Distorter-Funktion kodiert, ausgewählt ist aus der Gruppe bestehend aus
(a) einem Nukleinsäuremolekül umfassend oder bestehend aus dem Nukleinsäuremolekül wie in einer der SEQ ID NOs: 13 oder 14, 47 bis 50 oder 67 bis 72 dargestellt, oder einem Fragment davon, wobei das Fragment mindestens 70% der Distorter-Funktion behält;
(b) einem Nukleinsäuremolekül, das eine allelische Variante oder ein Ortholog des Nukleinsäuremoleküls nach (a) ist, wobei die allelische Variante oder das Ortholog mindestens 70% der Distorter-Funktion behält;
und
(c) einem Nukleinsäuremolekül, das durch die Degeneration des genetischen Codes mit dem Nukleinsäuremolekül nach (a) oder (b) verwandt ist,
wodurch die Vererbungsrate des/der genetischen Merkmale(s) erhöht wird.

12. Verfahren nach einem der Ansprüche 1 bis 11, des weiteren umfassend das Kreuzen des transgenen, nicht-menschlichen männlichen Säugetiers, das mittels der Methode nach einem der Ansprüche 1 bis 11 erhalten wurde, mit einem nicht-menschlichen, weiblichen Säugetier und Untersuchen des Nachwuchs dieser Kreuzung auf die Vererbung des/der genetischen Merkmale(s).

13. Verfahren nach einem der Ansprüche 1 bis 12, wobei das mindestens eine zweite Nukleinsäuremolekül, das ein Expressionsprodukt mit einer Distorter-Funktion kodiert, modifiziert ist, wodurch die Aktivität der Distorter-Funktion weiter vermindert oder erhöht wird.

14. Verfahren nach einem der Ansprüche 5 bis 13, wobei das erste Nukleinsäuremolekül, das ein Expressionsprodukt mit einer Responder-Funktion kodiert und das mindestens eine zweite Nukleinsäuremolekül, das ein Expressionsprodukt mit Distorter-Funktion kodiert, und/oder das mindestens eine zweite Nukleinsäuremolekül, das ein Expressionsprodukt kodiert, das gegen die Distorter-Funktion gerichtet ist, und/oder das zweite Nukleinsäuremolekül für die Inaktivierung der Distorter-Funktion durch homologe Rekombination und ein Promoter, der die Expression in der Spermatogenese und/oder der Spermiogenese aktiviert und/oder eine Stopp-Kassette in einem reversibel inaktiven Zustand der Expressionsfähigkeit in das X oder Y Chromosom oder in eines der Autosomen eingefügt wird/werden.

15. Nicht-menschliches männliches oder weibliches Säugetier, wobei das nicht-menschliche männliche oder weibliche Säugetier transgen ist für das Nukleinsäuremolekül, das ein Expressionsprodukt mit einer Responder-Funktion kodiert und das Nukleinsäuremolekül, das ein Expressionsprodukt mit einer Distorter-Funktion kodiert und/oder das Nukleinsäuremolekül, das ein Expressionsprodukt kodiert, das gegen die Distorter-Funktion gerichtet ist und/oder das Nukleinsäuremolekül für die Inaktivierung der Distorter-Funktion durch homologe Rekombination, wobei alle diese Nukleinsäuremoleküle wie in den Ansprüchen 1 bis 14 definiert sind, und wobei das Säugetier ausgewählt ist aus der Gruppe bestehend aus Rattus, Bos, Sus und Ovis.

16. Nicht-menschliches männliches oder weibliches Säugetier, wobei das nicht-menschliche männliche oder weibliche Säugetier transgen ist für das Nukleinsäuremolekül, das ein Expressionsprodukt mit einer Responder-Funktion kodiert und das Nukleinsäuremolekül, das ein Expressionsprodukt mit einer Distorter-Funktion kodiert und/oder das Nukleinsäuremolekül, das ein Expressionsprodukt kodiert, das gegen die Distorter-Funktion gerichtet ist und/oder das Nukleinsäuremolekül für die Inaktivierung der Distorter-Funktion durch homologe Rekombination, wobei alle diese Nukleinsäuremoleküle wie in den Ansprüchen 1 bis 5 und 7 bis 14 definiert sind, wobei das Säugetier Mus ist und wobei die Nukleinsäuremoleküle im X oder Y Chromosom sind.

17. Nicht-menschliches männliches oder weibliches Säugetier nach Anspruch 16, wobei das erste Nukleinsäuremolekül, das ein Expressionsprodukt mit einer Responder-Funktion kodiert und das mindestens eine zweite Nukleinsäuremolekül, das ein Expressionsprodukt mit einer Distorter-Funktion kodiert und/oder das mindestens eine zweite Nukleinsäuremolekül, das ein Expressionsprodukt kodiert, das gegen die Distorter-Funktion gerichtet ist und/oder das zweite Nukleinsäuremolekül für die Inaktivierung der Distorter-Funktion durch homologe Rekombination in das selbe Chromosom eingebracht sind.

18. Paar aus nicht-menschlichem männlichen und weiblichen Säugetier, wobei mindestens entweder das männliche Tier und/oder das weibliche Tier ein transgenes, nicht-menschliches Säugetier nach einem der Ansprüche 15 bis 17 ist.

19. Paar aus nicht-menschlichem männlichen und weiblichen Säugetier nach Anspruch 18, wobei das Nukleinsäuremolekül oder der Teil davon, das/der ein Expressionsprodukt mit einer Responder-Funktion kodiert und/oder das Nukleinsäuremolekül oder der Teil davon, das/der ein Expressionsprodukt mit einer Distorter-Funktion kodiert und/oder das Nukleinsäuremolekül oder der Teil davon, das/der ein Expressionsprodukt kodiert, das gegen die Distorter-Funktion gerichtet ist und/oder das Nukleinsäuremolekül oder der Teil davon für die Inaktivierung der Distorter-Funktion durch homologe Rekombination, wobei alle diese Nukleinsäuremoleküle wie in einem der Ansprüche 1 bis 14 definiert sind, durch Rekombinase-Erkennungsstellen flankiert ist/sind.

20. Paar aus nicht-menschlichem männlichen und weiblichen Säugetier nach Anspruch 18 oder 19, das des weiteren ein Nukleinsäuremolekül stabil in seine genomische DNA integriert hat, das eine ortsspezifische DNA-Rekombinase kodiert.

21. Paar aus nicht-menschlichem männlichen und weiblichen Säugetier nach Anspruch 20, wobei die DNA-Rekombinase Cre ist, wenn die Erkennungsstellen loxP-Stellen sind, oder flp ist, wenn die Erkennungsstellen FRT-Stellen sind oder Φc31 ist, wenn die Erkennungsstellen att-Stellen sind.

22. Paar aus nicht-menschlichem männlichen und weiblichen Säugetier nach Anspruch 20 oder 21, wobei die DNA-Rekombinase durch regulatorische Elemente kontrolliert wird, die vor der Spermiogenese aktiv sind.

23. Sperma erhältlich von einem männlichen Tier des transgenen, nicht-menschlichen Säugetiers wie definiert in Anspruch 15, 17 oder 18 bis 22, sofern sich diese auf Anspruch 15 oder 17 rückbeziehen, wobei das Sperma das Nukleinsäuremolekül, das ein Expressionsprodukt mit einer Responder-Funktion kodiert und das Nukleinsäuremolekül, das ein Expressionsprodukt mit einer Distorter-Funktion kodiert und/oder das Nukleinsäuremolekül, das ein Expressionsprodukt kodiert, das gegen die Distorter-Funktion gerichtet ist und/oder das Nukleinsäuremolekül für die Inaktivierung der Distorter-Funktion durch homologe Rekombination, wobei alle diese Nukleinsäuremoleküle wie in einem der Ansprüche 1 bis 14 definiert sind, umfasst.

24. Verwendung des Spermas nach Anspruch 23 für die Herstellung von Nachkommen.

25. Verwendung des Nukleinsäuremoleküls, das ein Expressionsprodukt mit einer Distorter-Funktion kodiert wie in einem der Ansprüche 1 bis 14 definiert, zur Identifizierung von Chemikalien oder biologischen Stoffen, die die (vorzeitige) Aktivierung oder Inhibierung der Responder-/Distorter-Signalkaskade auslösen können, wobei die Effektivität dieser Stoffe anhand ihres Effekts auf die Motilität von Sperma *in vitro* getestet wird.

26. Verwendung des Nukleinsäuremoleküls, das ein Expressionsprodukt mit einer Distorter-Funktion kodiert wie in einem der Ansprüche 1 bis 14 definiert, zur Isolierung von Rezeptormolekülen der Responder-/Distorter-Signalkaskade und/oder anderer Mitglieder der Responder-/Distorter-Signalkaskade, an die das Expressionsprodukt binden kann.

27. Verfahren zum Nachweis des Nukleinsäuremoleküls, das ein Expressionsprodukt mit einer Distorter-Funktion kodiert und/oder des Nukleinsäuremoleküls, das ein Expressionsprodukt kodiert, das gegen die Distorter-Funktion gerichtet ist und/oder des Nukleinsäuremoleküls für die Inaktivierung der Distorter-Funktion durch homologe Rekombination, wobei alle diese Nukleinsäuremoleküle wie in einem der Ansprüche 1 bis 14 definiert sind, in dem nicht-menschlichen männlichen oder weiblichen Säugetier wie definiert in einem der Ansprüche 15 bis 17, umfassend das Identifizieren des Nukleinsäuremoleküls in dem nicht-menschlichen männlichen oder weiblichen Säugetier mittels Polymerase-Kettenreaktion (PCR), Gen-(Mikro-)Array-Hybridisierung, Analyse von Einzel-Nukleotid Polymorphismen (SNP), und/oder Sequenzieren mit Primern, die an das Nukleinsäuremolekül hybridisieren.

28. Verfahren nach einem der Ansprüche 1 bis 14 oder 27, oder nicht-menschliches männliches oder weibliches Säugetier nach Anspruch 15 bis 17, oder Paar aus nicht-menschlichem männlichen und weiblichen Säugetier nach einem der Ansprüche 18 bis 22, oder Sperma nach Anspruch 23, oder Verwendung nach Anspruch 25 oder 26, wobei das Nukleinsäuremolekül, das ein Expressionsprodukt mit einer Distorter-Funktion kodiert, ausgewählt ist aus der Gruppe bestehend aus
(a) einem Nukleinsäuremolekül umfassend oder bestehend aus dem Nukleinsäuremolekül einer der SEQ ID NO: 3 bis 6 oder 12, oder einem Fragment davon, wobei das Expressionsprodukt, das von dem Fragment kodiert wird, mindestens 70% der Distorter-Funktion behält;
(b) einem Nukleinsäuremolekül, das eine allelische Variante oder ein Ortholog des Nukleinsäuremoleküls nach (a) ist, wobei das Expressionsprodukt, das von der allelischen Variante oder dem Ortholog kodiert wird, mindestens 70% der Distorter-Funktion behält;
(c) einem Nukleinsäuremolekül, das unter stringenten Bedingungen an das Nukleinsäuremolekül nach (a) mit einer der SEQ ID NOs: 3 bis 6 hybridisiert, wobei das Nukleinsäuremolekül ein Polypeptid kodiert, das
(i) an der Position, die der Position 49 der SEQ ID NO:17 entspricht, ein I hat;
(ii) an der Position, die der Position 144 der SEQ ID NO:17 entspricht, ein L hat;
(iii) an der Position, die der Position 323 der SEQ ID NO:17 entspricht, ein T hat; und
(iv) nach der Position 442 abbricht;
(d) einem Nukleinsäuremolekül, das unter stringenten Bedingungen an das Nukleinsäuremolekül nach (a) mit einer der SEQ ID NOs: 3 bis 6 hybridisiert, wobei das Nukleinsäuremolekül ein Polypeptid kodiert, das
(i) an der Position, die der Position 49 der SEQ ID NO:17 entspricht, ein I hat;
(ii) an der Position, die der Position 137 der SEQ ID NO:17 entspricht, ein E hat;
(iii) an der Position, die der Position 207 der SEQ ID NO:17 entspricht, ein F hat;
(iv) an der Position, die der Position 301 der SEQ ID NO:17 entspricht, ein M hat;
(v) an der Position, die der Position 323 der SEQ ID NO:17 entspricht, ein T hat;
(vi) an der Position, die der Position 332 der SEQ ID NO:17 entspricht, ein D hat;
(vii) an der Position, die der Position 407 bis 413 der SEQ ID NO:17 entspricht, eine interne Deletion hat;
(viii) an der Position, die der Position 440 der SEQ ID NO:17 entspricht, ein M hat;
(ix) an der Position, die der Position 471 der SEQ ID NO:17 entspricht, ein L hat;
(x) an der Position, die der Position 552 der SEQ ID NO:17 entspricht, ein I hat;
(xi) an der Position, die der Position 596 der SEQ ID NO:17 entspricht, ein K hat;
(xii) an der Position, die der Position 607 der SEQ ID NO:17 entspricht, ein R hat;
(xiii) an der Position, die der Position 610 der SEQ ID NO:17 entspricht, ein S hat; und
(xiv) an der Position, die der Position 703 der SEQ ID NO:17 entspricht, ein V hat;
(e) einem Nukleinsäuremolekül, das unter stringenten Bedingungen an das Nukleinsäuremolekül nach (a) mit einer der SEQ ID NOs: 3 bis 6 hybridisiert, wobei das Nukleinsäuremolekül ein Polypeptid kodiert, das
(i) an der Position, die der Position 49 der SEQ ID NO:17 entspricht, ein I hat;
(ii) an der Position, die der Position 54 der SEQ ID NO:17 entspricht, ein G hat;
(iii) an der Position, die der Position 137 der SEQ ID NO:17 entspricht, ein E hat;
(iv) an der Position, die der Position 173 der SEQ ID NO:17 entspricht, ein G hat;
(v) an der Position, die der Position 207 der SEQ ID NO:17 entspricht, ein F hat;
(vi) an der Position, die der Position 301 der SEQ ID NO:17 entspricht, ein M hat;
(vii) an der Position, die der Position 323 der SEQ ID NO:17 entspricht, ein T hat;
(viii) an der Position, die der Position 332 der SEQ ID NO:17 entspricht, ein D hat;
(ix) an der Position, die der Position 407 bis 413 der SEQ ID NO:17 entspricht, eine interne Deletion hat;
(x) an der Position, die der Position 440 der SEQ ID NO:17 entspricht, ein M hat;
(xi) an der Position, die der Position 471 der SEQ ID NO:17 entspricht, ein L hat;
(xii) an der Position, die der Position 508 der SEQ ID NO:17 entspricht, ein S hat;
(xiii) an der Position, die der Position 552 der SEQ ID NO:17 entspricht, ein I hat;
(xiv) an der Position, die der Position 596 der SEQ ID NO:17 entspricht, ein K hat;
(xv) an der Position, die der Position 607 der SEQ ID NO:17 entspricht, ein R hat;
(xvi) an der Position, die der Position 610 der SEQ ID NO:17 entspricht, ein S hat; und
(xvii) an der Position, die der Position 703 der SEQ ID NO:17 entspricht, ein V hat; und
(f) einem Nukleinsäuremolekül, das unter stringenten Bedingungen an das Nukleinsäuremolekül nach (a) mit einer der SEQ ID NOs: 3 bis 6 hybridisiert, wobei das Nukleinsäuremolekül ein Polypeptid kodiert, das
(i) an der Position, die der Position 49 der SEQ ID NO:17 entspricht, ein I hat;
(ii) an der Position, die der Position 137 der SEQ ID NO:17 entspricht, ein E hat;
(iii) an der Position, die der Position 207 der SEQ ID NO:17 entspricht, ein F hat;
(iv) an der Position, die der Position 301 der SEQ ID NO:17 entspricht, ein M hat;
(v) an der Position, die der Position 323 der SEQ ID NO:17 entspricht, ein T hat;
(vi) an der Position, die der Position 332 der SEQ ID NO:17 entspricht, ein D hat;
(vii) an der Position, die der Position 407 bis 413 der SEQ ID NO:17 entspricht, eine interne Deletion hat;
(viii) an der Position, die der Position 440 der SEQ ID NO:17 entspricht, ein M hat;
(ix) an der Position, die der Position 471 der SEQ ID NO:17 entspricht, ein L hat;
(x) an der Position, die der Position 530 der SEQ ID NO:17 entspricht, ein E hat;
(xi) an der Position, die der Position 552 der SEQ ID NO:17 entspricht, ein I hat;
(xii) an der Position, die der Position 573 der SEQ ID NO:17 entspricht, ein R hat;
(xiii) an der Position, die der Position 596 der SEQ ID NO:17 entspricht, ein K hat;
(xiv) an der Position, die der Position 607 der SEQ ID NO:17 entspricht, ein R hat;
(xv) an der Position, die der Position 610 der SEQ ID NO:17 entspricht, ein S hat; und
(xvi) an der Position, die der Position 703 der SEQ ID NO:17 entspricht, ein V hat;
wobei das Expressionsprodukt mit einer Distorter-Funktion ein im G-Protein-Signalweg involvierter Faktor ist.

29. Verfahren, oder nicht-menschliches männliches oder weibliches Säugetier, oder Paar aus nicht-menschlichem männlichem oder weiblichem Säugetier, oder Sperma oder Verwendung nach Anspruch 28, wobei das Nukleinsäuremolekül ein DNA Molekül ist.

30. Verfahren, oder nicht-menschliches männliches oder weibliches Säugetier, oder Paar aus nicht-menschlichem männlichem oder weiblichem Säugetier, oder Sperma oder Verwendung nach Anspruch 28 oder 29, wobei das Expressionsprodukt eine RNA oder ein (Poly)Peptid ist.

31. Verfahren, oder nicht-menschliches männliches oder weibliches Säugetier, oder Paar aus nicht-menschlichem männlichem oder weiblichem Säugetier, oder Sperma oder Verwendung nach Anspruch 28 bis 30, wobei der heterologe Promoter der Promoter von c-kit, ACE, Tcr oder Smok im Hoden ist.

32. Verfahren zur Identifizierung eines Expressionsproduktes eines Nukleinsäuremoleküls, das einen Distorter kodiert, umfassend die Schritte
(a) Isolieren eines Expressionsproduktes eines Nukleinsäuremoleküls, das einen potentiellen Distorter kodiert, mittels Protein-Protein-Interaktion mit einem bekannten Distorter, der aus dem Maus-t-Komplex abgeleitet ist, wobei der bekannte Distorter ausgewählt ist aus der Gruppe bestehend aus Tagap1, Tiam2 und Fgd2; und
(b) Testen des Nukleinsäuremoleküls, das das in (a) isolierte Expressionsprodukt kodiert, auf eine Veränderung in der Vererbungsrate des Responders wie in Anspruch 1 definiert oder eines genetischen Merkmals, das mit dem Responder verknüpft ist, in einem nicht-menschlichen Versuchstier, wobei das in (a) isolierte Expressionsprodukt ein Expressionsprodukt mit Distorter-Funktion ist, wenn die Vererbungsrate erhöht oder vermindert ist.

33. Verfahren nach Anspruch 32, wobei in Schritt (b) hypomorphe oder hypermorphe Allele des Nukleinsäuremoleküls für den Test auf eine Veränderung in der Vererbungsrate verwendet werden.

## Revendications

1. Méthode pour produire un animal mâle mammifère non humain transgénique, dans laquelle le ou les transgènes confèrent un changement dans le taux de transmission de un ou plusieurs caractères génétiques à la descendance dudit animal mâle mammifère non humain vers un taux non-mendélien, ladite méthode comprenant l'introduction
(a) d'une première molécule d'acide nucléique codant pour un produit d'expression ayant une fonction Répondeur dans un chromosome d'une cellule germinale mammifère non humaine, d'une cellule-oeuf (fécondée), d'une cellule embryonnaire ou d'une cellule dérivée des précédentes, appartenant à la même espèce que le mâle transgénique devant être préparé, ledit chromosome contenant ou conférant ledit ou lesdits caractères génétiques, liant ainsi sur ledit chromosome ladite fonction Répondeur au(x) caractère(s) génétique(s), dans laquelle ladite première molécule d'acide nucléique codant pour un produit d'expression ayant une fonction Répondeur est choisie dans le groupe constitué par
(aa) une molécule d'acide nucléique comprenant ou constituée par la molécule d'acide nucléique décrite dans SEQ ID NO: 15 ou 16 ou un fragment de celle-ci, dans laquelle le produit d'expression codé par le fragment conserve au moins 70 % de la fonction Répondeur ;
(ab) une molécule d'acide nucléique étant un variant allélique ou un orthologue de la molécule d'acide nucléique en (aa), dans laquelle le produit d'expression codé par le variant allélique ou l'orthologue conserve au moins 70 % de la fonction Répondeur ; et
(ac) une molécule d'acide nucléique qui est apparentée à la molécule d'acide nucléique en (aa) ou (ab) par la dégénérescence du code génétique ; et
(b) d'au moins une seconde molécule d'acide nucléique codant pour un produit d'expression ayant une fonction de Distorsion dans un ou plusieurs chromosomes d'une cellule germinale mammifère non humaine, d'une cellule-oeuf (fécondée), d'une cellule embryonnaire ou d'une cellule dérivée de la même espèce que le mâle transgénique devant être préparé, dans laquelle ledit produit d'expression ayant une fonction de Distorsion est choisi dans le groupe constitué par Tagap1, Tiam2 et Fgd2,
dans laquelle ladite première molécule d'acide nucléique codant pour un produit d'expression ayant une fonction Répondeur et ladite au moins une seconde molécule d'acide nucléique codant pour un produit d'expression ayant une fonction de Distorsion sont introduites dans le même chromosome ou dans des chromosomes différents et dans laquelle la transcription des molécules d'acide nucléique est activée par utilisation d'un promoteur qui active la transcription spécifiquement lors de la spermatogenèse et/ou de la spermiogenèse.

2. Méthode pour produire un animal mâle mammifère non humain transgénique, dans lequel le ou les transgènes confèrent un changement dans le taux de transmission de un ou plusieurs caractères génétiques à la descendance dudit animal mâle mammifère non humain vers un taux non-mendélien, ladite méthode comprenant l'introduction
(a) d'une première molécule d'acide nucléique codant pour un produit d'expression ayant une fonction Répondeur dans un chromosome d'une cellule germinale mammifère non humaine, d'une cellule-oeuf (fécondée), d'une cellule embryonnaire ou d'une cellule dérivée des précédentes, appartenant à la même espèce que le mâle transgénique devant être préparé, ledit chromosome contenant ou conférant ledit ou lesdits caractères génétiques, liant ainsi sur ledit chromosome ladite fonction Répondeur au(x) caractère(s) génétique(s), dans laquelle ladite première molécule d'acide nucléique codant pour un produit d'expression ayant une fonction Répondeur est choisie dans le groupe constitué par
(aa) une molécule d'acide nucléique comprenant ou constituée par la molécule d'acide nucléique décrite dans SEQ ID NO: 15 ou 16 ou un fragment de celle-ci, dans laquelle le produit d'expression codé par le fragment conserve au moins 70 % de la fonction Répondeur ;
(ab) une molécule d'acide nucléique étant un variant allélique ou un orthologue de la molécule d'acide nucléique en (aa), dans laquelle le produit d'expression codé par le variant allélique ou l'orthologue conserve au moins 70 % de la fonction Répondeur ; et
(ac) une molécule d'acide nucléique qui est apparentée à la molécule d'acide nucléique en (aa) ou (ab) par la dégénérescence du code génétique ; et
(b) d'au moins une seconde molécule d'acide nucléique codant pour un produit d'expression dirigé contre la fonction de Distorsion dans un ou des chromosomes d'une cellule germinale mammifère non humaine, d'une cellule-oeuf (fécondée), d'une cellule embryonnaire ou d'une cellule dérivée des précédentes, appartenant à la même espèce que le mâle transgénique devant être préparé, dans laquelle ladite fonction de Distorsion est un produit d'expression qui est codé par une molécule d'acide nucléique codant pour un produit d'expression ayant une fonction de Distorsion, ledit produit d'expression ayant une fonction de Distorsion étant choisi dans le groupe constitué par Tagap1, Tiam2 et Fgd2, dans laquelle ledit produit d'expression dirigé contre la fonction de Distorsion est un aptamère, un ARNsi ou un ARNsh ou un miARN, un ribozyme, ou une molécule d'acide nucléique antisens s'hybridant spécifiquement dans des conditions stringentes auxdites molécules d'acide nucléique codant pour un de Distorsion choisi dans le groupe constitué par Tagap1, Tiam2 et Fgd2 ; et/ou
(c) d'une seconde molécule d'acide nucléique inactivant la fonction de Distorsion par recombinaison homologue, dans laquelle ladite seconde molécule d'acide nucléique inactivant la fonction de Distorsion par recombinaison homologue est au moins partiellement identique à ladite molécule d'acide nucléique codant pour un produit d'expression ayant une fonction de Distorsion et dans laquelle ledit produit d'expression ayant une fonction de Distorsion est choisi dans le groupe constitué par Tagap1, Tiam2 et Fgd2,
dans lequel ladite première molécule d'acide nucléique codant pour un produit d'expression ayant une fonction Répondeur et ladite au moins une seconde molécule d'acide nucléique codant pour un produit d'expression dirigé contre la fonction de Distorsion et/ou ladite seconde molécule d'acide nucléique inactivant la fonction de Distorsion par recombinaison homologue sont introduites dans le même chromosome ou dans des chromosomes différents, et dans laquelle la transcription des molécules d'acide nucléique est activée par utilisation d'un promoteur qui active la transcription spécifiquement lors de la spermatogenèse et/ou de la spermiogenèse ; inactivant ainsi la fonction de Distorsion complètement ou à au moins 50 %.

3. Méthode selon la revendication 1 ou 2, dans laquelle ledit mammifère est choisi dans le groupe constitué par Mus, Rattus, Bos, Sus et Ovis.

4. Méthode selon l'une quelconque des revendications 1 à 3, dans laquelle ledit caractère génétique est le sexe.

5. Méthode selon l'une quelconque des revendications 1 à 4, dans laquelle ledit chromosome est un chromosome X ou Y.

6. Méthode selon l'une quelconque des revendications 1 à 4, dans laquelle ledit chromosome est un autosome.

7. Méthode selon l'une quelconque des revendications 1 à 6, dans laquelle ladite (au moins une) seconde molécule d'acide nucléique codant pour un produit d'expression ayant une fonction de Distorsion est choisie dans le groupe constitué par
(a) une molécule d'acide nucléique comprenant ou constituée par la molécule d'acide nucléique décrite dans l'une quelconque des SEQ ID NO: 1 à 14, 31 à 38, 47 à 50 ou 55 à 72 ou un fragment de celle-ci, dans laquelle le fragment conserve au moins 70 % de la fonction de Distorsion ;
(b) une molécule d'acide nucléique étant un variant allélique ou un orthologue des molécules d'acide nucléique en (a), dans laquelle le variant allélique ou l'orthologue conserve au moins 70 % de la fonction de Distorsion ;
et
(c) une molécule d'acide nucléique qui est apparentée à la molécule d'acide nucléique en (a) ou (b) par la dégénérescence du code génétique.

8. Méthode selon l'une quelconque des revendications 1 et 3 à 6, dans laquelle ladite au moins une seconde molécule d'acide nucléique codant pour un produit d'expression ayant une fonction de Distorsion est choisie dans le groupe constitué par
(a) une molécule d'acide nucléique comprenant ou constituée par la molécule d'acide nucléique décrite dans l'une quelconque des SEQ ID NO: 1 à 12, 31 à 38 ou 55 à 66 ou un fragment de celle-ci, dans laquelle le fragment conserve au moins 70 % de la fonction de Distorsion ;
(b) une molécule d'acide nucléique étant un variant allélique ou un orthologue de la molécule d'acide nucléique en (a), dans laquelle le variant allélique ou l'orthologue conserve au moins 70 % de la fonction de Distorsion ;
et
(c) une molécule d'acide nucléique qui est apparentée à la molécule d'acide nucléique en (a) ou (b) par la dégénérescence du code génétique,
améliorant ainsi ledit taux de transmission dudit ou desdits caractères génétiques.

9. Méthode selon l'une quelconque des revendications 1 et 3 à 6, dans laquelle ladite au moins une seconde molécule d'acide nucléique codant pour un produit d'expression ayant une fonction de Distorsion est choisie dans le groupe constitué par
(a) une molécule d'acide nucléique comprenant ou constituée par la molécule d'acide nucléique décrite dans SEQ ID NO: 13 ou 14, 47 à 50 ou 67 à 72 ou un fragment de celle-ci, dans laquelle le fragment conserve au moins 70 % de la fonction de Distorsion ;
(b) une molécule d'acide nucléique étant un variant allélique ou un orthologue de la molécule d'acide nucléique en (a), dans laquelle le variant allélique ou l'orthologue conserve au moins 70 % de la fonction de Distorsion ;
et
(c) une molécule d'acide nucléique qui est apparentée à la molécule d'acide nucléique en (a) ou (b) par la dégénérescence du code génétique,
réduisant ainsi ledit taux de transmission dudit ou desdits caractères génétiques.

10. Méthode selon l'une quelconque des revendications 2 à 6, dans laquelle ladite molécule d'acide nucléique codant pour un produit d'expression ayant une fonction de Distorsion est choisie dans le groupe constitué par
(a) une molécule d'acide nucléique comprenant ou constituée par la molécule d'acide nucléique décrite dans l'une quelconque des SEQ ID NO: 1 à 12, 31 à 38 ou 55 à 66 ou un fragment de celle-ci, dans laquelle le fragment conserve au moins 70 % de la fonction de Distorsion ;
(b) une molécule d'acide nucléique étant un variant allélique ou un orthologue de la molécule d'acide nucléique en (a), dans laquelle le variant allélique ou l'orthologue conserve au moins 70 % de la fonction de Distorsion ;
et
(c) une molécule d'acide nucléique qui est apparentée à la molécule d'acide nucléique en (a) ou (b) par la dégénérescence du code génétique,
réduisant ainsi ledit taux de transmission dudit ou desdits caractères génétiques.

11. Méthode selon l'une quelconque des revendications 2 à 6, dans laquelle ladite molécule d'acide nucléique codant pour un produit d'expression ayant une fonction de Distorsion est choisie dans le groupe constitué par
(a) une molécule d'acide nucléique comprenant ou constituée par la molécule d'acide nucléique décrite dans SEQ ID NO: 13 ou 14, 47 à 50 ou 67 à 72 ou un fragment de celle-ci, dans laquelle le fragment conserve au moins 70 % de la fonction de Distorsion ;
(b) une molécule d'acide nucléique étant un variant allélique ou un orthologue de la molécule d'acide nucléique en (a), dans laquelle le variant allélique ou l'orthologue conserve au moins 70 % de la fonction de Distorsion ;
et
(c) une molécule d'acide nucléique qui est apparentée à la molécule d'acide nucléique en (a) ou (b) par la dégénérescence du code génétique,
améliorant ainsi ledit taux de transmission dudit ou desdits caractères génétiques.

12. Méthode selon l'une quelconque des revendications 1 à 11 comprenant, en outre, le croisement du mammifère mâle non humain transgénique obtenu par la méthode selon l'une quelconque des revendications 1 à 11 avec un mammifère femelle non humain et l'analyse de la descendance dudit croisement en termes de transmission dudit ou desdits caractères génétiques.

13. Méthode selon l'une quelconque des revendications 1 à 12, dans laquelle ladite au moins une seconde molécule d'acide nucléique codant pour le produit d'expression ayant une fonction de Distorsion est modifiée, réduisant ainsi davantage ou améliorant davantage l'activité de la fonction de Distorsion.

14. Méthode selon l'une quelconque des revendications 5 à 13, dans laquelle ladite première molécule d'acide nucléique codant pour un produit d'expression ayant une fonction Répondeur et ladite au moins une seconde molécule d'acide nucléique codant pour un produit d'expression avec la fonction de Distorsion et/ou ladite au moins une seconde molécule d'acide nucléique codant pour un produit d'expression dirigé contre la fonction de Distorsion et/ou ladite seconde molécule d'acide nucléique inactivant la fonction de Distorsion par recombinaison homologue et un promoteur contrôlant l'expression lors de la spermatogenèse et/ou de la spermiogenèse et/ou une cassette d'arrêt sont intégrées dans ledit chromosome X ou Y ou dans l'un desdits autosomes dans un état d'expressivité inactif réversible.

15. Mammifère mâle ou femelle non humain, ledit mammifère mâle ou femelle non humain étant transgénique pour la molécule d'acide nucléique codant pour un produit d'expression ayant une fonction Répondeur et la molécule d'acide nucléique codant pour un produit d'expression ayant une fonction de Distorsion et/ou la molécule d'acide nucléique codant pour un produit d'expression dirigé contre la fonction de Distorsion et/ou la molécule d'acide nucléique inactivant la fonction de Distorsion par recombinaison homologue, toutes lesdites molécules d'acide nucléique étant telles que définies dans l'une quelconque des revendications 1 à 14, et ledit mammifère étant choisi dans le groupe constitué par Rattus, Bos, Sus et Ovis.

16. Mammifère mâle ou femelle non humain, ledit mammifère mâle ou femelle non humain étant transgénique pour la molécule d'acide nucléique codant pour un produit d'expression ayant une fonction Répondeur et la molécule d'acide nucléique codant pour un produit d'expression ayant une fonction de Distorsion et/ou la molécule d'acide nucléique codant pour un produit d'expression dirigé contre la fonction de Distorsion et/ou la molécule d'acide nucléique inactivant la fonction de Distorsion par recombinaison homologue, toutes lesdites molécules d'acide nucléique étant telles que définies dans l'une quelconque des revendications 1 à 5 et 7 à 14, ledit mammifère étant Mus et lesdites molécules d'acide nucléique étant dans un chromosome X ou Y.

17. Mammifère mâle ou femelle non humain selon la revendication 16, dans lequel ladite première molécule d'acide nucléique codant pour un produit d'expression ayant une fonction Répondeur et ladite au moins une seconde molécule d'acide nucléique codant pour un produit d'expression ayant une fonction de Distorsion et/ou ladite au moins une seconde molécule d'acide nucléique codant pour un produit d'expression dirigé contre la fonction de Distorsion et/ou ladite seconde molécule d'acide nucléique inactivant la fonction de Distorsion par recombinaison homologue sont introduites dans le même chromosome.

18. Couple d'animaux mammifères mâle et femelle non humains, dans lequel au moins le mâle et/ou la femelle est un mammifère non humain transgénique selon les revendications 15 à 17.

19. Couple d'animaux mammifères mâle et femelle non humains selon la revendication 18, dans lequel la molécule d'acide nucléique ou une partie de celle-ci codant pour un produit d'expression ayant une fonction Répondeur et/ou la molécule d'acide nucléique ou une partie de celle-ci codant pour un produit d'expression ayant une fonction de Distorsion et/ou la molécule d'acide nucléique ou une partie de celle-ci codant pour un produit d'expression dirigé contre la fonction de Distorsion et/ou la molécule d'acide nucléique ou une partie de celle-ci inactivant la fonction de Distorsion par recombinaison homologue, toutes lesdites molécules d'acide nucléique étant telles que définies dans l'une quelconque des revendications 1 à 14, est/sont encadrée(s) par des sites de reconnaissance de la recombinase.

20. Couple d'animaux mammifères mâle et femelle non humains selon la revendication 18 ou 19 ayant, en outre, une molécule d'acide nucléique codant pour un ADN recombinase spécifique de site, intégrée de manière stable dans leur ADN génomique.

21. Couple d'animaux mammifères mâle et femelle non humains selon la revendication 20, dans lequel ledit ADN recombinase est Cre, dans lequel lesdits sites de reconnaissance sont des sites loxP, ou flp, dans lequel lesdits sites de reconnaissance sont des sites FRT, ou Φc31, dans lequel lesdits sites de reconnaissance sont des sites att.

22. Couple d'animaux mammifères mâle et femelle non humains selon la revendication 20 ou 21, dans lequel ledit ADN recombinase est contrôlée par des éléments régulateurs qui sont actifs avant la spermiogenèse.

23. Sperme pouvant être obtenu à partir d'un animal mâle mammifère non humain transgénique tel que défini dans les revendications 15, 17 ou les revendications 18 à 22 quand elles se réfèrent aux revendications 15 ou 17, le sperme comprenant la molécule d'acide nucléique codant pour un produit d'expression ayant une fonction Répondeur et la molécule d'acide nucléique codant pour un produit d'expression ayant une fonction de Distorsion et/ou la molécule d'acide nucléique codant pour un produit d'expression dirigé contre la fonction de Distorsion et/ou la molécule d'acide nucléique inactivant la fonction de Distorsion par recombinaison homologue, toutes lesdites molécules d'acide nucléique étant telles que définies dans l'une quelconque des revendications 1 à 14.

24. Utilisation du sperme selon la revendication 23 pour la production de la descendance.

25. Utilisation de la molécule d'acide nucléique codant pour un produit d'expression ayant une fonction de Distorsion telle que définie dans l'une quelconque des revendications 1 à 14, pour identifier des agents chimiques ou des composés biologiques capables de déclencher l'activation ou l'inhibition (prématurée) de la cascade de signalisation Répondeur/Distorsion, dans laquelle l'efficacité de ces composés est testée par leur effet sur la motilité du sperme in vitro.

26. Utilisation de la molécule d'acide nucléique codant pour un produit d'expression ayant une fonction de Distorsion telle que définie dans l'une quelconque des revendications 1 à 14, pour isoler des molécules réceptrices de la cascade de signalisation Répondeur/Distorsion et/ou d'autres membres de la cascade de signalisation Répondeur/Distorsion auxquels ledit produit d'expression peut se lier.

27. Méthode de détection de la molécule d'acide nucléique codant pour un produit d'expression ayant une fonction de Distorsion et/ou de la molécule d'acide nucléique codant pour un produit d'expression dirigé contre la fonction de Distorsion et/ou de la molécule d'acide nucléique inactivant la fonction de Distorsion par recombinaison homologue, toutes lesdites molécules d'acide nucléique étant telles que définies dans l'une quelconque des revendications 1 à 14, chez le mammifère mâle ou femelle non humain tel que défini dans les revendications 15 à 17, comprenant l'identification de ladite molécule d'acide nucléique chez ledit mammifère mâle ou femelle non humain par réaction en chaîne de la polymérase (PCR), hybridation sur (micro)puce à gènes, analyse des polymorphismes d'un seul nucléotide (SNP), et/ou séquençage avec des amorces s'hybridant à ladite molécule d'acide nucléique.

28. Méthode selon l'une quelconque des revendications 1 à 14 ou 27 ou mammifère mâle ou femelle non humain selon les revendications 15 à 17, ou couple d'animaux mammifères mâle et femelle non humains selon l'une quelconque des revendications 18 à 22, ou sperme selon la revendication 23, ou utilisation selon les revendications 25 ou 26, où la molécule d'acide nucléique codant pour un produit d'expression ayant une fonction de Distorsion, où ledit produit d'expression ayant une fonction de Distorsion est un facteur impliqué dans la signalisation des protéines G, est choisie dans le groupe constitué par :
(a) une molécule d'acide nucléique comprenant ou constituée par la molécule d'acide nucléique selon l'une quelconque des SEQ ID NO: 3 à 6 et 12 ou un fragment de celle-ci, dans laquelle le produit d'expression codé par le fragment conserve au moins 70 % de la fonction de Distorsion ;
(b) une molécule d'acide nucléique qui est un variant allélique ou un orthologue de la molécule d'acide nucléique en (a), dans laquelle le produit d'expression codé par le variant allélique ou l'orthologue conserve au moins 70 % de la fonction de Distorsion ;
(c) une molécule d'acide nucléique qui s'hybride dans des conditions stringentes à la molécule d'acide nucléique en (a) se référant aux SEQ ID NO: 3 à 6, dans laquelle ladite molécule d'acide nucléique code pour un polypeptide qui a
(i) à la position correspondant à la position 49 de SEQ ID NO: 17 un I ;
(ii) à la position correspondant à la position 144 de SEQ ID NO: 17 un L ;
(iii) à la position correspondant à la position 323 de SEQ ID NO: 17 un T ; et
(iv) qui se termine après la position 442 ;
(d) une molécule d'acide nucléique qui s'hybride dans des conditions stringentes à la molécule d'acide nucléique en (a) se référant aux SEQ ID NO: 3 à 6, dans laquelle ladite molécule d'acide nucléique code pour un polypeptide qui a
(i) à la position correspondant à la position 49 de SEQ ID NO: 17 un I;
(ii) à la position correspondant à la position 137 de SEQ ID NO: 17 un E ;
(iii) à la position correspondant à la position 207 de SEQ ID NO: 17 un F ;
(iv) à la position correspondant à la position 301 de SEQ ID NO: 17 un M ;
(v) à la position correspondant à la position 323 de SEQ ID NO: 17 un T;
(vi) à la position correspondant à la position 332 de SEQ ID NO: 17 un D ;
(vii) à la position correspondant à la position 407-413 de SEQ ID NO: 17 une délétion interne ;
(viii) à la position correspondant à la position 440 de SEQ ID NO: 17 un M ;
(ix) à la position correspondant à la position 471 de SEQ ID NO: 17 un L ;
(x) à la position correspondant à la position 552 de SEQ ID NO: 17 un I ;
(xi) à la position correspondant à la position 596 de SEQ ID NO: 17 un K ;
(xii) à la position correspondant à la position 607 de SEQ ID NO: 17 un R ;
(xiii) à la position correspondant à la position 610 de SEQ ID NO: 17 un S ; et
(xiv) à la position correspondant à la position 703 de SEQ ID NO: 17 un V ;
(e) une molécule d'acide nucléique qui s'hybride dans des conditions stringentes à la molécule d'acide nucléique en (a) se référant aux SEQ ID NO: 3 à 6, dans laquelle ladite molécule d'acide nucléique code pour un polypeptide qui a
(i) à la position correspondant à la position 49 de SEQ ID NO: 17 un I ;
(ii) à la position correspondant à la position 54 de SEQ ID NO: 17 un G;
(iii) à la position correspondant à la position 137 de SEQ ID NO: 17 un E ;
(iv) à la position correspondant à la position 173 de SEQ ID NO: 17 un G;
(v) à la position correspondant à la position 207 de SEQ ID NO: 17 un F ;
(vi) à la position correspondant à la position 301 de SEQ ID NO: 17 un M ;
(vii) à la position correspondant à la position 323 de SEQ ID NO: 17 un T;
(viii) à la position correspondant à la position 332 de SEQ ID NO: 17 un D ;
(ix) à la position correspondant à la position 407-413 de SEQ ID NO: 17 une délétion interne ;
(x) à la position correspondant à la position 440 de SEQ ID NO: 17 un M ;
(xi) à la position correspondant à la position 471 de SEQ ID NO: 17 un L ;
(xii) à la position correspondant à la position 508 de SEQ ID NO: 17 un S;
(xiii) à la position correspondant à la position 552 de SEQ ID NO: 17 un I ;
(xiv) à la position correspondant à la position 596 de SEQ ID NO: 17 un K ;
(xv) à la position correspondant à la position 607 de SEQ ID NO: 17 un R ;
(xvi) à la position correspondant à la position 610 de SEQ ID NO: 17 un S ; et
(xvii) à la position correspondant à la position 703 de SEQ ID NO: 17 un V ; et
(f) une molécule d'acide nucléique qui s'hybride dans des conditions stringentes à la molécule d'acide nucléique en (a) se référant aux SEQ ID NO: 3 à 6, dans laquelle ladite molécule d'acide nucléique code pour un polypeptide qui a
(i) à la position correspondant à la position 49 de SEQ ID NO: 17 un I ;
(ii) à la position correspondant à la position 137 de SEQ ID NO: 17 un E ;
(iii) à la position correspondant à la position 207 de SEQ ID NO: 17 un F ;
(iv) à la position correspondant à la position 301 de SEQ ID NO: 17 un M ;
(v) à la position correspondant à la position 323 de SEQ ID NO: 17 un T;
(vi) à la position correspondant à la position 332 de SEQ ID NO: 17 un D ;
(vii) à la position correspondant à la position 407-413 de SEQ ID NO: 17 une délétion interne ;
(viii) à la position correspondant à la position 440 de SEQ ID NO: 17 un M ;
(ix) à la position correspondant à la position 471 de SEQ ID NO: 17 un L ;
(x) à la position correspondant à la position 530 de SEQ ID NO: 17 un E ;
(xi) à la position correspondant à la position 552 de SEQ ID NO: 17 un I ;
(xii) à la position correspondant à la position 573 de SEQ ID NO: 17 un R ;
(xiii) à la position correspondant à la position 596 de SEQ ID NO: 17 un K ;
(xiv) à la position correspondant à la position 607 de SEQ ID NO: 17 un R;
(xv) à la position correspondant à la position 610 de SEQ ID NO: 17 un S ; et
(xvi) à la position correspondant à la position 703 de SEQ ID NO: 17 un V.

29. Méthode, ou mammifère mâle ou femelle non humain, ou couple d'animaux mammifères mâle et femelle non humains, ou sperme, ou utilisation selon la revendication 28, où la molécule d'acide nucléique est une molécule d'ADN.

30. Méthode, ou mammifère mâle ou femelle non humain, ou couple d'animaux mammifères mâle et femelle non humains, ou sperme, ou utilisation selon la revendication 28 ou 29, où ledit produit d'expression est un ARN ou un (poly)peptide.

31. Méthode, ou mammifère mâle ou femelle non humain, ou couple d'animaux mammifères mâle et femelle non humains, ou sperme, ou utilisation selon la revendication 28 à 30, où le promoteur hétérologue est le promoteur testiculaire de c-kit, ACE, Tcr ou Smok.

32. Méthode d'identification d'un produit d'expression d'une molécule d'acide nucléique codant pour un de Distorsion, comprenant les étapes suivantes
(a) isoler un produit d'expression d'une molécule d'acide nucléique codant pour un de Distorsion candidat au moyen d'une interaction protéine-protéine avec un de Distorsion connu dérivé du complexe t murin, où ledit de Distorsion connu est choisi dans le groupe constitué par Tagap1, Tiam2 et Fgd2 ; et
(b) tester la molécule d'acide nucléique codant pour ledit produit d'expression isolé en (a) en termes de changement du taux de transmission du Répondeur tel que défini dans la revendication 1 ou de caractère génétique lié audit Répondeur chez un animal non humain expérimental, dans laquelle, quand ledit taux de transmission est amélioré ou réduit, ledit produit d'expression isolé en (a) est un produit d'expression ayant une fonction de Distorsion.

33. Méthode selon la revendication 32 dans laquelle, dans l'étape (b), les allèles hypomorphiques ou hypermorphiques de ladite molécule d'acide nucléique sont utilisés pour tester le changement de taux de transmission.


### SEQUENCE LISTING

<110> Max-Planck-Gesellschaft zur Förderung der Wissenschaften e.V.
<120> Isolation of the t-complex distorter 1 and applications thereof
<130> K 3062 PCT
<150> EP 05 01 7651.0
   <151> 2005-08-12
<160> 90
<170> PatentIn version 3.3
<210> 1
   <211> 2145
   <212> DNA
   <213> Tagap1 mouse wildtype FVB/N Accession Number NM_145968
<400> 1
   atgaagctga taagcagtct cgatggttca aaaacactta atgctaacaa catggagaca      60
   ttaattgaat gtcagtcaga gggtgatatc aaagtgcctc ccctgctgac atcatgtgag     120
   agtgaagaca gcatttgcca gctaactgaa attaagaaga gaaagaaagt gctgtcctgg     180
   ccatctctca tgagaaagct ctctccttca tccgacttct ctgggtcatt ggaaccagag     240
   ctgaaagtgt cgctgtttga ccaacccttg tcgatcatct gtggggagaa cgacacactt     300
   cccagaccca tccaggacat cctcaccatc ctctgcctta aaggtccttc aacggaaggg     360
   atattcagga aagctgccag cgagaaagcc cgcaaggagc tgaaggaggg gcttaactgt     420
   ggggtctccg tgaatctgaa gcagctccct gtgcacctcc tagctgtggt cttcaaggac     480
   ttcctccgag ggatacccct gaagctactc tcctgtgatc tctttgagga ctggatgggc     540
   gccctggaga agcccaccga ggaggacaga atcgaggccc tgaagcaggt tgctggtggc     600
   ctcccccggc ccaaccttct cctgctcagg cacttgctct acgtgctcca cctcatcagc     660
   aagaacgctg aggtcaacaa gatggactcc agcaacctcg ccatctgcat cgggcctaac     720
   atgctcactc tgaagaacga ccagagcctg tccttccagg cccagaagga cctgaacaat     780
   aaggttaaga tcttggtgga attcctcatc gacaactgct ttgagatatt tggggagaac     840
   attcggacgc gttcccgcat cacttctgac gactccctgg aacacactga cagttcagac     900
   gtgtcaactc tgcagaatga ctcggcctat gacagcaatg acccagatgt ggagcccaca     960
   agtggcgcag cctctcccaa caggcaactg gagggtccca ctcccacaat ggctggtctg    1020
   gatacccggg gccaacggga cacctgtgag tcaagctcag agtccagcgt tagcatggta    1080
   gtcaggctga aaagctccat tgtccaacaa gacaggcggt tctctgaacc caacatgtca    1140
   ccctcacgag agtgcctcgt gggcccaaca tccaagcaaa agctagcaag gagtgaggac    1200
   agcttcactc tgtcccagga cgcctcctgt tctgaaggcg atgaagctga agatcccttt    1260
   acagaggaag tcttcccagc agtggacagc aaacccaaga gacctgtgga tttgaaaata 1320
   aagaattgga cccaaggttt agcatctcca cagggacaca taaccaaagc tttctccaga 1380
   tcctccccag gcgaatcttt gggcagctca cctgtgcctt ctccatcctg ccccaagaga 1440
   aacttcttca ccagacacca gagtttcaca acaaagacag acaaaaccaa accccagaga 1500
   gaaattagaa agcactccat gtcgttttcc tttgcgtctc acaagaaagt gctgccccga 1560
   acctccagca ttgggtctga gaaatccaaa gacttttcta gagaccaact ccagaaggac 1620
   ttgaggaaag agagccaact ttctggcaga atcgtccagg aaaatgagtc agaaatccaa 1680
   agccaaacat ctctgggctt cagcttgtct ggcacctggg ccctctcagt cgacaacacg 1740
   ttccagttag tggatatgag gaaaccagga agcccaccat cttacgaaga ggccatttat 1800
   taccagacat caggactcac agcctacggt ggccagacag ttgggagtat gaggtcaaga 1860
   atgttcaagc caagcacagc agtgccccct gtgccttctc accatggagg tgacctcagt 1920
   gaagggacac ctggtggaca cagattgtct tctgtgactg agcactggac acacagtcag 1980
   actgtccatg tctctataga aactcagggg agatctgagc tacaccagtt gaggacagtg 2040
   tccgagtcca tgcagaaggc taagctggac tgtcttgggc cacaacacag ccacttagtc 2100
   tttgaggctg accaactctg ctgtgctaga gaatcctaca tttaa 2145
<210> 2
   <211> 2145
   <212> DNA
   <213> Tagap1 mouse wildtype BALB/c
<400> 2
   atgaagctga taagcagtct cgatggttca aaaacactta atgctaacaa catggagaca 60
   ttaattgaat gtcagtcaga gggtgatatc aaagtgcctc ccctgctgac atcatgtgag 120
   agtgaagaca gcatttgcca gctaactgaa attaagaaga gaaagaaagt gctgtcctgg 180
   ccatctctca tgagaaagct ctctccttca tccgacttct ctgggtcatt ggaaccagag 240
   ctgaaagtgt cgctgtttga ccaacccttg tcgatcatct gtggggagaa cgacacactt 300
   cccagaccca tccaggacat cctcaccatc ctctgcctta aaggtccttc aacggaaggg 360
   atattcagga aagctgccag cgagaaagcc cgcaaggagc tgaaggaggg gcttaactgt 420
   ggggtctccg tgaatctgaa gcagctccct gtgcacctcc tagctgtggt cttcaaggac 480
   ttcctccgag ggatacccct gaagctactc tcctgtgatc tctttgagga ctggatgggc 540
   gccctggaga agcccaccga ggaggacaga atcgaggccc tgaagcaggt tgctggtggc 600
   ctcccccggc ccaaccttct cctgctcagg cacttgctct acgtgctcca cctcatcagc 660
   aagaacgctg aggtcaacaa gatggactcc agcaacctcg ccatctgcat cgggcctaac 720
   atgctcactc tgaagaacga ccagagcctg tccttccagg cccagaagga cctgaacaat 780
   aaggttaaga tcttggtgga attcctcatc gacaactgct ttgagatatt tggggagaac 840
   attcggacgc gttcccgcat cacttctgac gactccctgg aacacactga cagttcagac     900
   gtgtcaactc tgcagaatga ctcggcctat gacagcaatg acccagatgt ggagcccaca     960
   agtggcgcag cctctcccaa caggcaactg gagggtccca ctcccacaat ggctggtctg    1020
   gatacccggg gccaccggga cacctgtgag tcaagctcag agtccagcgt tagcatggta    1080
   gtcaggctga aaagctccat tgtccaacaa gacaggcggt tctctgaacc caacatgtca    1140
   ccctcacgag agtgcctcgt gggcccaaca tccaagcaaa agctagcaag gagtgaggac    1200
   agcttcactc tgtcccagga cgcctcctgt tctgaaggcg atgaagctga agatcccttt    1260
   acagaggaag tcttcccagc agtggacagc aaacccaaga gacctgtgga tttgaaaata    1320
   aagaattgga cccaaggttt agcatctcca cagggacaca taaccaaagc tttctccaga    1380
   tcctccccag gcgaatcttt gggcagctca cctgtgcctt ctccatcctg ccccaagaga    1440
   aacttcttca ccagacacca gagtttcaca acaaagacag acaaaaccaa accccagaga    1500
   gaaattagaa agcactccat gtcgttttcc tttgcgtctc acaagaaagt gctgccccga    1560
   acctccagca ttgggtctga gaaatccaaa gacttttcta gagaccaact ccagaaggac    1620
   ttgaggaaag agagccaact ttctggcaga atcgtccagg aaaatgagtc agaaatccaa    1680
   agccaaacat ctctgggctt cagcttgtct ggcacctggg ccctctcagt cgacaacacg    1740
   ttccagttag tggatatgag gaaaccagga agcccaccat cttacgaaga ggccatttat    1800
   taccagacat caggactcac agcctacggt ggccagacag ttgggagtat gaggtcaaga    1860
   atgttcaagc caagcacagc agtgccccct gtgccttctc accatggagg tgacctcagt    1920
   gaagggacac ctggtggaca cagattgtct tctgtgactg agcactggac acacagtcag    1980
   actgtccatg tctctataga aactcagggg agatctgagc tacaccagtt gaggacagtg    2040
   tccgagtcca tgcagaaggc taagctggac tgtcttgggc cacaacacag ccacttagtc    2100
   tttgaggctg accaactctg ctgtgctaga gaatcctaca tttaa                    2145
<210> 3
   <211> 1329
   <212> DNA
   <213> Tagap1 t1 mouse
<400> 3
   atgaagctga taagcagtct cgatggttca aaaacactta atgctaacaa catggagaca      60
   ttaattgaat gtcagtcaga gggtgatatc aaagtgcctc ccctgctgac atcatgtgag     120
   agtgaagaca gcatttgcca gctaattgaa attaagaaga gaaagaaagt gctgtcctgg     180
   ccatctctca tgagaaagct ctctccttca tccgacttct ctgggtcatt ggaaccagag     240
   ctgaaagtgt cgctgtttga ccaacccttg tcgatcatct gtggggagaa cgacacactt     300
   cccagaccca tccaggacat cctcaccatc ctctgcctta aaggtccttc aacggaaggg     360
   atattcagga aagctgccag cgagaaagcc cgcaaggagc tgaaggaggg gcttaactgt     420
   ggggtctcct tgaatctgaa gcagctccct gtgcacctcc tagctgtggt cttcaaggac     480
   ttctttcgag ggatacccct gaagctactc tcctgtgatc tctttgagga ctggatgggc     540
   gccctggaga agcccaccga ggaggacaga atcgaggccc tgaagcaggt tgctggtggc     600
   ctcccccggc ccaaccttct cctgctcagg cacttgctct acgtgctcca cctcatcagc     660
   aagaacgctg aggtcaacaa gatggactcc agcaacctcg ccatctgcat cgggcctaac     720
   atgctcactc tgaagaacga ccagagcctg tccttccagg cccagaagga cctgaacaat     780
   aaggttaaga tcttggtgga attcctcatc gacaactgct ttgagatatt tggggagaac     840
   attcggacgc gttcccgcat cacttctgac gactccctgg aacacactga cagttcagac     900
   gtgtcaactc tgcagaatga ctcggcctat gacagcaatg acccagatgt ggagcccaca     960
   agtggcacag cctctcccaa caggcaactg gagggtccca ctcccacaat ggctggtctg    1020
   gatacccggg gccaccggga cacctgtgag tcaagctcag agtccagcgt tagcatggta    1080
   gtcaggctga aaagctccat tgtccaacaa gacaggcggt tctctgaacc caacatgtca    1140
   ccctcacgag agtgcctcgt gggcccaaca tccaaacaaa agctaacaag gagtgaggac    1200
   agcttcactc tgtcccagga cgcctcctgt tctgaaggcg atgaagctga agatcccttt    1260
   acagaggaag tcttcccagc agtggacagc aaacccaaga gacctgtgga tttgaaaata    1320
   aagaattga                                                            1329
<210> 4
   <211> 2124
   <212> DNA
   <213> Tagap1 t2 mouse
<400> 4
   atgaagctga taagcagtct cgatggttca aaaacactta atgctaacaa catggagaca      60
   ttaattgaat gtcagtcaga gggtgatatc aaagtgcctc ccctgctgac atcatgtgag     120
   agtgaagaca gcatttgcca gctaattgaa attaagaaga gaaaaaaagt gctgtcctgg     180
   ccatctctca tgagaaagct ctctccttca tccgacttct ctgggtcatt ggaaccagag     240
   ctgaaagtgt cgctgtttga ccaacccttg tcgatcatct gtggggagaa cgacacactt     300
   cccagaccca tccaggacat cctcaccatc ctctgcctta aaggtccttc aacggaaggg     360
   atattcagga aagctgccag cgagaaagcc cgcaaggagc tgaaggagga gcttaactgt     420
   ggggtctccg tgaatctgaa gcagctccct gtgcacctcc tagctgtggt cttcaaggac     480
   ttcctccgag ggatacccct gaagctactc tcctgtgatc tctttgagga ctggatgggc     540
   gccctggaga agcccacgga ggcggacaga atcgaggccc tgaagcaggt tgctggcggc     600
   ctcccccggc ccaacctttt cctgctcagg cacttgctct acgtgctcca cctcatcagc     660
   aagaacgctg aggtcaacaa gatggactcc agcaaccttg ccatctgcat cgggcctaac     720
   atgctcactc tgaagaacga ccagagcctg tccttccagg cccagaagga cctgaacaat 780
   aaggttaaga tcttggtgga attcctcatt gacaactgct ttgagatatt tggggagaac 840
   attcggacgc gttcccgcat cacttctgac gactccctgg aacacactga cagttcagac 900
   atgtcaactc tgcagaatga ctcggcctat gacagcaatg acccagatgt ggagcccaca 960
   agtggcacag cctctcccaa caggcaactg gaggatccca ctcccacaat ggctggtctg 1020
   gatacccggg gccaccggga cacctgtgag tcaagctcag agtccagcgt tagcatggta 1080
   gtcaggctga aaagctccat tgtccaacaa gacaggcggt tctctgaacc caacatgtca 1140
   ccctcacgag agtgcctcgt gggcccaaca tccaagcaaa agctaacaag gagtgaggac 1200
   agcttcactc tgtcccagga tgaagctgaa gatcccttta cagaggaagt cttcccagca 1260
   gtggacagca aacccaagag acctgtggat ttgaaaatga agaattggac ccaaggttta 1320
   gcatctccac agggacacat aaccaaagct ttctccagat cctccccagg cgaatctttg 1380
   ggcagctcac ttgtgccttc tccatcctgc cccaagagaa acttcttcac cagacaccag 1440
   agtttcacaa caaagacaga caaaaccaaa ccccagagag aaattagaaa gcactccatg 1500
   tcgttttcct ttgcgtctca caagaaagtg ctgccccgaa cctccagcat tgggtctgag 1560
   aaatccaaag acttttctag agaccaactc cagaaggact tgaggaaaga gagccaactt 1620
   tctggcagaa tcatccagga aaatgagtca gaaatccaaa gccaaacatc tctgggcttc 1680
   agcttgtctg gcacctgggc cctctcagtg gacaacacgt tccagttagt ggatatgagg 1740
   aaaccaggaa gcccaccatc ttacaaagag gccatttatt accagacatc aggactcaga 1800
   gcctacagtg gccagacagt tgggagtatg aggtcaagaa tgttcaagcc aagcacagcg 1860
   gtacctcctg tgccttctca ccatggaggt gacctcagtg aagggacacc tggtggacac 1920
   agattgtctt ctgtgactga gcactggaca cacagtcaga ctgtccatgt ctctatagaa 1980
   actcagggga gatctgagct acaccagttg aggacagtgt ccgagtccat gcagaaggct 2040
   aagctggact gtcttgggcc acaacacagc cacttagtct ttgaggttga ccaactctgc 2100
   tgtgctagag aatcctacat ttaa 2124
<210> 5
   <211> 2124
   <212> DNA
   <213> Tagap1 t3 mouse
<400> 5
   atgaagctga ttagcagtct cgatggttca aaaacactta atgctaacaa catggagaca 60
   ttaattgaat gtcagtcaga gggtgatatc aaagtgcctc ccctgctgac atcatgtgag 120
   agtgaagaca gcatttgcca gctaattgaa attaagaagg gaaaaaaagt gctgtcctgg 180
   ccatctctca tgagaaagct ctctccttca tccgacttct ctgggtcatt ggaaccagag 240
   ctgaaagtgt cgctgtttga ccaacccttg tcgatcatct gtggggagaa cgacacactt    300
   cccagaccca tccaggacat cctcaccatc ctctgcctta aaggtccttc aacggaaggg    360
   atattcagga aagctgccag cgagaaagcc cgcaaggagc tgaaggagga gcttaactgt    420
   ggggtctccg tgaatctgaa gcagctccct gtgcacctcc tagctgtggt cttcaaggac    480
   ttcctccgag ggatacccct gaagctactc tcctgtggtc tctttgagga ctggatgggc    540
   gccctggaga agcccacgga ggaggacaga atcgaggccc tgaagcaggt tgctggtggc    600
   ctcccccggc ccaacctttt cctgctcagg cacttgctct acgtgctcca cctcatcagc    660
   aagaacgctg aggtcaacaa gatggactcc agcaaccttg ccatctgcat cgggcctaac    720
   atgctcactc tgaagaacga ccagagcctg tccttccagg cccagaagga cctgaacaat    780
   aaggttaaga tcttggtgga attcctcatt gacaactgct ttgagatatt tggggagaac    840
   attcggacgc gttcccgcat cacttctgac gactccctgg aacacactga cagttcagac    900
   atgtcaactc tgcagaatga ctcggcctat gacagcaatg acccagatgt ggagcccaca    960
   agtggcacag cctctcccaa caggcagctg gaggatccca ctcccacaat ggctggtctg   1020
   gatacccggg gccaccggga cacctgtgag tcaagctcag agtccagcgt tagcatggta   1080
   gtcaggctga aaagctccat tgtccaacaa gacaggcggt tctctgaacc caacatgtca   1140
   ccctcacgag agtgcctcgt gggcccaaca tccaagcaaa agctaacaag gagtgaggac   1200
   agcttcactc tgtcccagga tgaagctgaa gatcccttta cagaggaagt cttcccagca   1260
   gtggacagca aacccaagag acctgtggat ttgaaaatga agaattggac ccaaggttta   1320
   gcatctccac agggacacat aaccaaagct ttctccagat cctccccagg cgaatctttg   1380
   ggcagctcac ttgtgccttc tccatcctgc cccaagagaa acttcttcac cagacaccag   1440
   agtttcacaa caaagacaga caaaaccaaa ccccagagag aaattagaaa gcactccatg   1500
   tcgttttcct ttgcgtctca caagaaagtg ctgccccgaa cctccagcat tgggtctgag   1560
   aaatccaaag acttttctag agaccaactc cagaaggact tgaggaaaga gagccaactt   1620
   tctggcagaa tcatccagga aaatgagtca gaaatccaaa gccaaacatc tctgggcttc   1680
   agcttgtctg gcacctgggc cctctcagtg gacaacacgt tccagttagt ggatatgagg   1740
   aaaccaggaa gcccaccatc ttacaaagag gccatttatt accagacatc aggactcaga   1800
   gcctacagtg gccagacagt tgggagtatg aggtcaagaa tgttcaagcc aagcacagcg   1860
   gtaccccctg tgccttctca ccatggaggt gacctcagtg aagggacacc tggtggacac   1920
   agattgtctt ctgtgactga gcactggaca cacagtcaga ctgtccatgt ctctatagaa   1980
   actcagggga gatctgagct acaccagttg aggacagtgt ccgagtccat gcagaaggct   2040
   aagctggact gtcttgggcc acaacacagc cacttagtct ttgaggttga ccaactctgc   2100
   tgtgctagag aatcctacat ttaa                                           2124
<210> 6
   <211> 2124
   <212> DNA
   <213> Tagap1 t4 mouse
<400> 6
   atgaagctga ttagcagtct cgatggttca aaaacactta atgctaacaa catggagaca   60
   ttaattgaat gtcagtcaga gggtgatatc aaagtgcctc ccctgctgac atcatgtgag  120
   agtgaagaca gcatttgcca gctaattgaa attaagaaga gaaagaaagt gctgtcctgg  180
   ccatctctca tgagaaagct ctctccttca tccgacttct ctgggtcatt ggaaccagag  240
   ctgaaagtgt cgctgtttga ccaacccttg tcgatcatct gtggggagaa cgacacactt  300
   cccagaccca tccaggacat cctcaccatc ctctgcctta aaggtccttc aacggaaggg  360
   atattcagga aagctgccag cgagaaagcc cgcaaggagc tgaaggagga gcttaactgt  420
   ggggtctccg tgaatctgaa gcagctccct gtgcacctcc tagctgtggt cttcaaggac  480
   ttcctccgag ggatacccct gaagctactc tcctgtgatc tctttgagga ctggatgggc  540
   gccctggaga agcccacgga ggaggacaga atcgaggccc tgaagcaggt tgctggtggc  600
   ctcccccggc ccaacctttt cctgctcagg cacttgctct acgtgctcca cctcatcagc  660
   aagaacgctg aggtcaacaa gatggactcc agcaaccttg ccatctgcat cgggcctaac  720
   atgctcactc tgaagaacga ccagagcctg tccttccagg cccagaagga cctgaacaat  780
   aaggttaaga tcttggtgga attcctcatt gacaactgct ttgagatatt tggggagaac  840
   attcggacgc gttcccgcat cacttctgac gactccctgg aacacactga cagttcagac  900
   atgtcaactc tgcagaatga ctcggcctat gacagcaatg acccagatgt ggagcccaca  960
   agtggcacag cctctcccaa caggcaactg gaggatccca ctcccacaat ggctggtctg 1020
   gatacccggg gccaccggga cacctgtgag tcaagctcag agtccagcgt tagcatggta 1080
   gtcaggctga aaagctccat tgtccaacaa gacaggcggt tctctgaacc caacatgtca 1140
   ccctcacgag agtgcctcgt gggcccaaca tccaagcaaa agctaacaag gagtgaggac 1200
   agcttcactc tgtcccagga tgaagctgaa gatcccttta cagaggaagt cttcccagca 1260
   gtggacagca aacccaagag acctgtggat ttgaaaatga agaattggac ccaaggttta 1320
   gcatctccac agggacacat aaccaaagct ttctccagat cctccccagg cgaatctttg 1380
   ggcagctcac ttgtgccttc tccatcctgc cccaagagaa acttcttcac cagacaccag 1440
   agtttcacaa caaagacaga caaaaccaaa ccccagagag aaattagaaa gcactccatg 1500
   tcgttttcct ttgcgtctca caagaaagtg ctgccccgaa cctccagcat tgggtctgag 1560
   aaatccgaag acttttctag agaccaactc cagaaggact tgaggaaaga gagccaactt 1620
   tctggcagaa tcatccagga aaatgagtca gaaatccaaa gccaaacatc tctgggcttc 1680
   agcttgtctg gcacccgggc cctctcagtg gacaacacgt tccagttagt ggatatgagg 1740
   aaaccaggaa gcccaccatc ttacaaagag gccatttatt accagacatc aggactcaga 1800
   gcctacagtg gccagacagt tgggagtatg aggtcaagaa tgttcaagcc aagcacagcg 1860
   gtaccccctg tgccttctca ccatggaggt gacctcagtg aagggacacc tggtggacac 1920
   agattgtctt ctgtgactga gcactggaca cacagtcaga ctgtccatgt ctctatagaa 1980
   actcagggga gatctgagct acaccagttg aggacagtgt ccgagtccat gcagaaggct 2040
   aagctggact gtcttgggcc acaacacagc cacttagtct ttgaggttga ccaactctgc 2100
   tgtgctagag aatcctacat ttaa 2124
<210> 7
   <211> 3363
   <212> DNA
   <213> Tagap1 homo sapiens; Accession number NM_054114
<400> 7
   gacatagctg ccctaaaagg aatgaggaag cgagagctct ccagtgtctg gctggctccg 60
   tccgtgtgac agcccatgat gttctttccg gtctctgtaa tattctgaat ttccacctgc 120
   ccgccccttc gcttataatg cagagcatgt gaagggagac cggctcggtc tctctctctc 180
   ccagtggact agaaggagca gagagttatg ctgtttctcc cattctttac agctcaccgg 240
   atgtaaaaga actctggcta gagaccctcc aaggacagag gcacagccac acgggagtga 300
   aatccacccc tggacagtca gccgcaatac tgatgaagct gagaagcagc cacaatgctt 360
   caaaaacact aaacgccaat aatatggaga cactaatcga atgtcaatca gagggtgata 420
   tcaaggaaca tcccctgttg gcatcatgtg agagtgaaga cagtatttgc cagctcattg 480
   aagttaagaa gagaaagaag gtgctgtcct ggccctttct catgagaagg ctctcccctg 540
   catcagattt ttctggggct ttggagacag acttgaaagc atcgctattt gatcagccct 600
   tgtcaattat ctgcggtgac agtgacacac tccccagacc catccaggac attctcacta 660
   ttctatgcct taaaggccct tcaacggaag ggatattcag gagagcagcc aacgagaaag 720
   cccgtaagga gctgaaggag gagctcaact ctggggatgc ggtggatctg gagaggctcc 780
   ccgtgcacct cctcgctgtg gtctttaagg acttcctcag aagtatcccc cggaagctac 840
   tttcaagcga cctctttgag gagtggatgg gtgctctgga gatgcaggac gaggaggaca 900
   gaatcgaggc cctgaaacag gttgcagata agctcccccg gcccaacctc ctgctactca 960
   agcacttggt ctatgtgctg cacctcatca gcaagaactc tgaggtgaac aggatggact 1020
   ccagcaatct ggccatctgc attggaccca acatgctcac cctggagaat gaccagagcc 1080
   tgtcatttga agcccagaag gacctgaaca acaaggtgaa gacactggtg gaattcctca 1140
   ttgataactg ctttgaaata tttggggaga acattccagt gcattccagt atcacttctg 1200
   atgactccct ggagcacact gacagttcag atgtgtcgac cctgcagaat gactcagcct 1260
   acgacagcaa cgaccctgat gtggaatcca acagcagcag tggcatcagc tctcccagca 1320
   ggcagcccca ggtgcccatg gccacagctg ctggcttgga tagcgcgggc ccacaggatg 1380
   cccgagaggt cagcccagag cccattgtga gcaccgtggc caggctgaaa agctccctcg 1440
   cacagcccga taggagatac tcagagccca gcatgccatc ctcccaggag tgcctcgaga 1500
   gccgggtgac aaaccaaaca ctaacaaaga gtgaagggga cttccccgtg ccccgggtag 1560
   gctctcgttt ggaaagtgag gaggctgaag acccatttcc agaggaggtc ttccctgcag 1620
   tgcaaggcaa aaccaagagg ccggtggacc tgaagatcaa gaacttggcc ccgggttcgg 1680
   tgctcccgcg ggcactggtt ctcaaagcct tctccagcag ctcgctggac gcgtcctctg 1740
   acagctcgcc cgtggcttct ccttccagtc ccaaaagaaa tttcttcagc agacatcagt 1800
   ctttcaccac aaagacagag aaaggcaagc ccagccgaga aattaaaaag cactccatgt 1860
   ctttcacctt tgcccctcac aaaaaagtgc tgaccaaaaa cctcagcgcg ggctctggga 1920
   aatcgcaaga ctttaccagg gaccacgtcc cgaggggtgt cagaaaggaa agccagcttg 1980
   ccggccgaat cgtgcaggaa aatgggtgtg aaacccacaa ccaaacagcc cgcggcttct 2040
   gcctgagacc ccacgccctc tcggtggatg atgtgttcca gggagctgac tgggagaggc 2100
   ctggaagccc accctcttat gaagaggcca tgcagggccc ggcagccaga ctagtggcct 2160
   ccgagagcca gaccgtgggg agcatgacgg tggggagcat gagggcgagg atgctggagg 2220
   cgcactgcct cctaccccct cttccacctg ctcaccacgt agaggactca agacacaggg 2280
   gcagcaaaga gccactccct ggccacggac tctctcccct gcctgagcga tggaaacaga 2340
   gcagaactgt ccatgcttct ggggactctc tggggcacgt gtctggccca gggagacctg 2400
   agctcctccc gctgaggacc gtctccgagt ccgtgcagag gaataagcgg gactgtctcg 2460
   tgcgacgatg tagccagccg gtctttgagg ctgaccaatt ccaatatgcc aaagaatcgt 2520
   atatttagga gggaggccat acgccatgcc atagcttgtg ctatctgtaa atatgagact 2580
   tgtaaagaac tgcctgtaga ttgtttttaa aaggtcttga ataagctcct tgagaaagtt 2640
   gtggaaagcc ctcctcagtg aggatagcta caccatggcc atggcgcatc agatagtctc 2700
   tgtgtacctg gatttgtgca atatgtaaaa atgtatcaaa tgtattatag ataaggtgtt 2760
   aggtgcaaag gatgtctaat aatccctgca cacgttttga acttgcagtg aagtacactg 2820
   ctgttccttg cttcctgggg cacttttctc ttggttagtg tttaaaaatt atcttcgctt 2880
   ttttaatgtg gcctcaaatg tcatgccaat tttcacatct tccacaaact ccatttaggg 2940
   agaaatgttt aaatctctgg tataagttta ctccatacca gagtaaacta tatattactc 3000
   tatataagca gtcttgcaat aactaatcac caccatagaa gaaagaaaca gactgcaagg 3060
   aacagagttg agtgtctgga gtcatcaaag gcattaaaaa ctccagtaaa agctggggcc 3120
   gtagcaaaaa tcatgaaaaa cacttcaacg tgtcctttca atcatccaat taaatgtggg 3180
   tagattaatg aaaatgtatt acatcaatat taactcatct atagcacttt gagtatcttt 3240
   gtagttcatg atatcctatc ctataatgtg gaggtaaatg attttatatg cattgggggt 3300
   catatataaa acttcaatgt aatttcacta caataaattg ccttccttat ttgaaagtaa 3360
   aaa 3363
<210> 8
   <211> 3027
   <212> DNA
   <213> Tagap1 Rattus norvegicus; Accession number XM_217791
<400> 8
   atggaattgt taagtgctcg cctgccccga gcaggagaaa catttgtccc aatatctgag 60
   catcagcact ggaagccaga gaacgcccac aacaacggag ctcagtgttt cccagagctc 120
   acgttaggcc ggacgcatca aggcaacaag gtcctggaca gtgagtgggt cacacagatg 180
   ctaaggacca cactagcctt tcggagggca ctttatatca agcttacagc atccattcta 240
   gaacttcaga acaggaagaa cgggaccttc catgtacttc ggggtgagaa ttacaagttc 300
   agcagtgttg aacacagttc tctggctggc atacagcagg tgttcagcaa gcattcccag 360
   tctgtccaag actgttctga cgttgaaaaa gttgtagcaa tagttgtggc tgagaaacct 420
   ggaatctcat tcaagaaccc accaccggct ttaatggctg gccccaggga cttggcagcc 480
   gcagagctgg agcgaggaga agcaaagaga cctccggaag agatccagtg cgatggggtg 540
   tcccagataa cagctcctct ggaatggagg gaaagcaggt tctatgagaa tagtgtgagt 600
   gataaaacag atgaattcct gtgcccacag atccgctaca agagctccac aaggggtcgc 660
   tgcaggcgcc acgcaggggg tcggtacatg cgcctagcaa ggcccctgcc actggataac 720
   aaccacctgg agactcatcg tgtgaggaca gtgctagtgt ctattttgtc cagacccctg 780
   agtcatcctg tgagggacgc tgcttcactt gggctcactc aaacacagaa ccaaagcctc 840
   gaggtagaga aaattcccct tggactgtca gccccgaatc caatgaagtt gataagcagt 900
   ctcgatggtg caaaaacgct taatgccaac aacatggaca cattaattga atgtcagtca 960
   gagggcgata tcaaggtgct tccattgctg acgtcatgtg agagtgaaga cagcatttgc 1020
   cagctaattg aagttaagaa gagaaagaaa gtgctgtcct ggccatctct catgagaaag 1080
   ctctctcctt caccagactt ctctgggtca ttggaaccag agctgaaagt gtcgctgttt 1140
   gatcaaccct tgtcaatcat ctgtaaggag aatgacacac tccctagacc catccaggac 1200
   atcctcacca tcctctgcct taaaggtcct tcaactgaag gaatattcag gaaagcagcc 1260
   agcgagaaag cccgcaagga gctgaaggag gagcttaact gtgggggctc tgtgaatctg 1320
   aaccagctcc ctgtgcacct cctggctgtg gtcttcaagg acttcctccg aggaatcccc 1380
   ctgaagctgc tctcctgtga cctctttgag gactggatgg gagccctgga gaagcccagt 1440
   gaggaggaca ggatcgaggc cctgaagcag gttgctgatc ggctcccccg gcccaacctc 1500
   cttctgctca ggaccttagt ctacgtgcta caccttatca gcaagaacgc cgaggtcaac 1560
   aagatggact ccagcaacct ggccatctgc atcggaccca acatgctcac actgaagaat 1620
   gaccagagcc tgtccttcca ggcccagagg gacctgaaca ataaggttaa gatcttggtg 1680
   gaattcctca ttgacaactg ccttgaaata tttggggaga acattccgac acatccccgc 1740
   atcacttctg atgactctct ggaacacact gacagctcag acgtgtcgac tctgcagaac 1800
   gactcagcct atgacagcaa tgacccggat gtagagcccg cgaatggagt tgcctctccc 1860
   tgcaggcagt tggagggtcc ctctaccaca gtggctggca tggatacccg ggggccccgg 1920
   aacacctgtg agtcgagctc agaatccagt gtcagcatgg tagccaggct gaaaagctcc 1980
   attggccagc aagacaggcg tttctctgaa cccaacatgc caccctcacg agagtgcttc 2040
   gtgggcccga taaccaagca aaagctaacg aggagcgagg acagcttcgt tctgccccag 2100
   gaagcctcct gttctgaagg caatgaagcc gaagatccct ttacagagga agtcttccca 2160
   gcagttgaag gcaaacccat gagaccagtg gatttgaaga taaagaactt gacccaaggt 2220
   ttagcatctc cacagggacc tgcaaacaaa gctttctcca gcttctccgc gggggaatct 2280
   ttggacagct cacctgtgcc ttctccatcc tgtcccaaga gaaacttctt caccagacac 2340
   cagagtttca ccacaaagac ggacaagacc aagccccaga gagaaattag aaagcactcc 2400
   atgtcatttt cctttgcgtc tcacaagaaa gtgctgcccc ggacctccag cattgggtct 2460
   gagaaatcca aagacttctc tagagaccag ctccagaagg acttgaggaa agagagccag 2520
   cttgccggca gaatcatccg ggaaaatgag tccgaaatcc aaagccaaac aaatctgggc 2580
   tccagcttgt ctggaacctg ggccctctca gttgataaca cgttccagtt cattgatgtg 2640
   aggaagccag gaagcccacc atcttatgaa gaggccattt attaccacac atcaggactc 2700
   acagcctaca gtggccagac agttgggagt atgagagcaa gaatgttgaa gcagagcatg 2760
   gcggtgcccc ctgtgccttc tcaccatgaa ggtgagctca gtgaagggat acctggtgga 2820
   cacagatcgt cttccgtgac tgagcactgg acacagagtc agactgtcca tgtctctgta 2880
   gaaactcggg ggagatctga gctacatcga ttgaggacag tgtctgagtc cgtgcagagg 2940
   gctaagctgg actaccttgg gcagcaacgc agccacttgg tctttgaggt tgaccaactc 3000
   cgatgtgcta aagaatccta catttag 3027
<210> 9
   <211> 2181
   <212> DNA
   <213> Fgd2 mouse transcript variant 3; Accession number AF017368
<400> 9
   atggagcgag cctgtgagaa gcaggactca gtgtgcaacc tggtggctgt gtttgagaac 60
   aacaggactc cgggagaagc acctggatcc catagccttg aggaccagcc ccatatccct 120
   gaacaccagc tgtccctgtc cccagagcct tgggaggcac cccctgtcaa ggaggccttg 180
   aagtctgaat tccggccagt gagcaggaca tatctgagtt ccctcaagaa caagctatcg 240
   agtggggctt ggaggagatc ctgtcagcct ggggtcagcc cggggccaga gacacaggaa 300
   cctgaggaga agagggtcgt gcgagagctt ctggagacgg agcaggccta tgtggctcgc 360
   ctgcacctgc ttgaccaggt gttcttccag gagctgctga gggaggcagg ccgcagcaag 420
   gccttccctg aggacgtggt gaagctcatt ttctccaaca tctcctccat ctatcgtttc 480
   cacgcccagt tcttccttcc cgagctgcag cggcgcgtgg atgactgggc agccacgccc 540
   cgcattgggg atgtgatcca gaagctggcc ccgtttctga aaatgtacag cgagtacgtg 600
   aagaactttg agcgggccgc ggaactgctg gccacgtgga tggacaagtc tcagcccttc 660
   caggaggtgg tcacccgcat ccagtgcagc gaggcctcca gcagcctgac cctgcagcac 720
   cacatgttgg agcctgtgca aagaatcccg cggtacgaac tgctgctcaa ggaatatgtg 780
   cagaagctgc cagcccaggc cccagacctc gaagatgccc agagagcact ggacatgatc 840
   ttctcagctg cacagcactc caatgcagcc attgcagaga tggagcggct gcagggcctg 900
   tgggatgtgt accagcgcct gggcttggag gatgacatcg tggacccctc caacaccctg 960
   ctccgagagg gccctgttct caagatctct ttccgccgca gcgacccaat ggaacgctac 1020
   ctggttttgt tcaacaacat gcttctgtat tgtgtacccc gagtcctcca agtgggtgcc 1080
   cagttccagg tgcggactcg catcgatgtg gccggcatga aggtgcggga gctgaccgat 1140
   gctgagttcc cacactcctt cctggtgtcc ggaaagcagc gcacactgga gctgcaggcc 1200
   cggtcccgag atgaaatggt ttcctggatg caggcctgcc aggcagccat tgaccaggtt 1260
   gagaagcgga gtgagacctt caaggctgct gtccagggac ctcaggggga cacgcaggag 1320
   cccaagccac aggtagagga gctgggtctc cgagcgcctc agtgggtccg ggacaagatg 1380
   gtgaccatgt gcatgcgctg ccaggagccc ttcaatgccc tgactcgtcg gcgccaccac 1440
   tgccgggctt gcggctatgt agtgtgtgcc aagtgctctg actaccgtgc ggagctgaaa 1500
   tatgacagca acaggcccaa ccgagtctgc ctgacctgct acacatttct cactggaaac 1560
   gtactccctc aaggcaagga ggacaagagg cggggcatct tggagaaaga ggcctcagca 1620
   gcacccgagc agagtctggt gtgcagcttc ctgcagctca taggggacaa gtgcagcagg 1680
   agccttcccc ggagctggtg tgtgatcccc cgggatgacc cccttgtcct gtatgtctat 1740
   gcagcccccc aggacacaaa ggctcacacc tccatccccc tactgggcta tcaggtgatt 1800
   tcagggcccc aggggacctc gggttttcca gctgcaacag tcaggcgagc agtacacctt 1860
   caaggccgag tctgtggagc tgcagggccg ctgggtgaca gctatcaagc gtgcgccagt 1920
   ggcacccctg aggacctgac gaagaagatg tgtctgactg aaccagcagc cagctgctca 1980
   tcgagagtcc atgacagcct tcccaggcct accccatggt tctatcactt caccccaagc 2040
   tgggctaccc ctgacccacc tgatgtctat accgagacaa ttcatcctga ctctgtatcc 2100
   agcagacaca ggccctttcc ttcaggaaga taccaccaag tgtcccagct ggctggagag 2160
   ggggcggata tccctggatc a 2181
<210> 10
   <211> 1965
   <212> DNA
   <213> Fgd2 mouse transcript variant 1; Accession number AK042260
<400> 10
   atggagcgag cctgtgagaa gcaggactca gtgtgcaacc tggtggctgt gtttgagaac 60
   aacaggactc cgggagaagc acctggatcc catagccttg aggaccagcc ccatagccct 120
   gaacaccagc tgtccctgtc cccagagcct tgggaggcac cccctgtcaa ggaggccttg 180
   aagtctgaat tccggccagt gagcaggaca tatctgagtt ccctcaagaa caagttatcg 240
   agtggggctt ggaggagatc ctgtcagcct ggggtcagcc cggggccaga gacacaggaa 300
   cctgaggaga agagggtcgt gcgagagctt ctggagacgg agcaggccta tgtggctcgc 360
   ctgcacctgc ttgaccaggt gttcttccag gagctgctga gggaggcagg ccgcagcaag 420
   gccttccctg aggacgtggt gaagctcatt ttctccaaca tctcctccat ctatcgtttc 480
   cacgcccagt tcttccttcc cgagctgcag cggcgcgtgg atgactgggc agccacgccc 540
   cgcattgggg atgtgatcca gaagctggcc ccgtttctga aaatgtacag cgagtacgtg 600
   aagaactttg agcgggccgc ggaactgctg gccacgtgga tggacaagtc tcagcccttc 660
   caggaggtgg tcacccgcat ccagtgcagc gaggcctcca gcagcctgac cctgcagcac 720
   cacatgttgg agcctgtgca aagaatcccg cggtacgaac tgctgctcaa ggaatatgtg 780
   cagaagctgc cagcccaggc cccagacctc gaagatgccc agagagcact ggacatgatc 840
   ttctcagctg cacagcactc caatgcagcc attgcagaga tggagcggct gcagggcctg 900
   tgggatgtgt accagcgcct gggcttggag gatgacatcg tggacccctc caacaccctg 960
   ctccgagagg gccctgttct caagatctct ttccgccgca gcgacccaat ggaacgctac 1020
   ctggttttgt tcaacaacat gcttctgtat tgtgtacccc gagtcctcca agtgggtgcc 1080
   cagttccagg tgcggactcg catcgatgtg gccggcatga aggtgcggga gctgaccgat 1140
   gctgagttcc cacactcctt cctggtgtcc ggaaagcagc gcacactgga gctgcaggcc 1200
   cggtcccgag atgaaatggt ttcctggatg caggcctgcc aggcagccat tgaccaggtt 1260
   gagaagcgga gtgagacctt caaggctgct gtccagggac ctcaggggga cacgcaggag 1320
   cccaagccac aggtagagga gctgggtctc cgagcgcctc agtgggtccg ggacaagatg 1380
   gtgaccatgt gcatgcgctg ccaggagccc ttcaatgccc tgactcgtcg gcgccaccac 1440
   tgccgggctt gcggctatgt agtgtgtgcc aagtgctctg actaccgtgc ggagctgaaa 1500
   tatgacagca acaggcccaa ccgagtctgc ctgacctgct acacatttct cactggaaac 1560
   gtactccctc aaggcaagga ggacaagagg cggggcatct tggagaaaga ggcctcagca   1620
   gcacccgagc agagtctggt gtgcagcttc ctgcagctca taggggacaa gtgcagcagg   1680
   agccttcccc ggagctggtg tgtgatcccc cgggatgacc cccttgtcct gtatgtctat   1740
   gcagcccccc aggacacaaa ggctcacacc tccatccccc tactgggcta tcaggtgatt   1800
   tcagggcccc agggggaccc tcgggttttc cagctgcaac agtcaggcca gcagtacacc   1860
   ttcaaggccg agtctgtgga gctgcagggc cgctgggtga cagctatcaa gcgtgcggcc   1920
   agtggccgga cccctgaggg acctgacgaa gaagatgtgt ctgac                   1965
<210> 11
   <211> 1387
   <212> DNA
   <213> Fgd2 mouse transcript variant 2; Accession number BC021845
<400> 11
<210> 12
   <211> 1968
   <212> DNA
   <213> Fgd2 mouse transcript variant 1 t6/tw5
<400> 12
   atggagcgag cctgtgagaa gcaggactca gtgtgcaacc tggtggctgt gtttgagaac     60
   aacagcagga ctccgggaga agcacctgga tcccatagcc ttgaggacca gctccatagc    120
   cctgaacacc agctgtccct gtccccagag ccttgggagg caccccctgt caaggaggcc    180
   ttgaagtctg aattccggcc agtgagcagg acatatctga gttccctcaa gaacaagcta    240
   tcgagtgggg cttggaggag atcctgccag cctggggtca gcccggggcc agagacacag    300
   gaacctgagg agaagagggt cgtgcgagag cttctggaga cggagcaggc ctatgtggct    360
   cgcctgcacc tgcttgacca ggtgttcttc caggagctgc tgagggaggc aggccgcagc    420
   aaggccttcc ctgaggacgt ggtgaagctc attttctcca acatctcctc catctatcgt    480
   ttccacgccc agttcttcct tcccgagctg cagcggcgcg tggatgactg ggcagccacg    540
   ccccgcattg gggatgtgat ccagaagctg gccccgtttc tgaaaatgta cagcgagtac    600
   gtgaagaact ttgagcgggc cgcggaactg ctggccacgt ggatggacaa gtctcagccc    660
   ttccaggagg tggtcacccg catccagtgc agcgaggcct cgggcagcct gaccctgcag    720
   caccacatgt tggagcctgt gcaaagaatc ccgcggtacg aactgctgct caaggaatat    780
   gtgcagaagc tgccagccca ggccccagac ctcgaagatg cccagagagc actggacatg    840
   atcttctcag ctgcacagca ctccaatgca gccattgcag agatggagcg gctgcagggc    900
   ctgtgggatg tgtaccagcg cctgggcttg gaggatgaca tcgtggaccc ctccaacacc    960
   ctgctccgag agggccctgt tctcaagatc tctttccgcc gcagcgaccc aatggaacgc   1020
   tacctggttt tgttcaacaa catgcttctg tattgtgtac cccgagtcct ccaagtgggt   1080
   gcccagttcc aggtgcggac tcgcatcgat gtggccggca tgaaggtgcg ggagctgacc   1140
   gatgctgagt tcccacactc cttcctggtg tccggaaagc agcgcacact ggagctgcag   1200
   gcccggtccc gagatgaaat ggtttcctgg atgcaggcct gccaggcagc cattgaccag   1260
   gttgagaagc ggagtgagac cttcaaggct gctgtccagg gacctcaggg ggacacgcag   1320
   gagcccaagc cacaggtaga ggagctgggt ctccgagcgc ctcagtgggt ccgggacaag   1380
   atggtgacca tgtgcatgcg ttgccaggag cccttcaatg ccctgactcg tcggcgccac   1440
   cactgccggg cttgcggcta tgtagtgtgt gccaagtgct ctgactaccg tgcggagctg   1500
   aaatatgaca gcaacaggcc caaccgagtc tgcctgacct gctacacatt tctcactgga   1560
   aacgtactcc ctcaaggcaa ggaggacaag aggcggggca tcttggagaa agaggcctca 1620
   gcagcacccg agcagagtct ggtgtgcagc ttcctgcagc tcataggaga caagtgcagc 1680
   aggagccttc cccggagctg gtgtgtgatc ccccgggatg acccccttgt gctgtatgtc 1740
   tatgcagccc cccaggacac aaaggctcac acctccatcc ccctactggg ctatcaggtg 1800
   atttcagggc cccaggggga ccctcgggtt ttccagctgc aacagtcagg ccagcagtac 1860
   accttcaagg ccgagtctgt ggagctgcag ggccgctggg tgacagctat caagcgtgcg 1920
   gccagtggcc ggacccctga gggacctgac gaagaagatg tgtctgac 1968
<210> 13
   <211> 5756
   <212> DNA
   <213> TIAM2 mouse wildtype; Accession number BC067048
<400> 13
   cggagctaga gaagccttga ccttcccggc tcagcgagtg gagaacaaag tgacggagaa 60
   aacttcgcgg agtgtgccct gagtccccac ggggcctcgc tcaggggcgc agagccgggc 120
   tcgaccggtc ctctgtgttg aaacttggac cgaggatcac ttcatggatt tgcctcacgt 180
   acctatgttt gttaatttcc tgtgtcgttc ccaagatcac gtaaaaccct ctgagcatta 240
   ttgacctccc tcccagcaaa agccaggctg cagtgtaatt tagaccatgg ctcatattga 300
   tacaactgtt ctcaattttg gttaaaatgg ggaactccga gagtcaatat accttccaag 360
   gatccaagaa tcatagtaat actgtcactg gtgctaagca aaagccttgc tctctgaaaa 420
   tacgcagcgt tcatgcaaaa gacgagaagt ccttacatgg ttggactcat gggagcagcg 480
   gtgcaggcta caagtccagg tccctagccc gaagctgcct ttctcacttt aagaatcacc 540
   agccttacgc caccagactc agtggaccca catgtaaagt ctcaaagggc accacctact 600
   ctaagcacag agcaaatacc ccgggaaatg atttccaggg caacagtggt gctttcttac 660
   ctgagaatgg cttccactat gttgaccgcg agtcagagga aagccatatc acctccaatg 720
   ggcaccttct cacctgctat gggagaaagg aaagcctcgc ctccactcct ccaggcgagg 780
   accacaggag ccccagggtg ctcatcaaga ccctgggaaa gctagacggg tgtttaagag 840
   tcgagttcca caacggtggc aacccccaca aggggacctc cgaggacccc agtggacctg 900
   tacggctgct gagatactcc cctaccttag catcggaaac ctgcccggtg cgggaaacca 960
   ggcggcattc cgctgcaggc tccccatcca gccagcggcc ctctcccact gactctcgcc 1020
   tgcgctccag caaaggcagc tccctgagct cagagtcatc ctggtatgac tccccctggg 1080
   gcaacgctgg ggaggtgagc gaggtggagg gctccttcct ggctcccagc actccagacc 1140
   ccagcctccc cagcagcttc ccacccagtg acaccaaaaa gcctttcaac caaagctctt 1200
   ccctctcctc cctccgggaa ttgtacaaag atcccaacct ggggtgccgc tcaccttccg 1260
   gcacctgcct ttcttccaat gagtacatca gctctcaagt cagcctgaac aaccgagtct 1320
   cctttgcgtc tgacatggat gtgccctcca gggtggatca cagggatccc ctgcactata 1380
   gttcctttac tctcccctgt cgcaagtcca aagccttaac tgaagatgca gctaagaaag 1440
   acaccctcaa agccagaatg cggcgcttca gtgactggac aggaagcctc tccaggaaga 1500
   agaggaaact gcaggaaccc aggtccatgg agggcagtga gtactttgat agccactcag 1560
   atggactgaa tgcagaaggg caggtgcccg cgcagacatc ttccttactg tggtcagggg 1620
   gctcggctca gaccctgcct cacagaagcg aatccactca cgctatcagc gtcgatcccc 1680
   tccgacagaa catctatgag aatttcatgc gagagctcga aatgagcagg agcaacacag 1740
   aacacgtgga aacttccaca gagaccatgg agtccagcag cgagtctgtc agctcactgg 1800
   agcagctgga tctgctcttt gagaaggagc agggagtggt ccggaaagcc gggtggctct 1860
   tcttcaaacc ccttgtcacc ttgcagaagg agaggaaact ggagctggtg gctcggagga 1920
   agtggaaaca atactgggtg accctgaaag gctgtactct gctgttttat gagacctatg 1980
   gaaagaattc cacagagcag aatagtgccc cacggtgtgc cctctttgca gaggacagca 2040
   tcgtgcagtc tgtcccagag catcccaaga aggaacacgt gttctgcctg agtaactcct 2100
   gtggggacgt ctacctattc caggccacta gccagacaga tctggaaaac tgggtcacag 2160
   ccatccactc gtgcgcatcc ctctttgcaa agaagcacgg aaaggaggac acggtgcgac 2220
   tgctaaagag ccagaccaga agcctgcttc agaagataga catggatagc aagatgaaga 2280
   agatggcaga gttgcagctg tctgtggtga gcgaccccaa gaacaggaag gccatcgaga 2340
   atcagatccg gcaatgggag cagaatctgg aaaaattcca catggacctg ttccgcatgc 2400
   gctgctattt ggcgagctta caaggtgggg agttaccaaa tcccaagagt ctccttgctg 2460
   ccaccagccg cccctccaag ctggctcttg gcaggctggg cgtcctgtct gtttcgtctt 2520
   tccatgctct ggtgtgttcc agagatgatt ccactctcag gaaaagaaca ctttccctta 2580
   cccagagagg aaaaagcaag aaaggcatat tttcttcatt gaaaggtctg gacaccctag 2640
   caagaaaggg tagggagaag agagcttcca taactcagat gtttgattct agccacagcc 2700
   atggatttct tggaactcag ctacctcaaa agtccactaa ctccaacaag gcccatgacc 2760
   tgcatctgta tggctccgca gtagacagcg cgctgcgaga cagcatgtgg gaagtccaga 2820
   cttatgtcca cttccaggat aacgaaggag ttactgtgac catcaagcca gagcacaggg 2880
   tggaagatgt tctggctttg gtgtgcaaga tgagacagtt ggaacccact cactatggtc 2940
   ttcagctccg aaaggtggtc gataaaagtg tggagtggtg tgtgcccgcg ctgtatgaat 3000
   acatgcaaga gcaggtttat gatgaaatcg aagttttccc actcagtgtg tatgacgtgc 3060
   agctaaccaa gactggggac atgactgact ttgggtttgc agtcacagtt caggtggacg 3120
   aacaccagca tctcaaccgg atatttatca gtgatgttct ccctgacagc ctggcatacg 3180
   gaggagggct gagaaagggc aacgaaatca caagcttaaa tggggaacca gtgtctgacc 3240
   ttgacatcca gcagatggag gctttgtttt ctgagaagag cgttggcctc actctagtcg 3300
   cccggcctgt gaccacaaga cgaaccctgt gtgcttcctg gtcagacagt gacctgttct 3360
   ccagggacca gaaaagtctg ccgccctctc ccaaccagtc ccagctgctg gaggaattcc 3420
   tggataactt tagaaaaacc gccacgagtg atttcagcaa tgtccctgag atcacaactg 3480
   gcttgaagag gagccagaca gaaggcaccc tggatcaagt gccccacagg gaaaagatgg 3540
   agcagacatt cctgagcgct gaccagattg cggagctctg cagggacttg aacaacaccc 3600
   acaccaacag tatggaagca ccaacagaga gccatgaccc acctcccagg cctctggctc 3660
   gtcacctctc agatgcagat cgcctccgga aagtcatcca ggagcttgta gacacagaga 3720
   agtcttacgt gaaggatctg agctgcctct ttgaactata cttggagcca cttcagaatg 3780
   agacctttct tacccaagat gagatggagt cactttttgg gagcctgcca gagatgctgg 3840
   agtttcaaaa ggtgttcctg gagacgttgg aggatgcgat ctccgcttcc tcggacttta 3900
   gtgtcctgga aaccccctca cagtttcgaa aattgctgtt ctcccttgga ggttctttcc 3960
   tctactatgc ggatcacttt aagctataca gtgggttctg tgccaaccac attaaagtac 4020
   agagggttct agagcgagct aaaacggaca aggccttcaa ggcttttctg gatgcccgaa 4080
   atcccaccaa gcagcactcc tccacgctgg agtcctatct catcaagcct gttcagagag 4140
   tgctcaagta tcctctgctt ctcaaggagc tagtgtcact gactgaccat gagagtgaag 4200
   aacactatca cctgacagaa gcactaaagg ccatggaaaa agtcgccagt cacatcaatg 4260
   agatgcagaa gatctacgag gattacggga tggtgtttga ccagctggtg gcagagcaga 4320
   gtggcacaga gaaggaagtg acagagctgt ccatggggga acttctgatg cactctacag 4380
   tttcctggtt gaatccgttc ctgtctctag gaaaagccag gaaggacatt gagctcacag 4440
   tatttgtttt taagagagct gtcatactgg tttataaaga aaactgcaag ctgaaaaaga 4500
   aactgccctc gaattcccgg cctgctcaca actctgctga cttggatcca tttaaattcc 4560
   gctggttgat tcccatatct gcgcttcaag ttagactggg gaacacggca gggactgaaa 4620
   ataattccac gtgggagctg attcatacca agtcggaaat tgaaggacgg ccagaaacca 4680
   tctttcaact gtgctgcagt gacagcgaga acaaaaccag cattgttaag gtgattcgtt 4740
   ctattctgag agagaacttc cggcgccaca taaagtgtga gctgccactg gagaagacgt 4800
   gtaaggaccg gctagtacct cttaagaacc gagttcctgt ttcagccaaa ttagcctcgt 4860
   ccaggtcgtt gaagggcctc agaacatcct ccagcagcga gtggcccagc gagcccagca 4920
   agggcaactc actggactca gatgagtgca gcctgagcag tggcacccag agtagcggct 4980
   gccccgtagc cgagagcagg cgagactcta agagcaccga gctggagaaa gacgctcagg 5040
   agggcctggc ggagtttcca gatggtctta tcaaagaaag cgacattctg agtgatgaag 5100
   atgaggactt ccaccaccct ctgaaacagg gtagccctac taaggacatt gagattcagt 5160
   tccagagact gaaaatctct gaggaatccg acgtgcaccc agttgggcag cagcctctca 5220
   cagagtcagg tgaacagccc aagctggtca ggggccattt ttgccccatt aaacggaaag 5280
   caaacagcac caagaggggc agaggaactt tgctcaaggc gcagactcgt caccagtccc 5340
   tggacagcca cccagaaact gccagcattg atctaaactt ggtcctggag agagaattca 5400
   gtgtccagag cttaacttca gtcgtcaatg aggagggttt ttatgaaaca cagagccatg 5460
   gcaaatcatg atttgtgtag tgtgcttaca atgctcattt gttttcaaat tggtggaaaa 5520
   gagaaactgc aggaaaacta ttcatgtttg agttttacgc agtatatttt ttccacaaaa 5580
   cctgtaaaga tttaagttat tttaatttat tgtgaatccg aaaactagat ttagttggtc 5640
   agaatctgta aagtactagc acctatccct ttggagcagc aatcaaatga tttagtcttg 5700
   taataaaatt gcattttaat taaaaaaaaa aaaaaaaaaa aaaaaaaaaa aaaaaa 5756
<210> 14
   <211> 5302
   <212> DNA
   <213> TIAM2 homo sapiens; Accession number NM_012454
<400> 14
<210> 15
   <211> 2076
   <212> DNA
   <213> Tcr, short 5'utr Mus musculus
<400> 15
   gtttgggagg agcttgtgtg tgtgagttgt gttttaagtt tatttgcgtg tgagtacctt 60
   tgggtttttg tgtgtgtctg tgtgtgtttg tgtgtgtata actgtgggtg actgtaagtg 120
   cacctgtgtg tttgtacgtg agtgtgtaag actgtgtgtg tgcacaagag cgtgtgtagg 180
   tgcacgtgtt gtaggtgtga gaacacctgt tgtgtttagg ccatcagtca gcttggtcat 240
   tgtttctaag gtagcattta tactttgtta cctcaagtgg gctctgggag tcaacagaag 300
   tcagaaaagc tcagatccaa gccccctttt tctgacatgg agaaatttca tgctcaatat 360
   gagatgctag agactattgg ccagggaggc tgcgcccagg tgaagctggc ccgacaccgc 420
   ctcacaggca cccacgtggc tgtcaaagtg attgtaaaga gggagtgttg gttcaaccct 480
   gtcatgtctg aggcagagtt actgatgatg accgatcatc cgaatatcat ctctctcctt 540
   caagtcattg agaccaagaa gaaagtatac ctcattatgg agttgtgcga gggtaaatca 600
   ctttaccaac acatccaaaa tgctggctac ctgcaggagg atgaagcacg cccattattc 660
   aagcagctct taagtgctat gaactactgc cacaaccagg gtatagttca cagggacctg 720
   acacctgaca atattatggt agaaaaagat gggaaagtga agatcattga ttttggactc 780
   ggcacccaag agaagccagg gcaaaaccac aacttattct gtgagattta cccatttagt 840
   actcctgagg tgctctttaa cagaccctat gatatgcgca agatcgatgt gtggggtctt 900
   ggagttgtgc tgtattttat ggtaactgga aagattctgt ttgatactgc cagcgtagaa 960
   aagctgcgaa agcaaattgt tgcagaaaag tgttctgttc cctgtagact gtcagtagag 1020
   ctccaagacc tgattagact tttaatgacg gacatccccg aacttaggcc cactgttgct 1080
   gaagttatgg tgcatccctg ggtcacagaa ggctcagggg tgttaccaga tccttgtgaa 1140
   gaacatatac ccctcaagcc agaccctgcg attgcaaaag caatgggatt tatcgggttc 1200
   caagctcaag acattgaaga ttcgttatgt cagagaaaat tcaacgaaac catggcatct 1260
   tattgtctac tgaaaaaaca gattcttaag gaatgtgaca ggccaatccg ggctcagccc 1320
   atgaatccat ctgtgacccc actctcttcc cttgttgatg ctcctacttt ccatctcgga 1380
   cttcggagga cagagactga acccacaggt ctcagattat ctgacaataa ggaagtgcct 1440
   gtctgtggca atagtactag taagaaaaga gagagaagtt tcagtgggcc gggtgttctc 1500
   agcaggccga ttaacacaac acccacaatg gaccaaacac acacccgtac ttggagtggt 1560
   ccctgcattt actcaaatgt ttgcacaatc catccaaaca gcatcaatga gagtacagaa 1620
   ggccacatca gtacctcagc agaggataag cctgtccaca gcagaggctg gcccagaggc 1680
   atcaagggct ggactaggaa gataggaaat gcaatgagga agctctgttg ctgtatccca 1740
   tccaaagaga catctcacct ggggcagaga agagtctgcc caaaaattta agacacagga 1800
   aggatgtcag gagaatgagc atccagcatg gcccagcctt tcagaccgaa ggcaagctct 1860
   acctgatcct ggacttcctg cggggaggtg acctcttcac caggctttcc aaagaggtga 1920
   tgttcacgga ggaggatgtc aagttctacc tggctgagct ggccttggct ctagaccacc 1980
   tccatggcct ggggatcatc tacagggatc tgaagccaga gaatatcctc ctggatgaag 2040
   agggacatat taagatcaca gattttggct tgagca 2076
<210> 16
   <211> 2827
   <212> DNA
   <213> Tcr, long 5'utr Mus musculus
<400> 16
   gagaggagtt ggtggagttg gtggagtttg gtggatttgg tggagttggt ggtgcccttt 60
   gcgatttcgt tgtatctagt gagccgtgtg tggattttgt gtttgattgg ttcgtgtgtg 120
   agcttttgtg tgtgtgtgtg tgtgtgtgtg tgtgtgtgtg tgtgtgtgtg tgtgtagatc 180
   agtgtgtgtt tgggaggagc ttgtgtgtgt gagttgtgtt ttaagtttat ttgcgtgtga 240
   gtacctttgg gtttttgtgt gtgtctgtgt gtgtttgtgt gtgtataact gtgggtgact 300
   gtaagtgcac ctgtgtgttt gtacgtgagt gtgtaagact gtgtgtgtgc acaagagcgt 360
   gtgtaggtgc acgtgttgta ggtgtgagaa cacctgttgt gtttaggcca tcagtcagct 420
   tggtcattgt ttctaaggta gcatttatac tttgttacct caagtgggct ctgggagtca 480
   acagaagtca gaaaagctca gatccaagcc ccctttttct gacatggaga aatttcatgc 540
   tcaatatgag atgctagaga ctattggcca gggaggctgc gcccaggtga agctggcccg 600
   acaccgcctc acaggcaccc acgtggctgt caaagtgatt gtaaagaggg agtgttggtt 660
   caaccctgtc atgtctgagg cagagttact gatgatgacc gatcatccga atatcatctc 720
   tctccttcaa gtcattgaga ccaagaagaa agtatacctc attatggagt tgtgcgaggg 780
   taaatcactt taccaacaca tccaaaatgc tggctacctg caggaggatg aagcacgccc 840
   attattcaag cagctcttaa gtgctatgaa ctactgccac aaccagggta tagttcacag 900
   ggacctgaca cctgacaata ttatggtaga aaaagatggg aaagtgaaga tcattgattt 960
   tggactcggc acccaagaga agccagggca aaaccacaac ttattctgtg agatttaccc 1020
   atttagtact cctgaggtgc tctttaacag accctatgat atgcgcaaga tcgatgtgtg 1080
   gggtcttgga gttgtgctgt attttatggt aactggaaag attctgtttg atactgccag 1140
   cgtagaaaag ctgcgaaagc aaattgttgc agaaaagtgt tctgttccct gtagactgtc 1200
   agtagagctc caagacctga ttagactttt aatgacggac atccccgaac ttaggcccac 1260
   tgttgctgaa gttatggtgc atccctgggt cacagaaggc tcaggggtgt taccagatcc 1320
   ttgtgaagaa catatacccc tcaagccaga ccctgcgatt gcaaaagcaa tgggatttat 1380
   cgggttccaa gctcaagaca ttgaagattc gttatgtcag agaaaattca acgaaaccat 1440
   ggcatcttat tgtctactga aaaaacagat tcttaaggaa tgtgacaggc caatccgggc 1500
   tcagcccatg aatccatctg tgaccccact ctcttccctt gttgatgctc ctactttcca 1560
   tctcggactt cggaggacag agactgaacc cacaggtctc agattatctg acaataagga 1620
   agtgcctgtc tgtggcaata gtactagtaa gaaaagagag agaagtttca gtgggccggg 1680
   tgttctcagc aggccgatta acacaacacc cacaatggac caaacacaca cccgtacttg 1740
   gagtggtccc tgcatttact caaatgtttg cacaatccat ccaaacagca tcaatgagag 1800
   tacagaaggc cacatcagta cctcagcaga ggataagcct gtccacagca gaggctggcc 1860
   cagaggcatc aagggctgga ctaggaagat aggaaatgca atgaggaagc tctgttgctg 1920
   tatcccatcc aaagagacat ctcacctggg gcagagaaga gtctgcccaa aaatttaaga 1980
   cacaggaagg atgtcaggag aatgagcatc cagcatggcc cagcctttca gaccgaaggc 2040
   aagctctacc tgatcctgga cttcctgcgg ggaggtgacc tcttcaccag gctttccaaa 2100
   gaggtgatgt tcacggagga ggatgtcaag ttctacctgg ctgagctggc cttggctcta 2160
   gaccacctcc atggcctggg gatcatctac agggatctga agccagagaa tatcctcctg 2220
   gatgaagagg gacatattaa gatcacagat tttggcttga gcaaggaggc caccgaccat 2280
   gacaagagag cctattcatt ttgtgggact attgaataca tggcgcccga ggtggtgaac 2340
   cggcgtggac acacacagag tgccgactgg tggtccttcg gtgtgctcat gttcgagatg 2400
   ctcacagggt ccctgccatt ccaggggaag gacaggaagg aaacaatggc ccgcatcctc 2460
   aaagcaaagc tgggtatgcc ttagttcctc agtgcggagg ctcagagcct gctcagggcc 2520
   cttttcaagc ggaacccctg caaccggcta ggtaagggtc cctgtgacac ccccacccca 2580
   ggaatgcaat gaggctgccc tctagacccc ccttaggaat gtgagaggcc accattctgt 2640
   tccccacggg atgtggagga cttcctcctt atgccccaac tctgaactgt atgcttttcc 2700
   ttgctaaggt tgcaggaagc agaggtaccc cgacgctggg gaaacactca catgtggcct 2760
   ggcgcccaca ggcacgtgga cttatcagga ttgctgaaag gcatttgaaa aaaaaaaaaa 2820
   aaaaaaa 2827
<210> 17
   <211> 714
   <212> PRT
   <213> Tagap1 mouse wildtype FVB/N
<400> 17
   Met Lys Leu Ile Ser Ser Leu Asp Gly Ser Lys Thr Leu Asn Ala Asn
   1               5                   10                  15
   Asn Met Glu Thr Leu Ile Glu Cys Gln Ser Glu Gly Asp Ile Lys Val
               20                  25                  30
   Pro Pro Leu Leu Thr Ser Cys Glu Ser Glu Asp Ser Ile Cys Gln Leu
           35                  40                  45
   Thr Glu Ile Lys Lys Arg Lys Lys Val Leu Ser Trp Pro Ser Leu Met
       50                  55                  60
   Arg Lys Leu Ser Pro Ser Ser Asp Phe Ser Gly Ser Leu Glu Pro Glu
   65                  70                  75                  80
   Leu Lys Val Ser Leu Phe Asp Gln Pro Leu Ser Ile Ile Cys Gly Glu
                   85                  90                  95
   Asn Asp Thr Leu Pro Arg Pro Ile Gln Asp Ile Leu Thr Ile Leu Cys
               100                 105                 110
   Leu Lys Gly Pro Ser Thr Glu Gly Ile Phe Arg Lys Ala Ala Ser Glu
           115                 120                 125
   Lys Ala Arg Lys Glu Leu Lys Glu Gly Leu Asn Cys Gly Val Ser Val
       130                 135                 140
   Asn Leu Lys Gln Leu Pro Val His Leu Leu Ala Val Val Phe Lys Asp
   145                 150                 155                 160
   Phe Leu Arg Gly Ile Pro Leu Lys Leu Leu Ser Cys Asp Leu Phe Glu
                   165                 170                 175
   Asp Trp Met Gly Ala Leu Glu Lys Pro Thr Glu Glu Asp Arg Ile Glu
               180                 185                 190
   Ala Leu Lys Gln Val Ala Gly Gly Leu Pro Arg Pro Asn Leu Leu Leu
           195                 200                 205
   Leu Arg His Leu Leu Tyr Val Leu His Leu Ile Ser Lys Asn Ala Glu
   210 215 220
   Val Asn Lys Met Asp Ser Ser Asn Leu Ala Ile Cys Ile Gly Pro Asn
   225 230 235 240
   Met Leu Thr Leu Lys Asn Asp Gln Ser Leu Ser Phe Gln Ala Gln Lys
   245 250 255
   Asp Leu Asn Asn Lys Val Lys Ile Leu Val Glu Phe Leu Ile Asp Asn
   260 265 270
   Cys Phe Glu Ile Phe Gly Glu Asn Ile Arg Thr Arg Ser Arg Ile Thr
   275 280 285
   Ser Asp Asp Ser Leu Glu His Thr Asp Ser Ser Asp Val Ser Thr Leu
   290 295 300
   Gln Asn Asp Ser Ala Tyr Asp Ser Asn Asp Pro Asp Val Glu Pro Thr
   305 310 315 320
   Ser Gly Ala Ala Ser Pro Asn Arg Gln Leu Glu Gly Pro Thr Pro Thr
   325 330 335
   Met Ala Gly Leu Asp Thr Arg Gly Gln Arg Asp Thr Cys Glu Ser Ser
   340 345 350
   Ser Glu Ser Ser Val Ser Met Val Val Arg Leu Lys Ser Ser Ile Val
   355 360 365
   Gln Gln Asp Arg Arg Phe Ser Glu Pro Asn Met Ser Pro Ser Arg Glu
   370 375 380
   Cys Leu Val Gly Pro Thr Ser Lys Gln Lys Leu Ala Arg Ser Glu Asp
   385 390 395 400
   Ser Phe Thr Leu Ser Gln Asp Ala Ser Cys Ser Glu Gly Asp Glu Ala
   405 410 415
   Glu Asp Pro Phe Thr Glu Glu Val Phe Pro Ala Val Asp Ser Lys Pro
   420 425 430
   Lys Arg Pro Val Asp Leu Lys Ile Lys Asn Trp Thr Gln Gly Leu Ala
   435 440 445
   Ser Pro Gln Gly His Ile Thr Lys Ala Phe Ser Arg Ser Ser Pro Gly
   450 455 460
   Glu Ser Leu Gly Ser Ser Pro Val Pro Ser Pro Ser Cys Pro Lys Arg
   465 470 475 480
   Asn Phe Phe Thr Arg His Gln Ser Phe Thr Thr Lys Thr Asp Lys Thr
   485 490 495
   Lys Pro Gln Arg Glu Ile Arg Lys His Ser Met Ser Phe Ser Phe Ala
   500 505 510
   Ser His Lys Lys Val Leu Pro Arg Thr Ser Ser Ile Gly Ser Glu Lys
   515 520 525
   Ser Lys Asp Phe Ser Arg Asp Gln Leu Gln Lys Asp Leu Arg Lys Glu
   530 535 540
   Ser Gln Leu Ser Gly Arg Ile Val Gln Glu Asn Glu Ser Glu Ile Gln
   545 550 555 560
   Ser Gln Thr Ser Leu Gly Phe Ser Leu Ser Gly Thr Trp Ala Leu Ser
   565 570 575
   Val Asp Asn Thr Phe Gln Leu Val Asp Met Arg Lys Pro Gly Ser Pro
   580 585 590
   Pro Ser Tyr Glu Glu Ala Ile Tyr Tyr Gln Thr Ser Gly Leu Thr Ala
   595 600 605
   Tyr Gly Gly Gln Thr Val Gly Ser Met Arg Ser Arg Met Phe Lys Pro
   610 615 620
   Ser Thr Ala Val Pro Pro Val Pro Ser His His Gly Gly Asp Leu Ser
   625 630 635 640
   Glu Gly Thr Pro Gly Gly His Arg Leu Ser Ser Val Thr Glu His Trp
   645 650 655
   Thr His Ser Gln Thr Val His Val Ser Ile Glu Thr Gln Gly Arg Ser
   660 665 670
   Glu Leu His Gln Leu Arg Thr Val Ser Glu Ser Met Gln Lys Ala Lys
   675 680 685
   Leu Asp Cys Leu Gly Pro Gln His Ser His Leu Val Phe Glu Ala Asp
   690 695 700
   Gln Leu Cys Cys Ala Arg Glu Ser Tyr Ile
   705 710
<210> 18
   <211> 714
   <212> PRT
   <213> Tagap1 mouse wildtype Balb/c
<400> 18
   Met Lys Leu Ile Ser Ser Leu Asp Gly Ser Lys Thr Leu Asn Ala Asn
   1               5                   10                  15
   Asn Met Glu Thr Leu Ile Glu Cys Gln Ser Glu Gly Asp Ile Lys Val
               20                  25                  30
   Pro Pro Leu Leu Thr Ser Cys Glu Ser Glu Asp Ser Ile Cys Gln Leu
           35                  40                  45
   Thr Glu Ile Lys Lys Arg Lys Lys Val Leu Ser Trp Pro Ser Leu Met
       50                  55                  60
   Arg Lys Leu Ser Pro Ser Ser Asp Phe Ser Gly Ser Leu Glu Pro Glu
   65                  70                  75                  80
   Leu Lys Val Ser Leu Phe Asp Gln Pro Leu Ser Ile Ile Cys Gly Glu
                   85                  90                  95
   Asn Asp Thr Leu Pro Arg Pro Ile Gln Asp Ile Leu Thr Ile Leu Cys
               100                 105                 110
   Leu Lys Gly Pro Ser Thr Glu Gly Ile Phe Arg Lys Ala Ala Ser Glu
           115                 120                 125
   Lys Ala Arg Lys Glu Leu Lys Glu Gly Leu Asn Cys Gly Val Ser Val
       130                 135                 140
   Asn Leu Lys Gln Leu Pro Val His Leu Leu Ala Val Val Phe Lys Asp
   145                 150                 155                 160
   Phe Leu Arg Gly Ile Pro Leu Lys Leu Leu Ser Cys Asp Leu Phe Glu
                   165                 170                 175
   Asp Trp Met Gly Ala Leu Glu Lys Pro Thr Glu Glu Asp Arg Ile Glu
               180                 185                 190
   Ala Leu Lys Gln Val Ala Gly Gly Leu Pro Arg Pro Asn Leu Leu Leu
           195                 200                 205
   Leu Arg His Leu Leu Tyr Val Leu His Leu Ile Ser Lys Asn Ala Glu
       210                 215                 220
   Val Asn Lys Met Asp Ser Ser Asn Leu Ala Ile Cys Ile Gly Pro Asn
   225                 230                 235                 240
   Met Leu Thr Leu Lys Asn Asp Gln Ser Leu Ser Phe Gln Ala Gln Lys
   245 250 255
   Asp Leu Asn Asn Lys Val Lys Ile Leu Val Glu Phe Leu Ile Asp Asn
   260 265 270
   Cys Phe Glu Ile Phe Gly Glu Asn Ile Arg Thr Arg Ser Arg Ile Thr
   275 280 285
   Ser Asp Asp Ser Leu Glu His Thr Asp Ser Ser Asp Val Ser Thr Leu
   290 295 300
   Gln Asn Asp Ser Ala Tyr Asp Ser Asn Asp Pro Asp Val Glu Pro Thr
   305 310 315 320
   Ser Gly Ala Ala Ser Pro Asn Arg Gln Leu Glu Gly Pro Thr Pro Thr
   325 330 335
   Met Ala Gly Leu Asp Thr Arg Gly His Arg Asp Thr Cys Glu Ser Ser
   340 345 350
   Ser Glu Ser Ser Val Ser Met Val Val Arg Leu Lys Ser Ser Ile Val
   355 360 365
   Gln Gln Asp Arg Arg Phe Ser Glu Pro Asn Met Ser Pro Ser Arg Glu
   370 375 380
   Cys Leu Val Gly Pro Thr Ser Lys Gln Lys Leu Ala Arg Ser Glu Asp
   385 390 395 400
   Ser Phe Thr Leu Ser Gln Asp Ala Ser Cys Ser Glu Gly Asp Glu Ala
   405 410 415
   Glu Asp Pro Phe Thr Glu Glu Val Phe Pro Ala Val Asp Ser Lys Pro
   420 425 430
   Lys Arg Pro Val Asp Leu Lys Ile Lys Asn Trp Thr Gln Gly Leu Ala
   435 440 445
   Ser Pro Gln Gly His Ile Thr Lys Ala Phe Ser Arg Ser Ser Pro Gly
   450 455 460
   Glu Ser Leu Gly Ser Ser Pro Val Pro Ser Pro Ser Cys Pro Lys Arg
   465 470 475 480
   Asn Phe Phe Thr Arg His Gln Ser Phe Thr Thr Lys Thr Asp Lys Thr
   485 490 495
   Lys Pro Gln Arg Glu Ile Arg Lys His Ser Met Ser Phe Ser Phe Ala
               500                 505                 510
   Ser His Lys Lys Val Leu Pro Arg Thr Ser Ser Ile Gly Ser Glu Lys
           515                 520                 525
   Ser Lys Asp Phe Ser Arg Asp Gln Leu Gln Lys Asp Leu Arg Lys Glu
       530                 535                 540
   Ser Gln Leu Ser Gly Arg Ile Val Gln Glu Asn Glu Ser Glu Ile Gln
   545                 550                 555                 560
   Ser Gln Thr Ser Leu Gly Phe Ser Leu Ser Gly Thr Trp Ala Leu Ser
                   565                 570                 575
   Val Asp Asn Thr Phe Gln Leu Val Asp Met Arg Lys Pro Gly Ser Pro
               580                 585                 590
   Pro Ser Tyr Glu Glu Ala Ile Tyr Tyr Gln Thr Ser Gly Leu Thr Ala
           595                 600                 605
   Tyr Gly Gly Gln Thr Val Gly Ser Met Arg Ser Arg Met Phe Lys Pro
       610                 615                 620
   Ser Thr Ala Val Pro Pro Val Pro Ser His His Gly Gly Asp Leu Ser
   625                 630                 635                 640
   Glu Gly Thr Pro Gly Gly His Arg Leu Ser Ser Val Thr Glu His Trp
                   645                 650                 655
   Thr His Ser Gln Thr Val His Val Ser Ile Glu Thr Gln Gly Arg Ser
               660                 665                 670
   Glu Leu His Gln Leu Arg Thr Val Ser Glu Ser Met Gln Lys Ala Lys
           675                 680                 685
   Leu Asp Cys Leu Gly Pro Gln His Ser His Leu Val Phe Glu Ala Asp
       690                 695                 700
   Gln Leu Cys Cys Ala Arg Glu Ser Tyr Ile
   705                 710
<210> 19
   <211> 442
   <212> PRT
   <213> Tagap1 t1 mouse
<400> 19
   Met Lys Leu Ile Ser Ser Leu Asp Gly Ser Lys Thr Leu Asn Ala Asn
   1               5                   10                  15
   Asn Met Glu Thr Leu Ile Glu Cys Gln Ser Glu Gly Asp Ile Lys Val
               20                  25                  30
   Pro Pro Leu Leu Thr Ser Cys Glu Ser Glu Asp Ser Ile Cys Gln Leu
           35                  40                  45
   Ile Glu Ile Lys Lys Arg Lys Lys Val Leu Ser Trp Pro Ser Leu Met
       50                  55                  60
   Arg Lys Leu Ser Pro Ser Ser Asp Phe Ser Gly Ser Leu Glu Pro Glu
   65                  70                  75                  80
   Leu Lys Val Ser Leu Phe Asp Gln Pro Leu Ser Ile Ile Cys Gly Glu
                   85                  90                  95
   Asn Asp Thr Leu Pro Arg Pro Ile Gln Asp Ile Leu Thr Ile Leu Cys
               100                 105                 110
   Leu Lys Gly Pro Ser Thr Glu Gly Ile Phe Arg Lys Ala Ala Ser Glu
           115                 120                 125
   Lys Ala Arg Lys Glu Leu Lys Glu Gly Leu Asn Cys Gly Val Ser Leu
       130                 135                 140
   Asn Leu Lys Gln Leu Pro Val His Leu Leu Ala Val Val Phe Lys Asp
   145                 150                 155                 160
   Phe Phe Arg Gly Ile Pro Leu Lys Leu Leu Ser Cys Asp Leu Phe Glu
                   165                 170                 175
   Asp Trp Met Gly Ala Leu Glu Lys Pro Thr Glu Glu Asp Arg Ile Glu
               180                 185                 190
   Ala Leu Lys Gln Val Ala Gly Gly Leu Pro Arg Pro Asn Leu Leu Leu
           195                 200                 205
   Leu Arg His Leu Leu Tyr Val Leu His Leu Ile Ser Lys Asn Ala Glu
       210                 215                 220
   Val Asn Lys Met Asp Ser Ser Asn Leu Ala Ile Cys Ile Gly Pro Asn
   225                 230                 235                 240
   Met Leu Thr Leu Lys Asn Asp Gln Ser Leu Ser Phe Gln Ala Gln Lys
                   245                 250                 255
   Asp Leu Asn Asn Lys Val Lys Ile Leu Val Glu Phe Leu Ile Asp Asn
               260                 265                 270
   Cys Phe Glu Ile Phe Gly Glu Asn Ile Arg Thr Arg Ser Arg Ile Thr
           275                 280                 285
   Ser Asp Asp Ser Leu Glu His Thr Asp Ser Ser Asp Val Ser Thr Leu
       290                 295                 300
   Gln Asn Asp Ser Ala Tyr Asp Ser Asn Asp Pro Asp Val Glu Pro Thr
   305                 310                 315                 320
   Ser Gly Thr Ala Ser Pro Asn Arg Gln Leu Glu Gly Pro Thr Pro Thr
                   325                 330                 335
   Met Ala Gly Leu Asp Thr Arg Gly His Arg Asp Thr Cys Glu Ser Ser
               340                 345                 350
   Ser Glu Ser Ser Val Ser Met Val Val Arg Leu Lys Ser Ser Ile Val
           355                 360                 365
   Gln Gln Asp Arg Arg Phe Ser Glu Pro Asn Met Ser Pro Ser Arg Glu
       370                 375                 380
   Cys Leu Val Gly Pro Thr Ser Lys Gln Lys Leu Thr Arg Ser Glu Asp
   385                 390                 395                 400
   Ser Phe Thr Leu Ser Gln Asp Ala Ser Cys Ser Glu Gly Asp Glu Ala
                   405                 410                 415
   Glu Asp Pro Phe Thr Glu Glu Val Phe Pro Ala Val Asp Ser Lys Pro
               420                 425                 430
   Lys Arg Pro Val Asp Leu Lys Ile Lys Asn
           435                 440
<210> 20
   <211> 707
   <212> PRT
   <213> Tagap1 t2 mouse
<400> 20
   Met Lys Leu Ile Ser Ser Leu Asp Gly Ser Lys Thr Leu Asn Ala Asn
   1               5                   10                  15
   Asn Met Glu Thr Leu Ile Glu Cys Gln Ser Glu Gly Asp Ile Lys Val
               20                  25                  30
   Pro Pro Leu Leu Thr Ser Cys Glu Ser Glu Asp Ser Ile Cys Gln Leu
           35                  40                  45
   Ile Glu Ile Lys Lys Arg Lys Lys Val Leu Ser Trp Pro Ser Leu Met
       50                  55                  60
   Arg Lys Leu Ser Pro Ser Ser Asp Phe Ser Gly Ser Leu Glu Pro Glu
   65                  70                  75                  80
   Leu Lys Val Ser Leu Phe Asp Gln Pro Leu Ser Ile Ile Cys Gly Glu
                   85                  90                  95
   Asn Asp Thr Leu Pro Arg Pro Ile Gln Asp Ile Leu Thr Ile Leu Cys
               100                 105                 110
   Leu Lys Gly Pro Ser Thr Glu Gly Ile Phe Arg Lys Ala Ala Ser Glu
           115                 120                 125
   Lys Ala Arg Lys Glu Leu Lys Glu Glu Leu Asn Cys Gly Val Ser Val
       130                 135                 140
   Asn Leu Lys Gln Leu Pro Val His Leu Leu Ala Val Val Phe Lys Asp
   145                 150                 155                 160
   Phe Leu Arg Gly Ile Pro Leu Lys Leu Leu Ser Cys Asp Leu Phe Glu
                   165                 170                 175
   Asp Trp Met Gly Ala Leu Glu Lys Pro Thr Glu Ala Asp Arg Ile Glu
               180                 185                 190
   Ala Leu Lys Gln Val Ala Gly Gly Leu Pro Arg Pro Asn Leu Phe Leu
           195                 200                 205
   Leu Arg His Leu Leu Tyr Val Leu His Leu Ile Ser Lys Asn Ala Glu
       210                 215                 220
   Val Asn Lys Met Asp Ser Ser Asn Leu Ala Ile Cys Ile Gly Pro Asn
   225                 230                 235                 240
   Met Leu Thr Leu Lys Asn Asp Gln Ser Leu Ser Phe Gln Ala Gln Lys
                   245                 250                 255
   Asp Leu Asn Asn Lys Val Lys Ile Leu Val Glu Phe Leu Ile Asp Asn
               260                 265                 270
   Cys Phe Glu Ile Phe Gly Glu Asn Ile Arg Thr Arg Ser Arg Ile Thr
           275                 280                 285
   Ser Asp Asp Ser Leu Glu His Thr Asp Ser Ser Asp Met Ser Thr Leu
   290 295 300
   Gln Asn Asp Ser Ala Tyr Asp Ser Asn Asp Pro Asp Val Glu Pro Thr
   305 310 315 320
   Ser Gly Thr Ala Ser Pro Asn Arg Gln Leu Glu Asp Pro Thr Pro Thr
   325 330 335
   Met Ala Gly Leu Asp Thr Arg Gly His Arg Asp Thr Cys Glu Ser Ser
   340 345 350
   Ser Glu Ser Ser Val Ser Met Val Val Arg Leu Lys Ser Ser Ile Val
   355 360 365
   Gln Gln Asp Arg Arg Phe Ser Glu Pro Asn Met Ser Pro Ser Arg Glu
   370 375 380
   Cys Leu Val Gly Pro Thr Ser Lys Gln Lys Leu Thr Arg Ser Glu Asp
   385 390 395 400
   Ser Phe Thr Leu Ser Gln Asp Glu Ala Glu Asp Pro Phe Thr Glu Glu
   405 410 415
   Val Phe Pro Ala Val Asp Ser Lys Pro Lys Arg Pro Val Asp Leu Lys
   420 425 430
   Met Lys Asn Trp Thr Gln Gly Leu Ala Ser Pro Gln Gly His Ile Thr
   435 440 445
   Lys Ala Phe Ser Arg Ser Ser Pro Gly Glu Ser Leu Gly Ser Ser Leu
   450 455 460
   Val Pro Ser Pro Ser Cys Pro Lys Arg Asn Phe Phe Thr Arg His Gln
   465 470 475 480
   Ser Phe Thr Thr Lys Thr Asp Lys Thr Lys Pro Gln Arg Glu Ile Arg
   485 490 495
   Lys His Ser Met Ser Phe Ser Phe Ala Ser His Lys Lys Val Leu Pro
   500 505 510
   Arg Thr Ser Ser Ile Gly Ser Glu Lys Ser Lys Asp Phe Ser Arg Asp
   515 520 525
   Gln Leu Gln Lys Asp Leu Arg Lys Glu Ser Gln Leu Ser Gly Arg Ile
   530 535 540
   Ile Gln Glu Asn Glu Ser Glu Ile Gln Ser Gln Thr Ser Leu Gly Phe
   545                 550                 555                 560
   Ser Leu Ser Gly Thr Trp Ala Leu Ser Val Asp Asn Thr Phe Gln Leu
                   565                 570                 575
   Val Asp Met Arg Lys Pro Gly Ser Pro Pro Ser Tyr Lys Glu Ala Ile
               580                 585                 590
   Tyr Tyr Gln Thr Ser Gly Leu Arg Ala Tyr Ser Gly Gln Thr Val Gly
           595                 600                 605
   Ser Met Arg Ser Arg Met Phe Lys Pro Ser Thr Ala Val Pro Pro Val
       610                 615                 620
   Pro Ser His His Gly Gly Asp Leu Ser Glu Gly Thr Pro Gly Gly His
   625                 630                 635                 640
   Arg Leu Ser Ser Val Thr Glu His Trp Thr His Ser Gln Thr Val His
                   645                 650                 655
   Val Ser Ile Glu Thr Gln Gly Arg Ser Glu Leu His Gln Leu Arg Thr
               660                 665                 670
   Val Ser Glu Ser Met Gln Lys Ala Lys Leu Asp Cys Leu Gly Pro Gln
           675                 680                 685
   His Ser His Leu Val Phe Glu Val Asp Gln Leu Cys Cys Ala Arg Glu
       690                 695                 700
   Ser Tyr Ile
   705
<210> 21
   <211> 707
   <212> PRT
   <213> Tagap1 t3 mouse
<210> 22
   <211> 707
   <212> PRT
   <213> Tagap1 t4 mouse
<400> 22
   Met Lys Leu Ile Ser Ser Leu Asp Gly Ser Lys Thr Leu Asn Ala Asn
   1 5 10 15
   Asn Met Glu Thr Leu Ile Glu Cys Gln Ser Glu Gly Asp Ile Lys Val
   20 25 30
   Pro Pro Leu Leu Thr Ser Cys Glu Ser Glu Asp Ser Ile Cys Gln Leu
   35 40 45
   Ile Glu Ile Lys Lys Arg Lys Lys Val Leu Ser Trp Pro Ser Leu Met
   50 55 60
   Arg Lys Leu Ser Pro Ser Ser Asp Phe Ser Gly Ser Leu Glu Pro Glu
   65 70 75 80
   Leu Lys Val Ser Leu Phe Asp Gln Pro Leu Ser Ile Ile Cys Gly Glu
                   85                  90                  95
   Asn Asp Thr Leu Pro Arg Pro Ile Gln Asp Ile Leu Thr Ile Leu Cys
               100                 105                 110
   Leu Lys Gly Pro Ser Thr Glu Gly Ile Phe Arg Lys Ala Ala Ser Glu
           115                 120                 125
   Lys Ala Arg Lys Glu Leu Lys Glu Glu Leu Asn Cys Gly Val Ser Val
       130                 135                 140
   Asn Leu Lys Gln Leu Pro Val His Leu Leu Ala Val Val Phe Lys Asp
   145                 150                 155                 160
   Phe Leu Arg Gly Ile Pro Leu Lys Leu Leu Ser Cys Asp Leu Phe Glu
                   165                 170                 175
   Asp Trp Met Gly Ala Leu Glu Lys Pro Thr Glu Glu Asp Arg Ile Glu
               180                 185                 190
   Ala Leu Lys Gln Val Ala Gly Gly Leu Pro Arg Pro Asn Leu Phe Leu
           195                 200                 205
   Leu Arg His Leu Leu Tyr Val Leu His Leu Ile Ser Lys Asn Ala Glu
       210                 215                 220
   Val Asn Lys Met Asp Ser Ser Asn Leu Ala Ile Cys Ile Gly Pro Asn
   225                 230                 235                 240
   Met Leu Thr Leu Lys Asn Asp Gln Ser Leu Ser Phe Gln Ala Gln Lys
                   245                 250                 255
   Asp Leu Asn Asn Lys Val Lys Ile Leu Val Glu Phe Leu Ile Asp Asn
               260                 265                 270
   Cys Phe Glu Ile Phe Gly Glu Asn Ile Arg Thr Arg Ser Arg Ile Thr
           275                 280                 285
   Ser Asp Asp Ser Leu Glu His Thr Asp Ser Ser Asp Met Ser Thr Leu
       290                 295                 300
   Gln Asn Asp Ser Ala Tyr Asp Ser Asn Asp Pro Asp Val Glu Pro Thr
   305                 310                 315                 320
   Ser Gly Thr Ala Ser Pro Asn Arg Gln Leu Glu Asp Pro Thr Pro Thr
                   325                 330                 335
   Met Ala Gly Leu Asp Thr Arg Gly His Arg Asp Thr Cys Glu Ser Ser
   340 345 350
   Ser Glu Ser Ser Val Ser Met Val Val Arg Leu Lys Ser Ser Ile Val
   355 360 365
   Gln Gln Asp Arg Arg Phe Ser Glu Pro Asn Met Ser Pro Ser Arg Glu
   370 375 380
   Cys Leu Val Gly Pro Thr Ser Lys Gln Lys Leu Thr Arg Ser Glu Asp
   385 390 395 400
   Ser Phe Thr Leu Ser Gln Asp Glu Ala Glu Asp Pro Phe Thr Glu Glu
   405 410 415
   Val Phe Pro Ala Val Asp Ser Lys Pro Lys Arg Pro Val Asp Leu Lys
   420 425 430
   Met Lys Asn Trp Thr Gln Gly Leu Ala Ser Pro Gln Gly His Ile Thr
   435 440 445
   Lys Ala Phe Ser Arg Ser Ser Pro Gly Glu Ser Leu Gly Ser Ser Leu
   450 455 460
   Val Pro Ser Pro Ser Cys Pro Lys Arg Asn Phe Phe Thr Arg His Gln
   465 470 475 480
   Ser Phe Thr Thr Lys Thr Asp Lys Thr Lys Pro Gln Arg Glu Ile Arg
   485 490 495
   Lys His Ser Met Ser Phe Ser Phe Ala Ser His Lys Lys Val Leu Pro
   500 505 510
   Arg Thr Ser Ser Ile Gly Ser Glu Lys Ser Glu Asp Phe Ser Arg Asp
   515 520 525
   Gln Leu Gln Lys Asp Leu Arg Lys Glu Ser Gln Leu Ser Gly Arg Ile
   530 535 540
   Ile Gln Glu Asn Glu Ser Glu Ile Gln Ser Gln Thr Ser Leu Gly Phe
   545 550 555 560
   Ser Leu Ser Gly Thr Arg Ala Leu Ser Val Asp Asn Thr Phe Gln Leu
   565 570 575
   Val Asp Met Arg Lys Pro Gly Ser Pro Pro Ser Tyr Lys Glu Ala Ile
   580 585 590
   Tyr Tyr Gln Thr Ser Gly Leu Arg Ala Tyr Ser Gly Gln Thr Val Gly
   595 600 605
   Ser Met Arg Ser Arg Met Phe Lys Pro Ser Thr Ala Val Pro Pro Val
   610 615 620
   Pro Ser His His Gly Gly Asp Leu Ser Glu Gly Thr Pro Gly Gly His
   625 630 635 640
   Arg Leu Ser Ser Val Thr Glu His Trp Thr His Ser Gln Thr Val His
   645 650 655
   Val Ser Ile Glu Thr Gln Gly Arg Ser Glu Leu His Gln Leu Arg Thr
   660 665 670
   Val Ser Glu Ser Met Gln Lys Ala Lys Leu Asp Cys Leu Gly Pro Gln
   675 680 685
   His Ser His Leu Val Phe Glu Val Asp Gln Leu Cys Cys Ala Arg Glu
   690 695 700
   Ser Tyr Ile
   705
<210> 23
   <211> 731
   <212> PRT
   <213> Tagap1 homo sapiens
<400> 23
   Met Lys Leu Arg Ser Ser His Asn Ala Ser Lys Thr Leu Asn Ala Asn
   1 5 10 15
   Asn Met Glu Thr Leu Ile Glu Cys Gln Ser Glu Gly Asp Ile Lys Glu
   20 25 30
   His Pro Leu Leu Ala Ser Cys Glu Ser Glu Asp Ser Ile Cys Gln Leu
   35 40 45
   Ile Glu Val Lys Lys Arg Lys Lys Val Leu Ser Trp Pro Phe Leu Met
   50 55 60
   Arg Arg Leu Ser Pro Ala Ser Asp Phe Ser Gly Ala Leu Glu Thr Asp
   65 70 75 80
   Leu Lys Ala Ser Leu Phe Asp Gln Pro Leu Ser Ile Ile Cys Gly Asp
   85 90 95
   Ser Asp Thr Leu Pro Arg Pro Ile Gln Asp Ile Leu Thr Ile Leu Cys
               100                 105                 110
   Leu Lys Gly Pro Ser Thr Glu Gly Ile Phe Arg Arg Ala Ala Asn Glu
           115                 120                 125
   Lys Ala Arg Lys Glu Leu Lys Glu Glu Leu Asn Ser Gly Asp Ala Val
       130                 135                 140
   Asp Leu Glu Arg Leu Pro Val His Leu Leu Ala Val Val Phe Lys Asp
   145                 150                 155                 160
   Phe Leu Arg Ser Ile Pro Arg Lys Leu Leu Ser Ser Asp Leu Phe Glu
                   165                 170                 175
   Glu Trp Met Gly Ala Leu Glu Met Gln Asp Glu Glu Asp Arg Ile Glu
               180                 185                 190
   Ala Leu Lys Gln Val Ala Asp Lys Leu Pro Arg Pro Asn Leu Leu Leu
           195                 200                 205
   Leu Lys His Leu Val Tyr Val Leu His Leu Ile Ser Lys Asn Ser Glu
       210                 215                 220
   Val Asn Arg Met Asp Ser Ser Asn Leu Ala Ile Cys Ile Gly Pro Asn
   225                 230                 235                 240
   Met Leu Thr Leu Glu Asn Asp Gln Ser Leu Ser Phe Glu Ala Gln Lys
                   245                 250                 255
   Asp Leu Asn Asn Lys Val Lys Thr Leu Val Glu Phe Leu Ile Asp Asn
               260                 265                 270
   Cys Phe Glu Ile Phe Gly Glu Asn Ile Pro Val His Ser Ser Ile Thr
           275                 280                 285
   Ser Asp Asp Ser Leu Glu His Thr Asp Ser Ser Asp Val Ser Thr Leu
       290                 295                 300
   Gln Asn Asp Ser Ala Tyr Asp Ser Asn Asp Pro Asp Val Glu Ser Asn
   305                 310                 315                 320
   Ser Ser Ser Gly Ile Ser Ser Pro Ser Arg Gln Pro Gln Val Pro Met
                   325                 330                 335
   Ala Thr Ala Ala Gly Leu Asp Ser Ala Gly Pro Gln Asp Ala Arg Glu
               340                 345                 350
   Val Ser Pro Glu Pro Ile Val Ser Thr Val Ala Arg Leu Lys Ser Ser
           355                 360                 365
   Leu Ala Gln Pro Asp Arg Arg Tyr Ser Glu Pro Ser Met Pro Ser Ser
       370                 375                 380
   Gln Glu Cys Leu Glu Ser Arg Val Thr Asn Gln Thr Leu Thr Lys Ser
   385                 390                 395                 400
   Glu Gly Asp Phe Pro Val Pro Arg Val Gly Ser Arg Leu Glu Ser Glu
                   405                 410                 415
   Glu Ala Glu Asp Pro Phe Pro Glu Glu Val Phe Pro Ala Val Gln Gly
               420                 425                 430
   Lys Thr Lys Arg Pro Val Asp Leu Lys Ile Lys Asn Leu Ala Pro Gly
           435                 440                 445
   Ser Val Leu Pro Arg Ala Leu Val Leu Lys Ala Phe Ser Ser Ser Ser
       450                 455                 460
   Leu Asp Ala Ser Ser Asp Ser Ser Pro Val Ala Ser Pro Ser Ser Pro
   465                 470                 475                 480
   Lys Arg Asn Phe Phe Ser Arg His Gln Ser Phe Thr Thr Lys Thr Glu
                   485                 490                 495
   Lys Gly Lys Pro Ser Arg Glu Ile Lys Lys His Ser Met Ser Phe Thr
               500                 505                 510
   Phe Ala Pro His Lys Lys Val Leu Thr Lys Asn Leu Ser Ala Gly Ser
           515                 520                 525
   Gly Lys Ser Gln Asp Phe Thr Arg Asp His Val Pro Arg Gly Val Arg
       530                 535                 540
   Lys Glu Ser Gln Leu Ala Gly Arg Ile Val Gln Glu Asn Gly Cys Glu
   545                 550                 555                 560
   Thr His Asn Gln Thr Ala Arg Gly Phe Cys Leu Arg Pro His Ala Leu
                   565                 570                 575
   Ser Val Asp Asp Val Phe Gln Gly Ala Asp Trp Glu Arg Pro Gly Ser
               580                 585                 590
   Pro Pro Ser Tyr Glu Glu Ala Met Gln Gly Pro Ala Ala Arg Leu Val
           595                 600                 605
   Ala Ser Glu Ser Gln Thr Val Gly Ser Met Thr Val Gly Ser Met Arg
   610 615 620
   Ala Arg Met Leu Glu Ala His Cys Leu Leu Pro Pro Leu Pro Pro Ala
   625 630 635 640
   His His Val Glu Asp Ser Arg His Arg Gly Ser Lys Glu Pro Leu Pro
   645 650 655
   Gly His Gly Leu Ser Pro Leu Pro Glu Arg Trp Lys Gln Ser Arg Thr
   660 665 670
   Val His Ala Ser Gly Asp Ser Leu Gly His Val Ser Gly Pro Gly Arg
   675 680 685
   Pro Glu Leu Leu Pro Leu Arg Thr Val Ser Glu Ser Val Gln Arg Asn
   690 695 700
   Lys Arg Asp Cys Leu Val Arg Arg Cys Ser Gln Pro Val Phe Glu Ala
   705 710 715 720
   Asp Gln Phe Gln Tyr Ala Lys Glu Ser Tyr Ile
   725 730
<210> 24
   <211> 1008
   <212> PRT
   <213> Tagap1 Rattus norvegicus
<400> 24
   Met Glu Leu Leu Ser Ala Arg Leu Pro Arg Ala Gly Glu Thr Phe Val
   1 5 10 15
   Pro Ile Ser Glu His Gln His Trp Lys Pro Glu Asn Ala His Asn Asn
   20 25 30
   Gly Ala Gln Cys Phe Pro Glu Leu Thr Leu Gly Arg Thr His Gln Gly
   35 40 45
   Asn Lys Val Leu Asp Ser Glu Trp Val Thr Gln Met Leu Arg Thr Thr
   50 55 60
   Leu Ala Phe Arg Arg Ala Leu Tyr Ile Lys Leu Thr Ala Ser Ile Leu
   65 70 75 80
   Glu Leu Gln Asn Arg Lys Asn Gly Thr Phe His Val Leu Arg Gly Glu
   85 90 95
   Asn Tyr Lys Phe Ser Ser Val Glu His Ser Ser Leu Ala Gly Ile Gln
               100                 105                 110
   Gln Val Phe Ser Lys His Ser Gln Ser Val Gln Asp Cys Ser Asp Val
           115                 120                 125
   Glu Lys Val Val Ala Ile Val Val Ala Glu Lys Pro Gly Ile Ser Phe
       130                 135                 140
   Lys Asn Pro Pro Pro Ala Leu Met Ala Gly Pro Arg Asp Leu Ala Ala
   145                 150                 155                 160
   Ala Glu Leu Glu Arg Gly Glu Ala Lys Arg Pro Pro Glu Glu Ile Gln
                   165                 170                 175
   Cys Asp Gly Val Ser Gln Ile Thr Ala Pro Leu Glu Trp Arg Glu Ser
               180                 185                 190
   Arg Phe Tyr Glu Asn Ser Val Ser Asp Lys Thr Asp Glu Phe Leu Cys
           195                 200                 205
   Pro Gln Ile Arg Tyr Lys Ser Ser Thr Arg Gly Arg Cys Arg Arg His
       210                 215                 220
   Ala Gly Gly Arg Tyr Met Arg Leu Ala Arg Pro Leu Pro Leu Asp Asn
   225                 230                 235                 240
   Asn His Leu Glu Thr His Arg Val Arg Thr Val Leu Val Ser Ile Leu
                   245                 250                 255
   Ser Arg Pro Leu Ser His Pro Val Arg Asp Ala Ala Ser Leu Gly Leu
               260                 265                 270
   Thr Gln Thr Gln Asn Gln Ser Leu Glu Val Glu Lys Ile Pro Leu Gly
           275                 280                 285
   Leu Ser Ala Pro Asn Pro Met Lys Leu Ile Ser Ser Leu Asp Gly Ala
       290                 295                 300
   Lys Thr Leu Asn Ala Asn Asn Met Asp Thr Leu Ile Glu Cys Gln Ser
   305                 310                 315                 320
   Glu Gly Asp Ile Lys Val Leu Pro Leu Leu Thr Ser Cys Glu Ser Glu
                   325                 330                 335
   Asp Ser Ile Cys Gln Leu Ile Glu Val Lys Lys Arg Lys Lys Val Leu
               340                 345                 350
   Ser Trp Pro Ser Leu Met Arg Lys Leu Ser Pro Ser Pro Asp Phe Ser
   355 360 365
   Gly Ser Leu Glu Pro Glu Leu Lys Val Ser Leu Phe Asp Gln Pro Leu
   370 375 380
   Ser Ile Ile Cys Lys Glu Asn Asp Thr Leu Pro Arg Pro Ile Gln Asp
   385 390 395 400
   Ile Leu Thr Ile Leu Cys Leu Lys Gly Pro Ser Thr Glu Gly Ile Phe
   405 410 415
   Arg Lys Ala Ala Ser Glu Lys Ala Arg Lys Glu Leu Lys Glu Glu Leu
   420 425 430
   Asn Cys Gly Gly Ser Val Asn Leu Asn Gln Leu Pro Val His Leu Leu
   435 440 445
   Ala Val Val Phe Lys Asp Phe Leu Arg Gly Ile Pro Leu Lys Leu Leu
   450 455 460
   Ser Cys Asp Leu Phe Glu Asp Trp Met Gly Ala Leu Glu Lys Pro Ser
   465 470 475 480
   Glu Glu Asp Arg Ile Glu Ala Leu Lys Gln Val Ala Asp Arg Leu Pro
   485 490 495
   Arg Pro Asn Leu Leu Leu Leu Arg Thr Leu Val Tyr Val Leu His Leu
   500 505 510
   Ile Ser Lys Asn Ala Glu Val Asn Lys Met Asp Ser Ser Asn Leu Ala
   515 520 525
   Ile Cys Ile Gly Pro Asn Met Leu Thr Leu Lys Asn Asp Gln Ser Leu
   530 535 540
   Ser Phe Gln Ala Gln Arg Asp Leu Asn Asn Lys Val Lys Ile Leu Val
   545 550 555 560
   Glu Phe Leu Ile Asp Asn Cys Leu Glu Ile Phe Gly Glu Asn Ile Pro
   565 570 575
   Thr His Pro Arg Ile Thr Ser Asp Asp Ser Leu Glu His Thr Asp Ser
   580 585 590
   Ser Asp Val Ser Thr Leu Gln Asn Asp Ser Ala Tyr Asp Ser Asn Asp
   595 600 605
   Pro Asp Val Glu Pro Ala Asn Gly Val Ala Ser Pro Cys Arg Gln Leu
       610                 615                 620
   Glu Gly Pro Ser Thr Thr Val Ala Gly Met Asp Thr Arg Gly Pro Arg
   625                 630                 635                 640
   Asn Thr Cys Glu Ser Ser Ser Glu Ser Ser Val Ser Met Val Ala Arg
                   645                 650                 655
   Leu Lys Ser Ser Ile Gly Gln Gln Asp Arg Arg Phe Ser Glu Pro Asn
               660                 665                 670
   Met Pro Pro Ser Arg Glu Cys Phe Val Gly Pro Ile Thr Lys Gln Lys
           675                 680                 685
   Leu Thr Arg Ser Glu Asp Ser Phe Val Leu Pro Gln Glu Ala Ser Cys
       690                 695                 700
   Ser Glu Gly Asn Glu Ala Glu Asp Pro Phe Thr Glu Glu Val Phe Pro
   705                 710                 715                 720
   Ala Val Glu Gly Lys Pro Met Arg Pro Val Asp Leu Lys Ile Lys Asn
                   725                 730                 735
   Leu Thr Gln Gly Leu Ala Ser Pro Gln Gly Pro Ala Asn Lys Ala Phe
               740                 745                 750
   Ser Ser Phe Ser Ala Gly Glu Ser Leu Asp Ser Ser Pro Val Pro Ser
           755                 760                 765
   Pro Ser Cys Pro Lys Arg Asn Phe Phe Thr Arg His Gln Ser Phe Thr
       770                 775                 780
   Thr Lys Thr Asp Lys Thr Lys Pro Gln Arg Glu Ile Arg Lys His Ser
   785                 790                 795                 800
   Met Ser Phe Ser Phe Ala Ser His Lys Lys Val Leu Pro Arg Thr Ser
                   805                 810                 815
   Ser Ile Gly Ser Glu Lys Ser Lys Asp Phe Ser Arg Asp Gln Leu Gln
               820                 825                 830
   Lys Asp Leu Arg Lys Glu Ser Gln Leu Ala Gly Arg Ile Ile Arg Glu
           835                 840                 845
   Asn Glu Ser Glu Ile Gln Ser Gln Thr Asn Leu Gly Ser Ser Leu Ser
       850                 855                 860
   Gly Thr Trp Ala Leu Ser Val Asp Asn Thr Phe Gln Phe Ile Asp Val
   865 870 875 880
   Arg Lys Pro Gly Ser Pro Pro Ser Tyr Glu Glu Ala Ile Tyr Tyr His
   885 890 895
   Thr Ser Gly Leu Thr Ala Tyr Ser Gly Gln Thr Val Gly Ser Met Arg
   900 905 910
   Ala Arg Met Leu Lys Gln Ser Met Ala Val Pro Pro Val Pro Ser His
   915 920 925
   His Glu Gly Glu Leu Ser Glu Gly Ile Pro Gly Gly His Arg Ser Ser
   930 935 940
   Ser Val Thr Glu His Trp Thr Gln Ser Gln Thr Val His Val Ser Val
   945 950 955 960
   Glu Thr Arg Gly Arg Ser Glu Leu His Arg Leu Arg Thr Val Ser Glu
   965 970 975
   Ser Val Gln Arg Ala Lys Leu Asp Tyr Leu Gly Gln Gln Arg Ser His
   980 985 990
   Leu Val Phe Glu Val Asp Gln Leu Arg Cys Ala Lys Glu Ser Tyr Ile
   995 1000 1005
<210> 25
   <211> 727
   <212> PRT
   <213> Fgd2 mouse transcript variant 3
<400> 25
   Met Glu Arg Ala Cys Glu Lys Gln Asp Ser Val Cys Asn Leu Val Ala
   1 5 10 15
   Val Phe Glu Asn Asn Arg Thr Pro Gly Glu Ala Pro Gly Ser His Ser
   20 25 30
   Leu Glu Asp Gln Pro His Ile Pro Glu His Gln Leu Ser Leu Ser Pro
   35 40 45
   Glu Pro Trp Glu Ala Pro Pro Val Lys Glu Ala Leu Lys Ser Glu Phe
   50 55 60
   Arg Pro Val Ser Arg Thr Tyr Leu Ser Ser Leu Lys Asn Lys Leu Ser
   65 70 75 80
   Ser Gly Ala Trp Arg Arg Ser Cys Gln Pro Gly Val Ser Pro Gly Pro
   85 90 95
   Glu Thr Gln Glu Pro Glu Glu Lys Arg Val Val Arg Glu Leu Leu Glu
   100 105 110
   Thr Glu Gln Ala Tyr Val Ala Arg Leu His Leu Leu Asp Gln Val Phe
   115 120 125
   Phe Gln Glu Leu Leu Arg Glu Ala Gly Arg Ser Lys Ala Phe Pro Glu
   130 135 140
   Asp Val Val Lys Leu Ile Phe Ser Asn Ile Ser Ser Ile Tyr Arg Phe
   145 150 155 160
   His Ala Gln Phe Phe Leu Pro Glu Leu Gln Arg Arg Val Asp Asp Trp
   165 170 175
   Ala Ala Thr Pro Arg Ile Gly Asp Val Ile Gln Lys Leu Ala Pro Phe
   180 185 190
   Leu Lys Met Tyr Ser Glu Tyr Val Lys Asn Phe Glu Arg Ala Ala Glu
   195 200 205
   Leu Leu Ala Thr Trp Met Asp Lys Ser Gln Pro Phe Gln Glu Val Val
   210 215 220
   Thr Arg Ile Gln Cys Ser Glu Ala Ser Ser Ser Leu Thr Leu Gln His
   225 230 235 240
   His Met Leu Glu Pro Val Gln Arg Ile Pro Arg Tyr Glu Leu Leu Leu
   245 250 255
   Lys Glu Tyr Val Gln Lys Leu Pro Ala Gln Ala Pro Asp Leu Glu Asp
   260 265 270
   Ala Gln Arg Ala Leu Asp Met Ile Phe Ser Ala Ala Gln His Ser Asn
   275 280 285
   Ala Ala Ile Ala Glu Met Glu Arg Leu Gln Gly Leu Trp Asp Val Tyr
   290 295 300
   Gln Arg Leu Gly Leu Glu Asp Asp Ile Val Asp Pro Ser Asn Thr Leu
   305 310 315 320
   Leu Arg Glu Gly Pro Val Leu Lys Ile Ser Phe Arg Arg Ser Asp Pro
   325 330 335
   Met Glu Arg Tyr Leu Val Leu Phe Asn Asn Met Leu Leu Tyr Cys Val
   340 345 350
   Pro Arg Val Leu Gln Val Gly Ala Gln Phe Gln Val Arg Thr Arg Ile
   355 360 365
   Asp Val Ala Gly Met Lys Val Arg Glu Leu Thr Asp Ala Glu Phe Pro
   370 375 380
   His Ser Phe Leu Val Ser Gly Lys Gln Arg Thr Leu Glu Leu Gln Ala
   385 390 395 400
   Arg Ser Arg Asp Glu Met Val Ser Trp Met Gln Ala Cys Gln Ala Ala
   405 410 415
   Ile Asp Gln Val Glu Lys Arg Ser Glu Thr Phe Lys Ala Ala Val Gln
   420 425 430
   Gly Pro Gln Gly Asp Thr Gln Glu Pro Lys Pro Gln Val Glu Glu Leu
   435 440 445
   Gly Leu Arg Ala Pro Gln Trp Val Arg Asp Lys Met Val Thr Met Cys
   450 455 460
   Met Arg Cys Gln Glu Pro Phe Asn Ala Leu Thr Arg Arg Arg His His
   465 470 475 480
   Cys Arg Ala Cys Gly Tyr Val Val Cys Ala Lys Cys Ser Asp Tyr Arg
   485 490 495
   Ala Glu Leu Lys Tyr Asp Ser Asn Arg Pro Asn Arg Val Cys Leu Thr
   500 505 510
   Cys Tyr Thr Phe Leu Thr Gly Asn Val Leu Pro Gln Gly Lys Glu Asp
   515 520 525
   Lys Arg Arg Gly Ile Leu Glu Lys Glu Ala Ser Ala Ala Pro Glu Gln
   530 535 540
   Ser Leu Val Cys Ser Phe Leu Gln Leu Ile Gly Asp Lys Cys Ser Arg
   545 550 555 560
   Ser Leu Pro Arg Ser Trp Cys Val Ile Pro Arg Asp Asp Pro Leu Val
   565 570 575
   Leu Tyr Val Tyr Ala Ala Pro Gln Asp Thr Lys Ala His Thr Ser Ile
   580 585 590
   Pro Leu Leu Gly Tyr Gln Val Ile Ser Gly Pro Gln Gly Thr Ser Gly
   595 600 605
   Phe Pro Ala Ala Thr Val Arg Arg Ala Val His Leu Gln Gly Arg Val
   610 615 620
   Cys Gly Ala Ala Gly Pro Leu Gly Asp Ser Tyr Gln Ala Cys Ala Ser
   625 630 635 640
   Gly Thr Pro Glu Asp Leu Thr Lys Lys Met Cys Leu Thr Glu Pro Ala
   645 650 655
   Ala Ser Cys Ser Ser Arg Val His Asp Ser Leu Pro Arg Pro Thr Pro
   660 665 670
   Trp Phe Tyr His Phe Thr Pro Ser Trp Ala Thr Pro Asp Pro Pro Asp
   675 680 685
   Val Tyr Thr Glu Thr Ile His Pro Asp Ser Val Ser Ser Arg His Arg
   690 695 700
   Pro Phe Pro Ser Gly Arg Tyr His Gln Val Ser Gln Leu Ala Gly Glu
   705 710 715 720
   Gly Ala Asp Ile Pro Gly Ser
   725
<210> 26
   <211> 655
   <212> PRT
   <213> Fgd2 mouse transcript variant 1
<400> 26
   Met Glu Arg Ala Cys Glu Lys Gln Asp Ser Val Cys Asn Leu Val Ala
   1 5 10 15
   Val Phe Glu Asn Asn Arg Thr Pro Gly Glu Ala Pro Gly Ser His Ser
   20 25 30
   Leu Glu Asp Gln Pro His Ser Pro Glu His Gln Leu Ser Leu Ser Pro
   35 40 45
   Glu Pro Trp Glu Ala Pro Pro Val Lys Glu Ala Leu Lys Ser Glu Phe
   50 55 60
   Arg Pro Val Ser Arg Thr Tyr Leu Ser Ser Leu Lys Asn Lys Leu Ser
   65 70 75 80
   Ser Gly Ala Trp Arg Arg Ser Cys Gln Pro Gly Val Ser Pro Gly Pro
   85 90 95
   Glu Thr Gln Glu Pro Glu Glu Lys Arg Val Val Arg Glu Leu Leu Glu
   100 105 110
   Thr Glu Gln Ala Tyr Val Ala Arg Leu His Leu Leu Asp Gln Val Phe
   115 120 125
   Phe Gln Glu Leu Leu Arg Glu Ala Gly Arg Ser Lys Ala Phe Pro Glu
   130 135 140
   Asp Val Val Lys Leu Ile Phe Ser Asn Ile Ser Ser Ile Tyr Arg Phe
   145 150 155 160
   His Ala Gln Phe Phe Leu Pro Glu Leu Gln Arg Arg Val Asp Asp Trp
   165 170 175
   Ala Ala Thr Pro Arg Ile Gly Asp Val Ile Gln Lys Leu Ala Pro Phe
   180 185 190
   Leu Lys Met Tyr Ser Glu Tyr Val Lys Asn Phe Glu Arg Ala Ala Glu
   195 200 205
   Leu Leu Ala Thr Trp Met Asp Lys Ser Gln Pro Phe Gln Glu Val Val
   210 215 220
   Thr Arg Ile Gln Cys Ser Glu Ala Ser Ser Ser Leu Thr Leu Gln His
   225 230 235 240
   His Met Leu Glu Pro Val Gln Arg Ile Pro Arg Tyr Glu Leu Leu Leu
   245 250 255
   Lys Glu Tyr Val Gln Lys Leu Pro Ala Gln Ala Pro Asp Leu Glu Asp
   260 265 270
   Ala Gln Arg Ala Leu Asp Met Ile Phe Ser Ala Ala Gln His Ser Asn
   275 280 285
   Ala Ala Ile Ala Glu Met Glu Arg Leu Gln Gly Leu Trp Asp Val Tyr
   290 295 300
   Gln Arg Leu Gly Leu Glu Asp Asp Ile Val Asp Pro Ser Asn Thr Leu
   305 310 315 320
   Leu Arg Glu Gly Pro Val Leu Lys Ile Ser Phe Arg Arg Ser Asp Pro
   325 330 335
   Met Glu Arg Tyr Leu Val Leu Phe Asn Asn Met Leu Leu Tyr Cys Val
   340 345 350
   Pro Arg Val Leu Gln Val Gly Ala Gln Phe Gln Val Arg Thr Arg Ile
           355                 360                 365
   Asp Val Ala Gly Met Lys Val Arg Glu Leu Thr Asp Ala Glu Phe Pro
       370                 375                 380
   His Ser Phe Leu Val Ser Gly Lys Gln Arg Thr Leu Glu Leu Gln Ala
   385                 390                 395                 400
   Arg Ser Arg Asp Glu Met Val Ser Trp Met Gln Ala Cys Gln Ala Ala
                   405                 410                 415
   Ile Asp Gln Val Glu Lys Arg Ser Glu Thr Phe Lys Ala Ala Val Gln
               420                 425                 430
   Gly Pro Gln Gly Asp Thr Gln Glu Pro Lys Pro Gln Val Glu Glu Leu
           435                 440                 445
   Gly Leu Arg Ala Pro Gln Trp Val Arg Asp Lys Met Val Thr Met Cys
       450                 455                 460
   Met Arg Cys Gln Glu Pro Phe Asn Ala Leu Thr Arg Arg Arg His His
   465                 470                 475                 480
   Cys Arg Ala Cys Gly Tyr Val Val Cys Ala Lys Cys Ser Asp Tyr Arg
                   485                 490                 495
   Ala Glu Leu Lys Tyr Asp Ser Asn Arg Pro Asn Arg Val Cys Leu Thr
               500                 505                 510
   Cys Tyr Thr Phe Leu Thr Gly Asn Val Leu Pro Gln Gly Lys Glu Asp
           515                 520                 525
   Lys Arg Arg Gly Ile Leu Glu Lys Glu Ala Ser Ala Ala Pro Glu Gln
       530                 535                 540
   Ser Leu Val Cys Ser Phe Leu Gln Leu Ile Gly Asp Lys Cys Ser Arg
   545                 550                 555                 560
   Ser Leu Pro Arg Ser Trp Cys Val Ile Pro Arg Asp Asp Pro Leu Val
                   565                 570                 575
   Leu Tyr Val Tyr Ala Ala Pro Gln Asp Thr Lys Ala His Thr Ser Ile
               580                 585                 590
   Pro Leu Leu Gly Tyr Gln Val Ile Ser Gly Pro Gln Gly Asp Pro Arg
           595                 600                 605
   Val Phe Gln Leu Gln Gln Ser Gly Gln Gln Tyr Thr Phe Lys Ala Glu
   610 615 620
   Ser Val Glu Leu Gln Gly Arg Trp Val Thr Ala Ile Lys Arg Ala Ala
   625 630 635 640
   Ser Gly Arg Thr Pro Glu Gly Pro Asp Glu Glu Asp Val Ser Asp
   645 650 655
<210> 27
   <211> 461
   <212> PRT
   <213> Fgd2 mouse transcript variant 2
<400> 27
   Met Tyr Ser Glu Tyr Val Lys Asn Phe Glu Arg Ala Ala Glu Leu Leu
   1 5 10 15
   Ala Thr Trp Met Asp Lys Ser Gln Pro Phe Gln Glu Val Val Thr Arg
   20 25 30
   Ile Gln Cys Ser Glu Ala Ser Ser Ser Leu Thr Leu Gln His His Met
   35 40 45
   Leu Glu Pro Val Gln Arg Ile Pro Arg Tyr Glu Leu Leu Leu Lys Glu
   50 55 60
   Tyr Val Gln Lys Leu Pro Ala Gln Ala Pro Asp Leu Glu Asp Ala Gln
   65 70 75 80
   Arg Ala Leu Asp Met Ile Phe Ser Ala Ala Gln His Ser Asn Ala Ala
   85 90 95
   Ile Ala Glu Met Glu Arg Leu Gln Gly Leu Trp Asp Val Tyr Gln Arg
   100 105 110
   Leu Gly Leu Glu Asp Asp Ile Val Asp Pro Ser Asn Thr Leu Leu Arg
   115 120 125
   Glu Gly Pro Val Leu Lys Ile Ser Phe Arg Arg Ser Asp Pro Met Glu
   130 135 140
   Arg Tyr Leu Val Leu Phe Asn Asn Met Leu Leu Tyr Cys Val Pro Arg
   145 150 155 160
   Val Leu Gln Val Gly Ala Gln Phe Gln Val Arg Thr Arg Ile Asp Val
   165 170 175
   Ala Gly Met Lys Val Arg Glu Leu Thr Asp Ala Glu Phe Pro His Ser
   180 185 190
   Phe Leu Val Ser Gly Lys Gln Arg Thr Leu Glu Leu Gln Ala Arg Ser
   195 200 205
   Arg Asp Glu Met Val Ser Trp Met Gln Ala Cys Gln Ala Ala Ile Asp
   210 215 220
   Gln Val Glu Lys Arg Ser Glu Thr Phe Lys Ala Ala Val Gln Gly Pro
   225 230 235 240
   Gln Gly Asp Thr Gln Glu Pro Lys Pro Gln Val Glu Glu Leu Gly Leu
   245 250 255
   Arg Ala Pro Gln Trp Val Arg Asp Lys Met Val Thr Met Cys Met Arg
   260 265 270
   Cys Gln Glu Pro Phe Asn Ala Leu Thr Arg Arg Arg His His Cys Arg
   275 280 285
   Ala Cys Gly Tyr Val Val Cys Ala Lys Cys Ser Asp Tyr Arg Ala Glu
   290 295 300
   Leu Lys Tyr Asp Ser Asn Arg Pro Asn Arg Val Cys Leu Thr Cys Tyr
   305 310 315 320
   Thr Phe Leu Thr Gly Asn Val Leu Pro Gln Gly Lys Glu Asp Lys Arg
   325 330 335
   Arg Gly Ile Leu Glu Lys Glu Ala Ser Ala Ala Pro Glu Gln Ser Leu
   340 345 350
   Val Cys Ser Phe Leu Gln Leu Ile Gly Asp Lys Cys Ser Arg Ser Leu
   355 360 365
   Pro Arg Ser Trp Cys Val Ile Pro Arg Asp Asp Pro Leu Val Leu Tyr
   370 375 380
   Val Tyr Ala Ala Pro Gln Asp Thr Lys Ala His Thr Ser Ile Pro Leu
   385 390 395 400
   Leu Gly Tyr Gln Val Ile Ser Gly Pro Gln Gly Asp Pro Arg Val Phe
   405 410 415
   Gln Leu Gln Gln Ser Gly Gln Gln Tyr Thr Phe Lys Ala Glu Ser Val
   420 425 430
   Glu Leu Gln Gly Arg Trp Val Thr Ala Ile Lys Arg Ala Ala Ser Gly
   435 440 445
   Arg Thr Pro Glu Gly Pro Asp Glu Glu Asp Val Ser Asp
   450 455 460
<210> 28
   <211> 656
   <212> PRT
   <213> Fgd2 mouse transcript variant 1 t6/tw5
<400> 28
   Met Glu Arg Ala Cys Glu Lys Gln Asp Ser Val Cys Asn Leu Val Ala
   1 5 10 15
   Val Phe Glu Asn Asn Ser Arg Thr Pro Gly Glu Ala Pro Gly Ser His
   20 25 30
   Ser Leu Glu Asp Gln Leu His Ser Pro Glu His Gln Leu Ser Leu Ser
   35 40 45
   Pro Glu Pro Trp Glu Ala Pro Pro Val Lys Glu Ala Leu Lys Ser Glu
   50 55 60
   Phe Arg Pro Val Ser Arg Thr Tyr Leu Ser Ser Leu Lys Asn Lys Leu
   65 70 75 80
   Ser Ser Gly Ala Trp Arg Arg Ser Cys Gln Pro Gly Val Ser Pro Gly
   85 90 95
   Pro Glu Thr Gln Glu Pro Glu Glu Lys Arg Val Val Arg Glu Leu Leu
   100 105 110
   Glu Thr Glu Gln Ala Tyr Val Ala Arg Leu His Leu Leu Asp Gln Val
   115 120 125
   Phe Phe Gln Glu Leu Leu Arg Glu Ala Gly Arg Ser Lys Ala Phe Pro
   130 135 140
   Glu Asp Val Val Lys Leu Ile Phe Ser Asn Ile Ser Ser Ile Tyr Arg
   145 150 155 160
   Phe His Ala Gln Phe Phe Leu Pro Glu Leu Gln Arg Arg Val Asp Asp
   165 170 175
   Trp Ala Ala Thr Pro Arg Ile Gly Asp Val Ile Gln Lys Leu Ala Pro
   180 185 190
   Phe Leu Lys Met Tyr Ser Glu Tyr Val Lys Asn Phe Glu Arg Ala Ala
           195                 200                 205
   Glu Leu Leu Ala Thr Trp Met Asp Lys Ser Gln Pro Phe Gln Glu Val
       210                 215                 220
   Val Thr Arg Ile Gln Cys Ser Glu Ala Ser Gly Ser Leu Thr Leu Gln
   225                 230                 235                 240
   His His Met Leu Glu Pro Val Gln Arg Ile Pro Arg Tyr Glu Leu Leu
                   245                 250                 255
   Leu Lys Glu Tyr Val Gln Lys Leu Pro Ala Gln Ala Pro Asp Leu Glu
               260                 265                 270
   Asp Ala Gln Arg Ala Leu Asp Met Ile Phe Ser Ala Ala Gln His Ser
           275                 280                 285
   Asn Ala Ala Ile Ala Glu Met Glu Arg Leu Gln Gly Leu Trp Asp Val
       290                 295                 300
   Tyr Gln Arg Leu Gly Leu Glu Asp Asp Ile Val Asp Pro Ser Asn Thr
   305                 310                 315                 320
   Leu Leu Arg Glu Gly Pro Val Leu Lys Ile Ser Phe Arg Arg Ser Asp
                   325                 330                 335
   Pro Met Glu Arg Tyr Leu Val Leu Phe Asn Asn Met Leu Leu Tyr Cys
               340                 345                 350
   Val Pro Arg Val Leu Gln Val Gly Ala Gln Phe Gln Val Arg Thr Arg
           355                 360                 365
   Ile Asp Val Ala Gly Met Lys Val Arg Glu Leu Thr Asp Ala Glu Phe
       370                 375                 380
   Pro His Ser Phe Leu Val Ser Gly Lys Gln Arg Thr Leu Glu Leu Gln
   385                 390                 395                 400
   Ala Arg Ser Arg Asp Glu Met Val Ser Trp Met Gln Ala Cys Gln Ala
                   405                 410                 415
   Ala Ile Asp Gln Val Glu Lys Arg Ser Glu Thr Phe Lys Ala Ala Val
               420                 425                 430
   Gln Gly Pro Gln Gly Asp Thr Gln Glu Pro Lys Pro Gln Val Glu Glu
           435                 440                 445
   Leu Gly Leu Arg Ala Pro Gln Trp Val Arg Asp Lys Met Val Thr Met
   450 455 460
   Cys Met Arg Cys Gln Glu Pro Phe Asn Ala Leu Thr Arg Arg Arg His
   465 470 475 480
   His Cys Arg Ala Cys Gly Tyr Val Val Cys Ala Lys Cys Ser Asp Tyr
   485 490 495
   Arg Ala Glu Leu Lys Tyr Asp Ser Asn Arg Pro Asn Arg Val Cys Leu
   500 505 510
   Thr Cys Tyr Thr Phe Leu Thr Gly Asn Val Leu Pro Gln Gly Lys Glu
   515 520 525
   Asp Lys Arg Arg Gly Ile Leu Glu Lys Glu Ala Ser Ala Ala Pro Glu
   530 535 540
   Gln Ser Leu Val Cys Ser Phe Leu Gln Leu Ile Gly Asp Lys Cys Ser
   545 550 555 560
   Arg Ser Leu Pro Arg Ser Trp Cys Val Ile Pro Arg Asp Asp Pro Leu
   565 570 575
   Val Leu Tyr Val Tyr Ala Ala Pro Gln Asp Thr Lys Ala His Thr Ser
   580 585 590
   Ile Pro Leu Leu Gly Tyr Gln Val Ile Ser Gly Pro Gln Gly Asp Pro
   595 600 605
   Arg Val Phe Gln Leu Gln Gln Ser Gly Gln Gln Tyr Thr Phe Lys Ala
   610 615 620
   Glu Ser Val Glu Leu Gln Gly Arg Trp Val Thr Ala Ile Lys Arg Ala
   625 630 635 640
   Ala Ser Gly Arg Thr Pro Glu Gly Pro Asp Glu Glu Asp Val Ser Asp
   645 650 655
<210> 29
   <211> 1714
   <212> PRT
   <213> TIAM2 mouse wildtype
<400> 29
   Met Gly Asn Ser Glu Ser Gln Tyr Thr Phe Gln Gly Ser Lys Asn His
   1 5 10 15
   Ser Asn Thr Val Thr Gly Ala Lys Gln Lys Pro Cys Ser Leu Lys Ile
   20 25 30
   Arg Ser Val His Ala Lys Asp Glu Lys Ser Leu His Gly Trp Thr His
   35 40 45
   Gly Ser Ser Gly Ala Gly Tyr Lys Ser Arg Ser Leu Ala Arg Ser Cys
   50 55 60
   Leu Ser His Phe Lys Asn His Gln Pro Tyr Ala Thr Arg Leu Ser Gly
   65 70 75 80
   Pro Thr Cys Lys Val Ser Lys Gly Thr Thr Tyr Ser Lys His Arg Ala
   85 90 95
   Asn Thr Pro Gly Asn Asp Phe Gln Gly Asn Ser Gly Ala Phe Leu Pro
   100 105 110
   Glu Asn Gly Phe His Tyr Val Asp Arg Glu Ser Glu Glu Ser His Ile
   115 120 125
   Thr Ser Asn Gly His Leu Leu Thr Cys Tyr Gly Arg Lys Glu Ser Leu
   130 135 140
   Ala Ser Thr Pro Pro Gly Glu Asp His Arg Ser Pro Arg Val Leu Ile
   145 150 155 160
   Lys Thr Leu Gly Lys Leu Asp Gly Cys Leu Arg Val Glu Phe His Asn
   165 170 175
   Gly Gly Asn Pro His Lys Gly Thr Ser Glu Asp Pro Ser Gly Pro Val
   180 185 190
   Arg Leu Leu Arg Tyr Ser Pro Thr Leu Ala Ser Glu Thr Cys Pro Val
   195 200 205
   Arg Glu Thr Arg Arg His Ser Ala Ala Gly Ser Pro Ser Ser Gln Arg
   210 215 220
   Pro Ser Pro Thr Asp Ser Arg Leu Arg Ser Ser Lys Gly Ser Ser Leu
   225 230 235 240
   Ser Ser Glu Ser Ser Trp Tyr Asp Ser Pro Trp Gly Asn Ala Gly Glu
   245 250 255
   Val Ser Glu Val Glu Gly Ser Phe Leu Ala Pro Ser Thr Pro Asp Pro
   260 265 270
   Ser Leu Pro Ser Ser Phe Pro Pro Ser Asp Thr Lys Lys Pro Phe Asn
   275 280 285
   Gln Ser Ser Ser Leu Ser Ser Leu Arg Glu Leu Tyr Lys Asp Pro Asn
   290 295 300
   Leu Gly Cys Arg Ser Pro Ser Gly Thr Cys Leu Ser Ser Asn Glu Tyr
   305 310 315 320
   Ile Ser Ser Gln Val Ser Leu Asn Asn Arg Val Ser Phe Ala Ser Asp
   325 330 335
   Met Asp Val Pro Ser Arg Val Asp His Arg Asp Pro Leu His Tyr Ser
   340 345 350
   Ser Phe Thr Leu Pro Cys Arg Lys Ser Lys Ala Leu Thr Glu Asp Ala
   355 360 365
   Ala Lys Lys Asp Thr Leu Lys Ala Arg Met Arg Arg Phe Ser Asp Trp
   370 375 380
   Thr Gly Ser Leu Ser Arg Lys Lys Arg Lys Leu Gln Glu Pro Arg Ser
   385 390 395 400
   Met Glu Gly Ser Glu Tyr Phe Asp Ser His Ser Asp Gly Leu Asn Ala
   405 410 415
   Glu Gly Gln Val Pro Ala Gln Thr Ser Ser Leu Leu Trp Ser Gly Gly
   420 425 430
   Ser Ala Gln Thr Leu Pro His Arg Ser Glu Ser Thr His Ala Ile Ser
   435 440 445
   Val Asp Pro Leu Arg Gln Asn Ile Tyr Glu Asn Phe Met Arg Glu Leu
   450 455 460
   Glu Met Ser Arg Ser Asn Thr Glu His Val Glu Thr Ser Thr Glu Thr
   465 470 475 480
   Met Glu Ser Ser Ser Glu Ser Val Ser Ser Leu Glu Gln Leu Asp Leu
   485 490 495
   Leu Phe Glu Lys Glu Gln Gly Val Val Arg Lys Ala Gly Trp Leu Phe
   500 505 510
   Phe Lys Pro Leu Val Thr Leu Gln Lys Glu Arg Lys Leu Glu Leu Val
   515 520 525
   Ala Arg Arg Lys Trp Lys Gln Tyr Trp Val Thr Leu Lys Gly Cys Thr
           530                 535                 540
   Leu Leu Phe Tyr Glu Thr Tyr Gly Lys Asn Ser Thr Glu Gln Asn Ser
   545                 550                 555                 560
   Ala Pro Arg Cys Ala Leu Phe Ala Glu Asp Ser Ile Val Gln Ser Val
                   565                 570                 575
   Pro Glu His Pro Lys Lys Glu His Val Phe Cys Leu Ser Asn Ser Cys
               580                 585                 590
   Gly Asp Val Tyr Leu Phe Gln Ala Thr Ser Gln Thr Asp Leu Glu Asn
           595                 600                 605
   Trp Val Thr Ala Ile His Ser Cys Ala Ser Leu Phe Ala Lys Lys His
       610                 615                 620
   Gly Lys Glu Asp Thr Val Arg Leu Leu Lys Ser Gln Thr Arg Ser Leu
   625                 630                 635                 640
   Leu Gln Lys Ile Asp Met Asp Ser Lys Met Lys Lys Met Ala Glu Leu
                   645                 650                 655
   Gln Leu Ser Val Val Ser Asp Pro Lys Asn Arg Lys Ala Ile Glu Asn
               660                 665                 670
   Gln Ile Arg Gln Trp Glu Gln Asn Leu Glu Lys Phe His Met Asp Leu
           675                 680                 685
   Phe Arg Met Arg Cys Tyr Leu Ala Ser Leu Gln Gly Gly Glu Leu Pro
       690                 695                 700
   Asn Pro Lys Ser Leu Leu Ala Ala Thr Ser Arg Pro Ser Lys Leu Ala
   705                 710                 715                 720
   Leu Gly Arg Leu Gly Val Leu Ser Val Ser Ser Phe His Ala Leu Val
                   725                 730                 735
   Cys Ser Arg Asp Asp Ser Thr Leu Arg Lys Arg Thr Leu Ser Leu Thr
               740                 745                 750
   Gln Arg Gly Lys Ser Lys Lys Gly Ile Phe Ser Ser Leu Lys Gly Leu
           755                 760                 765
   Asp Thr Leu Ala Arg Lys Gly Arg Glu Lys Arg Ala Ser Ile Thr Gln
       770                 775                 780
   Met Phe Asp Ser Ser His Ser His Gly Phe Leu Gly Thr Gln Leu Pro
   785 790 795 800
   Gln Lys Ser Thr Asn Ser Asn Lys Ala His Asp Leu His Leu Tyr Gly
   805 810 815
   Ser Ala Val Asp Ser Ala Leu Arg Asp Ser Met Trp Glu Val Gln Thr
   820 825 830
   Tyr Val His Phe Gln Asp Asn Glu Gly Val Thr Val Thr Ile Lys Pro
   835 840 845
   Glu His Arg Val Glu Asp Val Leu Ala Leu Val Cys Lys Met Arg Gln
   850 855 860
   Leu Glu Pro Thr His Tyr Gly Leu Gln Leu Arg Lys Val Val Asp Lys
   865 870 875 880
   Ser Val Glu Trp Cys Val Pro Ala Leu Tyr Glu Tyr Met Gln Glu Gln
   885 890 895
   Val Tyr Asp Glu Ile Glu Val Phe Pro Leu Ser Val Tyr Asp Val Gln
   900 905 910
   Leu Thr Lys Thr Gly Asp Met Thr Asp Phe Gly Phe Ala Val Thr Val
   915 920 925
   Gln Val Asp Glu His Gln His Leu Asn Arg Ile Phe Ile Ser Asp Val
   930 935 940
   Leu Pro Asp Ser Leu Ala Tyr Gly Gly Gly Leu Arg Lys Gly Asn Glu
   945 950 955 960
   Ile Thr Ser Leu Asn Gly Glu Pro Val Ser Asp Leu Asp Ile Gln Gln
   965 970 975
   Met Glu Ala Leu Phe Ser Glu Lys Ser Val Gly Leu Thr Leu Val Ala
   980 985 990
   Arg Pro Val Thr Thr Arg Arg Thr Leu Cys Ala Ser Trp Ser Asp Ser
   995 1000 1005
   Asp Leu Phe Ser Arg Asp Gln Lys Ser Leu Pro Pro Ser Pro Asn
   1010 1015 1020
   Gln Ser Gln Leu Leu Glu Glu Phe Leu Asp Asn Phe Arg Lys Thr
   1025 1030 1035
   Ala Thr Ser Asp Phe Ser Asn Val Pro Glu Ile Thr Thr Gly Leu
   1040 1045 1050
   Lys Arg Ser Gln Thr Glu Gly Thr Leu Asp Gln Val Pro His Arg
   1055 1060 1065
   Glu Lys Met Glu Gln Thr Phe Leu Ser Ala Asp Gln Ile Ala Glu
   1070 1075 1080
   Leu Cys Arg Asp Leu Asn Asn Thr His Thr Asn Ser Met Glu Ala
   1085 1090 1095
   Pro Thr Glu Ser His Asp Pro Pro Pro Arg Pro Leu Ala Arg His
   1100 1105 1110
   Leu Ser Asp Ala Asp Arg Leu Arg Lys Val Ile Gln Glu Leu Val
   1115 1120 1125
   Asp Thr Glu Lys Ser Tyr Val Lys Asp Leu Ser Cys Leu Phe Glu
   1130 1135 1140
   Leu Tyr Leu Glu Pro Leu Gln Asn Glu Thr Phe Leu Thr Gln Asp
   1145 1150 1155
   Glu Met Glu Ser Leu Phe Gly Ser Leu Pro Glu Met Leu Glu Phe
   1160 1165 1170
   Gln Lys Val Phe Leu Glu Thr Leu Glu Asp Ala Ile Ser Ala Ser
   1175 1180 1185
   Ser Asp Phe Ser Val Leu Glu Thr Pro Ser Gln Phe Arg Lys Leu
   1190 1195 1200
   Leu Phe Ser Leu Gly Gly Ser Phe Leu Tyr Tyr Ala Asp His Phe
   1205 1210 1215
   Lys Leu Tyr Ser Gly Phe Cys Ala Asn His Ile Lys Val Gln Arg
   1220 1225 1230
   Val Leu Glu Arg Ala Lys Thr Asp Lys Ala Phe Lys Ala Phe Leu
   1235 1240 1245
   Asp Ala Arg Asn Pro Thr Lys Gln His Ser Ser Thr Leu Glu Ser
   1250 1255 1260
   Tyr Leu Ile Lys Pro Val Gln Arg Val Leu Lys Tyr Pro Leu Leu
   1265 1270 1275
   Leu Lys Glu Leu Val Ser Leu Thr Asp His Glu Ser Glu Glu His
   1280 1285 1290
   Tyr His Leu Thr Glu Ala Leu Lys Ala Met Glu Lys Val Ala Ser
   1295 1300 1305
   His Ile Asn Glu Met Gln Lys Ile Tyr Glu Asp Tyr Gly Met Val
   1310 1315 1320
   Phe Asp Gln Leu Val Ala Glu Gln Ser Gly Thr Glu Lys Glu Val
   1325 1330 1335
   Thr Glu Leu Ser Met Gly Glu Leu Leu Met His Ser Thr Val Ser
   1340 1345 1350
   Trp Leu Asn Pro Phe Leu Ser Leu Gly Lys Ala Arg Lys Asp Ile
   1355 1360 1365
   Glu Leu Thr Val Phe Val Phe Lys Arg Ala Val Ile Leu Val Tyr
   1370 1375 1380
   Lys Glu Asn Cys Lys Leu Lys Lys Lys Leu Pro Ser Asn Ser Arg
   1385 1390 1395
   Pro Ala His Asn Ser Ala Asp Leu Asp Pro Phe Lys Phe Arg Trp
   1400 1405 1410
   Leu Ile Pro Ile Ser Ala Leu Gln Val Arg Leu Gly Asn Thr Ala
   1415 1420 1425
   Gly Thr Glu Asn Asn Ser Thr Trp Glu Leu Ile His Thr Lys Ser
   1430 1435 1440
   Glu Ile Glu Gly Arg Pro Glu Thr Ile Phe Gln Leu Cys Cys Ser
   1445 1450 1455
   Asp Ser Glu Asn Lys Thr Ser Ile Val Lys Val Ile Arg Ser Ile
   1460 1465 1470
   Leu Arg Glu Asn Phe Arg Arg His Ile Lys Cys Glu Leu Pro Leu
   1475 1480 1485
   Glu Lys Thr Cys Lys Asp Arg Leu Val Pro Leu Lys Asn Arg Val
   1490 1495 1500
   Pro Val Ser Ala Lys Leu Ala Ser Ser Arg Ser Leu Lys Gly Leu
   1505 1510 1515
   Arg Thr Ser Ser Ser Ser Glu Trp Pro Ser Glu Pro Ser Lys Gly
   1520 1525 1530
   Asn Ser Leu Asp Ser Asp Glu Cys Ser Leu Ser Ser Gly Thr Gln
   1535 1540 1545
   Ser Ser Gly Cys Pro Val Ala Glu Ser Arg Arg Asp Ser Lys Ser
   1550 1555 1560
   Thr Glu Leu Glu Lys Asp Ala Gln Glu Gly Leu Ala Glu Phe Pro
   1565 1570 1575
   Asp Gly Leu Ile Lys Glu Ser Asp Ile Leu Ser Asp Glu Asp Glu
   1580 1585 1590
   Asp Phe His His Pro Leu Lys Gln Gly Ser Pro Thr Lys Asp Ile
   1595 1600 1605
   Glu Ile Gln Phe Gln Arg Leu Lys Ile Ser Glu Glu Ser Asp Val
   1610 1615 1620
   His Pro Val Gly Gln Gln Pro Leu Thr Glu Ser Gly Glu Gln Pro
   1625 1630 1635
   Lys Leu Val Arg Gly His Phe Cys Pro Ile Lys Arg Lys Ala Asn
   1640 1645 1650
   Ser Thr Lys Arg Gly Arg Gly Thr Leu Leu Lys Ala Gln Thr Arg
   1655 1660 1665
   His Gln Ser Leu Asp Ser His Pro Glu Thr Ala Ser Ile Asp Leu
   1670 1675 1680
   Asn Leu Val Leu Glu Arg Glu Phe Ser Val Gln Ser Leu Thr Ser
   1685 1690 1695
   Val Val Asn Glu Glu Gly Phe Tyr Glu Thr Gln Ser His Gly Lys
   1700 1705 1710
   Ser
<210> 30
   <211> 1701
   <212> PRT
   <213> TIAM2 homo sapiens
<400> 30
   Met Gly Asn Ser Asp Ser Gln Tyr Thr Leu Gln Gly Ser Lys Asn His
   1 5 10 15
   Ser Asn Thr Ile Thr Gly Ala Lys Gln Ile Pro Cys Ser Leu Lys Ile
               20                  25                  30
   Arg Gly Ile His Ala Lys Glu Glu Lys Ser Leu His Gly Trp Gly His
           35                  40                  45
   Gly Ser Asn Gly Ala Gly Tyr Lys Ser Arg Ser Leu Ala Arg Ser Cys
       50                  55                  60
   Leu Ser His Phe Lys Ser Asn Gln Pro Tyr Ala Ser Arg Leu Gly Gly
   65                  70                  75                  80
   Pro Thr Cys Lys Val Ser Arg Gly Val Ala Tyr Ser Thr His Arg Thr
                   85                  90                  95
   Asn Ala Pro Gly Lys Asp Phe Gln Gly Ile Ser Ala Ala Phe Ser Thr
               100                 105                 110
   Glu Asn Gly Phe His Ser Val Gly His Glu Leu Ala Asp Asn His Ile
           115                 120                 125
   Thr Ser Arg Asp Cys Asn Gly His Leu Leu Asn Cys Tyr Gly Arg Asn
       130                 135                 140
   Glu Ser Ile Ala Ser Thr Pro Pro Gly Glu Asp Arg Lys Ser Pro Arg
   145                 150                 155                 160
   Val Leu Ile Lys Thr Leu Gly Lys Leu Asp Gly Cys Leu Arg Val Glu
                   165                 170                 175
   Phe His Asn Gly Gly Asn Pro Ser Lys Val Pro Ala Glu Asp Cys Ser
               180                 185                 190
   Glu Pro Val Gln Leu Leu Arg Tyr Ser Pro Thr Leu Ala Ser Glu Thr
           195                 200                 205
   Ser Pro Val Pro Glu Ala Arg Arg Gly Ser Ser Ala Asp Ser Leu Pro
       210                 215                 220
   Ser His Arg Pro Ser Pro Thr Asp Ser Arg Leu Arg Ser Ser Lys Gly
   225                 230                 235                 240
   Ser Ser Leu Ser Ser Glu Ser Ser Trp Tyr Asp Ser Pro Trp Gly Asn
                   245                 250                 255
   Ala Gly Glu Leu Ser Glu Ala Glu Gly Ser Phe Leu Ala Pro Gly Met
               260                 265                 270
   Pro Asp Pro Ser Leu His Ala Ser Phe Pro Pro Gly Asp Ala Lys Lys
   275 280 285
   Pro Phe Asn Gln Ser Ser Ser Leu Ser Ser Leu Arg Glu Leu Tyr Lys
   290 295 300
   Asp Ala Asn Leu Gly Ser Leu Ser Pro Ser Gly Ile Arg Leu Ser Asp
   305 310 315 320
   Glu Tyr Met Gly Thr His Ala Ser Leu Ser Asn Arg Val Ser Phe Ala
   325 330 335
   Ser Asp Ile Asp Val Pro Ser Arg Val Ala His Gly Asp Pro Ile Gln
   340 345 350
   Tyr Ser Ser Phe Thr Leu Pro Cys Arg Lys Pro Lys Ala Phe Val Glu
   355 360 365
   Asp Thr Ala Lys Lys Asp Ser Leu Lys Ala Arg Met Arg Arg Ile Ser
   370 375 380
   Asp Trp Thr Gly Ser Leu Ser Arg Lys Lys Arg Lys Leu Gln Glu Pro
   385 390 395 400
   Arg Ser Lys Glu Gly Ser Asp Tyr Phe Asp Ser Arg Ser Asp Gly Leu
   405 410 415
   Asn Thr Asp Val Gln Gly Ser Ser Gln Ala Ser Ala Phe Leu Trp Ser
   420 425 430
   Gly Gly Ser Thr Gln Ile Leu Ser Gln Arg Ser Glu Ser Thr His Ala
   435 440 445
   Ile Gly Ser Asp Pro Leu Arg Gln Asn Ile Tyr Glu Asn Phe Met Arg
   450 455 460
   Glu Leu Glu Met Ser Arg Thr Asn Thr Glu Asn Ile Glu Thr Ser Thr
   465 470 475 480
   Glu Thr Ala Glu Ser Ser Ser Glu Ser Leu Ser Ser Leu Glu Gln Leu
   485 490 495
   Asp Leu Leu Phe Glu Lys Glu Gln Gly Val Val Arg Lys Ala Gly Trp
   500 505 510
   Leu Phe Phe Lys Pro Leu Val Thr Val Gln Lys Glu Arg Lys Leu Glu
   515 520 525
   Leu Val Ala Arg Arg Lys Trp Lys Gln Tyr Trp Val Thr Leu Lys Gly
   530 535 540
   Cys Thr Leu Leu Phe Tyr Glu Thr Tyr Gly Lys Asn Ser Met Asp Gln
   545 550 555 560
   Ser Ser Ala Pro Arg Cys Ala Leu Phe Ala Glu Asp Ser Ile Val Gln
   565 570 575
   Ser Val Pro Glu His Pro Lys Lys Glu Asn Val Phe Cys Leu Ser Asn
   580 585 590
   Ser Phe Gly Asp Val Tyr Leu Phe Gln Ala Thr Ser Gln Thr Asp Leu
   595 600 605
   Glu Asn Trp Val Thr Ala Val His Ser Ala Cys Ala Ser Leu Phe Ala
   610 615 620
   Lys Lys His Gly Lys Glu Asp Thr Leu Arg Leu Leu Lys Asn Gln Thr
   625 630 635 640
   Lys Asn Leu Leu Gln Lys Ile Asp Met Asp Ser Lys Met Lys Lys Met
   645 650 655
   Ala Glu Leu Gln Leu Ser Val Val Ser Asp Pro Lys Asn Arg Lys Ala
   660 665 670
   Ile Glu Asn Gln Ile Gln Gln Trp Glu Gln Asn Leu Glu Lys Phe His
   675 680 685
   Met Asp Leu Phe Arg Met Arg Cys Tyr Leu Ala Ser Leu Gln Gly Gly
   690 695 700
   Glu Leu Pro Asn Pro Lys Ser Leu Leu Ala Ala Ala Ser Arg Pro Ser
   705 710 715 720
   Lys Leu Ala Leu Gly Arg Leu Gly Ile Leu Ser Val Ser Ser Phe His
   725 730 735
   Ala Leu Val Cys Ser Arg Asp Asp Ser Ala Leu Arg Lys Arg Thr Leu
   740 745 750
   Ser Leu Thr Gln Arg Gly Arg Asn Lys Lys Gly Ile Phe Ser Ser Leu
   755 760 765
   Lys Gly Leu Asp Thr Leu Ala Arg Lys Gly Lys Glu Lys Arg Pro Ser
   770 775 780
   Ile Thr Gln Val Asp Glu Leu Leu His Ile Tyr Gly Ser Thr Val Asp
   785                 790                 795                 800
   Gly Val Pro Arg Asp Asn Ala Trp Glu Ile Gln Thr Tyr Val His Phe
                   805                 810                 815
   Gln Asp Asn His Gly Val Thr Val Gly Ile Lys Pro Glu His Arg Val
               820                 825                 830
   Glu Asp Ile Leu Thr Leu Ala Cys Lys Met Arg Gln Leu Glu Pro Ser
           835                 840                 845
   His Tyr Gly Leu Gln Leu Arg Lys Leu Val Asp Asp Asn Val Glu Tyr
       850                 855                 860
   Cys Ile Pro Ala Pro Tyr Glu Tyr Met Gln Gln Gln Val Tyr Asp Glu
   865                 870                 875                 880
   Ile Glu Val Phe Pro Leu Asn Val Tyr Asp Val Gln Leu Thr Lys Thr
                   885                 890                 895
   Gly Ser Val Cys Asp Phe Gly Phe Ala Val Thr Ala Gln Val Asp Glu
               900                 905                 910
   Arg Gln His Leu Ser Arg Ile Phe Ile Ser Asp Val Leu Pro Asp Gly
           915                 920                 925
   Leu Ala Tyr Gly Glu Gly Leu Arg Lys Gly Asn Glu Ile Met Thr Leu
       930                 935                 940
   Asn Gly Glu Ala Val Ser Asp Leu Asp Leu Lys Gln Met Glu Ala Leu
   945                 950                 955                 960
   Phe Ser Glu Lys Ser Val Gly Leu Thr Leu Ile Ala Arg Pro Pro Asp
                   965                 970                 975
   Thr Lys Ala Thr Leu Cys Thr Ser Trp Ser Asp Ser Asp Leu Phe Ser
               980                 985                 990
   Arg Asp Gln Lys Ser Leu Leu Pro  Pro Pro Asn Gln Ser  Gln Leu Leu
           995                 1000                1005
   Glu Glu  Phe Leu Asp Asn Phe  Lys Lys Asn Thr Ala  Asn Asp Phe
       1010                1015                1020
   Ser Asn  Val Pro Asp Ile Thr  Thr Gly Leu Lys Arg  Ser Gln Thr
       1025                1030                1035
   Asp Gly Thr Leu Asp Gln Val Ser His Arg Glu Lys Met Glu Gln
   1040 1045 1050
   Thr Phe Arg Ser Ala Glu Gln Ile Thr Ala Leu Cys Arg Ser Phe
   1055 1060 1065
   Asn Asp Ser Gln Ala Asn Gly Met Glu Gly Pro Arg Glu Asn Gln
   1070 1075 1080
   Asp Pro Pro Pro Arg Pro Leu Ala Arg His Leu Ser Asp Ala Asp
   1085 1090 1095
   Arg Leu Arg Lys Val Ile Gln Glu Leu Val Asp Thr Glu Lys Ser
   1100 1105 1110
   Tyr Val Lys Asp Leu Ser Cys Leu Phe Glu Leu Tyr Leu Glu Pro
   1115 1120 1125
   Leu Gln Asn Glu Thr Phe Leu Thr Gln Asp Glu Met Glu Ser Leu
   1130 1135 1140
   Phe Gly Ser Leu Pro Glu Met Leu Glu Phe Gln Lys Val Phe Leu
   1145 1150 1155
   Glu Thr Leu Glu Asp Gly Ile Ser Ala Ser Ser Asp Phe Asn Thr
   1160 1165 1170
   Leu Glu Thr Pro Ser Gln Phe Arg Lys Leu Leu Phe Ser Leu Gly
   1175 1180 1185
   Gly Ser Phe Leu Tyr Tyr Ala Asp His Phe Lys Leu Tyr Ser Gly
   1190 1195 1200
   Phe Cys Ala Asn His Ile Lys Val Gln Lys Val Leu Glu Arg Ala
   1205 1210 1215
   Lys Thr Asp Lys Ala Phe Lys Ala Phe Leu Asp Ala Arg Asn Pro
   1220 1225 1230
   Thr Lys Gln His Ser Ser Thr Leu Glu Ser Tyr Leu Ile Lys Pro
   1235 1240 1245
   Val Gln Arg Val Leu Lys Tyr Pro Leu Leu Leu Lys Glu Leu Val
   1250 1255 1260
   Ser Leu Thr Asp Gln Glu Ser Glu Glu His Tyr His Leu Thr Glu
   1265 1270 1275
   Ala Leu Lys Ala Met Glu Lys Val Ala Ser His Ile Asn Glu Met
   1280 1285 1290
   Gln Lys Ile Tyr Glu Asp Tyr Gly Thr Val Phe Asp Gln Leu Val
   1295 1300 1305
   Ala Glu Gln Ser Gly Thr Glu Lys Glu Val Thr Glu Leu Ser Met
   1310 1315 1320
   Gly Glu Leu Leu Met His Ser Thr Val Ser Trp Leu Asn Pro Phe
   1325 1330 1335
   Leu Ser Leu Gly Lys Ala Arg Lys Asp Leu Glu Leu Thr Val Phe
   1340 1345 1350
   Val Phe Lys Arg Ala Val Ile Leu Val Tyr Lys Glu Asn Cys Lys
   1355 1360 1365
   Leu Lys Lys Lys Leu Pro Ser Asn Ser Arg Pro Ala His Asn Ser
   1370 1375 1380
   Thr Asp Leu Asp Pro Phe Lys Phe Arg Trp Leu Ile Pro Ile Ser
   1385 1390 1395
   Ala Leu Gln Val Arg Leu Gly Asn Pro Ala Gly Thr Glu Asn Asn
   1400 1405 1410
   Ser Ile Trp Glu Leu Ile His Thr Lys Ser Glu Ile Glu Gly Arg
   1415 1420 1425
   Pro Glu Thr Ile Phe Gln Leu Cys Cys Ser Asp Ser Glu Ser Lys
   1430 1435 1440
   Thr Asn Ile Val Lys Val Ile Arg Ser Ile Leu Arg Glu Asn Phe
   1445 1450 1455
   Arg Arg His Ile Lys Cys Glu Leu Pro Leu Glu Lys Thr Cys Lys
   1460 1465 1470
   Asp Arg Leu Val Pro Leu Lys Asn Arg Val Pro Val Ser Ala Lys
   1475 1480 1485
   Leu Ala Ser Ser Arg Ser Leu Lys Val Leu Lys Asn Ser Ser Ser
   1490 1495 1500
   Asn Glu Trp Thr Gly Glu Thr Gly Lys Gly Thr Leu Leu Asp Ser
   1505 1510 1515
   Asp Glu Gly Ser Leu Ser Ser Gly Thr Gln Ser Ser Gly Cys Pro
   1520 1525 1530
   Thr Ala Glu Gly Arg Gln Asp Ser Lys Ser Thr Ser Pro Gly Lys
   1535 1540 1545
   Tyr Pro His Pro Gly Leu Ala Asp Phe Ala Asp Asn Leu Ile Lys
   1550 1555 1560
   Glu Ser Asp Ile Leu Ser Asp Glu Asp Asp Asp His Arg Gln Thr
   1565 1570 1575
   Val Lys Gln Gly Ser Pro Thr Lys Asp Ile Glu Ile Gln Phe Gln
   1580 1585 1590
   Arg Leu Arg Ile Ser Glu Asp Pro Asp Val His Pro Glu Ala Glu
   1595 1600 1605
   Gln Gln Pro Gly Pro Glu Ser Gly Glu Gly Gln Lys Gly Gly Glu
   1610 1615 1620
   Gln Pro Lys Leu Val Arg Gly His Phe Cys Pro Ile Lys Arg Lys
   1625 1630 1635
   Ala Asn Ser Thr Lys Arg Asp Arg Gly Thr Leu Leu Lys Ala Gln
   1640 1645 1650
   Ile Arg His Gln Ser Leu Asp Ser Gln Ser Glu Asn Ala Thr Ile
   1655 1660 1665
   Asp Leu Asn Ser Val Leu Glu Arg Glu Phe Ser Val Gln Ser Leu
   1670 1675 1680
   Thr Ser Val Val Ser Glu Glu Cys Phe Tyr Glu Thr Glu Ser His
   1685 1690 1695
   Gly Lys Ser
   1700
<210> 31
   <211> 2148
   <212> DNA
   <213> Tagap1 Bos taurus; Ensembl Transcript ENSBTAT00000025952
<400> 31
   atactgattc tgaaaaaaat taatggacaa ctaactgttg atatgaataa aatggtgaat 60
   atattttgtg gggaaagagg taatatcaag gaacatcctt tgttggcatc atgtgagagt 120
   gaagataata tttgccagct aattgaaatc aagaagagaa agaaggtgtc taactggctc 180
   cttctcatga gaaggctttc ttcttcatca gatgtttctg cagcttcgga gccagaattg 240
   aagacatccc ttttcgatca gcccttgtca gccatctgca gtgacaacac gcttccggga 300
   cccattcagg atattctcac gattctatgc ctgaaaggcc cttccactga aggaatattc 360
   aggaaagcag ccaacgagaa agcccgcaaa gagctgaagg aggagctcag ctccggaggt 420
   gtggtggacc tgagaagcct ccctgtgcac ctcctggcgg tggtcctcaa ggacttcctc 480
   agaagtattc cactgaagct cctgtcctgt gacctgtttg aggagtggat gggtgccctg 540
   gccaagcaga gcgaggagga ccggatcgag gccctgaaac aggttgcaga taagctcccg 600
   cggcccaacc acctgctgct caagcatctg gtctccgtgc tccacgtgat cagcaagaac 660
   tccgaggtca accggatgga cgccagcaat ctcgccatct gcattgggcc caacgtgctg 720
   agcccggaaa acgagcacaa tctatcgctg gaagcccgga gagacctgaa cgataaggtt 780
   aagacactgg tggaattcct catcgataac tgctttgaaa tatttgggga agactttcca 840
   gcacattcca gaattgcttc tgatgactcc ctggaacaca cggacagttc agacatgtcg 900
   accctgcaga acgactcagc ctacgacagc aacgaccctg accacgacgt ggagcctgcg 960
   ggctccccaa gctcgcagcc cccagggccc ccggaactgg ctgctggcgg cgtggaaccc 1020
   agagccccgc tgcgcccttg ggagcccgtg gtcaacacca cggccagact aaagggcttc 1080
   ctcgggcaac ctgaccggag gtactcggac cccagcacca cattctcccc ggagtgcctc 1140
   gagggcagaa gagcaaaccc gaaactcacg cgaagcgagg acgacttcac tgccgtggct 1200
   caggcagcct cccgctttgc cggcgaggaa gccgaggacc cgtttccaga ggaggtgttt 1260
   cctgcagccg aaggccgggc ccagaggccc cgggacctgg gggagtggag cccgactcag 1320
   ggctccgtgt caccgtgcgc acgggtcccc aaagccccct ccagcagctc tctggacgct 1380
   ttctccgaca gctcgcccct ggcctctcct tccagcccca aaagaaactt cttcaccaga 1440
   caccagtctt tcaccaaggc tgagaaaagc aagcccaaca gagaaattaa aaagcactcc 1500
   atgtcattct ccttcgcctc tcaccaaaga gggctgacca aaatgcgcag ctttggagcc 1560
   acgaagtcca agggctgccc ccgagaccaa gagaagcgag gttccaagaa agaaagccag 1620
   ctcgccggcc ggatcgtcca agaaagctcg tcggatgccc ccggccaagc cgtgctgggc 1680
   tttaactcgg gggcctacgc cctctcggtg gaggatgtgt tccggctggt ggatcagagg 1740
   caccccggcc gccccccctc ttacgaggag gccgtgaggc tccaggcgct ggagctcgcc 1800
   ccccgcgggg gccagacagt gggcagcctg agggcccgcg tgctgagcct ggacgcgggg 1860
   ctcctgcctc ccctccctgc ccacccccac ggggactcga gaaacatccg cgggccggag 1920
   cccctggacg ggctccgagg ggggctgggg actgagacct ggaggcagag ctgcgcccct 1980
   aaggacacgg caggacgggt gatggtcccc gggacatccg agctgcagcg gctgagaacc 2040
   gcatccgagt cgcagcagaa gggcaggcag gccgtcctgg cccggcggtg tagccagccc 2100
   gtgtttgacg ccgagcagct ccgattcgct aaggaatcct acatctag 2148
<210> 32
   <211> 2055
   <212> DNA
   <213> Tagap1 Canis familiaris; Ensembl Transcript ENSCAFT00000001032
<400> 32
   tcaaaaacac taaatgccaa taatatggag acattgattg aatgtcagtc agagggtgat 60
   atcaaggaac atcctctgtt ggcatcatgt gagagtgaag ataacatttg ccagctaatt 120
   gaaattaaga agagaaagaa ggtgctgtct tggccatttc tcatgagaag gctttctact 180
   gtgtcagatt ttcccggggc ttcagaacca gaattgaaag tgccactatt tgatcagccc 240
   ttgtcaatca tctgtggtga ggacgacaca ctccccagac ccattcagga tattctcact 300
   attttgtgcc ttaaaggacc ttccaccgaa gggatattca ggaaagcagc caatgagaaa 360
   gcccgcaaag agctaaagga agatctcaac tcgggaggca tggtagatct gaaaagtctc 420
   cccgtgcacc tcctggctgc aatctttaag gacttcctca gaagtatccc actgaagcta 480
   ctttcatgtg acctgtttga agaatggatg gacgccctgg agaggcagaa tgaagaggat 540
   agaattgagg ccctgaaaca ggttgcagag aagctcccgc ggcccaacct cctgctgctc 600
   aagcacttga tctccgtgct ctatctgatc agcaaaaact cggagatcaa taagatggat 660
   gccagcaatt tagctatctg catcggaccc aacatgctga cccgagagaa tgaccaacac 720
   ctgtctttcg aagcccagaa agacttgaac aacaaggtta agtcactggt ggagttcctc 780
   attgataact gcttggaaat atttggggag aacattccca cacaccccag tactgcttct 840
   gatgactctc tggaacacac cgacagttca gacctgtcaa tgctgcagaa tgactcagcc 900
   tatgacagca atgatcctga cgtggaatcc agcggtgcca ccagctcccc aaacaggagg 960
   ccccaggtgc ccttggagac ggctgccagc tgggagtcca gaggcccaca gcttgcttgg 1020
   gagttgagcc cggagcccat tgtcagcacc atagccgggc tgaaaaactc cctcagtgaa 1080
   ccagacagga gctactcaga gcccagcatg tcttcctcac aagagagcct ggagagccag 1140
   aaaacacacc aaaaactaac acggagtgag gatgacttca ccatggctca ggcaggggct 1200
   catttggaaa gtgaggaggc tgaagaccca tttccagagg aggtgtttcc tgcagttgaa 1260
   ggcaaaatcc aaagtccaca ggacctgaag gtgaagaact caactcaggg tttggtgtca 1320
   ctatggggac taatgcccaa agccatctcc agtggctctc tggatgcttc ctctgacagc 1380
   tcacccatgg cttctccttc cagtcctaaa agaaatttct tcacaagaca tcagtctttc 1440
   acaaagacag agaaaagtaa gcccaacaga gaaatcaaaa aacactccat gtcattctcc 1500
   tttgcctctc acaaaagagt gcttaccaaa acccctagtt gcgtgtctgt gaaatccaaa 1560
   ggctttacca gagaccaagt aaagaaaggt tttaaaaaag aaagccagct tgctgggcga 1620
   atcattcagg aaaacttaag ccctggaagt ccgccatctt atgaagaggc cattaggtgc 1680
   caggcattgg acctctcggc ctatgggggc caaacagttg gcagcatgag agctagaatg 1740
   ctcagccagg acactgaact accacctctc ctaccttttc gtcatggagg gaattcaaga 1800
   aatataggca gtgaagagcc acttgatggg cacagactat ctcccaggac tgagagttgg 1860
   gaacagagca ggactgtcca tgcttctgca gaaacaatag gacaagtgac tgttacaagg 1920
   agaccagagc tgtaccgcct aagaactcta tctgaatcaa aacagaagag taggctggac 1980
   cacctagtgc agcaatgtag tcagccggtc tttgaggctg accaactcca gtatgctaaa 2040
   gaatcctaca tttag 2055
<210> 33
   <211> 2082
   <212> DNA
   <213> Tagap1 Gallus gallus; Ensembl Transcript ENSGALT00000022338
<400> 33
   atgaaagtgc taagcagctg taatacttcg aagacactaa atgctggaaa catggagagt 60
   ttgattgagt gcccatcaga ggctgatgcc aagaaatgcc ccctgttggg cccagctgac 120
   actgaggatg gactttgcca gttggctgat gaaatgaaga aaagaaagaa ggtgatatcg 180
   tggccatttg ctctgagacg cacctccacc agtggggatt ccccggggca gctggactct 240
   ggcctcaaga tcaccttgtt tggccagcct ctggcaatta tttgtgggga agacgacaca 300
   ctgccccagc cagtccagga tctcctagct atattgtaca tgaaaggacc ttccactgaa 360
   ggtatattca gaaaagctgc caatgagaaa gcacgcaagg agttgaaaga ggatctaaac 420
   aaaggcggga atgttgattt ggaaagcaaa actgtgcatc tactggcagt ggttttgaag 480
   gacttcctcc gaaatattcc ctccaaactt ctgtcagatg atctgtatga caagtggatg 540
   ctcgctctgg agaagccaag caagcaagaa aaaatcgaag aactgaaaga ggtggctgac 600
   aaactgccca gaccaaacct ggtcttgctg aagcacttgc tctctgtgct ccaccgcatc 660
   agccagaatg ctgacaccaa caggatggat gccaacaacc ttgccatctg cgtaggccca 720
   aacatgctga gcccagggac aggcagcatg ctgccgctgg aggtgcagaa ggagatgaat 780
   gacaaggtga cggtgttggt ggagttcctc atagaaaaca gctcagaaat atttggggag 840
   gacattctat ggcctgtcag caccttggct gtggagtcgc cagagcatat agacagctcc 900
   acagaacacc tatgtgctac tcatcagaat gactctgcct atgacagccc agatccggaa 960
   gctgaatgct gtacttctga gctagagcag cccaaaggaa gaagcactgg tttgagcaga 1020
   agatatccaa catgtgtctc tgccacatca ctgactaatt gcaaaaatga catcaacatg 1080
   atggacagga ggtactcaga accagaccta tccttccagg accgttttga aagcaaaata 1140
   aggaaacaga agctaaacaa aagtgaggac agttttccag ttcagcagaa gcagctaggt 1200
   ctggaaaatg aggtactgga caaacggctt gcaatcttac ctccacaatt atcaagtgac   1260
   tctctatcca aaacatcctc cagttgctca ctagagagct ctgatggctc agtcttcacc   1320
   agctccccat tagtttcacc ctctagtccc aaaaaaacct tcttaaatag gccccagtcc   1380
   ttttgcacca agactcatga agactgcagc acagctaggc gagaggtcaa aaagcattct   1440
   atgtcattct cttttgcaaa ccaccggaaa acactaacaa aacagcagag ctgggggcct   1500
   ggaaaacaca tgggtttcca gagggacagt ttcacaaaga aagatgatca gttctcctgc   1560
   agaattgtac aggaaaacag ccctgaggac gacaaaccac cgcacgtacc atatcagcga   1620
   aggtcacgtt tcaggtcagc tgatgaagtg ttcagagagg tagaccagag gaatcctgga   1680
   agaccaccct cttatgaaga agctactaag aactgtgagg ccacccacgt tccctcctgc   1740
   aatctcacag ttcagactat gagattaaag gtgtcaaacc aggacacttt gccatctgat   1800
   ccacgtacca gccttgcaca gggcatagcc tgtacagctt cgaaggacct acccagtggc   1860
   agggtttctg cagtgaatga ttctgatgca gaaactgaaa ctctcagtgt taccgttgga   1920
   ataaactccc gtgtgagttt acctgtgacc cctggagtgt accgattgag agccatgtct   1980
   gaatcctgtc aaaagaacaa acatgagtat gtggcacggc ggtgcagcca gcccattttt   2040
   gaggtagagc agatacagta tgctaaggaa tcctatgttt aa                      2082
<210> 34
   <211> 1383
   <212> DNA
   <213> Fgd2 mouse transcript variant 2 t6/tw5
<400> 34
   atgtacagcg agtacgtgaa gaactttgag cgggccgcgg aactgctggc cacgtggatg     60
   gacaagtctc agcccttcca ggaggtggtc acccgcatcc agtgcagcga ggcctcgggc    120
   agcctgaccc tgcagcacca catgttggag cctgtgcaaa gaatcccgcg gtacgaactg    180
   ctgctcaagg aatatgtgca gaagctgcca gcccaggccc cagacctcga agatgcccag    240
   agagcactgg acatgatctt ctcagctgca cagcactcca atgcagccat tgcagagatg    300
   gagcggctgc agggcctgtg ggatgtgtac cagcgcctgg gcttggagga tgacatcgtg    360
   gacccctcca acaccctgct ccgagagggc cctgttctca agatctcttt ccgccgcagc    420
   gacccaatgg aacgctacct ggttttgttc aacaacatgc ttctgtattg tgtaccccga    480
   gtcctccaag tgggtgccca gttccaggtg cggactcgca tcgatgtggc cggcatgaag    540
   gtgcgggagc tgaccgatgc tgagttccca cactccttcc tggtgtccgg aaagcagcgc    600
   acactggagc tgcaggcccg gtcccgagat gaaatggttt cctggatgca ggcctgccag    660
   gcagccattg accaggttga gaagcggagt gagaccttca aggctgctgt ccagggacct    720
   cagggggaca cgcaggagcc caagccacag gtagaggagc tgggtctccg agcgcctcag    780
   tgggtccggg acaagatggt gaccatgtgc atgcgttgcc aggagccctt caatgccctg    840
   actcgtcggc gccaccactg ccgggcttgc ggctatgtag tgtgtgccaa gtgctctgac 900
   taccgtgcgg agctgaaata tgacagcaac aggcccaacc gagtctgcct gacctgctac 960
   acatttctca ctggaaacgt actccctcaa ggcaaggagg acaagaggcg gggcatcttg 1020
   gagaaagagg cctcagcagc acccgagcag agtctggtgt gcagcttcct gcagctcata 1080
   ggagacaagt gcagcaggag ccttccccgg agctggtgtg tgatcccccg ggatgacccc 1140
   cttgtgctgt atgtctatgc agccccccag gacacaaagg ctcacacctc catcccccta 1200
   ctgggctatc aggtgatttc agggccccag ggggaccctc gggttttcca gctgcaacag 1260
   tcaggccagc agtacacctt caaggccgag tctgtggagc tgcagggccg ctgggtgaca 1320
   gctatcaagc gtgcggccag tggccggacc cctgagggac ctgacgaaga agatgtgtct 1380
   gac 1383
<210> 35
   <211> 1971
   <212> DNA
   <213> Fgd2 Bos taurus; Transcript ENSBTAT00000018834
<400> 35
   atggaggggg caagtgagga aactctgaca tctgtgtcca gtctggtgac tgtgtttgag 60
   aatagcagga tgacaggagc agtgcccaga gtccacggcc tggaggccgg gcatcagcag 120
   cctgggtgca gggctcctac gtctgcaggg ccctgggaga agcccactgt ggaggaggcc 180
   ctggaatctg ggtccaggac tgtcagcagg aggtacctga gctccctgaa gaacaagctg 240
   tccagcggag cttggaggaa gtcttaccag cccaggacct gccctggctc ggggacacag 300
   gagcctgagg agaagaagat tgtccgggag ctgctggaga ctgagcaggc ctacgtggcc 360
   cgcctccacc tgctggacca ggtcttcttc caggagctgc tgaaggaggc ccgcagcagc 420
   aaggccttcc ctgaggacgt ggtcaggctc atcttctcca acatctcctc catctaccag 480
   ttccactcac agttcttcct cccagagctg cagcggcggc tggacgactg gactaccacc 540
   ccccgcatcg gtgatgtgat ccagaagctg gcgcccttcc tgaagatgta cagtgagtat 600
   gtcaagaact tcgagcgagc catcgagctg ctggccacct ggacagacaa atccccacct 660
   ttccaggagg ttatcacccg catccagagc agcgaggcct cagctagcct gaccctgcag 720
   caccacatgc tggaacctgt gcagagaatc ccacgctacg agctgctgct caaggagtac 780
   gtccagaagc tgccaggcca ggccccagac ctggctgatg cccagaaagc ccttgacatg 840
   atcttctcgg ccgctcagca ctccaatgca gccatcactg aaatggagcg gctgcaggaa 900
   ctgtgggacg tgtaccagcg cctgggcctg gaggacgata tagtcgaccc ctccaacacc 960
   ctgctccgcg agggccccgt cctcaagatc tccttccgcc gcagcggccc catggagcgc 1020
   taccttttct tgttcaacaa catgctgctc tactgtgtgc ccagggtcat ccaggtgggc 1080
   gcccacttcc aggtcaggac ccgcatcgac gtggccggca tgaaggtgcg ggagctgact 1140
   gacgcagagt ttccccactc cttcctggtg tccgggaagc agcgaaccct ggagctgcga 1200
   gcccggtccc aggaggaaat gatctcctgg ttgcaggcct gccaagccgc cattgaccaa 1260
   attgagaagc ggaatgaaac cttcaaggct gcagtccaga gccctgaggg agacccccag 1320
   gagcaggagc tgcagtccgt ggagctgggc ctccgggcgc cccagtgggt gcgggacaag 1380
   atggtaacca tgtgcatgcg ctgccaggag cccttcaacg ccctgacgcg ccgccgccac 1440
   cactgccggg cctgcggcta cgtggtgtgt gccaggtgct cggactaccg ggcggagctc 1500
   aagtacgatg ccaacaggcc caaccgagtc tgcctggact gctacacttt cctcaccgga 1560
   aacgtactcc ctgaggaaaa ggaggacaag agacggggca tcctggagaa aggagccgtg 1620
   gcggggtccg agcagagcct gatatgcagc tttctgcagc ttctggggga caagtggggc 1680
   aagagcgggc cccggggctg gtgtgtcatc ccccgggatg accccctcgt gctctacgtc 1740
   tatgctgccc ctcaggacat gcgggcccac acctccatcc ccctgctggg ctaccaggtg 1800
   accgctgggc cccaggcgga cccccgggtc ttccagctgc agcagtcagg ccagctctac 1860
   accttcaaag cggaaagcga ggagctgaag ggccgctggg tgaaggccat ggagcgggcg 1920
   gccagcggct ggatccctgg ggggcccgat gacaaggacc tgtcggactg a 1971
<210> 36
   <211> 1994
   <212> DNA
   <213> Fgd2 Canis familiaris; Transcript ENSCAFT00000002252
<400> 36
   atggagggag agagtaagga gaagctggca tctgtgtcca atctggtgac tgtgtttgag 60
   aacagcagga ttccaggagc agcatccagt gtcagcaagc tggagattga acatcaccac 120
   cctggatgca ggtctccccc acccgcagag ccattgaagg agcctaacct tggagagacc 180
   ctgagggggt ctgagcccag gatggtcagc aggaggtacc tgagctccct gaagaacaag 240
   ctgtccagtg gggcctggag aaaatcttgc cagcctggga ccgaccctgg gccagggaca 300
   caggagcctg aggaaaagag gatcgtccag gagctactgg agacagagaa ggcctatgtg 360
   gcacgccttc atctgctaga ccaggtgttc tttcaggagc tactgaggga agcccgcagc 420
   agcaaggcct tccctgagga tgtggtcagg ctcatcttct ccaacatctc ctccatctac 480
   cagttccacg ctcagttctt cctcccggag ctgcagcggc gactggatga ctggacagcc 540
   accccccgca tcggcgacgt gatccagaaa ctggccccat tcctgaagat gtacagtgag 600
   tatgtcaaga actttgagcg agctgctgag ctgctggcca cctggactga caagtctgca 660
   cccttccagg aggtgatcac ccgcattcag agcagcgagg cctccggcag cctgactctg 720
   caacaccaca tgctggaacc cgtgcagaga atcccacgct acgagctgct gctcaaggag 780
   tatgttcaga agctgccagc ccaggcccca gaccgggccg atgctcagaa agcgctcgac 840
   atgatcttct cagccgctca gcactccaac gcggccatca gtgagatgga gcggctgcag     900
   gacctgtggg aggtgtacca gcgcctgggc ctcgagggcg acatagtgga cccctccaac     960
   accctgctcc gcgaaggccc tgtcctcaag atctcctttc gccgcagcga ccccatggaa    1020
   cgctaccttt tcctgttcaa caacatgctg ctctactgta tacccaaggt catccaggtg    1080
   ggcgcccaat tccaggtgag gacccgcatc gacgtggctg gaatgaaggt gcgggagctg    1140
   acggatgcag agttccccca ctccttcctg gtgtccggga agcagcgcac cctggagctg    1200
   caagcccggt cccaggaaga aatgatttcc tggatgcagg cctgccaagc agccattgac    1260
   caaatcgaga agcggaatga aaccttcaag gctgcggtcc agggcccaga gggagacacc    1320
   caggagcagg agctgcagtc cgaggagctg ggcctccggg cgccgcagtg ggtccgggac    1380
   aagatggtga ccatgtgcat gcgctgccgg gagcccttca acgccctgcg gcgccgccgc    1440
   caccactgcc gagcctgcgg cttcgtggtg tgtgccaagt gctccgacta ccgggctgag    1500
   ctcaaatacg atgacaaccg gcccaaccgg gtctgcttcc actgctacac gttcctcacc    1560
   ggaaacgtgc tcccggagga cagggaggac aggaggcggg gcattctgga gaaagggtcc    1620
   atgacagggt ctgagcagag cctgatgtgc agcttccttc agctggttgg ggacaaatgg    1680
   ggcaagagca gcccccgggg ctggtgtgtg atcccccggg acgaccccct ggtcctctat   1740
   gtctacgctg cccctcagga catgcgggct cacacctcca tccccctgct gggctaccag    1800
   gtgactgctg ggacccaggc agatccccgg gtcttccagc tgcaacagtc agggcagctt    1860
   tacaccttca aggctgaaac tgaggagctg agggaccgct gggtgaaggc catggagcgg    1920
   gcggccagtg gctggagccc cagggggccc aaggatggag acctgtctga ctgacccaca   1980
   gccggccact ctcc                                                      1994
<210> 37
   <211> 1974
   <212> DNA
   <213> Fgd2 Rattus norvegicus; Transcript ENSRNOT00000000636
<400> 37
   atggagcaag cctgtgcgaa gcaggactca gtgtgcaacc tggtggctgt gtttgagaac      60
   agcaggtctc cgggagcagc acctggaccc catagttttg aggaccagcc ccatagccct     120
   gaacatcagc tgcccctgtc cccagagcct tgggaggcac cccctgccgg ggaggccttg     180
   acgtctgagt tccggccagt cagcaggaca tatctgaatt ccctcaagaa taagctatcg     240
   agtggggctt ggaggaaatc ctgccaacct ggggccagtc cagggccgga gactcaggaa     300
   cctgaggaga agagggtcgt gcaggagctt ctggagacgg agcaggccta cgtggctcgc     360
   ctgcacttgc ttgaccaggc cactgtgttc ttccaggagc tgctgaggga ggcgagccgt     420
   agcaaggcct tccctgagga cgtggtgaag ctcatcttct ccaatatctc ctccatctac     480
   cgtttccacg cacagttctt cctcccggag ctgcagaggc gcgtggatga ctggacagcc   540
   acaccacgca tcggggatgt gatccagaaa ctggccccct ttctgaaaat gtacagcgag   600
   tacgtgaaga actttgagcg ggcggcagaa ctgctggcca cctggatgga caagtcacag   660
   cccttccagg aggtggtcac ccgcatccag cgcagtgagg cctccggcag cctgaccctg   720
   cagcaccaca tgttggaacc tgtgcagaga atcccacggt acgaactgct gctcaaggaa   780
   tatgtgcaga agctgccagc ccaggcccca gacctcgaag atgcccagag agcgctggac   840
   atgatcttct cagccgcaca gcactcgaac gcagccattg ctgagatgga gcggctgcag   900
   ggcctgtggg atgtgtacca gcgcctgggc ctggaggatg acattgtgga cccttccaac   960
   accctgctcc gagagggccc tgttctcaag atctctttcc gccgcagcga cccaatggag  1020
   cgctacctgg ttttgttcaa caacatgctt ctgtactgcg tgccccgggt cctccaagtg  1080
   ggtgcccagt tccaggtgag gacccgcatc gacgtggctg gcatgaaggt gcgggagctg  1140
   acggacgctg agttcccgca ctccttcctg gtatccggca agcagagaac gttggagctg  1200
   caggcccggt ctcgagagga aatggtttct tggatacagg cctgccaggc agctattgac  1260
   caagtcgaga agcgaagtga aaccttcaag gctgctgtcc agggacctca gggggacaca  1320
   caggagccca agccacaggc agaggagctg ggtctccgag cacctcagtg ggtccgagac  1380
   aagatggtga ccatgtgtat gcgctgccag gagcccttca atgccctgac tcgtcggcgt  1440
   caccactgcc gggcttgtgg ctacgtagtg tgtgcaaagt gttctgacta ccgtgcggag  1500
   ctgaaatatg acggcaacag gcccaaccga gtctgcctga cctgctacac gttcctcacc  1560
   ggaaacctgc tccctgacag caaggaggac aagaggcgag gcatcttgga gaaagagacc  1620
   tccgcagggc ccgaccagag tgtggtgtgc agtttcctgc agctcatggg ggacaagagt  1680
   atcaggagca ttccccggag ctggtgtgtg atcccccggg atgacccgct tgtgctgtat  1740
   gtctatgcag ccccccagga catgaaggct cacacctcca tccccttgct gggttatcag  1800
   gtgacttcag ggccccaggg ggaccctcgg gttttccagc tgcaacagtc aggccagcag  1860
   tacaccttca aggctgagtc tgtggagctg cagggccgct gggtgagagc tatcaagcgt  1920
   gcggccagcg gctggacccc cgagggacct gacgaagaag acatgtctga ctga        1974
<210> 38
   <211> 1740
   <212> DNA
   <213> Fgd2 (splice variant) Rattus norvegicus; Transcript ENSRNOT00000041665
<400> 38
   atggagcaag cctgtgcgaa gcaggactca gtgtgcaacc tggtggctgt gtttgagaac    60
   agcaggaacc tgaggagaag agggtcgtgc aggagcttct ggagacggag caggcctacg   120
   tggctcgcct gcacttgctt gaccaggcca gtgttcttcc aggagctgct gagggaggcg   180
   agccgtagca aggccttccc tgaggacgtg gtgaagctca tcttctccaa tatctcctcc   240
   atctaccgtt tccacgcaca gttcttcctc ccggagctgc agaggcgcgt ggatgactgg    300
   acagccacac cacgcatcgg ggatgtgatc cagaaactgg ccccctttct gaaaatgtac    360
   agcgagtacg tgaagaactt tgagcgggcg gcagaactgc tggccacctg gatggacaag    420
   tcacagccct tccaggaggt ggtcacccgc atccagcgca gtgaggcctc cggcagcctg    480
   accctgcagc accacatgtt ggaacctgtg cagagaatcc cacggtacga actgctgctc    540
   aaggaatatg tgcagaagct gccagcccag gccccagacc tcgaagatgc ccagagagcg    600
   ctggacatga tcttctcagc cgcacagcac tcgaacgcag ccattgctga gatggagcgg    660
   ctgcagggcc tgtgggatgt gtaccagcgc ctgggcctgg aggatgacat tgtggaccct    720
   tccaacaccc tgctccgaga gggccctgtt ctcaagatct ctttccgccg cagcgaccca    780
   atggagcgct acctggtttt gttcaacaac atgcttctgt actgcgtgcc ccgggtcctc    840
   caagtgggtg cccagttcca ggtgaggacc cgcatcgacg tggctggcat gaaggtgcgg    900
   gagctgacgg acgctgagtt cccgcactcc ttcctggtat ccggcaagca gagaacgttg    960
   gagctgcagg cccggtctcg agaggaaatg gtttcttgga tacaggcctg ccaggcagct   1020
   attgaccaag tcgagaagcg aagtgaaacc ttcaaggctg ctgtccaggg acctcagggg   1080
   gacacacagg agcccaagcc acaggcagag gagctgggtc tccgagcacc tcagtgggtc   1140
   cgagacaaga tggtgaccat gtgtatgcgc tgccaggagc ccttcaatgc cctgactcgt   1200
   cggcgtcacc actgccgggc ttgtggctac gtagtgtgtg caaagtgttc tgactaccgt   1260
   gcggagctga aatatgacgg caacaggccc aaccgagtct gcctgacctg ctacacgttc   1320
   ctcaccggaa acctgctccc tgacagcaag gaggacaaga ggcgaggcat cttggagaaa   1380
   gagacctccg cagggcccga ccagagtgtg gtgtgcagtt tcctgcagct catgggggac   1440
   aagagtatca ggagcattcc ccggagctgg tgtgtgatcc cccgggatga cccgcttgtg   1500
   ctgtatgtct atgcagcccc ccaggacatg aaggctcaca cctccatccc cttgctgggt   1560
   tatcaggtga cttcagggcc ccagggggac cctcgggttt tccagctgca acagtcaggc   1620
   cagcagtaca ccttcaaggc tgagtctgtg gagctgcagg gccgctgggt gagagctatc   1680
   aagcgtgcgg ccagcggctg gacccccgag ggacctgacg aagaagacat gtctgactga   1740
<210> 39
   <211> 715
   <212> PRT
   <213> Tagap1 Bos taurus; deduced from Ensembl Transcript ENSBTAT00000025952
<400> 39
   Ile Leu Ile Leu Lys Lys Ile Asn Gly Gln Leu Thr Val Asp Met Asn
   1               5                   10                  15
   Lys Met Val Asn Ile Phe Cys Gly Glu Arg Gly Asn Ile Lys Glu His
   20 25 30
   Pro Leu Leu Ala Ser Cys Glu Ser Glu Asp Asn Ile Cys Gln Leu Ile
   35 40 45
   Glu Ile Lys Lys Arg Lys Lys Val Ser Asn Trp Leu Leu Leu Met Arg
   50 55 60
   Arg Leu Ser Ser Ser Ser Asp Val Ser Ala Ala Ser Glu Pro Glu Leu
   65 70 75 80
   Lys Thr Ser Leu Phe Asp Gln Pro Leu Ser Ala Ile Cys Ser Asp Asn
   85 90 95
   Thr Leu Pro Gly Pro Ile Gln Asp Ile Leu Thr Ile Leu Cys Leu Lys
   100 105 110
   Gly Pro Ser Thr Glu Gly Ile Phe Arg Lys Ala Ala Asn Glu Lys Ala
   115 120 125
   Arg Lys Glu Leu Lys Glu Glu Leu Ser Ser Gly Gly Val Val Asp Leu
   130 135 140
   Arg Ser Leu Pro Val His Leu Leu Ala Val Val Leu Lys Asp Phe Leu
   145 150 155 160
   Arg Ser Ile Pro Leu Lys Leu Leu Ser Cys Asp Leu Phe Glu Glu Trp
   165 170 175
   Met Gly Ala Leu Ala Lys Gln Ser Glu Glu Asp Arg Ile Glu Ala Leu
   180 185 190
   Lys Gln Val Ala Asp Lys Leu Pro Arg Pro Asn His Leu Leu Leu Lys
   195 200 205
   His Leu Val Ser Val Leu His Val Ile Ser Lys Asn Ser Glu Val Asn
   210 215 220
   Arg Met Asp Ala Ser Asn Leu Ala Ile Cys Ile Gly Pro Asn Val Leu
   225 230 235 240
   Ser Pro Glu Asn Glu His Asn Leu Ser Leu Glu Ala Arg Arg Asp Leu
   245 250 255
   Asn Asp Lys Val Lys Thr Leu Val Glu Phe Leu Ile Asp Asn Cys Phe
   260 265 270
   Glu Ile Phe Gly Glu Asp Phe Pro Ala His Ser Arg Ile Ala Ser Asp
   275 280 285
   Asp Ser Leu Glu His Thr Asp Ser Ser Asp Met Ser Thr Leu Gln Asn
   290 295 300
   Asp Ser Ala Tyr Asp Ser Asn Asp Pro Asp His Asp Val Glu Pro Ala
   305 310 315 320
   Gly Ser Pro Ser Ser Gln Pro Pro Gly Pro Pro Glu Leu Ala Ala Gly
   325 330 335
   Gly Val Glu Pro Arg Ala Pro Leu Arg Pro Trp Glu Pro Val Val Asn
   340 345 350
   Thr Thr Ala Arg Leu Lys Gly Phe Leu Gly Gln Pro Asp Arg Arg Tyr
   355 360 365
   Ser Asp Pro Ser Thr Thr Phe Ser Pro Glu Cys Leu Glu Gly Arg Arg
   370 375 380
   Ala Asn Pro Lys Leu Thr Arg Ser Glu Asp Asp Phe Thr Ala Val Ala
   385 390 395 400
   Gln Ala Ala Ser Arg Phe Ala Gly Glu Glu Ala Glu Asp Pro Phe Pro
   405 410 415
   Glu Glu Val Phe Pro Ala Ala Glu Gly Arg Ala Gln Arg Pro Arg Asp
   420 425 430
   Leu Gly Glu Trp Ser Pro Thr Gln Gly Ser Val Ser Pro Cys Ala Arg
   435 440 445
   Val Pro Lys Ala Pro Ser Ser Ser Ser Leu Asp Ala Phe Ser Asp Ser
   450 455 460
   Ser Pro Leu Ala Ser Pro Ser Ser Pro Lys Arg Asn Phe Phe Thr Arg
   465 470 475 480
   His Gln Ser Phe Thr Lys Ala Glu Lys Ser Lys Pro Asn Arg Glu Ile
   485 490 495
   Lys Lys His Ser Met Ser Phe Ser Phe Ala Ser His Gln Arg Gly Leu
   500 505 510
   Thr Lys Met Arg Ser Phe Gly Ala Thr Lys Ser Lys Gly Cys Pro Arg
   515 520 525
   Asp Gln Glu Lys Arg Gly Ser Lys Lys Glu Ser Gln Leu Ala Gly Arg
   530 535 540
   Ile Val Gln Glu Ser Ser Ser Asp Ala Pro Gly Gln Ala Val Leu Gly
   545 550 555 560
   Phe Asn Ser Gly Ala Tyr Ala Leu Ser Val Glu Asp Val Phe Arg Leu
   565 570 575
   Val Asp Gln Arg His Pro Gly Arg Pro Pro Ser Tyr Glu Glu Ala Val
   580 585 590
   Arg Leu Gln Ala Leu Glu Leu Ala Pro Arg Gly Gly Gln Thr Val Gly
   595 600 605
   Ser Leu Arg Ala Arg Val Leu Ser Leu Asp Ala Gly Leu Leu Pro Pro
   610 615 620
   Leu Pro Ala His Pro His Gly Asp Ser Arg Asn Ile Arg Gly Pro Glu
   625 630 635 640
   Pro Leu Asp Gly Leu Arg Gly Gly Leu Gly Thr Glu Thr Trp Arg Gln
   645 650 655
   Ser Cys Ala Pro Lys Asp Thr Ala Gly Arg Val Met Val Pro Gly Thr
   660 665 670
   Ser Glu Leu Gln Arg Leu Arg Thr Ala Ser Glu Ser Gln Gln Lys Gly
   675 680 685
   Arg Gln Ala Val Leu Ala Arg Arg Cys Ser Gln Pro Val Phe Asp Ala
   690 695 700
   Glu Gln Leu Arg Phe Ala Lys Glu Ser Tyr Ile
   705 710 715
<210> 40
   <211> 684
   <212> PRT
   <213> Tagap1 Canis familiaris; deduced from Ensembl Transcript ENSCAFT00000001032
<210> 41
   <211> 693
   <212> PRT
   <213> Tagap1 Gallus gallus; deduced from Ensembl Transcript ENSGALT00000022338
<400> 41
   Met Lys Val Leu Ser Ser Cys Asn Thr Ser Lys Thr Leu Asn Ala Gly
   1               5                   10                  15
   Asn Met Glu Ser Leu Ile Glu Cys Pro Ser Glu Ala Asp Ala Lys Lys
               20                  25                  30
   Cys Pro Leu Leu Gly Pro Ala Asp Thr Glu Asp Gly Leu Cys Gln Leu
           35                  40                  45
   Ala Asp Glu Met Lys Lys Arg Lys Lys Val Ile Ser Trp Pro Phe Ala
       50                  55                  60
   Leu Arg Arg Thr Ser Thr Ser Gly Asp Ser Pro Gly Gln Leu Asp Ser
   65                  70                  75                  80
   Gly Leu Lys Ile Thr Leu Phe Gly Gln Pro Leu Ala Ile Ile Cys Gly
                   85                  90                  95
   Glu Asp Asp Thr Leu Pro Gln Pro Val Gln Asp Leu Leu Ala Ile Leu
   100 105 110
   Tyr Met Lys Gly Pro Ser Thr Glu Gly Ile Phe Arg Lys Ala Ala Asn
   115 120 125
   Glu Lys Ala Arg Lys Glu Leu Lys Glu Asp Leu Asn Lys Gly Gly Asn
   130 135 140
   Val Asp Leu Glu Ser Lys Thr Val His Leu Leu Ala Val Val Leu Lys
   145 150 155 160
   Asp Phe Leu Arg Asn Ile Pro Ser Lys Leu Leu Ser Asp Asp Leu Tyr
   165 170 175
   Asp Lys Trp Met Leu Ala Leu Glu Lys Pro Ser Lys Gln Glu Lys Ile
   180 185 190
   Glu Glu Leu Lys Glu Val Ala Asp Lys Leu Pro Arg Pro Asn Leu Val
   195 200 205
   Leu Leu Lys His Leu Leu Ser Val Leu His Arg Ile Ser Gln Asn Ala
   210 215 220
   Asp Thr Asn Arg Met Asp Ala Asn Asn Leu Ala Ile Cys Val Gly Pro
   225 230 235 240
   Asn Met Leu Ser Pro Gly Thr Gly Ser Met Leu Pro Leu Glu Val Gln
   245 250 255
   Lys Glu Met Asn Asp Lys Val Thr Val Leu Val Glu Phe Leu Ile Glu
   260 265 270
   Asn Ser Ser Glu Ile Phe Gly Glu Asp Ile Leu Trp Pro Val Ser Thr
   275 280 285
   Leu Ala Val Glu Ser Pro Glu His Ile Asp Ser Ser Thr Glu His Leu
   290 295 300
   Cys Ala Thr His Gln Asn Asp Ser Ala Tyr Asp Ser Pro Asp Pro Glu
   305 310 315 320
   Ala Glu Cys Cys Thr Ser Glu Leu Glu Gln Pro Lys Gly Arg Ser Thr
   325 330 335
   Gly Leu Ser Arg Arg Tyr Pro Thr Cys Val Ser Ala Thr Ser Leu Thr
   340 345 350
   Asn Cys Lys Asn Asp Ile Asn Met Met Asp Arg Arg Tyr Ser Glu Pro
   355 360 365
   Asp Leu Ser Phe Gln Asp Arg Phe Glu Ser Lys Ile Arg Lys Gln Lys
   370 375 380
   Leu Asn Lys Ser Glu Asp Ser Phe Pro Val Gln Gln Lys Gln Leu Gly
   385 390 395 400
   Leu Glu Asn Glu Val Leu Asp Lys Arg Leu Ala Ile Leu Pro Pro Gln
   405 410 415
   Leu Ser Ser Asp Ser Leu Ser Lys Thr Ser Ser Ser Cys Ser Leu Glu
   420 425 430
   Ser Ser Asp Gly Ser Val Phe Thr Ser Ser Pro Leu Val Ser Pro Ser
   435 440 445
   Ser Pro Lys Lys Thr Phe Leu Asn Arg Pro Gln Ser Phe Cys Thr Lys
   450 455 460
   Thr His Glu Asp Cys Ser Thr Ala Arg Arg Glu Val Lys Lys His Ser
   465 470 475 480
   Met Ser Phe Ser Phe Ala Asn His Arg Lys Thr Leu Thr Lys Gln Gln
   485 490 495
   Ser Trp Gly Pro Gly Lys His Met Gly Phe Gln Arg Asp Ser Phe Thr
   500 505 510
   Lys Lys Asp Asp Gln Phe Ser Cys Arg Ile Val Gln Glu Asn Ser Pro
   515 520 525
   Glu Asp Asp Lys Pro Pro His Val Pro Tyr Gln Arg Arg Ser Arg Phe
   530 535 540
   Arg Ser Ala Asp Glu Val Phe Arg Glu Val Asp Gln Arg Asn Pro Gly
   545 550 555 560
   Arg Pro Pro Ser Tyr Glu Glu Ala Thr Lys Asn Cys Glu Ala Thr His
   565 570 575
   Val Pro Ser Cys Asn Leu Thr Val Gln Thr Met Arg Leu Lys Val Ser
   580 585 590
   Asn Gln Asp Thr Leu Pro Ser Asp Pro Arg Thr Ser Leu Ala Gln Gly
   595 600 605
   Ile Ala Cys Thr Ala Ser Lys Asp Leu Pro Ser Gly Arg Val Ser Ala
   610 615 620
   Val Asn Asp Ser Asp Ala Glu Thr Glu Thr Leu Ser Val Thr Val Gly
   625 630 635 640
   Ile Asn Ser Arg Val Ser Leu Pro Val Thr Pro Gly Val Tyr Arg Leu
   645 650 655
   Arg Ala Met Ser Glu Ser Cys Gln Lys Asn Lys His Glu Tyr Val Ala
   660 665 670
   Arg Arg Cys Ser Gln Pro Ile Phe Glu Val Glu Gln Ile Gln Tyr Ala
   675 680 685
   Lys Glu Ser Tyr Val
   690
<210> 42
   <211> 461
   <212> PRT
   <213> Fgd2 mouse transcript variant 2 t6/tw5; deduced sequence
<400> 42
   Met Tyr Ser Glu Tyr Val Lys Asn Phe Glu Arg Ala Ala Glu Leu Leu
   1 5 10 15
   Ala Thr Trp Met Asp Lys Ser Gln Pro Phe Gln Glu Val Val Thr Arg
   20 25 30
   Ile Gln Cys Ser Glu Ala Ser Gly Ser Leu Thr Leu Gln His His Met
   35 40 45
   Leu Glu Pro Val Gln Arg Ile Pro Arg Tyr Glu Leu Leu Leu Lys Glu
   50 55 60
   Tyr Val Gln Lys Leu Pro Ala Gln Ala Pro Asp Leu Glu Asp Ala Gln
   65 70 75 80
   Arg Ala Leu Asp Met Ile Phe Ser Ala Ala Gln His Ser Asn Ala Ala
   85 90 95
   Ile Ala Glu Met Glu Arg Leu Gln Gly Leu Trp Asp Val Tyr Gln Arg
   100 105 110
   Leu Gly Leu Glu Asp Asp Ile Val Asp Pro Ser Asn Thr Leu Leu Arg
   115 120 125
   Glu Gly Pro Val Leu Lys Ile Ser Phe Arg Arg Ser Asp Pro Met Glu
   130 135 140
   Arg Tyr Leu Val Leu Phe Asn Asn Met Leu Leu Tyr Cys Val Pro Arg
   145 150 155 160
   Val Leu Gln Val Gly Ala Gln Phe Gln Val Arg Thr Arg Ile Asp Val
   165 170 175
   Ala Gly Met Lys Val Arg Glu Leu Thr Asp Ala Glu Phe Pro His Ser
   180 185 190
   Phe Leu Val Ser Gly Lys Gln Arg Thr Leu Glu Leu Gln Ala Arg Ser
   195 200 205
   Arg Asp Glu Met Val Ser Trp Met Gln Ala Cys Gln Ala Ala Ile Asp
   210 215 220
   Gln Val Glu Lys Arg Ser Glu Thr Phe Lys Ala Ala Val Gln Gly Pro
   225 230 235 240
   Gln Gly Asp Thr Gln Glu Pro Lys Pro Gln Val Glu Glu Leu Gly Leu
   245 250 255
   Arg Ala Pro Gln Trp Val Arg Asp Lys Met Val Thr Met Cys Met Arg
   260 265 270
   Cys Gln Glu Pro Phe Asn Ala Leu Thr Arg Arg Arg His His Cys Arg
   275 280 285
   Ala Cys Gly Tyr Val Val Cys Ala Lys Cys Ser Asp Tyr Arg Ala Glu
   290 295 300
   Leu Lys Tyr Asp Ser Asn Arg Pro Asn Arg Val Cys Leu Thr Cys Tyr
   305 310 315 320
   Thr Phe Leu Thr Gly Asn Val Leu Pro Gln Gly Lys Glu Asp Lys Arg
   325 330 335
   Arg Gly Ile Leu Glu Lys Glu Ala Ser Ala Ala Pro Glu Gln Ser Leu
   340 345 350
   Val Cys Ser Phe Leu Gln Leu Ile Gly Asp Lys Cys Ser Arg Ser Leu
   355 360 365
   Pro Arg Ser Trp Cys Val Ile Pro Arg Asp Asp Pro Leu Val Leu Tyr
   370 375 380
   Val Tyr Ala Ala Pro Gln Asp Thr Lys Ala His Thr Ser Ile Pro Leu
   385                 390                 395                 400
   Leu Gly Tyr Gln Val Ile Ser Gly Pro Gln Gly Asp Pro Arg Val Phe
                   405                 410                 415
   Gln Leu Gln Gln Ser Gly Gln Gln Tyr Thr Phe Lys Ala Glu Ser Val
               420                 425                 430
   Glu Leu Gln Gly Arg Trp Val Thr Ala Ile Lys Arg Ala Ala Ser Gly
           435                 440                 445
   Arg Thr Pro Glu Gly Pro Asp Glu Glu Asp Val Ser Asp
       450                 455                 460
<210> 43
   <211> 656
   <212> PRT
   <213> Fgd2 Bos taurus; deduced from Transcript ENSBTAT00000018834
<400> 43
   Met Glu Gly Ala Ser Glu Glu Thr Leu Thr Ser Val Ser Ser Leu Val
   1               5                   10                  15
   Thr Val Phe Glu Asn Ser Arg Met Thr Gly Ala Val Pro Arg Val His
               20                  25                  30
   Gly Leu Glu Ala Gly His Gln Gln Pro Gly Cys Arg Ala Pro Thr Ser
           35                  40                  45
   Ala Gly Pro Trp Glu Lys Pro Thr Val Glu Glu Ala Leu Glu Ser Gly
       50                  55                  60
   Ser Arg Thr Val Ser Arg Arg Tyr Leu Ser Ser Leu Lys Asn Lys Leu
   65                  70                  75                  80
   Ser Ser Gly Ala Trp Arg Lys Ser Tyr Gln Pro Arg Thr Cys Pro Gly
                   85                  90                  95
   Ser Gly Thr Gln Glu Pro Glu Glu Lys Lys Ile Val Arg Glu Leu Leu
               100                 105                 110
   Glu Thr Glu Gln Ala Tyr Val Ala Arg Leu His Leu Leu Asp Gln Val
           115                 120                 125
   Phe Phe Gln Glu Leu Leu Lys Glu Ala Arg Ser Ser Lys Ala Phe Pro
       130                 135                 140
   Glu Asp Val Val Arg Leu Ile Phe Ser Asn Ile Ser Ser Ile Tyr Gln
   145 150 155 160
   Phe His Ser Gln Phe Phe Leu Pro Glu Leu Gln Arg Arg Leu Asp Asp
   165 170 175
   Trp Thr Thr Thr Pro Arg Ile Gly Asp Val Ile Gln Lys Leu Ala Pro
   180 185 190
   Phe Leu Lys Met Tyr Ser Glu Tyr Val Lys Asn Phe Glu Arg Ala Ile
   195 200 205
   Glu Leu Leu Ala Thr Trp Thr Asp Lys Ser Pro Pro Phe Gln Glu Val
   210 215 220
   Ile Thr Arg Ile Gln Ser Ser Glu Ala Ser Ala Ser Leu Thr Leu Gln
   225 230 235 240
   His His Met Leu Glu Pro Val Gln Arg Ile Pro Arg Tyr Glu Leu Leu
   245 250 255
   Leu Lys Glu Tyr Val Gln Lys Leu Pro Gly Gln Ala Pro Asp Leu Ala
   260 265 270
   Asp Ala Gln Lys Ala Leu Asp Met Ile Phe Ser Ala Ala Gln His Ser
   275 280 285
   Asn Ala Ala Ile Thr Glu Met Glu Arg Leu Gln Glu Leu Trp Asp Val
   290 295 300
   Tyr Gln Arg Leu Gly Leu Glu Asp Asp Ile Val Asp Pro Ser Asn Thr
   305 310 315 320
   Leu Leu Arg Glu Gly Pro Val Leu Lys Ile Ser Phe Arg Arg Ser Gly
   325 330 335
   Pro Met Glu Arg Tyr Leu Phe Leu Phe Asn Asn Met Leu Leu Tyr Cys
   340 345 350
   Val Pro Arg Val Ile Gln Val Gly Ala His Phe Gln Val Arg Thr Arg
   355 360 365
   Ile Asp Val Ala Gly Met Lys Val Arg Glu Leu Thr Asp Ala Glu Phe
   370 375 380
   Pro His Ser Phe Leu Val Ser Gly Lys Gln Arg Thr Leu Glu Leu Arg
   385 390 395 400
   Ala Arg Ser Gln Glu Glu Met Ile Ser Trp Leu Gln Ala Cys Gln Ala
                   405                 410                 415
   Ala Ile Asp Gln Ile Glu Lys Arg Asn Glu Thr Phe Lys Ala Ala Val
               420                 425                 430
   Gln Ser Pro Glu Gly Asp Pro Gln Glu Gln Glu Leu Gln Ser Val Glu
           435                 440                 445
   Leu Gly Leu Arg Ala Pro Gln Trp Val Arg Asp Lys Met Val Thr Met
       450                 455                 460
   Cys Met Arg Cys Gln Glu Pro Phe Asn Ala Leu Thr Arg Arg Arg His
   465                 470                 475                 480
   His Cys Arg Ala Cys Gly Tyr Val Val Cys Ala Arg Cys Ser Asp Tyr
                   485                 490                 495
   Arg Ala Glu Leu Lys Tyr Asp Ala Asn Arg Pro Asn Arg Val Cys Leu
               500                 505                 510
   Asp Cys Tyr Thr Phe Leu Thr Gly Asn Val Leu Pro Glu Glu Lys Glu
           515                 520                 525
   Asp Lys Arg Arg Gly Ile Leu Glu Lys Gly Ala Val Ala Gly Ser Glu
       530                 535                 540
   Gln Ser Leu Ile Cys Ser Phe Leu Gln Leu Leu Gly Asp Lys Trp Gly
   545                 550                 555                 560
   Lys Ser Gly Pro Arg Gly Trp Cys Val Ile Pro Arg Asp Asp Pro Leu
                   565                 570                 575
   Val Leu Tyr Val Tyr Ala Ala Pro Gln Asp Met Arg Ala His Thr Ser
               580                 585                 590
   Ile Pro Leu Leu Gly Tyr Gln Val Thr Ala Gly Pro Gln Ala Asp Pro
           595                 600                 605
   Arg Val Phe Gln Leu Gln Gln Ser Gly Gln Leu Tyr Thr Phe Lys Ala
       610                 615                 620
   Glu Ser Glu Glu Leu Lys Gly Arg Trp Val Lys Ala Met Glu Arg Ala
   625                 630                 635                 640
   Ala Ser Gly Trp Ile Pro Gly Gly Pro Asp Asp Lys Asp Leu Ser Asp
                   645                 650                 655
<210> 44
   <211> 657
   <212> PRT
   <213> Fgd2 Canis familiaris; deduced from Transcript ENSCAFT00000002252
<400> 44
   Met Glu Gly Glu Ser Lys Glu Lys Leu Ala Ser Val Ser Asn Leu Val
   1               5                   10                  15
   Thr Val Phe Glu Asn Ser Arg Ile Pro Gly Ala Ala Ser Ser Val Ser
               20                  25                  30
   Lys Leu Glu Ile Glu His His His Pro Gly Cys Arg Ser Pro Pro Pro
           35                  40                  45
   Ala Glu Pro Leu Lys Glu Pro Asn Leu Gly Glu Thr Leu Arg Gly Ser
       50                  55                  60
   Glu Pro Arg Met Val Ser Arg Arg Tyr Leu Ser Ser Leu Lys Asn Lys
   65                  70                  75                  80
   Leu Ser Ser Gly Ala Trp Arg Lys Ser Cys Gln Pro Gly Thr Asp Pro
                   85                  90                  95
   Gly Pro Gly Thr Gln Glu Pro Glu Glu Lys Arg Ile Val Gln Glu Leu
               100                 105                 110
   Leu Glu Thr Glu Lys Ala Tyr Val Ala Arg Leu His Leu Leu Asp Gln
           115                 120                 125
   Val Phe Phe Gln Glu Leu Leu Arg Glu Ala Arg Ser Ser Lys Ala Phe
       130                 135                 140
   Pro Glu Asp Val Val Arg Leu Ile Phe Ser Asn Ile Ser Ser Ile Tyr
   145                 150                 155                 160
   Gln Phe His Ala Gln Phe Phe Leu Pro Glu Leu Gln Arg Arg Leu Asp
                   165                 170                 175
   Asp Trp Thr Ala Thr Pro Arg Ile Gly Asp Val Ile Gln Lys Leu Ala
               180                 185                 190
   Pro Phe Leu Lys Met Tyr Ser Glu Tyr Val Lys Asn Phe Glu Arg Ala
           195                 200                 205
   Ala Glu Leu Leu Ala Thr Trp Thr Asp Lys Ser Ala Pro Phe Gln Glu
       210                 215                 220
   Val Ile Thr Arg Ile Gln Ser Ser Glu Ala Ser Gly Ser Leu Thr Leu
   225                 230                 235                 240
   Gln His His Met Leu Glu Pro Val Gln Arg Ile Pro Arg Tyr Glu Leu
                   245                 250                 255
   Leu Leu Lys Glu Tyr Val Gln Lys Leu Pro Ala Gln Ala Pro Asp Arg
               260                 265                 270
   Ala Asp Ala Gln Lys Ala Leu Asp Met Ile Phe Ser Ala Ala Gln His
           275                 280                 285
   Ser Asn Ala Ala Ile Ser Glu Met Glu Arg Leu Gln Asp Leu Trp Glu
       290                 295                 300
   Val Tyr Gln Arg Leu Gly Leu Glu Gly Asp Ile Val Asp Pro Ser Asn
   305                 310                 315                 320
   Thr Leu Leu Arg Glu Gly Pro Val Leu Lys Ile Ser Phe Arg Arg Ser
                   325                 330                 335
   Asp Pro Met Glu Arg Tyr Leu Phe Leu Phe Asn Asn Met Leu Leu Tyr
               340                 345                 350
   Cys Ile Pro Lys Val Ile Gln Val Gly Ala Gln Phe Gln Val Arg Thr
           355                 360                 365
   Arg Ile Asp Val Ala Gly Met Lys Val Arg Glu Leu Thr Asp Ala Glu
       370                 375                 380
   Phe Pro His Ser Phe Leu Val Ser Gly Lys Gln Arg Thr Leu Glu Leu
   385                 390                 395                 400
   Gln Ala Arg Ser Gln Glu Glu Met Ile Ser Trp Met Gln Ala Cys Gln
                   405                 410                 415
   Ala Ala Ile Asp Gln Ile Glu Lys Arg Asn Glu Thr Phe Lys Ala Ala
               420                 425                 430
   Val Gln Gly Pro Glu Gly Asp Thr Gln Glu Gln Glu Leu Gln Ser Glu
           435                 440                 445
   Glu Leu Gly Leu Arg Ala Pro Gln Trp Val Arg Asp Lys Met Val Thr
       450                 455                 460
   Met Cys Met Arg Cys Arg Glu Pro Phe Asn Ala Leu Arg Arg Arg Arg
   465                 470                 475                 480
   His His Cys Arg Ala Cys Gly Phe Val Val Cys Ala Lys Cys Ser Asp
                   485                 490                 495
   Tyr Arg Ala Glu Leu Lys Tyr Asp Asp Asn Arg Pro Asn Arg Val Cys
               500                 505                 510
   Phe His Cys Tyr Thr Phe Leu Thr Gly Asn Val Leu Pro Glu Asp Arg
           515                 520                 525
   Glu Asp Arg Arg Arg Gly Ile Leu Glu Lys Gly Ser Met Thr Gly Ser
       530                 535                 540
   Glu Gln Ser Leu Met Cys Ser Phe Leu Gln Leu Val Gly Asp Lys Trp
   545                 550                 555                 560
   Gly Lys Ser Ser Pro Arg Gly Trp Cys Val Ile Pro Arg Asp Asp Pro
                   565                 570                 575
   Leu Val Leu Tyr Val Tyr Ala Ala Pro Gln Asp Met Arg Ala His Thr
               580                 585                 590
   Ser Ile Pro Leu Leu Gly Tyr Gln Val Thr Ala Gly Thr Gln Ala Asp
           595                 600                 605
   Pro Arg Val Phe Gln Leu Gln Gln Ser Gly Gln Leu Tyr Thr Phe Lys
       610                 615                 620
   Ala Glu Thr Glu Glu Leu Arg Asp Arg Trp Val Lys Ala Met Glu Arg
   625                 630                 635                 640
   Ala Ala Ser Gly Trp Ser Pro Arg Gly Pro Lys Asp Gly Asp Leu Ser
                   645                 650                 655
   Asp
<210> 45
   <211> 657
   <212> PRT
   <213> Fgd2 Rattus norvegicus; deduced from Transcript ENSRNOT00000000636
<400> 45
   Met Glu Gln Ala Cys Ala Lys Gln Asp Ser Val Cys Asn Leu Val Ala
   1               5                   10                  15
   Val Phe Glu Asn Ser Arg Ser Pro Gly Ala Ala Pro Gly Pro His Ser
               20                  25                  30
   Phe Glu Asp Gln Pro His Ser Pro Glu His Gln Leu Pro Leu Ser Pro
           35                  40                  45
   Glu Pro Trp Glu Ala Pro Pro Ala Gly Glu Ala Leu Thr Ser Glu Phe
       50                  55                  60
   Arg Pro Val Ser Arg Thr Tyr Leu Asn Ser Leu Lys Asn Lys Leu Ser
   65                  70                  75                  80
   Ser Gly Ala Trp Arg Lys Ser Cys Gln Pro Gly Ala Ser Pro Gly Pro
                   85                  90                  95
   Glu Thr Gln Glu Pro Glu Glu Lys Arg Val Val Gln Glu Leu Leu Glu
               100                 105                 110
   Thr Glu Gln Ala Tyr Val Ala Arg Leu His Leu Leu Asp Gln Ala Thr
           115                 120                 125
   Val Phe Phe Gln Glu Leu Leu Arg Glu Ala Ser Arg Ser Lys Ala Phe
       130                 135                 140
   Pro Glu Asp Val Val Lys Leu Ile Phe Ser Asn Ile Ser Ser Ile Tyr
   145                 150                 155                 160
   Arg Phe His Ala Gln Phe Phe Leu Pro Glu Leu Gln Arg Arg Val Asp
                   165                 170                 175
   Asp Trp Thr Ala Thr Pro Arg Ile Gly Asp Val Ile Gln Lys Leu Ala
               180                 185                 190
   Pro Phe Leu Lys Met Tyr Ser Glu Tyr Val Lys Asn Phe Glu Arg Ala
           195                 200                 205
   Ala Glu Leu Leu Ala Thr Trp Met Asp Lys Ser Gln Pro Phe Gln Glu
       210                 215                 220
   Val Val Thr Arg Ile Gln Arg Ser Glu Ala Ser Gly Ser Leu Thr Leu
   225                 230                 235                 240
   Gln His His Met Leu Glu Pro Val Gln Arg Ile Pro Arg Tyr Glu Leu
                   245                 250                 255
   Leu Leu Lys Glu Tyr Val Gln Lys Leu Pro Ala Gln Ala Pro Asp Leu
               260                 265                 270
   Glu Asp Ala Gln Arg Ala Leu Asp Met Ile Phe Ser Ala Ala Gln His
           275                 280                 285
   Ser Asn Ala Ala Ile Ala Glu Met Glu Arg Leu Gln Gly Leu Trp Asp
       290                 295                 300
   Val Tyr Gln Arg Leu Gly Leu Glu Asp Asp Ile Val Asp Pro Ser Asn
   305 310 315 320
   Thr Leu Leu Arg Glu Gly Pro Val Leu Lys Ile Ser Phe Arg Arg Ser
   325 330 335
   Asp Pro Met Glu Arg Tyr Leu Val Leu Phe Asn Asn Met Leu Leu Tyr
   340 345 350
   Cys Val Pro Arg Val Leu Gln Val Gly Ala Gln Phe Gln Val Arg Thr
   355 360 365
   Arg Ile Asp Val Ala Gly Met Lys Val Arg Glu Leu Thr Asp Ala Glu
   370 375 380
   Phe Pro His Ser Phe Leu Val Ser Gly Lys Gln Arg Thr Leu Glu Leu
   385 390 395 400
   Gln Ala Arg Ser Arg Glu Glu Met Val Ser Trp Ile Gln Ala Cys Gln
   405 410 415
   Ala Ala Ile Asp Gln Val Glu Lys Arg Ser Glu Thr Phe Lys Ala Ala
   420 425 430
   Val Gln Gly Pro Gln Gly Asp Thr Gln Glu Pro Lys Pro Gln Ala Glu
   435 440 445
   Glu Leu Gly Leu Arg Ala Pro Gln Trp Val Arg Asp Lys Met Val Thr
   450 455 460
   Met Cys Met Arg Cys Gln Glu Pro Phe Asn Ala Leu Thr Arg Arg Arg
   465 470 475 480
   His His Cys Arg Ala Cys Gly Tyr Val Val Cys Ala Lys Cys Ser Asp
   485 490 495
   Tyr Arg Ala Glu Leu Lys Tyr Asp Gly Asn Arg Pro Asn Arg Val Cys
   500 505 510
   Leu Thr Cys Tyr Thr Phe Leu Thr Gly Asn Leu Leu Pro Asp Ser Lys
   515 520 525
   Glu Asp Lys Arg Arg Gly Ile Leu Glu Lys Glu Thr Ser Ala Gly Pro
   530 535 540
   Asp Gln Ser Val Val Cys Ser Phe Leu Gln Leu Met Gly Asp Lys Ser
   545 550 555 560
   Ile Arg Ser Ile Pro Arg Ser Trp Cys Val Ile Pro Arg Asp Asp Pro
                   565                 570                 575
   Leu Val Leu Tyr Val Tyr Ala Ala Pro Gln Asp Met Lys Ala His Thr
               580                 585                 590
   Ser Ile Pro Leu Leu Gly Tyr Gln Val Thr Ser Gly Pro Gln Gly Asp
           595                 600                 605
   Pro Arg Val Phe Gln Leu Gln Gln Ser Gly Gln Gln Tyr Thr Phe Lys
       610                 615                 620
   Ala Glu Ser Val Glu Leu Gln Gly Arg Trp Val Arg Ala Ile Lys Arg
   625                 630                 635                 640
   Ala Ala Ser Gly Trp Thr Pro Glu Gly Pro Asp Glu Glu Asp Met Ser
                   645                 650                 655
   Asp
<210> 46
   <211> 579
   <212> PRT
   <213> Fgd2 (splice variant) Rattus norvegicus; deduced from Transcript ENSRNOT00000041665
<400> 46
   Met Glu Gln Ala Cys Ala Lys Gln Asp Ser Val Cys Asn Leu Val Ala
   1               5                   10                  15
   Val Phe Glu Asn Ser Arg Asn Leu Arg Arg Arg Gly Ser Cys Arg Ser
               20                  25                  30
   Phe Trp Arg Arg Ser Arg Pro Thr Trp Leu Ala Cys Thr Cys Leu Thr
           35                  40                  45
   Arg Pro Val Phe Phe Gln Glu Leu Leu Arg Glu Ala Ser Arg Ser Lys
       50                  55                  60
   Ala Phe Pro Glu Asp Val Val Lys Leu Ile Phe Ser Asn Ile Ser Ser
   65                  70                  75                  80
   Ile Tyr Arg Phe His Ala Gln Phe Phe Leu Pro Glu Leu Gln Arg Arg
                   85                  90                  95
   Val Asp Asp Trp Thr Ala Thr Pro Arg Ile Gly Asp Val Ile Gln Lys
               100                 105                 110
   Leu Ala Pro Phe Leu Lys Met Tyr Ser Glu Tyr Val Lys Asn Phe Glu
           115                 120                 125
   Arg Ala Ala Glu Leu Leu Ala Thr Trp Met Asp Lys Ser Gln Pro Phe
       130                 135                 140
   Gln Glu Val Val Thr Arg Ile Gln Arg Ser Glu Ala Ser Gly Ser Leu
   145                 150                 155                 160
   Thr Leu Gln His His Met Leu Glu Pro Val Gln Arg Ile Pro Arg Tyr
                   165                 170                 175
   Glu Leu Leu Leu Lys Glu Tyr Val Gln Lys Leu Pro Ala Gln Ala Pro
               180                 185                 190
   Asp Leu Glu Asp Ala Gln Arg Ala Leu Asp Met Ile Phe Ser Ala Ala
           195                 200                 205
   Gln His Ser Asn Ala Ala Ile Ala Glu Met Glu Arg Leu Gln Gly Leu
       210                 215                 220
   Trp Asp Val Tyr Gln Arg Leu Gly Leu Glu Asp Asp Ile Val Asp Pro
   225                 230                 235                 240
   Ser Asn Thr Leu Leu Arg Glu Gly Pro Val Leu Lys Ile Ser Phe Arg
                   245                 250                 255
   Arg Ser Asp Pro Met Glu Arg Tyr Leu Val Leu Phe Asn Asn Met Leu
               260                 265                 270
   Leu Tyr Cys Val Pro Arg Val Leu Gln Val Gly Ala Gln Phe Gln Val
           275                 280                 285
   Arg Thr Arg Ile Asp Val Ala Gly Met Lys Val Arg Glu Leu Thr Asp
       290                 295                 300
   Ala Glu Phe Pro His Ser Phe Leu Val Ser Gly Lys Gln Arg Thr Leu
   305                 310                 315                 320
   Glu Leu Gln Ala Arg Ser Arg Glu Glu Met Val Ser Trp Ile Gln Ala
                   325                 330                 335
   Cys Gln Ala Ala Ile Asp Gln Val Glu Lys Arg Ser Glu Thr Phe Lys
               340                 345                 350
   Ala Ala Val Gln Gly Pro Gln Gly Asp Thr Gln Glu Pro Lys Pro Gln
           355                 360                 365
   Ala Glu Glu Leu Gly Leu Arg Ala Pro Gln Trp Val Arg Asp Lys Met
       370                 375                 380
   Val Thr Met Cys Met Arg Cys Gln Glu Pro Phe Asn Ala Leu Thr Arg
   385                 390                 395                 400
   Arg Arg His His Cys Arg Ala Cys Gly Tyr Val Val Cys Ala Lys Cys
                   405                 410                 415
   Ser Asp Tyr Arg Ala Glu Leu Lys Tyr Asp Gly Asn Arg Pro Asn Arg
               420                 425                 430
   Val Cys Leu Thr Cys Tyr Thr Phe Leu Thr Gly Asn Leu Leu Pro Asp
           435                 440                 445
   Ser Lys Glu Asp Lys Arg Arg Gly Ile Leu Glu Lys Glu Thr Ser Ala
       450                 455                 460
   Gly Pro Asp Gln Ser Val Val Cys Ser Phe Leu Gln Leu Met Gly Asp
   465                 470                 475                 480
   Lys Ser Ile Arg Ser Ile Pro Arg Ser Trp Cys Val Ile Pro Arg Asp
                   485                 490                 495
   Asp Pro Leu Val Leu Tyr Val Tyr Ala Ala Pro Gln Asp Met Lys Ala
               500                 505                 510
   His Thr Ser Ile Pro Leu Leu Gly Tyr Gln Val Thr Ser Gly Pro Gln
           515                 520                 525
   Gly Asp Pro Arg Val Phe Gln Leu Gln Gln Ser Gly Gln Gln Tyr Thr
       530                 535                 540
   Phe Lys Ala Glu Ser Val Glu Leu Gln Gly Arg Trp Val Arg Ala Ile
   545                 550                 555                 560
   Lys Arg Ala Ala Ser Gly Trp Thr Pro Glu Gly Pro Asp Glu Glu Asp
                   565                 570                 575
   Met Ser Asp
<210> 47
   <211> 5121
   <212> DNA
   <213> Tiam2 Bos taurus; Transcript ENSBTAT00000013256
<400> 47
   ctcttactga cactgctgtt cttaattcag gtgacgatgg gaaactccga gagtcagtac 60
   accctacaag gacctaaaaa tcatagcaat actattactg gtgctaaaca aaagccttgc 120
   tccctgaaaa tccgtggcct tcatgccaag gatgacaagt cattgcacgg atggggtcat 180
   ggaagcagcg gagcggggta caagtccagg tcgttggccc gaagctgcct ttctcacttt 240
   aagagcaatc agccttacgc atcccgacca gggggcccca cgtgcaaagc ctccaaaggc 300
   agtgcctacg ccaagcatag gacaggtgcc gcagggtcag atttccaggg caccgatgcc 360
   gctttctcgc ccgagaacgg cttccactat gtcggacgcc cgcccgagga gaaccactcg 420
   gcttcgaggg actgccgcaa tgggcacctt ctcaactgct acggcgggaa cgagagcatc 480
   gcatccaccc cgcctggcga ggaccgcaag agcccccggg tgctcatcaa gacgctgggc 540
   aagctggacg ggtgcctgcg ggtagagttc cacagcggcg ccgcgccccc gggggcctcc 600
   ccatcgggcg gcccggtgca gctgctgcgc tactcggccg gttccgcgcc cagccccagc 660
   gcctccccgg cagccgccgc gcgcccccgc tccagcaagg gcagctcgct cagctccgag 720
   tcctcctggt acgactcgcc ctggggcccg gccggcgagg tcagtgaggc tgagggctcc 780
   ttcgtggcgc cagacacgcc tgagcccggc ctccacactg gcttcccgac cagggatgct 840
   ccaaagcctt tcagccaaag cgcctccctc tcatccctcc gggaccccta ccccaacgcc 900
   tcccttggga gcctggcccc cgcggccctg cggctttccg atgactacat gggcacccgc 960
   gccagcctca gtgcccgcgt ctccttcgcc tccgacatgg acgtgccggc ccgcgtggag 1020
   cgcggggagc ccgggcagtt cgcgtccttc accctccctt gccgcaagtc cagggccctg 1080
   ggcgaggagt cctccaagaa ggacaccctg aaagccagga tgcgccgcat cagcgactgg 1140
   acgggaagcc tctccagaaa gaaaagaaga ctccaggagc cgaggtccaa ggagggcagt 1200
   gactactttg acagccgttc ggatgggctg aacgtggaca cgcaagggcc ctcccaaggg 1260
   tctgtgtccc tgtggtcagg gggctctgct cagatcctgt cccatagaag tgaatctgct 1320
   catgcgatcg gcagcgaccc cctccagcag aacatttatg agaatttcat gcgagagctg 1380
   gagatgagca ggaccaacac ggagaacctc gagacgtcca cggagacggc cgagtccagc 1440
   agcgagtcgc tcagctcttt ggagcaactg gacctgctct ttgagaagga acaaggggtg 1500
   gtgcgcaaag ccgggtggct cttcttcaaa cccctcgtca ctctgcagaa ggaaaggaag 1560
   ctggaactgg tggcccggag gaaatggaag cagtattggg tgacacttaa aggatgcacg 1620
   ctgctgtttt acgagaccta cgggaagaat tctatggatc agagcagtgc cccccgctgt 1680
   gccctctttg cagaagacag catagtgcag tctgtcccgg aacatcccaa gaaggaaaat 1740
   gtcttctgcc tcagcaactc cttcggggat gtctacctat tccaggccac cagccagaca 1800
   gacctggaga actgggtcac cgccatccac tcggcctgcg catccctctt cgcaaagaaa 1860
   cacggcaaag aggacactgt ccggctgctg aagaaccaga ccagaaacct cctccagaag 1920
   atagacatgg acagcaagat gaagaagatg gcagagttgc agctgtccgt ggtgagcgac 1980
   ccaaagaaca ggaaggccat agagaaccag aatagggcct taaaaaattg gcaagagatt 2040
   ctcctggaca cccaacttgg agttttcagg tccttatgtg ttgtcaaatg tttaggtcag 2100
   ggagaggagc tgcccttgac cgccctcccc acccctccat cagggaccta ctatgctccg 2160
   ataattctca gggtcgctct ttatctcaca cactgttttg tgtgttctag agacgactct 2220
   gcccttcgga aaagaactct ctcgctgact cagcggggaa gacacaagaa ggggttgttt 2280
   tcttcattaa aaggcctgga cacacttgca agaaaaggga aggagaaaag accgtctata 2340
   actcagatat tcgattccag tggcagccat ggattttccg gaactcagct acctcaaagc 2400
   tccaataact ccagtgaggt ggatgacctg ctacacttat acgggtcagc agtggatggc 2460
   gttccccgag acggcacgtg ggagagccag gttgacgttc actttccgga tcgtcaagtc 2520
   gtgactgtga tggtcaagcc ggaaaccagg gtagaagatg ttctgactct ggcctgcaag 2580
   atgaggcagt tggaacccag ccattatggc ctacaacttc ggaagttagt ggacgagaac 2640
   gttgagtact gtattcctgc accctacgaa tatatgcagg accaggttta tgatgagata 2700
   gaagtctttc cactaagcgt gtatgatgtg cagctgacaa agaccggggg tgtgtctgac 2760
   ttcgggtttg cagtgactgc tcaggtggac gagcaccagc atctcagccg catctttata 2820
   agtgacgtcc tccctgacgg cctggcgtat ggggaagggt tgagaaaggg caatgaaatc 2880
   atgaccttaa atggggaagc cgtgtctgat cttgacctca agcagatgga ggctctgttt 2940
   tctgagaaga gtgtcgggct cactctgatt gccaggccac cagacacaaa agcgactctg 3000
   tgttcttcct ggtcagacag tgacctgttc tcccgggacc agaagggtct gctgccccct 3060
   cctaaccagt cccaacttct ggaggaattc ctggatcact ttaaaaagga tacggcaaat 3120
   gatttcagca acgtccccga tgtcacaact ggcctgaaaa ggagtcagac tgatggcaca 3180
   ctggatcagg tttcccacag ggagaagaag gagcagacat tcaggagtgc tgagcagatc 3240
   actgcgctgt gtaggaattt taacgacacc caggccaacg gcatggaagg gccaagggag 3300
   ggtcaggacc ctgctccaag gccactggct cgccacctct ctgatgcaga tcgcctccgg 3360
   aaagtcatcc aggagctcat ggacacagag aagtcctatg tgaaggatct gagctgcctc 3420
   ttcgacttat atttggagcc acttcagagt gagaccatcc tggtcttcac tcagatggag 3480
   tcactttttg gaagcttgcc agagatgctt gaatttcaaa aggtgtttct cgagaccctg 3540
   gaggatggga tttcagcgtc gtctgacttt aacatacttg aaaccccctc gcagttcaga 3600
   aaactgctgt tttccctcgg aggctctttc ctttattatg cggaccactt taaactgtat 3660
   agcggattct gtgctaatca tattaaagta cagaaggttc tagagcgagc taaaactgat 3720
   aaagccttta aggcttttct ggatgcccgg aaccccacca agcagcactc ctccacgctg 3780
   gagtcttacc tcatcaagcc agttcagagg gtgctcaagt acccgctgct gctcaaggag 3840
   ctggtgtccc tgacggacca cgagagcgag gagcactatc atctgacgga agcactaaag   3900
   gcaatggaaa aagtagccag ccacatcaat gagatgcaga agatatatga agattatggg   3960
   accgtatttg accagcttgt agcagagcag agtggaacag agaaggaggt cacagaactt   4020
   tcaatggggg agcttctgat gcactctaca gtgtcctggt tgaatccatt tctgtctcta   4080
   ggaaaagcca gaaaggacct tgaactcaca gtatttgttt ttaagagagc tgtcatattg   4140
   gtttataaag aaaactgcaa actgaaaaag aaattgccct caaattcccg gcctgcacac   4200
   agcaatgctg acctggatcc gtttaagttc cgctggttga tccccatatc cgcgcttcaa   4260
   gtcagactgg gaaatacagc agggacagaa aataattcct tatgggagct gatccacacc   4320
   aagtcagaaa tagaaggacg gccagagacc atctttcagt tgtgctgcag tgacaatgaa   4380
   agcaaaacca acatcgtcaa ggtgattcga tctattctga gggaaaattt caggcgtcac   4440
   ataaagtgtg aattacccct ggagaaaaca tgtaaggatc gcctggtacc ccttaagaac   4500
   cgcgttcctg tctcagccaa attagcttca tccaggtcct taaaagtcct taagaattcc   4560
   tccagcagcg agtggccggg ggagccgggc aagggcagct ccctggactc ggacgagggc   4620
   agcctgagca gcagcaccca gagcagcggc tgcccccctg ccggaagggg gcaggacacc   4680
   cagcagcacc cccacgcggg cctggccgac ttctccgaca gtctcatcaa agagagcgac   4740
   attctgagcg acgacgagga cgactacccc caggctcgga ggcagggcag ccccaccaaa   4800
   gacatcgaga tccagttcca gagactgagg atctctgagg accccgatgc tcacccggct   4860
   gaagagccgc ccagcaagga tgggcagccc aagctggtgc gggggcactt ctgcgccatc   4920
   aaacgcaagg ccaacagcac caagcgggac aggggcaccc tgctgaaggc gcagacgcgt   4980
   caccagtccc tggacagtca tcctggaaac gccaaccttg atctcagctc tgtccttgag   5040
   cgagaattca gcgtccagag cttaacatcg gttgtcaacg aagagtgctt ttatgaaaca   5100
   gagagccatg gaaagtccta g                                             5121
<210> 48
   <211> 5188
   <212> DNA
   <213> Tiam2 Gallus gallus; Transcript ENSGALG00000013645
<400> 48
   atgggaaatt cagaaagcca atacagtctt cagggatcaa aaaatcatgc tgctgcttca     60
   actggttcca agcagaagcc ttgctctcta aaaattcgca gcattcatgc taaagatgaa    120
   aagtcttgct ccctgcatgg atggggacat accaatagtg gctcaaacta caagtcaagg    180
   tctcttgcta gaagctgcct ttcacacttc aagagtagcc aaccttattc agctagactc    240
   agtgacactg tggtgaaggc ctctaaaagt aacgtccttg ccaaacacag gacacacacc    300
   tcaggggact actgtccagg aaataatgca gtgtttttgc ctgataatgg tttccactat    360
   attggccttc aagctggaag taatcatgct gcaccccgag attgcaatgg gcacatttta    420
   aaatgctatg gaaagaatga gagtcttgca tcaacctctc catcagagga caggaggagt     480
   ccaaaagtac tcattaaaac actggggaag ctggatggtt gcttgagagt cgaattccac     540
   aacagtagca acagcaaagt accaactgag gagtccagtg ggccagtcca gctgctgagg     600
   tattcaccta ccttggaatc taagtcaaat aacctgctag atgtcaggag gaactccagc     660
   gcagactgtt cttcaaacca tcgtctgtca cctactgatt caaggcttcg atctagtaag     720
   ggaagctccc taagctccga gtcttcatgg tatgactctc tctggggaaa tgctggggat     780
   atcagtgagt tggatggtcc atatttgact aggagcactc cagatacaag cattcatgcc     840
   agtttcccag caagtgacaa gaaatccttc aatcaaagtt catctctgtc ctcacttcga     900
   gatctctata aggatacaaa tttggaaggc actcctccac ctgggatcag gctgtctgat     960
   gagtatattg acactcatgg tagcctaagc aaccgtgtat catttgcttc agacattgat    1020
   gttccctcca gggtagagca gggaagtcct gctcattatt cctcttacac tctcccatgt    1080
   agaaagtcca agccactcgc tgaggatgca tccaagaagg acacattaaa aagccgcatg    1140
   aggcgcatca gcgattggac aggaagtctc tcgaggaaga aaaggaagtt gcaggaaccc    1200
   aaatgtaaag atgggagcga atactttgac agcagaatgg acaacttcag cacagacacg    1260
   ctggcaccat cacagcagtc tactttgttg tggtcaccca gctccagtca catcctgtcc    1320
   cagagaagtg agtccaccaa tgcagtcagc agtgatgccc tgaggcagaa catttatgaa    1380
   aacttcatgc gggagctgga gatgagcagg acaaacctgg agaacactga gacctcatca    1440
   gaaacggagg actccagcag tgagtctctc agctccttgg agcaactgga tttgttgtac    1500
   gagaaggagc aaggagtggt gcgcaaagca gggtggctgt tcttcaaacc actggtgacc    1560
   ctgcagaaag agaagaagct ggagctggtc acacggcgaa agtggaagca gtactgggta    1620
   acactaaaag gttgtactct gctcttctat gagacctatg gaaggaattc aatggagcag    1680
   agtagtttgc ctcgatacgc cctgtttgct gaagacagta tagtccagtc tgttccagaa    1740
   catcccaaga aagaaaacgt gttctgtctc agcaattctt ttggagatgt ctacctattt    1800
   caggcaacaa gtcagacaga tctggaaaac tgggttactg ccatacattc agcctgtgct    1860
   tccctctttg caaaaaagct tgggaaggag gacacagttc ggctactgaa aaatcagacc    1920
   aagagtctct tccagaagat tgatatggac agcaagatga aaaagatggc agagttgcag    1980
   ctctcaattg ttagcgatcc aaaaaacagg aaggcgatag agaatcagat ccagcaatgg    2040
   gaacagaacc tggaaaaatt taacatggac cttttccgaa tgcgatgtta cctagccagt    2100
   ttgcaaggtg gggagcttcc gaacccaaag agccttttgg ctgctgccag tcgtccttca    2160
   aaattggcac tgggaaggct cggcattttc tcagtgtcat ccttccatgc actgatctgc    2220
   tccagggatg aagctgctct caggaagcgt accctgtctc tgtctcaaag agtccgaaat    2280
   aagaagggtt tgttttcttc actaaaagga ctggacacac tggcaagaaa aggaaaagag    2340
   aagcgacctt ccataacgca gatttttgac tccactggag gacatggatt tgccggtgtc 2400
   cagaattcag ccaactctgc tgagcaagtt gatgaatttt tgaatgtcta ctgctcagta 2460
   ccggacagca tccagaagga gaatgcttgg gaaacacaaa cgtatgttca cttctgtgac 2520
   ggtcaaggag tagctttaac tctgaaacca gaccacaggg tggaagatgt tctgtctctg 2580
   gcatgcaaga tgaaacaact ggagccaagg aactacggtc tacaactcag aagattggtt 2640
   gatgaaaata ctgaatactg tgctcctgaa ccatacgaat atatagtaga ccaggtgtat 2700
   gatgaaatag aaatctgccc attaaatgtt tatcacattc atcttacaaa gactgaaaat 2760
   ataacagatt ttggttttgc tgtcacagct caagttgatg aaaatcagca tctcacacac 2820
   atatttgtaa gtgatgttct tcctgatggg cttgcataca gggaagggct acgagtaggc 2880
   aatgagatcc tgagcataaa tggagagtct gtgtctgatc ttgacctcag gcagatggag 2940
   ctattgtttt cagagagaag cgtaatgctc actttgagaa tgagccactg tgggaatcag 3000
   caacccttgt gtgcatcctg gtcatcagat ggtgacattt ccagggttcc aaaaagtttg 3060
   ttgccgcctc caaaccagtc acagctcctg gaagagtttt tagataactt cagaaaaaac 3120
   acagaaaatg atgcagattt taataacgtg cctgatgtca catccagctt gaaaaggagc 3180
   agtactgatg gcactctgga ccaagtacca cacagggaga agactgatcc acctttcagg 3240
   agtgcagagc agatcagtgc gttgtgccgc aacttccaag aggtccagac gagcagcatg 3300
   gaagggcaga aggacaacca ggatccgcct ccacgaccac tggctcgcca cctttctgat 3360
   gcagacagat tgaggaaagt catccaagaa cttatggata ctgagaaatc ttatgtcaag 3420
   gacttgagtt gcctctttga gctatacttg gagcctcttc aaaatgaaac cttccttaca 3480
   caagatgaga tggagtcctt gttcggcagt ctgccagaaa tgctggattt tcagaaggtg 3540
   tttttggaga cccttgaaga tggaatatct tcttcctcgg actttaacac actggagaca 3600
   ccgtctcagt tccggaaatt gctgttttcc ctgggaggat cgtttctgta ttatgctgac 3660
   cacttcaaac tgtacagtgg cttttgtgcc aaccacatca aagttcagaa agttcttgag 3720
   agagccaaaa cagacagtgc atttaaggcc ttcttggatg ctcgcaatcc tacaaagcaa 3780
   cactcctcta cgctggagtc gtatctcata aagcctgttc agagagtgct gaaatatcct 3840
   ctgcttttga aagagctggt gtccctgaca gacaacgaga gtgaggagca ctaccatttg 3900
   acagaagcgc tgaaggcaat ggaaaaagta gcaagtcaca tcaatgagat gcagaagata 3960
   tatgaagatt atggcactgt atttgatcaa ctggttgcag atcaaagtgg aacagagaag 4020
   gaggtgactg aactttccat gggagaactt ctgatgcact ctacagtttc ctggctaaat 4080
   cccttcccat cactgggcaa agcaagaaaa gaccttgaac ttacagtgtt tgcctttaag 4140
   aagagggctg taatactggt gtataaagag aactacaaac tgaaaaagaa aatgcctact 4200
   aatgttcgtg ctgcccataa ttatggtgac ttggatccat ttaagtttcg ctggctgatt 4260
   cctctatctg ctcttcaagt tcggcttggg aacacagcag gcacaggaac agagaacagc 4320
   tgtatctggg aactgattca cacaaagtca gaactggaag gcaggccaga aaccattttt 4380
   cagttatgca gcagcgactg cgagaacaag actaacatcg tgaaggtgat ccgttctatc 4440
   ttgcgggaga atttcagacg tcacataaaa tgtgagctgc ctctggacaa aacctgtaaa 4500
   gatcgcctca ttccactgaa gaaccgtgtg cctgcgacag ctaaactggc ttccaccagg 4560
   tccttgaagg tactgaagaa ttcacccagc agtgagtgga acggtgacca ggggaaaggc 4620
   accttccagg actctgatga ctgcagcctg agcagcagca cccagagcag cagctgcaac 4680
   accacagaga gcatacagga gcccaaaact tcatccccgg atcaacacgt acagagctgt 4740
   tcctccgact tttccaatgc tcttgttaaa gaatccgata ttcttagtga tgatgatgat 4800
   gacgactatc agagcctaaa gaagggcagc cctactaaag acattgaaat tcagttccag 4860
   cggctgaaga tttcagagga acccagtact gactctgaac gagatcaagc tgcagaaaag 4920
   gaggaaggag atggtttcaa gatgggagaa catccaaagc tgatacgtgg ccatttctgc 4980
   ccagtgaagc gaaaagtaaa cagtacgaag cgtaacaggg gaactttaac ggcaatgcag 5040
   gaacgtcacc agtctcttga cagccactct gatgctgcaa acttggacct gaactctatt 5100
   ttggagaggg aatttagcgt ccagagttta acatctgtag ttaacgagga ctgtttttat 5160
   gaagctgtgg agagacatgg aaagtcct 5188
<210> 49
   <211> 5163
   <212> DNA
   <213> Tiam2 Rattus norvegicus; Transcript ENSRNOG00000016728
<400> 49
   gttaagatgg ggaactccga gagtcaatat accttccaag gatccaagaa tcaaagcaat 60
   actgtcactg gtgctaagca gaagccgtgc tctctgaaga tacgcagcat tcatgcaaaa 120
   gatgagaagt ccttgcatgg ttgggctcac gggagcggcg gagcaggcta caagtccaga 180
   tccctagcca ggagctgcct ttctcacttt aagagtcacc agccttacgc caccagactc 240
   aatggaccca cgtgtaaagt ctcaaagggc actgcctact ctaagcacag agcaaatgcc 300
   ccaggaaatg attttcaggg caacaatggt gctttcttac ccgagaatgg cttccactat 360
   gttgggcgcg agtcagggga gagccacatc acctccaggg actgcaatgg acaccttctc 420
   acctgctatg ggagacacga cagcattgcc tccactcctc caggtgagga ccgcaggagc 480
   cccagggtgc tcatcaagac actggggaag ctagatgggt gtttaagagt tgagttccac 540
   aatggcggca acccccacaa agggccctca gaggacccca gtgaacctgt acagctgcta 600
   agatactccc ctacgttagc agcggaaacc tgcccggtgc cggaaagtag gcggctttcg 660
   ggtgcaggct cctcagccag ccagcgccct tctcccactg actcttgcct gcgctccagt 720
   aaaggcagct ccctgagctc ggagtcctcc tggtatgact ccccctgggg caacgcaggg 780
   gaggcgagtg aagtggacgg caccttcctg gctcccagtg ctctagaccc cagcctcccc 840
   agcagcttcc cacccagtga caccaaaaag cctttcaacc aaagctcttc cctctcctct 900
   ctccgggaac tgtacaaaga tgccaacctg gggtgccgct caccttccgg cacctgcctt 960
   tcttccaacg agtacattag ctcccaagtc ggcctgaaca accgagtctc ctttgcttct 1020
   gacatggatg tgccctccag ggtggatcac agggacacca tgcagtacag ctcctttact 1080
   ctcccgtgtc gcaagtccaa agccttaact gaggatgcag ctaagaaaga caccctcaaa 1140
   gccagaatgc ggcgcttcag tgactggaca ggaagccttt ccaggaagaa gaggaaattg 1200
   caggaaccca gatccacgga gggcagtgag tacttcgata gccactcgga tggactgaat 1260
   gcagatgtgc aggggcccac gcagacatct gccttactgt ggtcaggggg ctcagctcag 1320
   accctgcctc acagaagcga atccactcac gcaatcggca gcgatcccct ccaacagaac 1380
   atctacgaga atttcatgcg agagctggaa atgagcagga ccaacatgga gaacgcggaa 1440
   acgtccacag agactgtgga gtccagcagc gagtccctca gctcgctgga gcagctggat 1500
   ctgctctttg agaaggagca gggggtggtc cgaaaagctg ggtggctctt cttcaaaccc 1560
   cttgtcacct tgcagaagga gaggaaactg gagctggtgg ctcggaggaa gtggaaacag 1620
   tactgggtga ccctgaaagg ctgtactctg atgttttatg agacctacgg aaagaattcc 1680
   acagaccaga atagcgcccc acggtgtgcc ctctttgcag aggacagcat tgtgcagtct 1740
   gtcccagagc atcccaagaa ggagcacgtg ttctgcctga gtaactccta tggagatgtc 1800
   taccttttcc aggccactag ccagacggat ctggaaaact ggatcactgc catacactcg 1860
   gcgtgcgcat ccctctttgc aaagaagcat gggaaagaag acacggtgcg gctgctgaag 1920
   agccagacca gaggcctgct tcagaagata gacatggaca gcaagatgaa gaagatggca 1980
   gagctgcagc tgtctgtggt gagcgacccc aagaacagga aggccatcga aaaccagatc 2040
   cggcaatggg agcagaacct ggaaaaattc cacatggacc tgttccgcat gcgctgctat 2100
   ttggcaagct tacaaggtgg ggagttacca aaccccaaga gtctccttgc agccaccagc 2160
   cgcccctcca agctggctct tggcagactg ggcgtcctgt ctgtttcatc tttccatgct 2220
   ctggtatgtt ccagagacga ttctgctctc aggaaaagaa cactttcctt tacccagaga 2280
   gcaaaaagca agaaaggcct attttcttca ttgaaaggtc tggacactct agcaagaaaa 2340
   gggagggaga agagagcttc tataactcag atgtttgatt cgagccacag ccatggatat 2400
   cttggaactc aactacctca aaactccact aactccagtg aggtccatga cctgcatctg 2460
   tatggctcct cagcagacag cactctccga gacagcacgt gggaagtaca gacttatgtc 2520
   cacttccagg ataacgaagg agtcactgtg atcatcaagc cagagcacag ggtggaagat 2580
   gttctggctt tggcatgcaa gatgaggcac ttggaaccca ctcgctatgg tcttcagctc 2640
   cgaaaggtgg tggatgaaag tgtggagtgg tgtgtgcccg cgttgtatga gtacatgcag 2700
   gagcaggcaa gttgtgttta tgatgaaatt gaagtcttcc cactcagtgt gtatgacgtg 2760
   cagctaacca agacggggga catgactgac tttgggtttg cagtcacagc ccaggtggac 2820
   gagcaccagc atctcagccg gatatttatc agcgatgttc tccctgacag cctggcgtat 2880
   ggaggaggac tgagaaaggg caatgaaatc aggagcttaa atggggaagc agtgtctgac 2940
   cttgacctca agcagatgga ggctttgttt tctgagaaga gcgttggact cactctagtc 3000
   gcccggcctg cagacacaag agcaagcctg tgtacttcct gctcagacag tgacctgttc 3060
   tccagggacc agaaaagtct gctgccctct cccaaccagt gccaactgct ggaggaattc 3120
   ctggataact ttaaaaaaac ctccacaaac ggtaaggctg atgtacccag acctttgtct 3180
   ggctgcctta gacagatgga aacgcttggt gttccggata aggtgcacca tgacctgccc 3240
   tatcttgtca cctctcagag tgctgagcag attgtggagc tctgcagagg caccaccagt 3300
   atggaagcac cgacagacag ccgcgacccg cctcccaggc ctctggctcg tcacctctca 3360
   gatgcagacc gcctccgaaa agtcatccag gagcttgtag acaccgagaa gtcttacgtg 3420
   aaggatctga gctgcctctt tgaactgtac ttggagcccc ttcagaacga gacctttctt 3480
   acccaagatg agatggagtc actttttggg agcttgccag agatgctgga atttcaaaag 3540
   gtgttcctgg agactctgga ggatgggatc tctgcttcct cagactttag tgtcctggaa 3600
   accccctcac agtttcggaa attgctgttc tctctcggag gctctttcct ctactacgca 3660
   gatcacttta aactgtacag tggattctgc gccaaccaca ttaaagtaca gaaggtccta 3720
   gagcgagcta aaaccgacag agctttcaag gcttttctgg acgcgcggaa ccccacgaag 3780
   cagcactcct ccacgctgga gtcatatctc atcaagcctg ttcagagagt gctcaagtat 3840
   cctctgctcc tcaaggagct cgtgtcactg actgaccatg agagcgaaga acactatcac 3900
   ctgacagaag ctctaaaggc catggaaaaa gtagccagtc acatcaatga gatgcagaag 3960
   atctacgagg actatgggac ggtgtttgac cagctggtgg cagagcagag tggcacagag 4020
   aaggaggtaa cagagctgtc catgggggaa cttctgatgc actctacagt ttcctggttg 4080
   aatccgttcc tgtctctggg aaaagccagg aaggacattg agctcacagt gtttgttttt 4140
   aagagagctg tcatactggt ttataaagaa aactgcaagc tgaaaaagaa actgccctcc 4200
   aattcccggc ctgctcacaa ctctgctgac ttggacccat ttaaattccg ctggttgatc 4260
   cccatatctg cgcttcaagt tagactgggg aacacggcag ggactgaaaa caattccacc 4320
   tgggagctga ttcataccaa gtcagaaatt gaaggacggc cagagaccat ctttcaactg 4380
   tgctgcagtg acagcgagag caaaaccagc atcgttaagg tgatccgttc tattctgaga 4440
   gagaacttca ggcgccacat aaagtgtgag ttaccaatgg agaagacatg taaggaccgg 4500
   ctggtacctc ttaagaaccg agttcctgtt tcagccaaat tagcctcatc caggtctttg 4560
   aaggtcctca gaacatcctc cagcagcgag tggcccagcg accccagcaa gggcaactca 4620
   ctggactcgg atgagtgcag cctgagcagt ggcacccaga gcagtggctg cccagtagct 4680
   gagagcagac aagactgtaa gagcaccgtg ctggagaaag acgctcatga ggtcctggca 4740
   gagtttccag atggtctcat caaagaaagc gacattctaa gtgatgaaga ggaggacttc 4800
   caccaccctc tgaaacaggg cagccctact aaggacattg agcttcagtt ccagagactg 4860
   cgaatctctg aggaacccga tatgcactca actgggcagc agccgcctct cacagggccg 4920
   ggtgaacagc ccaagctggt caggggccac ttttgcccca ttaagcgtaa agcaaacagc 4980
   actaagaggg gccgaggaac tttgctcaag gcgcagacac gtcaccagtc cctggacagc 5040
   cacccggaaa ccgccagcat cgatctaaat ttggttctgg agagagaatt cagtgtccag 5100
   agcttaactt cagttgttaa tgaggagtgt ttttatgaaa cacagagcca tggcaaatcc 5160
   tag 5163
<210> 50
   <211> 3298
   <212> DNA
   <213> Tiam2 Canis familiaris; Transcript ENSCAFG00000000577
<400> 50
<210> 51
   <211> 1706
   <212> PRT
   <213> Tiam2 Bos taurus; deduced from Transcript ENSBTAT00000013256
<400> 51
   Leu Leu Leu Thr Leu Leu Phe Leu Ile Gln Val Thr Met Gly Asn Ser
   1               5                   10                  15
   Glu Ser Gln Tyr Thr Leu Gln Gly Pro Lys Asn His Ser Asn Thr Ile
               20                  25                  30
   Thr Gly Ala Lys Gln Lys Pro Cys Ser Leu Lys Ile Arg Gly Leu His
           35                  40                  45
   Ala Lys Asp Asp Lys Ser Leu His Gly Trp Gly His Gly Ser Ser Gly
       50                  55                  60
   Ala Gly Tyr Lys Ser Arg Ser Leu Ala Arg Ser Cys Leu Ser His Phe
   65                  70                  75                  80
   Lys Ser Asn Gln Pro Tyr Ala Ser Arg Pro Gly Gly Pro Thr Cys Lys
                   85                  90                  95
   Ala Ser Lys Gly Ser Ala Tyr Ala Lys His Arg Thr Gly Ala Ala Gly
               100                 105                 110
   Ser Asp Phe Gln Gly Thr Asp Ala Ala Phe Ser Pro Glu Asn Gly Phe
           115                 120                 125
   His Tyr Val Gly Arg Pro Pro Glu Glu Asn His Ser Ala Ser Arg Asp
       130                 135                 140
   Cys Arg Asn Gly His Leu Leu Asn Cys Tyr Gly Gly Asn Glu Ser Ile
   145                 150                 155                 160
   Ala Ser Thr Pro Pro Gly Glu Asp Arg Lys Ser Pro Arg Val Leu Ile
                   165                 170                 175
   Lys Thr Leu Gly Lys Leu Asp Gly Cys Leu Arg Val Glu Phe His Ser
   180 185 190
   Gly Ala Ala Pro Pro Gly Ala Ser Pro Ser Gly Gly Pro Val Gln Leu
   195 200 205
   Leu Arg Tyr Ser Ala Gly Ser Ala Pro Ser Pro Ser Ala Ser Pro Ala
   210 215 220
   Ala Ala Ala Arg Pro Arg Ser Ser Lys Gly Ser Ser Leu Ser Ser Glu
   225 230 235 240
   Ser Ser Trp Tyr Asp Ser Pro Trp Gly Pro Ala Gly Glu Val Ser Glu
   245 250 255
   Ala Glu Gly Ser Phe Val Ala Pro Asp Thr Pro Glu Pro Gly Leu His
   260 265 270
   Thr Gly Phe Pro Thr Arg Asp Ala Pro Lys Pro Phe Ser Gln Ser Ala
   275 280 285
   Ser Leu Ser Ser Leu Arg Asp Pro Tyr Pro Asn Ala Ser Leu Gly Ser
   290 295 300
   Leu Ala Pro Ala Ala Leu Arg Leu Ser Asp Asp Tyr Met Gly Thr Arg
   305 310 315 320
   Ala Ser Leu Ser Ala Arg Val Ser Phe Ala Ser Asp Met Asp Val Pro
   325 330 335
   Ala Arg Val Glu Arg Gly Glu Pro Gly Gln Phe Ala Ser Phe Thr Leu
   340 345 350
   Pro Cys Arg Lys Ser Arg Ala Leu Gly Glu Glu Ser Ser Lys Lys Asp
   355 360 365
   Thr Leu Lys Ala Arg Met Arg Arg Ile Ser Asp Trp Thr Gly Ser Leu
   370 375 380
   Ser Arg Lys Lys Arg Arg Leu Gln Glu Pro Arg Ser Lys Glu Gly Ser
   385 390 395 400
   Asp Tyr Phe Asp Ser Arg Ser Asp Gly Leu Asn Val Asp Thr Gln Gly
   405 410 415
   Pro Ser Gln Gly Ser Val Ser Leu Trp Ser Gly Gly Ser Ala Gln Ile
   420 425 430
   Leu Ser His Arg Ser Glu Ser Ala His Ala Ile Gly Ser Asp Pro Leu
   435 440 445
   Gln Gln Asn Ile Tyr Glu Asn Phe Met Arg Glu Leu Glu Met Ser Arg
   450 455 460
   Thr Asn Thr Glu Asn Leu Glu Thr Ser Thr Glu Thr Ala Glu Ser Ser
   465 470 475 480
   Ser Glu Ser Leu Ser Ser Leu Glu Gln Leu Asp Leu Leu Phe Glu Lys
   485 490 495
   Glu Gln Gly Val Val Arg Lys Ala Gly Trp Leu Phe Phe Lys Pro Leu
   500 505 510
   Val Thr Leu Gln Lys Glu Arg Lys Leu Glu Leu Val Ala Arg Arg Lys
   515 520 525
   Trp Lys Gln Tyr Trp Val Thr Leu Lys Gly Cys Thr Leu Leu Phe Tyr
   530 535 540
   Glu Thr Tyr Gly Lys Asn Ser Met Asp Gln Ser Ser Ala Pro Arg Cys
   545 550 555 560
   Ala Leu Phe Ala Glu Asp Ser Ile Val Gln Ser Val Pro Glu His Pro
   565 570 575
   Lys Lys Glu Asn Val Phe Cys Leu Ser Asn Ser Phe Gly Asp Val Tyr
   580 585 590
   Leu Phe Gln Ala Thr Ser Gln Thr Asp Leu Glu Asn Trp Val Thr Ala
   595 600 605
   Ile His Ser Ala Cys Ala Ser Leu Phe Ala Lys Lys His Gly Lys Glu
   610 615 620
   Asp Thr Val Arg Leu Leu Lys Asn Gln Thr Arg Asn Leu Leu Gln Lys
   625 630 635 640
   Ile Asp Met Asp Ser Lys Met Lys Lys Met Ala Glu Leu Gln Leu Ser
   645 650 655
   Val Val Ser Asp Pro Lys Asn Arg Lys Ala Ile Glu Asn Gln Asn Arg
   660 665 670
   Ala Leu Lys Asn Trp Gln Glu Ile Leu Leu Asp Thr Gln Leu Gly Val
   675 680 685
   Phe Arg Ser Leu Cys Val Val Lys Cys Leu Gly Gln Gly Glu Glu Leu
   690 695 700
   Pro Leu Thr Ala Leu Pro Thr Pro Pro Ser Gly Thr Tyr Tyr Ala Pro
   705 710 715 720
   Ile Ile Leu Arg Val Ala Leu Tyr Leu Thr His Cys Phe Val Cys Ser
   725 730 735
   Arg Asp Asp Ser Ala Leu Arg Lys Arg Thr Leu Ser Leu Thr Gln Arg
   740 745 750
   Gly Arg His Lys Lys Gly Leu Phe Ser Ser Leu Lys Gly Leu Asp Thr
   755 760 765
   Leu Ala Arg Lys Gly Lys Glu Lys Arg Pro Ser Ile Thr Gln Ile Phe
   770 775 780
   Asp Ser Ser Gly Ser His Gly Phe Ser Gly Thr Gln Leu Pro Gln Ser
   785 790 795 800
   Ser Asn Asn Ser Ser Glu Val Asp Asp Leu Leu His Leu Tyr Gly Ser
   805 810 815
   Ala Val Asp Gly Val Pro Arg Asp Gly Thr Trp Glu Ser Gln Val Asp
   820 825 830
   Val His Phe Pro Asp Arg Gln Val Val Thr Val Met Val Lys Pro Glu
   835 840 845
   Thr Arg Val Glu Asp Val Leu Thr Leu Ala Cys Lys Met Arg Gln Leu
   850 855 860
   Glu Pro Ser His Tyr Gly Leu Gln Leu Arg Lys Leu Val Asp Glu Asn
   865 870 875 880
   Val Glu Tyr Cys Ile Pro Ala Pro Tyr Glu Tyr Met Gln Asp Gln Val
   885 890 895
   Tyr Asp Glu Ile Glu Val Phe Pro Leu Ser Val Tyr Asp Val Gln Leu
   900 905 910
   Thr Lys Thr Gly Gly Val Ser Asp Phe Gly Phe Ala Val Thr Ala Gln
   915 920 925
   Val Asp Glu His Gln His Leu Ser Arg Ile Phe Ile Ser Asp Val Leu
   930 935 940
   Pro Asp Gly Leu Ala Tyr Gly Glu Gly Leu Arg Lys Gly Asn Glu Ile
   945 950 955 960
   Met Thr Leu Asn Gly Glu Ala Val Ser Asp Leu Asp Leu Lys Gln Met
   965 970 975
   Glu Ala Leu Phe Ser Glu Lys Ser Val Gly Leu Thr Leu Ile Ala Arg
   980 985 990
   Pro Pro Asp Thr Lys Ala Thr Leu Cys Ser Ser Trp Ser Asp Ser Asp
   995 1000 1005
   Leu Phe Ser Arg Asp Gln Lys Gly Leu Leu Pro Pro Pro Asn Gln
   1010 1015 1020
   Ser Gln Leu Leu Glu Glu Phe Leu Asp His Phe Lys Lys Asp Thr
   1025 1030 1035
   Ala Asn Asp Phe Ser Asn Val Pro Asp Val Thr Thr Gly Leu Lys
   1040 1045 1050
   Arg Ser Gln Thr Asp Gly Thr Leu Asp Gln Val Ser His Arg Glu
   1055 1060 1065
   Lys Lys Glu Gln Thr Phe Arg Ser Ala Glu Gln Ile Thr Ala Leu
   1070 1075 1080
   Cys Arg Asn Phe Asn Asp Thr Gln Ala Asn Gly Met Glu Gly Pro
   1085 1090 1095
   Arg Glu Gly Gln Asp Pro Ala Pro Arg Pro Leu Ala Arg His Leu
   1100 1105 1110
   Ser Asp Ala Asp Arg Leu Arg Lys Val Ile Gln Glu Leu Met Asp
   1115 1120 1125
   Thr Glu Lys Ser Tyr Val Lys Asp Leu Ser Cys Leu Phe Asp Leu
   1130 1135 1140
   Tyr Leu Glu Pro Leu Gln Ser Glu Thr Ile Leu Val Phe Thr Gln
   1145 1150 1155
   Met Glu Ser Leu Phe Gly Ser Leu Pro Glu Met Leu Glu Phe Gln
   1160 1165 1170
   Lys Val Phe Leu Glu Thr Leu Glu Asp Gly Ile Ser Ala Ser Ser
   1175 1180 1185
   Asp Phe Asn Ile Leu Glu Thr Pro Ser Gln Phe Arg Lys Leu Leu
       1190                1195                1200
   Phe Ser Leu Gly Gly Ser Phe Leu Tyr Tyr Ala Asp His Phe Lys
       1205                1210                1215
   Leu Tyr Ser Gly Phe Cys Ala Asn His Ile Lys Val Gln Lys Val
       1220                1225                1230
   Leu Glu Arg Ala Lys Thr Asp Lys Ala Phe Lys Ala Phe Leu Asp
       1235                1240                1245
   Ala Arg Asn Pro Thr Lys Gln His Ser Ser Thr Leu Glu Ser Tyr
       1250                1255                1260
   Leu Ile Lys Pro Val Gln Arg Val Leu Lys Tyr Pro Leu Leu Leu
       1265                1270                1275
   Lys Glu Leu Val Ser Leu Thr Asp His Glu Ser Glu Glu His Tyr
       1280                1285                1290
   His Leu Thr Glu Ala Leu Lys Ala Met Glu Lys Val Ala Ser His
       1295                1300                1305
   Ile Asn Glu Met Gln Lys Ile Tyr Glu Asp Tyr Gly Thr Val Phe
       1310                1315                1320
   Asp Gln Leu Val Ala Glu Gln Ser Gly Thr Glu Lys Glu Val Thr
       1325                1330                1335
   Glu Leu Ser Met Gly Glu Leu Leu Met His Ser Thr Val Ser Trp
       1340                1345                1350
   Leu Asn Pro Phe Leu Ser Leu Gly Lys Ala Arg Lys Asp Leu Glu
       1355                1360                1365
   Leu Thr Val Phe Val Phe Lys Arg Ala Val Ile Leu Val Tyr Lys
       1370                1375                1380
   Glu Asn Cys Lys Leu Lys Lys Lys Leu Pro Ser Asn Ser Arg Pro
       1385                1390                1395
   Ala His Ser Asn Ala Asp Leu Asp Pro Phe Lys Phe Arg Trp Leu
       1400                1405                1410
   Ile Pro Ile Ser Ala Leu Gln Val Arg Leu Gly Asn Thr Ala Gly
       1415                1420                1425
   Thr Glu Asn Asn Ser Leu Trp Glu Leu Ile His Thr Lys Ser Glu
       1430                1435                1440
   Ile Glu Gly Arg Pro Glu Thr Ile Phe Gln Leu Cys Cys Ser Asp
       1445                1450                1455
   Asn Glu Ser Lys Thr Asn Ile Val Lys Val Ile Arg Ser Ile Leu
       1460                1465                1470
   Arg Glu Asn Phe Arg Arg His Ile Lys Cys Glu Leu Pro Leu Glu
       1475                1480                1485
   Lys Thr Cys Lys Asp Arg Leu Val Pro Leu Lys Asn Arg Val Pro
       1490                1495                1500
   Val Ser Ala Lys Leu Ala Ser Ser Arg Ser Leu Lys Val Leu Lys
       1505                1510                1515
   Asn Ser Ser Ser Ser Glu Trp Pro Gly Glu Pro Gly Lys Gly Ser
       1520                1525                1530
   Ser Leu Asp Ser Asp Glu Gly Ser Leu Ser Ser Ser Thr Gln Ser
       1535                1540                1545
   Ser Gly Cys Pro Pro Ala Gly Arg Gly Gln Asp Thr Gln Gln His
       1550                1555                1560
   Pro His Ala Gly Leu Ala Asp Phe Ser Asp Ser Leu Ile Lys Glu
       1565                1570                1575
   Ser Asp Ile Leu Ser Asp Asp Glu Asp Asp Tyr Pro Gln Ala Arg
       1580                1585                1590
   Arg Gln Gly Ser Pro Thr Lys Asp Ile Glu Ile Gln Phe Gln Arg
       1595                1600                1605
   Leu Arg Ile Ser Glu Asp Pro Asp Ala His Pro Ala Glu Glu Pro
       1610                1615                1620
   Pro Ser Lys Asp Gly Gln Pro Lys Leu Val Arg Gly His Phe Cys
       1625                1630                1635
   Ala Ile Lys Arg Lys Ala Asn Ser Thr Lys Arg Asp Arg Gly Thr
       1640                1645                1650
   Leu Leu Lys Ala Gln Thr Arg His Gln Ser Leu Asp Ser His Pro
       1655                1660                1665
   Gly Asn Ala Asn Leu Asp Leu Ser Ser Val Leu Glu Arg Glu Phe
           1670                    1675                    1680
   Ser Val Gln Ser Leu Thr Ser Val Val Asn Glu Glu Cys Phe Tyr
       1685                    1690                    1695
   Glu Thr Glu Ser His Gly Lys Ser
       1700                    1705
<210> 52
   <211> 1729
   <212> PRT
   <213> Tiam2 Gallus gallus; deduced from ENSGALG00000013645
<400> 52
   Met Gly Asn Ser Glu Ser Gln Tyr Ser Leu Gln Gly Ser Lys Asn His
   1 5 10 15
   Ala Ala Ala Ser Thr Gly Ser Lys Gln Lys Pro Cys Ser Leu Lys Ile
   20 25 30
   Arg Ser Ile His Ala Lys Asp Glu Lys Ser Cys Ser Leu His Gly Trp
   35 40 45
   Gly His Thr Asn Ser Gly Ser Asn Tyr Lys Ser Arg Ser Leu Ala Arg
   50 55 60
   Ser Cys Leu Ser His Phe Lys Ser Ser Gln Pro Tyr Ser Ala Arg Leu
   65 70 75 80
   Ser Asp Thr Val Val Lys Ala Ser Lys Ser Asn Val Leu Ala Lys His
   85 90 95
   Arg Thr His Thr Ser Gly Asp Tyr Cys Pro Gly Asn Asn Ala Val Phe
   100 105 110
   Leu Pro Asp Asn Gly Phe His Tyr Ile Gly Leu Gln Ala Gly Ser Asn
   115 120 125
   His Ala Ala Pro Arg Asp Cys Asn Gly His Ile Leu Lys Cys Tyr Gly
   130 135 140
   Lys Asn Glu Ser Leu Ala Ser Thr Ser Pro Ser Glu Asp Arg Arg Ser
   145 150 155 160
   Pro Lys Val Leu Ile Lys Thr Leu Gly Lys Leu Asp Gly Cys Leu Arg
   165 170 175
   Val Glu Phe His Asn Ser Ser Asn Ser Lys Val Pro Thr Glu Glu Ser
   180 185 190
   Ser Gly Pro Val Gln Leu Leu Arg Tyr Ser Pro Thr Leu Glu Ser Lys
           195                 200                 205
   Ser Asn Asn Leu Leu Asp Val Arg Arg Asn Ser Ser Ala Asp Cys Ser
       210                 215                 220
   Ser Asn His Arg Leu Ser Pro Thr Asp Ser Arg Leu Arg Ser Ser Lys
   225                 230                 235                 240
   Gly Ser Ser Leu Ser Ser Glu Ser Ser Trp Tyr Asp Ser Leu Trp Gly
                   245                 250                 255
   Asn Ala Gly Asp Ile Ser Glu Leu Asp Gly Pro Tyr Leu Thr Arg Ser
               260                 265                 270
   Thr Pro Asp Thr Ser Ile His Ala Ser Phe Pro Ala Ser Asp Lys Lys
           275                 280                 285
   Ser Phe Asn Gln Ser Ser Ser Leu Ser Ser Leu Arg Asp Leu Tyr Lys
       290                 295                 300
   Asp Thr Asn Leu Glu Gly Thr Pro Pro Pro Gly Ile Arg Leu Ser Asp
   305                 310                 315                 320
   Glu Tyr Ile Asp Thr His Gly Ser Leu Ser Asn Arg Val Ser Phe Ala
                   325                 330                 335
   Ser Asp Ile Asp Val Pro Ser Arg Val Glu Gln Gly Ser Pro Ala His
               340                 345                 350
   Tyr Ser Ser Tyr Thr Leu Pro Cys Arg Lys Ser Lys Pro Leu Ala Glu
           355                 360                 365
   Asp Ala Ser Lys Lys Asp Thr Leu Lys Ser Arg Met Arg Arg Ile Ser
       370                 375                 380
   Asp Trp Thr Gly Ser Leu Ser Arg Lys Lys Arg Lys Leu Gln Glu Pro
   385                 390                 395                 400
   Lys Cys Lys Asp Gly Ser Glu Tyr Phe Asp Ser Arg Met Asp Asn Phe
                   405                 410                 415
   Ser Thr Asp Thr Leu Ala Pro Ser Gln Gln Ser Thr Leu Leu Trp Ser
               420                 425                 430
   Pro Ser Ser Ser His Ile Leu Ser Gln Arg Ser Glu Ser Thr Asn Ala
           435                 440                 445
   Val Ser Ser Asp Ala Leu Arg Gln Asn Ile Tyr Glu Asn Phe Met Arg
   450 455 460
   Glu Leu Glu Met Ser Arg Thr Asn Leu Glu Asn Thr Glu Thr Ser Ser
   465 470 475 480
   Glu Thr Glu Asp Ser Ser Ser Glu Ser Leu Ser Ser Leu Glu Gln Leu
   485 490 495
   Asp Leu Leu Tyr Glu Lys Glu Gln Gly Val Val Arg Lys Ala Gly Trp
   500 505 510
   Leu Phe Phe Lys Pro Leu Val Thr Leu Gln Lys Glu Lys Lys Leu Glu
   515 520 525
   Leu Val Thr Arg Arg Lys Trp Lys Gln Tyr Trp Val Thr Leu Lys Gly
   530 535 540
   Cys Thr Leu Leu Phe Tyr Glu Thr Tyr Gly Arg Asn Ser Met Glu Gln
   545 550 555 560
   Ser Ser Leu Pro Arg Tyr Ala Leu Phe Ala Glu Asp Ser Ile Val Gln
   565 570 575
   Ser Val Pro Glu His Pro Lys Lys Glu Asn Val Phe Cys Leu Ser Asn
   580 585 590
   Ser Phe Gly Asp Val Tyr Leu Phe Gln Ala Thr Ser Gln Thr Asp Leu
   595 600 605
   Glu Asn Trp Val Thr Ala Ile His Ser Ala Cys Ala Ser Leu Phe Ala
   610 615 620
   Lys Lys Leu Gly Lys Glu Asp Thr Val Arg Leu Leu Lys Asn Gln Thr
   625 630 635 640
   Lys Ser Leu Phe Gln Lys Ile Asp Met Asp Ser Lys Met Lys Lys Met
   645 650 655
   Ala Glu Leu Gln Leu Ser Ile Val Ser Asp Pro Lys Asn Arg Lys Ala
   660 665 670
   Ile Glu Asn Gln Ile Gln Gln Trp Glu Gln Asn Leu Glu Lys Phe Asn
   675 680 685
   Met Asp Leu Phe Arg Met Arg Cys Tyr Leu Ala Ser Leu Gln Gly Gly
   690 695 700
   Glu Leu Pro Asn Pro Lys Ser Leu Leu Ala Ala Ala Ser Arg Pro Ser
   705 710 715 720
   Lys Leu Ala Leu Gly Arg Leu Gly Ile Phe Ser Val Ser Ser Phe His
   725 730 735
   Ala Leu Ile Cys Ser Arg Asp Glu Ala Ala Leu Arg Lys Arg Thr Leu
   740 745 750
   Ser Leu Ser Gln Arg Val Arg Asn Lys Lys Gly Leu Phe Ser Ser Leu
   755 760 765
   Lys Gly Leu Asp Thr Leu Ala Arg Lys Gly Lys Glu Lys Arg Pro Ser
   770 775 780
   Ile Thr Gln Ile Phe Asp Ser Thr Gly Gly His Gly Phe Ala Gly Val
   785 790 795 800
   Gln Asn Ser Ala Asn Ser Ala Glu Gln Val Asp Glu Phe Leu Asn Val
   805 810 815
   Tyr Cys Ser Val Pro Asp Ser Ile Gln Lys Glu Asn Ala Trp Glu Thr
   820 825 830
   Gln Thr Tyr Val His Phe Cys Asp Gly Gln Gly Val Ala Leu Thr Leu
   835 840 845
   Lys Pro Asp His Arg Val Glu Asp Val Leu Ser Leu Ala Cys Lys Met
   850 855 860
   Lys Gln Leu Glu Pro Arg Asn Tyr Gly Leu Gln Leu Arg Arg Leu Val
   865 870 875 880
   Asp Glu Asn Thr Glu Tyr Cys Ala Pro Glu Pro Tyr Glu Tyr Ile Val
   885 890 895
   Asp Gln Val Tyr Asp Glu Ile Glu Ile Cys Pro Leu Asn Val Tyr His
   900 905 910
   Ile His Leu Thr Lys Thr Glu Asn Ile Thr Asp Phe Gly Phe Ala Val
   915 920 925
   Thr Ala Gln Val Asp Glu Asn Gln His Leu Thr His Ile Phe Val Ser
   930 935 940
   Asp Val Leu Pro Asp Gly Leu Ala Tyr Arg Glu Gly Leu Arg Val Gly
   945 950 955 960
   Asn Glu Ile Leu Ser Ile Asn Gly Glu Ser Val Ser Asp Leu Asp Leu
   965 970 975
   Arg Gln Met Glu Leu Leu Phe Ser Glu Arg Ser Val Met Leu Thr Leu
   980 985 990
   Arg Met Ser His Cys Gly Asn Gln Gln Pro Leu Cys Ala Ser Trp Ser
   995 1000 1005
   Ser Asp Gly Asp Ile Ser Arg Val Pro Lys Ser Leu Leu Pro Pro
   1010 1015 1020
   Pro Asn Gln Ser Gln Leu Leu Glu Glu Phe Leu Asp Asn Phe Arg
   1025 1030 1035
   Lys Asn Thr Glu Asn Asp Ala Asp Phe Asn Asn Val Pro Asp Val
   1040 1045 1050
   Thr Ser Ser Leu Lys Arg Ser Ser Thr Asp Gly Thr Leu Asp Gln
   1055 1060 1065
   Val Pro His Arg Glu Lys Thr Asp Pro Pro Phe Arg Ser Ala Glu
   1070 1075 1080
   Gln Ile Ser Ala Leu Cys Arg Asn Phe Gln Glu Val Gln Thr Ser
   1085 1090 1095
   Ser Met Glu Gly Gln Lys Asp Asn Gln Asp Pro Pro Pro Arg Pro
   1100 1105 1110
   Leu Ala Arg His Leu Ser Asp Ala Asp Arg Leu Arg Lys Val Ile
   1115 1120 1125
   Gln Glu Leu Met Asp Thr Glu Lys Ser Tyr Val Lys Asp Leu Ser
   1130 1135 1140
   Cys Leu Phe Glu Leu Tyr Leu Glu Pro Leu Gln Asn Glu Thr Phe
   1145 1150 1155
   Leu Thr Gln Asp Glu Met Glu Ser Leu Phe Gly Ser Leu Pro Glu
   1160 1165 1170
   Met Leu Asp Phe Gln Lys Val Phe Leu Glu Thr Leu Glu Asp Gly
   1175 1180 1185
   Ile Ser Ser Ser Ser Asp Phe Asn Thr Leu Glu Thr Pro Ser Gln
   1190 1195 1200
   Phe Arg Lys Leu Leu Phe Ser Leu Gly Gly Ser Phe Leu Tyr Tyr
   1205 1210 1215
   Ala Asp His Phe Lys Leu Tyr Ser Gly Phe Cys Ala Asn His Ile
   1220 1225 1230
   Lys Val Gln Lys Val Leu Glu Arg Ala Lys Thr Asp Ser Ala Phe
   1235 1240 1245
   Lys Ala Phe Leu Asp Ala Arg Asn Pro Thr Lys Gln His Ser Ser
   1250 1255 1260
   Thr Leu Glu Ser Tyr Leu Ile Lys Pro Val Gln Arg Val Leu Lys
   1265 1270 1275
   Tyr Pro Leu Leu Leu Lys Glu Leu Val Ser Leu Thr Asp Asn Glu
   1280 1285 1290
   Ser Glu Glu His Tyr His Leu Thr Glu Ala Leu Lys Ala Met Glu
   1295 1300 1305
   Lys Val Ala Ser His Ile Asn Glu Met Gln Lys Ile Tyr Glu Asp
   1310 1315 1320
   Tyr Gly Thr Val Phe Asp Gln Leu Val Ala Asp Gln Ser Gly Thr
   1325 1330 1335
   Glu Lys Glu Val Thr Glu Leu Ser Met Gly Glu Leu Leu Met His
   1340 1345 1350
   Ser Thr Val Ser Trp Leu Asn Pro Phe Pro Ser Leu Gly Lys Ala
   1355 1360 1365
   Arg Lys Asp Leu Glu Leu Thr Val Phe Ala Phe Lys Lys Arg Ala
   1370 1375 1380
   Val Ile Leu Val Tyr Lys Glu Asn Tyr Lys Leu Lys Lys Lys Met
   1385 1390 1395
   Pro Thr Asn Val Arg Ala Ala His Asn Tyr Gly Asp Leu Asp Pro
   1400 1405 1410
   Phe Lys Phe Arg Trp Leu Ile Pro Leu Ser Ala Leu Gln Val Arg
   1415 1420 1425
   Leu Gly Asn Thr Ala Gly Thr Gly Thr Glu Asn Ser Cys Ile Trp
   1430 1435 1440
   Glu Leu Ile His Thr Lys Ser Glu Leu Glu Gly Arg Pro Glu Thr
   1445 1450 1455
   Ile Phe Gln Leu Cys Ser Ser Asp Cys Glu Asn Lys Thr Asn Ile
   1460 1465 1470
   Val Lys Val Ile Arg Ser Ile Leu Arg Glu Asn Phe Arg Arg His
   1475 1480 1485
   Ile Lys Cys Glu Leu Pro Leu Asp Lys Thr Cys Lys Asp Arg Leu
   1490 1495 1500
   Ile Pro Leu Lys Asn Arg Val Pro Ala Thr Ala Lys Leu Ala Ser
   1505 1510 1515
   Thr Arg Ser Leu Lys Val Leu Lys Asn Ser Pro Ser Ser Glu Trp
   1520 1525 1530
   Asn Gly Asp Gln Gly Lys Gly Thr Phe Gln Asp Ser Asp Asp Cys
   1535 1540 1545
   Ser Leu Ser Ser Ser Thr Gln Ser Ser Ser Cys Asn Thr Thr Glu
   1550 1555 1560
   Ser Ile Gln Glu Pro Lys Thr Ser Ser Pro Asp Gln His Val Gln
   1565 1570 1575
   Ser Cys Ser Ser Asp Phe Ser Asn Ala Leu Val Lys Glu Ser Asp
   1580 1585 1590
   Ile Leu Ser Asp Asp Asp Asp Asp Asp Tyr Gln Ser Leu Lys Lys
   1595 1600 1605
   Gly Ser Pro Thr Lys Asp Ile Glu Ile Gln Phe Gln Arg Leu Lys
   1610 1615 1620
   Ile Ser Glu Glu Pro Ser Thr Asp Ser Glu Arg Asp Gln Ala Ala
   1625 1630 1635
   Glu Lys Glu Glu Gly Asp Gly Phe Lys Met Gly Glu His Pro Lys
   1640 1645 1650
   Leu Ile Arg Gly His Phe Cys Pro Val Lys Arg Lys Val Asn Ser
   1655 1660 1665
   Thr Lys Arg Asn Arg Gly Thr Leu Thr Ala Met Gln Glu Arg His
   1670 1675 1680
   Gln Ser Leu Asp Ser His Ser Asp Ala Ala Asn Leu Asp Leu Asn
   1685 1690 1695
   Ser Ile Leu Glu Arg Glu Phe Ser Val Gln Ser Leu Thr Ser Val
   1700 1705 1710
   Val Asn Glu Asp Cys Phe Tyr Glu Ala Val Glu Arg His Gly Lys
   1715 1720 1725
   Ser
<210> 53
   <211> 1720
   <212> PRT
   <213> Rattus norvegicus; deduced from ENSRNOG00000016728
<400> 53
   Val Lys Met Gly Asn Ser Glu Ser Gln Tyr Thr Phe Gln Gly Ser Lys
   1 5 10 15
   Asn Gln Ser Asn Thr Val Thr Gly Ala Lys Gln Lys Pro Cys Ser Leu
   20 25 30
   Lys Ile Arg Ser Ile His Ala Lys Asp Glu Lys Ser Leu His Gly Trp
   35 40 45
   Ala His Gly Ser Gly Gly Ala Gly Tyr Lys Ser Arg Ser Leu Ala Arg
   50 55 60
   Ser Cys Leu Ser His Phe Lys Ser His Gln Pro Tyr Ala Thr Arg Leu
   65 70 75 80
   Asn Gly Pro Thr Cys Lys Val Ser Lys Gly Thr Ala Tyr Ser Lys His
   85 90 95
   Arg Ala Asn Ala Pro Gly Asn Asp Phe Gln Gly Asn Asn Gly Ala Phe
   100 105 110
   Leu Pro Glu Asn Gly Phe His Tyr Val Gly Arg Glu Ser Gly Glu Ser
   115 120 125
   His Ile Thr Ser Arg Asp Cys Asn Gly His Leu Leu Thr Cys Tyr Gly
   130 135 140
   Arg His Asp Ser Ile Ala Ser Thr Pro Pro Gly Glu Asp Arg Arg Ser
   145 150 155 160
   Pro Arg Val Leu Ile Lys Thr Leu Gly Lys Leu Asp Gly Cys Leu Arg
   165 170 175
   Val Glu Phe His Asn Gly Gly Asn Pro His Lys Gly Pro Ser Glu Asp
   180 185 190
   Pro Ser Glu Pro Val Gln Leu Leu Arg Tyr Ser Pro Thr Leu Ala Ala
   195 200 205
   Glu Thr Cys Pro Val Pro Glu Ser Arg Arg Leu Ser Gly Ala Gly Ser
   210 215 220
   Ser Ala Ser Gln Arg Pro Ser Pro Thr Asp Ser Cys Leu Arg Ser Ser
   225 230 235 240
   Lys Gly Ser Ser Leu Ser Ser Glu Ser Ser Trp Tyr Asp Ser Pro Trp
   245 250 255
   Gly Asn Ala Gly Glu Ala Ser Glu Val Asp Gly Thr Phe Leu Ala Pro
   260 265 270
   Ser Ala Leu Asp Pro Ser Leu Pro Ser Ser Phe Pro Pro Ser Asp Thr
   275 280 285
   Lys Lys Pro Phe Asn Gln Ser Ser Ser Leu Ser Ser Leu Arg Glu Leu
   290 295 300
   Tyr Lys Asp Ala Asn Leu Gly Cys Arg Ser Pro Ser Gly Thr Cys Leu
   305 310 315 320
   Ser Ser Asn Glu Tyr Ile Ser Ser Gln Val Gly Leu Asn Asn Arg Val
   325 330 335
   Ser Phe Ala Ser Asp Met Asp Val Pro Ser Arg Val Asp His Arg Asp
   340 345 350
   Thr Met Gln Tyr Ser Ser Phe Thr Leu Pro Cys Arg Lys Ser Lys Ala
   355 360 365
   Leu Thr Glu Asp Ala Ala Lys Lys Asp Thr Leu Lys Ala Arg Met Arg
   370 375 380
   Arg Phe Ser Asp Trp Thr Gly Ser Leu Ser Arg Lys Lys Arg Lys Leu
   385 390 395 400
   Gln Glu Pro Arg Ser Thr Glu Gly Ser Glu Tyr Phe Asp Ser His Ser
   405 410 415
   Asp Gly Leu Asn Ala Asp Val Gln Gly Pro Thr Gln Thr Ser Ala Leu
   420 425 430
   Leu Trp Ser Gly Gly Ser Ala Gln Thr Leu Pro His Arg Ser Glu Ser
   435 440 445
   Thr His Ala Ile Gly Ser Asp Pro Leu Gln Gln Asn Ile Tyr Glu Asn
   450 455 460
   Phe Met Arg Glu Leu Glu Met Ser Arg Thr Asn Met Glu Asn Ala Glu
   465 470 475 480
   Thr Ser Thr Glu Thr Val Glu Ser Ser Ser Glu Ser Leu Ser Ser Leu
   485 490 495
   Glu Gln Leu Asp Leu Leu Phe Glu Lys Glu Gln Gly Val Val Arg Lys
   500 505 510
   Ala Gly Trp Leu Phe Phe Lys Pro Leu Val Thr Leu Gln Lys Glu Arg
   515 520 525
   Lys Leu Glu Leu Val Ala Arg Arg Lys Trp Lys Gln Tyr Trp Val Thr
   530 535 540
   Leu Lys Gly Cys Thr Leu Met Phe Tyr Glu Thr Tyr Gly Lys Asn Ser
   545 550 555 560
   Thr Asp Gln Asn Ser Ala Pro Arg Cys Ala Leu Phe Ala Glu Asp Ser
   565 570 575
   Ile Val Gln Ser Val Pro Glu His Pro Lys Lys Glu His Val Phe Cys
   580 585 590
   Leu Ser Asn Ser Tyr Gly Asp Val Tyr Leu Phe Gln Ala Thr Ser Gln
   595 600 605
   Thr Asp Leu Glu Asn Trp Ile Thr Ala Ile His Ser Ala Cys Ala Ser
   610 615 620
   Leu Phe Ala Lys Lys His Gly Lys Glu Asp Thr Val Arg Leu Leu Lys
   625 630 635 640
   Ser Gln Thr Arg Gly Leu Leu Gln Lys Ile Asp Met Asp Ser Lys Met
   645 650 655
   Lys Lys Met Ala Glu Leu Gln Leu Ser Val Val Ser Asp Pro Lys Asn
   660 665 670
   Arg Lys Ala Ile Glu Asn Gln Ile Arg Gln Trp Glu Gln Asn Leu Glu
           675                 680                 685
   Lys Phe His Met Asp Leu Phe Arg Met Arg Cys Tyr Leu Ala Ser Leu
       690                 695                 700
   Gln Gly Gly Glu Leu Pro Asn Pro Lys Ser Leu Leu Ala Ala Thr Ser
   705                 710                 715                 720
   Arg Pro Ser Lys Leu Ala Leu Gly Arg Leu Gly Val Leu Ser Val Ser
                   725                 730                 735
   Ser Phe His Ala Leu Val Cys Ser Arg Asp Asp Ser Ala Leu Arg Lys
               740                 745                 750
   Arg Thr Leu Ser Phe Thr Gln Arg Ala Lys Ser Lys Lys Gly Leu Phe
           755                 760                 765
   Ser Ser Leu Lys Gly Leu Asp Thr Leu Ala Arg Lys Gly Arg Glu Lys
       770                 775                 780
   Arg Ala Ser Ile Thr Gln Met Phe Asp Ser Ser His Ser His Gly Tyr
   785                 790                 795                 800
   Leu Gly Thr Gln Leu Pro Gln Asn Ser Thr Asn Ser Ser Glu Val His
                   805                 810                 815
   Asp Leu His Leu Tyr Gly Ser Ser Ala Asp Ser Thr Leu Arg Asp Ser
               820                 825                 830
   Thr Trp Glu Val Gln Thr Tyr Val His Phe Gln Asp Asn Glu Gly Val
           835                 840                 845
   Thr Val Ile Ile Lys Pro Glu His Arg Val Glu Asp Val Leu Ala Leu
       850                 855                 860
   Ala Cys Lys Met Arg His Leu Glu Pro Thr Arg Tyr Gly Leu Gln Leu
   865                 870                 875                 880
   Arg Lys Val Val Asp Glu Ser Val Glu Trp Cys Val Pro Ala Leu Tyr
                   885                 890                 895
   Glu Tyr Met Gln Glu Gln Ala Ser Cys Val Tyr Asp Glu Ile Glu Val
               900                 905                 910
   Phe Pro Leu Ser Val Tyr Asp Val Gln Leu Thr Lys Thr Gly Asp Met
           915                 920                 925
   Thr Asp Phe Gly Phe Ala Val Thr Ala Gln Val Asp Glu His Gln His
       930                 935                 940
   Leu Ser Arg Ile Phe Ile Ser Asp Val Leu Pro Asp Ser Leu Ala Tyr
   945                 950                 955                 960
   Gly Gly Gly Leu Arg Lys Gly Asn Glu Ile Arg Ser Leu Asn Gly Glu
                   965                 970                 975
   Ala Val Ser Asp Leu Asp Leu Lys Gln Met Glu Ala Leu Phe Ser Glu
               980                 985                 990
   Lys Ser Val Gly Leu Thr Leu Val  Ala Arg Pro Ala Asp  Thr Arg Ala
           995                 1000                1005
   Ser Leu  Cys Thr Ser Cys Ser  Asp Ser Asp Leu Phe  Ser Arg Asp
       1010                1015                1020
   Gln Lys  Ser Leu Leu Pro Ser  Pro Asn Gln Cys Gln  Leu Leu Glu
       1025                1030                1035
   Glu Phe  Leu Asp Asn Phe Lys  Lys Thr Ser Thr Asn  Gly Lys Ala
       1040                1045                1050
   Asp Val  Pro Arg Pro Leu Ser  Gly Cys Leu Arg Gln  Met Glu Thr
       1055                1060                1065
   Leu Gly  Val Pro Asp Lys Val  His His Asp Leu Pro  Tyr Leu Val
       1070                1075                1080
   Thr Ser  Gln Ser Ala Glu Gln  Ile Val Glu Leu Cys  Arg Gly Thr
       1085                1090                1095
   Thr Ser  Met Glu Ala Pro Thr  Asp Ser Arg Asp Pro  Pro Pro Arg
       1100                1105                1110
   Pro Leu  Ala Arg His Leu Ser  Asp Ala Asp Arg Leu  Arg Lys Val
       1115                1120                1125
   Ile Gln  Glu Leu Val Asp Thr  Glu Lys Ser Tyr Val  Lys Asp Leu
       1130                1135                1140
   Ser Cys  Leu Phe Glu Leu Tyr  Leu Glu Pro Leu Gln  Asn Glu Thr
       1145                1150                1155
   Phe Leu  Thr Gln Asp Glu Met  Glu Ser Leu Phe Gly  Ser Leu Pro
       1160                1165                1170
   Glu Met  Leu Glu Phe Gln Lys  Val Phe Leu Glu Thr  Leu Glu Asp
       1175                 1180                 1185
   Gly Ile  Ser Ala Ser Ser Asp  Phe Ser Val Leu Glu  Thr Pro Ser
       1190                 1195                 1200
   Gln Phe  Arg Lys Leu Leu Phe  Ser Leu Gly Gly Ser  Phe Leu Tyr
       1205                 1210                 1215
   Tyr Ala  Asp His Phe Lys Leu  Tyr Ser Gly Phe Cys  Ala Asn His
       1220                 1225                 1230
   Ile Lys  Val Gln Lys Val Leu  Glu Arg Ala Lys Thr  Asp Arg Ala
       1235                 1240                 1245
   Phe Lys  Ala Phe Leu Asp Ala  Arg Asn Pro Thr Lys  Gln His Ser
       1250                 1255                 1260
   Ser Thr  Leu Glu Ser Tyr Leu  Ile Lys Pro Val Gln  Arg Val Leu
       1265                 1270                 1275
   Lys Tyr  Pro Leu Leu Leu Lys  Glu Leu Val Ser Leu  Thr Asp His
       1280                 1285                 1290
   Glu Ser  Glu Glu His Tyr His  Leu Thr Glu Ala Leu  Lys Ala Met
       1295                 1300                 1305
   Glu Lys  Val Ala Ser His Ile  Asn Glu Met Gln Lys  Ile Tyr Glu
       1310                 1315                 1320
   Asp Tyr  Gly Thr Val Phe Asp  Gln Leu Val Ala Glu  Gln Ser Gly
       1325                 1330                 1335
   Thr Glu  Lys Glu Val Thr Glu  Leu Ser Met Gly Glu  Leu Leu Met
       1340                 1345                 1350
   His Ser  Thr Val Ser Trp Leu  Asn Pro Phe Leu Ser  Leu Gly Lys
       1355                 1360                 1365
   Ala Arg  Lys Asp Ile Glu Leu  Thr Val Phe Val Phe  Lys Arg Ala
       1370                 1375                 1380
   Val Ile  Leu Val Tyr Lys Glu  Asn Cys Lys Leu Lys  Lys Lys Leu
       1385                 1390                 1395
   Pro Ser  Asn Ser Arg Pro Ala  His Asn Ser Ala Asp  Leu Asp Pro
       1400                 1405                 1410
   Phe Lys Phe Arg Trp Leu Ile Pro Ile Ser Ala Leu Gln Val Arg
   1415 1420 1425
   Leu Gly Asn Thr Ala Gly Thr Glu Asn Asn Ser Thr Trp Glu Leu
   1430 1435 1440
   Ile His Thr Lys Ser Glu Ile Glu Gly Arg Pro Glu Thr Ile Phe
   1445 1450 1455
   Gln Leu Cys Cys Ser Asp Ser Glu Ser Lys Thr Ser Ile Val Lys
   1460 1465 1470
   Val Ile Arg Ser Ile Leu Arg Glu Asn Phe Arg Arg His Ile Lys
   1475 1480 1485
   Cys Glu Leu Pro Met Glu Lys Thr Cys Lys Asp Arg Leu Val Pro
   1490 1495 1500
   Leu Lys Asn Arg Val Pro Val Ser Ala Lys Leu Ala Ser Ser Arg
   1505 1510 1515
   Ser Leu Lys Val Leu Arg Thr Ser Ser Ser Ser Glu Trp Pro Ser
   1520 1525 1530
   Asp Pro Ser Lys Gly Asn Ser Leu Asp Ser Asp Glu Cys Ser Leu
   1535 1540 1545
   Ser Ser Gly Thr Gln Ser Ser Gly Cys Pro Val Ala Glu Ser Arg
   1550 1555 1560
   Gln Asp Cys Lys Ser Thr Val Leu Glu Lys Asp Ala His Glu Val
   1565 1570 1575
   Leu Ala Glu Phe Pro Asp Gly Leu Ile Lys Glu Ser Asp Ile Leu
   1580 1585 1590
   Ser Asp Glu Glu Glu Asp Phe His His Pro Leu Lys Gln Gly Ser
   1595 1600 1605
   Pro Thr Lys Asp Ile Glu Leu Gln Phe Gln Arg Leu Arg Ile Ser
   1610 1615 1620
   Glu Glu Pro Asp Met His Ser Thr Gly Gln Gln Pro Pro Leu Thr
   1625 1630 1635
   Gly Pro Gly Glu Gln Pro Lys Leu Val Arg Gly His Phe Cys Pro
   1640 1645 1650
   Ile Lys Arg Lys Ala Asn Ser Thr Lys Arg Gly Arg Gly Thr Leu
   1655 1660 1665
   Leu Lys Ala Gln Thr Arg His Gln Ser Leu Asp Ser His Pro Glu
   1670 1675 1680
   Thr Ala Ser Ile Asp Leu Asn Leu Val Leu Glu Arg Glu Phe Ser
   1685 1690 1695
   Val Gln Ser Leu Thr Ser Val Val Asn Glu Glu Cys Phe Tyr Glu
   1700 1705 1710
   Thr Gln Ser His Gly Lys Ser
   1715 1720
<210> 54
   <211> 933
   <212> PRT
   <213> Tiam2 Canis familiaris; deduced from ENSCAFG00000000577
<400> 54
   Met Arg Glu Leu Glu Met Ser Arg Thr Asn Thr Glu Asn Val Glu Thr
   1 5 10 15
   Ser Thr Asp Thr Ala Asp Ser Ser Ser Gly Ser Leu Ser Ser Leu Glu
   20 25 30
   Gln Leu Asp Leu Leu Phe Glu Lys Glu Gln Gly Val Val Arg Lys Ala
   35 40 45
   Gly Trp Leu Phe Phe Lys Pro Leu Val Thr Leu Gln Lys Glu Arg Lys
   50 55 60
   Leu Glu Leu Val Ala Arg Arg Lys Trp Lys Gln Tyr Trp Val Thr Leu
   65 70 75 80
   Lys Gly Cys Thr Leu Leu Phe Tyr Glu Thr Tyr Gly Lys Asn Ser Met
   85 90 95
   Asp Gln Ser Asn Ala Pro Arg Cys Ala Leu Phe Ala Glu Asp Ser Ile
   100 105 110
   Val Gln Ser Val Pro Glu His Pro Lys Lys Glu Asn Val Phe Cys Leu
   115 120 125
   Ser Asn Ser Phe Gly Asp Val Tyr Leu Phe Gln Ala Thr Ser Gln Thr
   130 135 140
   Asp Leu Glu Asn Trp Val Thr Ala Ile His Ser Ala Cys Ala Ser Leu
   145 150 155 160
   Phe Ala Lys Lys His Gly Lys Glu Asp Thr Val Arg Leu Leu Lys Asn
   165 170 175
   Gln Thr Lys Asn Leu Val Gln Lys Ile Asp Met Asp Ser Lys Met Lys
   180 185 190
   Lys Met Ala Glu Leu Gln Leu Ser Val Val Ser Asp Pro Lys Asn Arg
   195 200 205
   Lys Ala Ile Glu Asn Gln Ile Gln Gln Trp Glu Gln Asn Leu Glu Lys
   210 215 220
   Phe His Met Asp Leu Phe Arg Met Arg Cys Tyr Leu Ala Ser Leu Gln
   225 230 235 240
   Gly Gly Glu Leu Pro Asn Pro Lys Ser Leu Leu Ala Ala Ala Ser Arg
   245 250 255
   Pro Ser Lys Leu Ala Leu Gly Arg Leu Gly Ile Leu Ser Val Ser Ser
   260 265 270
   Phe His Ala Leu Val Cys Ser Arg Asp Asp Ser Ala Phe Arg Lys Arg
   275 280 285
   Thr Leu Ser Leu Thr Gln Arg Gly Arg Asn Lys Lys Gly Ile Phe Ser
   290 295 300
   Ser Leu Lys Gly Leu Asp Thr Leu Ala Arg Lys Gly Lys Glu Lys Arg
   305 310 315 320
   Pro Ser Val Thr Gln Ala Ser Ser Ser Val Asp Glu Leu Leu His Ile
   325 330 335
   Tyr Gly Ser Thr Ala Asp Gly Val Pro Arg Asp Asn Thr Trp Glu Ala
   340 345 350
   Gln Thr Tyr Val His Phe Gln Asp Asn Gln Gly Val Thr Val Met Ile
   355 360 365
   Lys Pro Glu His Arg Val Glu Asp Ile Leu Thr Leu Ala Cys Lys Met
   370 375 380
   Arg Gln Leu Glu Pro Ser His Tyr Gly Leu Gln Leu Arg Lys Leu Val
   385 390 395 400
   Asp Glu Asn Thr Glu Tyr Cys Ile Pro Ala Pro Tyr Glu Tyr Met Gln
   405 410 415
   Glu Gln Ala Ser Val Tyr Asp Glu Ile Glu Ile Phe Pro Leu Ser Val
   420 425 430
   Tyr Asp Val Gln Leu Thr Lys Thr Gly Ser Val Ser Asp Phe Gly Phe
   435 440 445
   Ala Val Thr Ala Gln Val Asp Glu His Gln His Leu Ser Arg Ile Phe
   450 455 460
   Ile Ser Asp Val Leu Pro Asp Gly Leu Ala Tyr Gly Glu Gly Leu Arg
   465 470 475 480
   Lys Gly Asn Glu Ile Met Thr Leu Asn Gly Glu Ala Val Ser Asp Leu
   485 490 495
   Asp Leu Lys Gln Met Glu Ala Leu Phe Ser Glu Lys Ser Val Gly Leu
   500 505 510
   Thr Leu Ile Ala Arg Pro Pro Asp Arg Lys Ser Thr Leu Cys Ser Ser
   515 520 525
   Trp Ser Asp Ser Asp Leu Phe Ser Arg Asp Gln Lys Ser Leu Leu Pro
   530 535 540
   Pro Pro Asn Gln Ser Gln Leu Leu Glu Glu Phe Leu Asp Asn Phe Lys
   545 550 555 560
   Lys Asn Thr Thr Asn Asp Phe Ser Asn Val Pro Asp Val Thr Ser Gly
   565 570 575
   Leu Lys Arg Ser Gln Thr Asp Gly Ala Leu Ala Arg Val Pro Arg Glu
   580 585 590
   Glu Asp Thr Ala Gln Thr Leu Gly Ser Ala Glu Gln Ile Thr Val Leu
   595 600 605
   Cys Arg Ser Phe Asn Asp Thr Gln Ser Asn Gly Met Glu Gly Pro Arg
   610 615 620
   Gly Thr Arg Asp Pro Pro Pro Arg Pro Leu Ala Arg His Leu Ser Asp
   625 630 635 640
   Ala Asp Arg Leu Arg Lys Val Ile Gln Glu Leu Met Asp Thr Glu Lys
   645 650 655
   Ser Tyr Val Lys Asp Leu Ser Cys Leu Phe Glu Leu Tyr Leu Glu Pro
   660 665 670
   Leu Gln Asn Glu Thr Phe Leu Thr Gln Asp Glu Met Glu Ser Leu Phe
   675 680 685
   Gly Ser Leu Pro Glu Met Leu Glu Phe Gln Lys Val Phe Leu Glu Thr
   690 695 700
   Leu Glu Asp Gly Leu Ser Ala Ser Ser Asp Phe Asn Ile Leu Glu Thr
   705 710 715 720
   Pro Ser Gln Phe Arg Lys Leu Leu Phe Ser Leu Gly Gly Ser Phe Leu
   725 730 735
   Tyr Tyr Ala Asp His Phe Lys Leu Tyr Ser Gly Phe Cys Ala Asn His
   740 745 750
   Ile Lys Val Gln Lys Val Leu Glu Arg Ala Lys Thr Asp Lys Ala Phe
   755 760 765
   Lys Ala Phe Leu Asp Ala Arg Asn Pro Thr Lys Gln His Ser Ser Thr
   770 775 780
   Leu Glu Ser Tyr Leu Ile Lys Pro Val Gln Arg Val Leu Lys Tyr Pro
   785 790 795 800
   Leu Leu Leu Lys Glu Leu Val Ser Leu Thr Asp His Glu Ser Glu Glu
   805 810 815
   His Tyr His Leu Thr Glu Ala Leu Lys Ala Met Glu Lys Val Ala Ser
   820 825 830
   His Ile Asn Glu Met Gln Lys Ile Tyr Glu Asp Tyr Gly Thr Val Phe
   835 840 845
   Asp Gln Leu Val Ala Glu Gln Ser Gly Thr Glu Lys Glu Pro Val Ser
   850 855 860
   Val Val Ser Met Ser Ser Leu Ser Pro Val Met Ile Gln Val Thr Glu
   865 870 875 880
   Leu Ser Met Gly Glu Leu Leu Met His Ser Ala Val Ser Trp Leu Asn
   885 890 895
   Pro Phe Leu Ser Leu Gly Lys Ala Arg Lys Asp Leu Glu Leu Thr Val
   900 905 910
   Phe Val Phe Lys Arg Ala Val Ile Leu Val Tyr Lys Glu Asn Cys Lys
           915                 920                 925
   Leu Lys Lys Lys Leu
       930
<210> 55
   <211> 1965
   <212> DNA
   <213> Fgd2 mouse transcript variant 1a t6/tw5
<400> 55
   atggagcgag cctgtgagaa gcaggactca gtgtgcaacc tggtggctgt gtttgagaac      60
   aacaggactc cgggagaagc acctggatcc catagccttg aggaccagct ccatagccct     120
   gaacaccagc tgtccctgtc cccagagcct tgggaggcac cccctgtcaa ggaggccttg     180
   aagtctgaat tccggccagt gagcaggaca tatctgagtt ccctcaagaa caagctatcg     240
   agtggggctt ggaggagatc ctgccagcct ggggtcagcc cggggccaga gacacaggaa     300
   cctgaggaga agagggtcgt gcgagagctt ctggagacgg agcaggccta tgtggctcgc     360
   ctgcacctgc ttgaccaggt gttcttccag gagctgctga gggaggcagg ccgcagcaag     420
   gccttccctg aggacgtggt gaagctcatt ttctccaaca tctcctccat ctatcgtttc     480
   cacgcccagt tcttccttcc cgagctgcag cggcgcgtgg atgactgggc agccacgccc     540
   cgcattgggg atgtgatcca gaagctggcc ccgtttctga aaatgtacag cgagtacgtg     600
   aagaactttg agcgggccgc ggaactgctg gccacgtgga tggacaagtc tcagcccttc     660
   caggaggtgg tcacccgcat ccagtgcagc gaggcctcgg gcagcctgac cctgcagcac     720
   cacatgttgg agcctgtgca aagaatcccg cggtacgaac tgctgctcaa ggaatatgtg     780
   cagaagctgc cagcccaggc cccagacctc gaagatgccc agagagcact ggacatgatc     840
   ttctcagctg cacagcactc caatgcagcc attgcagaga tggagcggct gcagggcctg     900
   tgggatgtgt accagcgcct gggcttggag gatgacatcg tggacccctc caacaccctg     960
   ctccgagagg gccctgttct caagatctct ttccgccgca gcgacccaat ggaacgctac    1020
   ctggttttgt tcaacaacat gcttctgtat tgtgtacccc gagtcctcca agtgggtgcc    1080
   cagttccagg tgcggactcg catcgatgtg gccggcatga aggtgcggga gctgaccgat    1140
   gctgagttcc cacactcctt cctggtgtcc ggaaagcagc gcacactgga gctgcaggcc    1200
   cggtcccgag atgaaatggt ttcctggatg caggcctgcc aggcagccat tgaccaggtt    1260
   gagaagcgga gtgagacctt caaggctgct gtccagggac ctcaggggga cacgcaggag    1320
   cccaagccac aggtagagga gctgggtctc cgagcgcctc agtgggtccg ggacaagatg    1380
   gtgaccatgt gcatgcgttg ccaggagccc ttcaatgccc tgactcgtcg gcgccaccac    1440
   tgccgggctt gcggctatgt agtgtgtgcc aagtgctctg actaccgtgc ggagctgaaa    1500
   tatgacagca acaggcccaa ccgagtctgc ctgacctgct acacatttct cactggaaac 1560
   gtactccctc aaggcaagga ggacaagagg cggggcatct tggagaaaga ggcctcagca 1620
   gcacccgagc agagtctggt gtgcagcttc ctgcagctca taggagacaa gtgcagcagg 1680
   agccttcccc ggagctggtg tgtgatcccc cgggatgacc cccttgtgct gtatgtctat 1740
   gcagcccccc aggacacaaa ggctcacacc tccatccccc tactgggcta tcaggtgatt 1800
   tcagggcccc agggggaccc tcgggttttc cagctgcaac agtcaggcca gcagtacacc 1860
   ttcaaggccg agtctgtgga gctgcagggc cgctgggtga cagctatcaa gcgtgcggcc 1920
   agtggccgga cccctgaggg acctgacgaa gaagatgtgt ctgac 1965
<210> 56
   <211> 3484
   <212> DNA
   <213> Tagap1 Danio rerio; Ensembl Transcript ENSDART00000063826
<400> 56
   caacatcatc atcatcacgc gaaatacgac tactgtggat tactaactca agccatgctt 60
   ttttctgcat tgcagcttgc ctgagctgag ggatcactgg ttacagactt tacacaggaa 120
   aactgtggag gcaaggttgt tagcgggcag cacttcccca ccccccagcg tcctcatgaa 180
   ggtgctgagc ggcaatacca cgaataaaac gctaagtgga gatgggatgg actctgactt 240
   acttgttgat agcaacggca aaaactccgc cctttcaaaa gagttgcata accaggaggc 300
   acatgcacag cagccgattg gggaaaacgg aggcaaaaag tcctttaaaa tcccatttaa 360
   gctgaagaga agctccacaa gcccaagtgt tactgctcat cctgagtcca gtacaaagaa 420
   cttgctgttc ggacgaaatc tccaagatga cactgcgctc ccaaaaccaa ttgctgaaat 480
   actcctgttg ttgttcagga aggggccagt tactgagggg gtctttagag tctcatgtaa 540
   cagcaaaaac ctgcatactc tcagaaatca gctcaattct ggggctgagg tggacatgga 600
   tgccctgcct gtgacactct tggttggttt gctgaagatt tttctgaggg aactgcctgg 660
   aggtttgctg atgtctgagc attatgaaag ctggattggt gctctggaga aagagcggac 720
   agaggaggtg caaagtgagc tgagaggaat ggccgagaag ttgcccaaag caaacagtct 780
   cctcctgcag cacctgttgt gcctttttca ccacatcagc cagcgatcag aaaccaacaa 840
   aatggatgcc aagaatctag ctgtgtgcat tgctccaacc ttgctgcatc gtgacagcca 900
   gcccctagac gtagatgttg tggaaaaggt cacaaaactt atacagttcc tgactgagaa 960
   ttgctgtgag atatttggac atgatatcct gacgttgttt ggggatctag aagatcggaa 1020
   ggccactgac aagtcggatt cagcctcctt gatgtcccct gatatatctt tcgaagtcca 1080
   ccaacatgac tctgcatacg acagcacaga tcctgatgcg gattgtgact gtgtggaagc 1140
   tgagagctat gaagaaggca tgatggcaca tgggagccta ggcctaagca aaaagggacg 1200
   ctctgacatt cagtcttgtt cctctgatgc tatatttgac acattcacca aaactttcag 1260
   ccgacgaagc tctgagccct ccattttatc tactctccct ataacgggtt tgagagagct 1320
   tgctcgcagc catgatgact tttcaacaga gaaggaacac tttgacaatc agccgcttaa 1380
   aaagcagaac tcagatgact ctttcctgct tcccaaccgc tctgagaaca gaagatccct 1440
   caaaaaactg gctgggagtt ttaacatgga cttgcctatc attgtgtctt ctccaacatc 1500
   taagattggt tcctgtccat cattctgttc ctcagatagc agctcctcaa atcactcaga 1560
   gcagtcaaaa actccttcac ctctgccatc ccctgcaaac cctcgcaaaa cccagtccac 1620
   caggcatgca tcgttcatga ttaaatctag acataaccat gctcaaggcg accaagaagt 1680
   tacccgccga tccctctcaa tgagagccaa gagtctcgga aactttacat ttaacagaag 1740
   cagcctgaag aaaggggact ctcagaagga agtggtcttc ccatgtgaaa ctctccagga 1800
   ggactcgcaa aatgagacag agaacccaga tgaacttgtt cgccgtcgac gacctctttc 1860
   tgccattgaa gtcttccagc aggtggacag ccggatgcca tgcagccccc cgtcctacga 1920
   acaggcactt cagactggag ttcaccaggc cccaccacaa tacagagaaa tgacagtcca 1980
   acacgccaga gaactaggca agaaatctcg tcccatatcc atgaacgaca acctactaga 2040
   catttacaaa gtaaacgagt ccacagaatg cttagaaaca ttcacagaga gcattcaatt 2100
   agataaaccg cagctggtga cattccggca gagggccatg tccgagtcgg tatcccagtt 2160
   gaaacatgag aaagtgtcac gcaggtgtag tcagcctgtt tttgaggagt tctcctatgc 2220
   taaggaatct tatgtgtaag tgattctttc ttaattgcaa tattaaacgc cttattaaag 2280
   gatcagttca cccacatttt tagttatgct attaattatt taccatcatg tcattttaac 2340
   tcctcttcag aacacaaatg aaatatttta gatcaaatct gagagcttcc tcatcctccg 2400
   taggcagcaa tggtcctgag ttaaaagtct tgaaaaagaa ccaaaaacat tgtcaaagca 2460
   ttccatatga ccttagtggt tcaactgtaa tcatacgaag ctacaagaac actattgtgc 2520
   accaaaaaca tgtcatattg ttcgtagtaa atgtccaccc tgatctaatg tagtagacat 2580
   agccaaaagg tttttacagt ttttatgtgc acaaaagtgt tcatggagct tcgaatgact 2640
   acagttgaac cactgaagtc acataaaatg ctttgacaac tttgttggac ttttatggac 2700
   ttttgggtac tgttgccatc tataaaggat gagggagctc tcagattttg tctaaaatac 2760
   cttaatttat gttctgacaa tgaacgaagg gatttaagtg atatgaggga gagtaattaa 2820
   taacagaatt ctcatgtttg ggatgaacta atcctttaat attttaaagt gaagtgtgcg 2880
   atttttgtgc aaaaaaatcc tcaaggaaat ctgcaactgt ttcccaaact ctttccatcg 2940
   gtccaatagt ccaaccctaa actcacttcg ttggttaacc cactgttgct atgtcaaaat 3000
   aaggtactca tactttgctg cattggttgc cactatcttt ccctaccaat cgcttgcttg 3060
   ttaatatgct gtaaaatgtg agtttgtttg attattaata cttttcaagc atcacactga 3120
   gcactcctgc aaacgtaatg tggaatgtga ttaaataaga ttaatttgac tgcattctaa 3180
   taccaaacag gactttaagt ttatttgaga taggataacc tattttaata tagtgaaact 3240
   gcacacttta gctttaaaat accttgctaa gctgaactgt agtgacacat taaatgtgtt 3300
   acactgatct catatgctaa tattgaatct tgccagtatg aagctatgta aatagttaca 3360
   gtaattaaat tgtacaacat aaaaaatagt ctgcaatttt gatgtagtat gtacagctgt 3420
   aaaatacaat atttctgtat aatttactct ttatttcact acaataaaaa tctttaaggc 3480
   caca 3484
<210> 57
   <211> 3364
   <212> DNA
   <213> Tagap1 Macacca mulatta; Ensembl Transcript ENSMMUT00000016796
<400> 57
   gacagagctg ccctaaaagg aatgaggaag tgagagctct ccagtgtctg gctggcttcg 60
   tctgtgtgac agcccatgat gttctttccg gtctctgtaa tattctgaat ttccacctgc 120
   ccgccccctc gcttataatg cagagcatgt gaagggagac cggctcagtc tccctctctc 180
   ccagtggact agaagcagca gagtgtcatg ctctttctcc cattatttac agctcaccgg 240
   atgtaaaaga actctggcta gagaccctcc gaggacagag ccacagccgc acaggagtga 300
   aattcactcc cggacagtca gtcccaatac tgatgaagct gagaagcagc cacaatgctt 360
   caaaaacact aaatgccagt aatatggaga cgctaatcga atgtcaatca gagggtgaca 420
   tcaaggaaca tcccctgttg gcatcatgtg agagtgaaga cagtatttgc cagctcattg 480
   aagttaagaa gagaaagaag gtgctgtcct ggccctttct catgagaagg ctctcccctg 540
   catcagattt ttctggggct ttggagacag acttgaaagc atcactattt gatcagccct 600
   tgtcaattat ctgtggtggc agcgacacac tccccagacc catccaggac attctcacta 660
   ttctatgcct taaaggccct tcaacggaag ggatattcag gagagcagcc aacgagaaag 720
   cccgcaagga gctgaaggag gagctcaact ctggggatgc ggtggatctg gagagcctcc 780
   ccgtgcacct cctcgctgtg gtctttaagg acttcctcag aagtatcccc cggaagctac 840
   tttcaagcga cctctttgag gagtggatgg gcactctgga gatgcaggac gaggaggaca 900
   gaatcgaggc cctgaaacag gttgcagata agctccccca gcccaacctc ctgctgctca 960
   agcacttggt ctatgtgcta cacctcatca gcaagaactc tgaggtcaac aggatggact 1020
   ccagcaacct ggccatctgc attggaccca acatgctcac cctggagaat gaccagtgtc 1080
   tgtcatttga agcccagaag gacctgaaca acaaggtgaa gacactggtg gaattcctca 1140
   ttgataactg ctttgaaata tttggggaga acattccagt acattccagt atcacttctg 1200
   ttgactccct ggagcacact gacagttcag atgtgtcgac cctgcagaat gactcagcct 1260
   acgacagcaa cgatcctgat gtggaatcca acagcagcag tggcatcagc tctcccagca 1320
   ggcagcccca ggtgcccatg gccacagctg ctggctttga tagtgggggc ccacaggatg 1380
   cccgagaggt cagcccagag cccattgtga gcaccgtggc caggctgaaa agctccctcg 1440
   cacagcccga taggaggtac tcagagccca gcatgccgtc ctcccaggag tgccttgaga 1500
   gccgggtgac aaaccaaaca ctaacaaaga gtgaagggga cttccccgtg ccccgggtag 1560
   gctctcgttt ggaaagggag gaggctgaag acccatttcc agaggaggtc ttccctgcag 1620
   tgcaaggcaa aaccaagagg ccggtggacc tgaagatcaa gaacttgacc ccgggttcgg 1680
   tgctcccaag ggcactggtt cccaaagcct tctccagcag ctcgctggac gcgtcctctg 1740
   acagctcacc cgtggcttct ccttccagtc ccaaaagaaa tttcttcagc agacatcagt 1800
   ctttcaccac aaagacagaa aaaggcaagc ccagcagaga aattaaaaag cactccatgt 1860
   ctttctcctt tgcccctcac aaaaaagtgc tgaccaaaaa cctcagcgca gggtctggga 1920
   aatcgcaaga ctttaccagg gaccacgtcc caaggggtgt tagaaaggaa agccagcttg 1980
   ctggccgaat cgtgcaggaa aatgggtctg aaacccacaa tcaaacaacc cgcgccttct 2040
   gcctgagacc ccacgccctc tcggtggatg atgtgttcca gggagctgac tgggagaggc 2100
   ctggaagccc accctcttat gaagaggcca tgcagggccc ggcagcccga ctcgcggcct 2160
   acgggagcca gaccgtgggg agcatgactg tggggagcat gagggcgagg atgctggagg 2220
   tggactccct cctaccccct cttccacctg ctcaccacac aggagactca agacacaggg 2280
   acagcaaaga gccactccct ggccacggac cctctcccct gcctgagcga tggatacaga 2340
   gcagaactgt ccatgcttct tgggactctg tggggcacgt gtctggccca gggagacctg 2400
   agctcctccg gctgaggacc gtctccgagt ccgtgcagag gaataagcgg gactgtctca 2460
   tgcgacgatg tagccagccg gtctttgagg ctgaccagtt ccaacatgcc aaagaatcgt 2520
   atatttagga gggaggccat acaccatgcc acagcttgtg ctatctgtaa atatgagact 2580
   tgtaaagaac tgcctttaga ttgtggtttt aaaggtcttg aataagctcc tttagaacgc 2640
   tgtgcaaagc cctcctcagt gaggatagct acaccatggc catggtgcat cagatagtct 2700
   gtgtgtaccc ggatttgtgc aatatgtaac aaatgtataa aatgtattat agataaggtg 2760
   ttaggtgcaa agaatgtcta ataatccctg catacgtttg tggatttgca gtgaagtaca 2820
   ttgctgttcc ttgcttcctg gggcactttt ctcttggcta gtgtttgaaa attatctttg 2880
   cttttataat gtggcctcaa atgtcatgcc aattttcaca ttttccacaa actccattta 2940
   gggagaagtg tttaaacccc cggtaagttt actctatacc agagtaaact atatattact 3000
   ccatataagc agccttgcaa taactaatca ccaccataga cgaaagaaac agactgcaag 3060
   gaacagagct gagtgtctgg agccatcaaa ggcattaaaa actccagcaa aagcagaggt 3120
   catagcaaaa atcatgaaaa acacttcaac ctgtcctttc aatcatccaa ttaaatttgg 3180
   gtaaattaat gaaaatgtat tatatcaata ttaactcatc tacagcactt tgagtttctt 3240
   tgtaattcat gatgtcctat cctatagtgt ggaggtaaat gattttatat gcattggggg 3300
   tcatatataa aacttcaatg taatttcact acaataaatt gccttcctta tttgaaagta 3360
   aaaa 3364
<210> 58
   <211> 2175
   <212> DNA
   <213> Tagap1 Monodelphis domestica; Ensembl Transcript ENSMODT00000009606
<400> 58
   atgaaggtgt taagtggctg caatgcttcc aaaacactaa atgccagtga catggaaact 60
   ttaattgaat gtcagccaga gggcaatgtg aagcaacacc acttgctggc acaatgtgat 120
   agtgaagatg ggatttgcca cctgattgaa aacaagaaga gaaaggtgat agcctggcct 180
   tttcccatga gaagaacagc ttcttcttca gattcttctg gatctttaac accagaatgg 240
   aaggcttccc tgtttgacca gcccttgtca gcaatctgca ctgatgaaga tatccttccc 300
   caaccaattc aggaaattct taatattctt caaaacaaag gcccttcaac tgaaggaata 360
   ttcagaaagg ctgccaatga aaaggcccgg aaagaactta aggaagagct caactctggg 420
   ggaatggtca atttggaaac caaatctgtt cacctactgg ctgcagtttt aaaggatttc 480
   ctcaggagta tcccactcaa actactttca tcagatctat ttgaagaatg gatgacagcc 540
   ttagaaaggc caaatgagga tgacaaaatt gagagcttga aacaggttgt aaagaaacta 600
   ccaaggacaa atatcctgtt gctcaaacat ttggtctatg tgctttacaa catcagtaag 660
   aattcggatg tcagcaagat ggactcaagc aatcttgcca tttgtattgg acccaacatg 720
   ctgtccctca acactgacca aagcctatct tttgatgctc agaaggagtt gaacaacaag 780
   attaaaactt tggtggaatt tttcattgat aactgctttg aaatatttga agaggacatc 840
   cctggacatt cccccaggtt caattctgat gattcactgg aacacatcaa caattcagat 900
   atgtcaactc tgcaaaatga ctcagcctat gagagtacag atgctgacac agaatgcagc 960
   aacagttttg gctctcaaaa caagccgtcc ctagacactg caggactgtc cagtgacttg 1020
   gacaacagaa agcatctgta tgagtcaaga ccagggtctg ttgtctctta caccaactta 1080
   ctaaaaagct cccttagcac tcaagaaagg aggtactcag agcctagcac accatccacg 1140
   aaagactgcc ttgaaagcag gatcaccagc cacaaattga ctaaaagtga ggacagcttc 1200
   gctgtccccc aggcaagttc ctgttttggg ggtcaagaaa taaaagaatc atttccagag 1260
   gaatgtttcc ctagtctaca acacaggaaa caaaagtcat taggtcttca aataaaggaa 1320
   ggaactttat gcacagaatc acttgttgag ccttccccta aaacctcctc ctgtggttcc 1380
   ctggatagtt cctctgatag ctcagtcttt gccaactcgc cagtggtttc tccttccagt 1440
   ccaaaaagga actttttcac caggcatcaa tcctttacaa caaagactgc tggggcggac 1500
   agtaagctga cccgagagag aaaaaagcac tccatgtcat tctcttttgc tactcataag 1560
   aaggtgccaa acaaaactgc aaattggcgc tttcccagag accagggcaa gaaagacctc 1620
   aaaaaggaga gccaactcac tggcaggatt gtccaagaag tctgtgttgg agctatagat 1680
   gactaccaac caactccaga atgtgggagt tcaaggtcac atcttctttc agttgaagaa 1740
   gtatttcaac ttgtagatca gaaaaaccct ggaagccccc catcctatga ggaggcagtt 1800
   caatattgcc ggctttccaa aattcctcct tatgagagcc ggacagtcca aagtatgaga 1860
   gacactatgc aaagccagaa ttctagactg ccatctcttt cacttttgga ctatggtgag 1920
   catgccaaga atacacacgg caaagaaaca ctcaacagag acagtgcatc atctgtgggt 1980
   gaaacttggg tacagaatgg agcttccaat gtggctatgg aaaggaaagg acaaatacca 2040
   aaatctgagg ttcgccggtt aagggtcttg tctgaatcct tgctaaagaa taaacaggac 2100
   tgtgtcatta gaaggtgtag tcagccaatc tttgaggttg accaaatcca atatgccaag 2160
   gaatcctatg tttag 2175
<210> 59
   <211> 2109
   <212> DNA
   <213> Tagap1 Xenopus tropicalis; Ensembl Transcript ENSXETT00000021546
<400> 59
   atgaaggtgc tcacaggctg caatgctttt aaagcaatca ataccagcaa catggaagac 60
   ttaatagaat gcccaacaga ggcagatgcc aagaaatacc agttattggc agcaatgata 120
   agtgaagatg gcatgtgcca tgtaattgaa aacaacaaaa aaagaaaggc tgtcatatcc 180
   tggccattca cattcagaag aagctcaaca ctatcagaaa cttcagttcc acctgaacta 240
   aaagcaactt tgtttgacca accactttct attgtctgtg aagaggacgc ccttcccaaa 300
   ccaatacttg aaatccttac tattctgtgc cagcagggcc catccactga gggcattttc 360
   cgtaaagctg ccaacgagaa agcacgcaaa gaactcaagg aggatctcaa tagtggaaag 420
   acggtggatt taaaaagcaa gcatgtgcat ttattggctg tggttctcaa ggtaaatata 480
   ttgtctacgc aaaaagaaat gatcatcaat aacaactttt tcttttttct taacaacttc 540
   attagtgatg agcgaatatg ttctgtttca cgtgttgcgg ataaactgcc cagacctaac 600
   tggattctac tgcaacattt aatctgcgtc ctctatcaca tcagcaaagc ctcaacttta 660
   aacaagatgg attcaaacaa tctagctgtc tgtattggcc caaacatgct ccaacctcac 720
   catgactata atctttccct agaagctcag aagcaggcca atgacagggt tatttcccta 780
   gttgagttct tcatagacaa ttgctttgat ctgtttggtc aaaatgtatc acagtgtcta 840
   agtacctcaa aggaggagtt gctggaggac actgatgtct cagaaatccc ttttcaacaa 900
   aatgactcag catatgacag cacagatcct gaatatgagg gacataatag taccatcacc 960
   agctgtcaaa aagacttggg gattaacagt gggagagagg ttctaaatag ctcaatggat 1020
   caggatataa atctgactca atcttctata aacttgttga aagtcagcaa tatggacagg 1080
   aggaagtcag aaccacacat attcccctca caggatacca aggtggtcgc aggcagaaaa 1140
   ctgaccagaa gccatgatga tgtcactgtt cgtaaaagtg gagaaccttt aactaaggag 1200
   gacctatcag agcaagtgtc agaaatagcc ttatataaga aaaagatgcc aaaaggtctg 1260
   acagttaata ccagttattc agaagatgtg ttagatgatg tactgcagaa tgctctttcc 1320
   agttgttcac tagagagttg cttttcagac tgttctgtgt ttaccagctc accgcttgct 1380
   tctcccataa gtccaaagaa caactgtttg ataagacatc aatcatgttc ttcgaagagt 1440
   ggaattagaa atgacattaa attatcaagc agagaaataa aaaagcattc aaagtcattt 1500
   tcctatgtga atcaaaagaa aaagttggca aagacacaga gctgggggcc tgaaggacaa 1560
   aatccaggct tacagaggta catgtttaac agcagtctca gaaatagaca tcaatatgaa 1620
   aatccacagg ctaaatgttt tcagcagcct gctgttgtta ggcttagaag gccacaatct 1680
   gcccgcaaaa tgtcagttga tgaggtgttc cgaatagtag accagagaaa ccctggcaaa 1740
   cctccatcat atgaggaagc cattcataaa aatgttccac cctttaaagg tatgacagtc 1800
   caaactgtga gagctactgt ttcaaataat gaatattcat cttcccatgt tgtcagtgat 1860
   acccccatgg acagagaatg tagaactcat gcagagaaac agacaacaat aataaatgaa 1920
   gggccaacag aaaaaaactg tgccgaggaa atgcaacagc tctgtcagtt tggaaaaact 1980
   aaaagtgttg ttatacgaac tatgtctgag tctgttcaga aacacaagca tgaaacctta 2040
   agtcggagat gcagccagcc ttttgagctt tatgatcaga ttcagtatgc caaggaatcc 2100
   tatgtttag 2109
<210> 60
   <211> 3354
   <212> DNA
   <213> Tagap1 Pan troglodytes; Ensembl Transcript ENSPTRT00000039625
<400> 60
   gacatagctg ccctaaaagg aatgaggaag cgagagctct ccagtgtctg gctggctccg     60
   tccgtgtgac agcccatgat gttctttccg gtctctgtaa tattctgaat ttccacctgc    120
   ccgccccctc gcttataatg cagagcatgt gaagggagac cggctcagtc tctctctctc    180
   ccagtggact agaaggagca gagtgttacg ctgtttctcc cattctttac agctcaccgg    240
   atgtaaaaga actctggcta gagaccctcc aaggacagag gcacagccac acgggagtga    300
   aatccacccc tggacagtca gccgcaatac tgatgaagct gagaagcagc cacaatgctt    360
   caaaaacact aaacgccaat aatatggaga ctctaatcga atgtcaatca gagggtgata    420
   tcaaggaaca tcccctgttg gcatcatgtg agagtgaaga cagtatttgc cagctcattg    480
   aagttaagaa gagaaagaag gtgctgtcct ggccctttct catgagaagg ctctcccctg    540
   catcagattt ttctggggct ttggagacag acttgaaagc atcgctattt gatcagccct    600
   tgtcaattat ctgcggtgac agtgacacac tccccagacc catccaggac attctcacta 660
   ttctatgcct taaaggccct tcaacggaag ggatattcag gagagcagcc aacgagaaag 720
   cccgtaagga gctgaaggag gagctcaact ctggggatgc ggtggatctg gagaggctcc 780
   ccgtgcacct ccttgctgtg gtctttaagg acttcctcag aagtataccc cggaagctac 840
   tttcaagcga cctctttgag gagtggatgg gtgctctgga gatgcaggac gaggaggaca 900
   gaatcgaggc cctgaaacag gttgcagata agctccccca gcccaacctc ctgctactca 960
   agcacttggt ctatgtgctg cacctcatca gcaagaactc tgaggtgaac aggatggact 1020
   ccagcaatct ggccatctgc attggaccca acatgctcac cctggagaat gaccagagcc 1080
   tgtcatttga agcccagaag gacctgaaca acaaggtgaa gacactggtg gaattcctca 1140
   ttgataactg ctttgaaata tttggggaga acattccagt gcattccagt atcacttctg 1200
   atgactccct ggagcacact gacagttcag atgtgtccac cctgcagaat gactcagcct 1260
   acgacagcaa cgaccctgat gtggaatcca acagcagcag tggcatcagc tctcccagca 1320
   ggcagcccca ggtgcccatg gccacagctg ctggcttgga tagcgcgggc ccacaggatg 1380
   cccgagaggt cagcccagag cccattgtga gcaccgtggc caggctgaaa agctccctcg 1440
   cacagcccga taggagatac tcagagccca gcatgccatc ctcccaggag tgcctcgaga 1500
   gccgggtgac aaaccaaaca ctaacaaaga gtgaagggga cttccccgtg ccccgggtag 1560
   gctctcgttt ggaaagtgag gaggctgaag acccatttcc agaggaggtc ttccctgcag 1620
   tgcaaggcaa aaccaagagg ccggtggacc tgaagatcaa gaacttggcc ccgggttcgg 1680
   tgctcccgcg ggcactggtt ctcaaagcct tctccagcag ctcgctggac gcgtcctctg 1740
   acagctcgcc cgtggcttct ccttccagtc ccaaaagaaa tttcttcagc agacatcagt 1800
   ctttcaccac aaagacggag aaaggcaagc ccagccgaga aattaaaaag cactccatgt 1860
   ctttctcctt cgcccctcac aaaaaagtgc tgaccaaaaa cctcagcgcg ggctctggga 1920
   aatcgcaaga ctttaccagg gaccacgtcc cgaggggtgt cagaaaggaa agccagcttg 1980
   ccggccgaat cgtgcaggaa aatgggtgtg aaacccacaa ccaaacagcc cgcggcttct 2040
   gcctgagacc ccacgccctc tcggtggatg atgtgttcca gggagctgac tgggagaggc 2100
   ctggaagccc accctcttat gaagaggcca tgcagggccc ggcagccaga ctagtggcct 2160
   acgagagcca gaccgtgggg agcatgacgg tggggagcat gagggcgagg atgctggagg 2220
   cgcactgcct cctaccccct cttccacctg ctcaccacgt agaggactca agacacaggg 2280
   gcagcaaaga gccactccct ggacacggac tctctcccct gcctgagcga tggaaacaga 2340
   gcagaactgt ccatgcttct ggggactctc tggggcacgt gtctggccca gggagacctg 2400
   agctcctccc gctgaggact gtctccgagt ccatgcagag gaataagcgg gactgtctcg 2460
   tgcgacgatg tagccagccg gtctttgagg ctgaccaatt ccaatatgcc aaagaatcgt 2520
   atatttagga gggacgccat acgccatgcc atagcttgtg ctatctgtaa atatgagact   2580
   tgtaaagaac tgcctgtaga ttgcttttaa aaggtcttga ataagctcct tgagaaagtt   2640
   gtggaaagcc ctcctcagtg aggatagcta caccatggcc atggcgcatc agatagtctc   2700
   tgtgtacctg gatttgtgca atatgtaaaa atgtatcaaa tgtattatag ataaggtgtt   2760
   aggtgcaaag gatgtctaat aatccctgca cacgtttgtg aacttgcagt gaagtacact   2820
   gctgttcctt gcttcctggg gcacttttct cttggttggt gtttaaaaat tatcttcgct   2880
   tttttaatgt ggcctcaaat gtcatgccaa ttttcacatt ttccacaaac tccatttagg   2940
   gagaaatgtt taaatctctg gtataagttt actccatacc agagtaaact atatattact   3000
   ctatataagc agccttgcaa taactaatca ccaccataga agaaagaaac agactgcaag   3060
   gaacagagtt gagtgtctgg agtcatcaaa ggcattaaaa actccagtaa aagctggggc   3120
   catagcagaa atcatgaaaa acacttcaac atgtcctttc aatcatccag ttaaatttgg   3180
   gtagattaat gaaaatgtat tacatcaata ttaactcatc tatagcactt tgagtttctt   3240
   tgtagttcat gatatcctat cctataatgt ggaggtaaat gattttatat gcattggggg   3300
   tcacatataa aacttcaatg taatttcact acaataaatt gccttcctta tttg         3354
<210> 61
   <211> 2480
   <212> DNA
   <213> Fgd2 macacca mulatta transcript variant 1; Transcript ENSMMDT00000090740
<400> 61
   agaacagatt cacgggtgat ttagcctatc tgtcccaggc caaggtggct gcatgtgcta     60
   gctggaggcc tctctctctg cttcaagggt agctgagatc caccccggaa accggcagga    120
   tgaaggaggc aagtgaggag aagctggcgt ctgtgtccaa cctggtcact gtgtttgaga    180
   atagcaggac tccagaagca gcacccagag gccacaggct agaggacgcg catcaccacc    240
   ctgagtgcag gcctcccagg tccccaggac cgtgggagaa gctgaatgtt ggggaggcca    300
   tggggtctga gcccaggaca gtcagcggga ggtacctgaa ctccctgaag aacaagctgt    360
   ccagtggagc ctggaggaaa tcttgccagc ctgtgaccct ctcaggatcg gggatgcagg    420
   agccagagaa gaagatcgtc caggagctgc tggagacaga gcaggcctac gtggcacgcc    480
   tccacctgct agaccaggcc atggtgtttt tccaggagct gctgagggcg gcccgcagga    540
   gcaaggcctt ccccgaggac gtggtcaggg tcatcttctc caacatctcc tccatctatc    600
   agttccattc tcagttcttc ctcccagagc tgcagcggcg cctggatgac tggacagcca    660
   acccccgcat cggcgacgtg atacagaagc tggccccctt cctgaagatg tacggtgagt    720
   atgtcaagaa ctttgagcga gcggctgagc tgctggccac ctggaccgag aagtctccac    780
   tcttccagga ggttctcact cgcatccaga gcggtgaggc ttcgggcagc ctgaccctgc    840
   agcaccacat gctggaacca gtgcagagaa ttccacgcta cgagctgctg ctcaaggagt    900
   acgtccagaa gctgccagcc caggccccag accgggccga tgctcagaaa gccctggaca    960
   tgatcttctc agctgcccag cactccaatg cagccatcac tgagatggag cggctgcagg   1020
   acctgtggga ggtgtaccag cgcctgggcc tcgaggacga catagtagac ccttctaaca   1080
   ccctgctccg tgagggcccg gtcttcaaga tctccttccg ccgcaacgat cccatggagc   1140
   gctacctttt cttgttcaac aacatgctgc tctactgcgt gcccagggtg atccaggtgg   1200
   gtgcccagtt ccaggtgagg acccgcatcg acgtggccgg gatgaaggtg cgggagctga   1260
   tggacgctga gttcccccac tccttcctgg tgtctgggaa gcagcgcact ctggagctgc   1320
   aagcccggtc ccaggaggaa aagatttcct ggatgcaggc cttccaagca gctgttgacc   1380
   aaattgagaa gcggaatgaa accttcaagg cggcggccca ggggcctgag ggagacaccc   1440
   aggagcagga gctgcagtct gaggagctgg gcctccgagc accacagtgg gtccgggaca   1500
   aaatggtgac catgtgcatg cgctgccagg agcccttcaa cgctctgacg cgccgtcgcc   1560
   accactgccg ggcctgcggc tatgtggtgt gtgccaggtg ctccgactac cgggccgagc   1620
   tgaaatataa cgacaatagg caaaaccgag tctgcctcca ctgctacacg ttcctcactg   1680
   gaaacgtgct ccctgaggcc aaggaggaca agaggcgggg catcctggag aaagggtcct   1740
   cagccacgcc tgaccagagc ctgatgtgca gcttcctgca gctcatcggg gacaagtggg   1800
   gcaagagtgg cccccggggc tggtgtgtga tccctcgaga tgaccccctt gtgctctatg   1860
   tctacgctgc ccctcaggac atgagggctc acacctccat ccccctgctg ggctaccagg   1920
   tgaccgttgg gccccagggg gaccctcggg tcttccagct acagcagtca ggccaactct   1980
   acactttcaa ggccgagacg gaggagctga agggccgctg ggtgaaggcc atggagcggg   2040
   cggccagtgg atggagtccc agctggccca acgatgggga cctgtccgac tgagccactg   2100
   ccagccactc tcctgcccac ctctccccac cctgaaccca gctcctgcca cagactgacc   2160
   ctgtggcctc aggacccact gccccaaggg gtgctttcac agaattgatt cagccatctg   2220
   cgctcaggcc atgtgtcccg atctgggatg cagaaaatat gggtccattc ctttctagaa   2280
   aggggacaac caagtgtctc agtttgcctt gcggggaggg ggctcctggg ccatgggact   2340
   tccagtgctg aaactgggaa agccccaggt aaccccggac tggtggtacc agagtgtggt   2400
   tttcaaccgg ggctgctcat ttgtgtctcc cggggagctt ttaaagagta ctggtgaaaa   2460
   acacatagta aattaatttt                                                2480
<210> 62
   <211> 2471
   <212> DNA
   <213> Fgd2 _b macacca mulatta transcript variant 2; transcript ENSMMUT00000040739
<400> 62
   acagattcac gggtgattta gcctatctgt cccaggccaa ggtggctgca tgtgctagct 60
   ggaggcctct ctctctgctt caagggtagc tgagatccac cccggaaacc ggcaggatga 120
   aggaggcaag tgaggagaag ctggcgtctg tgtccaacct ggtcactgtg tttgagaata 180
   gcaggactcc agaagcagca cccagaggcc acaggctaga ggacgcgcat caccaccctg 240
   agtgcaggcc tcccaggtcc ccaggaccgt gggagaagct gaatgttggg gaggccatgg 300
   ggtctgagcc caggacagtc agcgggaggt acctgaactc cctgaagaac aagctgtcca 360
   gtggagcctg gaggaaatct tgccagcctg tgaccctctc aggatcgggg atgcaggagc 420
   cagagaagaa gatcgtccag gagctgctgg agacagagca ggcctacgtg gcacgcctcc 480
   acctgctaga ccaggtgttt ttccaggagc tgctgagggc ggcccgcagg agcaaggcct 540
   tccccgagga cgtggtcagg gtcatcttct ccaacatctc ctccatctat cagttccatt 600
   ctcagttctt cctcccagag ctgcagcggc gcctggatga ctggacagcc aacccccgca 660
   tcggcgacgt gatacagaag ctggccccct tcctgaagat gtacggtgag tatgtcaaga 720
   actttgagcg agcggctgag ctgctggcca cctggaccga gaagtctcca ctcttccagg 780
   aggttctcac tcgcatccag agcggtgagg cttcgggcag cctgaccctg cagcaccaca 840
   tgctggaacc agtgcagaga attccacgct acgagctgct gctcaaggag tacgtccaga 900
   agctgccagc ccaggcccca gaccgggccg atgctcagaa agccctggac atgatcttct 960
   cagctgccca gcactccaat gcagccatca ctgagatgga gcggctgcag gacctgtggg 1020
   aggtgtacca gcgcctgggc ctcgaggacg acatagtaga cccttctaac accctgctcc 1080
   gtgagggccc ggtcttcaag atctccttcc gccgcaacga tcccatggag cgctaccttt 1140
   tcttgttcaa caacatgctg ctctactgcg tgcccagggt gatccaggtg ggtgcccagt 1200
   tccaggtgag gacccgcatc gacgtggccg ggatgaaggt gcgggagctg atggacgctg 1260
   agttccccca ctccttcctg gtgtctggga agcagcgcac tctggagctg caagcccggt 1320
   cccaggagga aaagatttcc tggatgcagg ccttccaagc agctgttgac caaattgaga 1380
   agcggaatga aaccttcaag gcggcggccc aggggcctga gggagacacc caggagcagg 1440
   agctgcagtc tgaggagctg ggcctccgag caccacagtg ggtccgggac aaaatggtga 1500
   ccatgtgcat gcgctgccag gagcccttca acgctctgac gcgccgtcgc caccactgcc 1560
   gggcctgcgg ctatgtggtg tgtgccaggt gctccgacta ccgggccgag ctgaaatata 1620
   acgacaatag gcaaaaccga gtctgcctcc actgctacac gttcctcact ggaaacgtgc 1680
   tccctgaggc caaggaggac aagaggcggg gcatcctgga gaaagggtcc tcagccacgc 1740
   ctgaccagag cctgatgtgc agcttcctgc agctcatcgg ggacaagtgg ggcaagagtg 1800
   gcccccgggg ctggtgtgtg atccctcgag atgaccccct tgtgctctat gtctacgctg 1860
   cccctcagga catgagggct cacacctcca tccccctgct gggctaccag gtgaccgttg 1920
   ggccccaggg ggaccctcgg gtcttccagc tacagcagtc aggccaactc tacactttca   1980
   aggccgagac ggaggagctg aagggccgct gggtgaaggc catggagcgg gcggccagtg   2040
   gatggagtcc cagctggccc aacgatgggg acctgtccga ctgagccact gccagccact   2100
   ctcctgccca cctctcccca ccctgaaccc agctcctgcc acagactgac cctgtggcct   2160
   caggacccac tgccccaagg ggtgctttca cagaattgat tcagccatct gcgctcaggc   2220
   catgtgtccc gatctgggat gcagaaaata tgggtccatt cctttctaga aaggggacaa   2280
   ccaagtgtct cagtttgcct tgcggggagg gggctcctgg gccatgggac ttccagtgct   2340
   gaaactggga aagccccagg taaccccgga ctggtggtac cagagtgtgg ttttcaaccg   2400
   gggctgctca tttgtgtctc ccggggagct tttaaagagt actggtgaaa aacacatagt   2460
   aaattaattt t                                                        2471
<210> 63
   <211> 2043
   <212> DNA
   <213> Fgd2 Macacca mulatta transcript variant 3; Ensembl ENSMMUT00000031213
<400> 63
   atgtttccaa agaaagccag gcacccaggg ttccctgcac tggagatttg caccaggcaa     60
   ccatccacac cagggacttg ctcgtgtttt ccctgttctc cagggaggaa accctcaggt    120
   ctgtctcttc tcctcaggac tccagaagca gcacccagag gccacaggct agaggacgcg    180
   catcaccacc ctgagtgcag gcctcccagg tccccaggac cgtgggagaa gctgaatgtt    240
   ggggaggcca tggggtctga gcccaggaca gtcagcggga ggtacctgaa ctccctgaag    300
   aacaagctgt ccagtggagc ctggaggaaa tcttgccagc ctgtgaccct ctcaggatcg    360
   gggatgcagg agccagagaa gaagatcgtc caggagctgc tggagacaga gcaggcctac    420
   gtggcacgcc tccacctgct agaccaggcc atggtgtttt tccaggagct gctgagggcg    480
   gcccgcagga gcaaggcctt ccccgaggac gtggtcaggg tcatcttctc caacatctcc    540
   tccatctatc agttccattc tcagttcttc ctcccagagc tgcagcggcg cctggatgac    600
   tggacagcca acccccgcat cggcgacgtg atacagaagc tggccccctt cctgaagatg    660
   tacggtgagt atgtcaagaa ctttgagcga gcggctgagc tgctggccac ctggaccgag    720
   aagtctccac tcttccagga ggttctcact cgcatccaga gcggtgaggc ttcgggcagc    780
   ctgaccctgc agcaccacat gctggaacca gtgcagagaa ttccacgcta cgagctgctg    840
   ctcaaggagt acgtccagaa gctgccagcc caggccccag accgggccga tgctcagaaa    900
   gccctggaca tgatcttctc agctgcccag cactccaatg cagccatcac tgagatggag    960
   cggctgcagg acctgtggga ggtgtaccag cgcctgggcc tcgaggacga catagtagac   1020
   ccttctaaca ccctgctccg tgagggcccg gtcttcaaga tctccttccg ccgcaacgat   1080
   cccatggagc gctacctttt cttgttcaac aacatgctgc tctactgcgt gcccagggtg   1140
   atccaggtgg gtgcccagtt ccaggtgagg acccgcatcg acgtggccgg gatgaaggtg 1200
   cgggagctga tggacgctga gttcccccac tccttcctgg tgtctgggaa gcagcgcact 1260
   ctggagctgc aagcccggtc ccaggaggaa aagatttcct ggatgcaggc cttccaagca 1320
   gctgttgacc aaattgagaa gcggaatgaa accttcaagg cggcggccca ggggcctgag 1380
   ggagacaccc aggagcagga gctgcagtct gaggagctgg gcctccgagc accacagtgg 1440
   gtccgggaca aaatggtgac catgtgcatg cgctgccagg agcccttcaa cgctctgacg 1500
   cgccgtcgcc accactgccg ggcctgcggc tatgtggtgt gtgccaggtg ctccgactac 1560
   cgggccgagc tgaaatataa cgacaatagg caaaaccgag tctgcctcca ctgctacacg 1620
   ttcctcactg gaaacgtgct ccctgaggcc aaggaggaca agaggcgggg catcctggag 1680
   aaagggtcct cagccacgcc tgaccagagc ctgatgtgca gcttcctgca gctcatcggg 1740
   gacaagtggg gcaagagtgg cccccggggc tggtgtgtga tccctcgaga tgaccccctt 1800
   gtgctctatg tctacgctgc ccctcaggac atgagggctc acacctccat ccccctgctg 1860
   ggctaccagg tgaccgttgg gccccagggg gaccctcggg tcttccagct acagcagtca 1920
   ggccaactct acactttcaa ggccgagacg gaggagctga agggccgctg ggtgaaggcc 1980
   atggagcggg cggccagtgg atggagtccc agctggccca acgatgggga cctgtccgac 2040
   tga 2043
<210> 64
   <211> 1890
   <212> DNA
   <213> Fgd2 Monodelphis domestica; transcript ENSMODT00000017147
<400> 64
   agcaactcca tctcccagat gccccagact ctgatggagc cccattttga taggactcca 60
   ggaccctggg aaagggacag tctggaagat agaccaggac ctggccactg ggcagtcagc 120
   accaggctac tctcctctct aagaaataag atctccaaca gcggctggag gaagtcctgt 180
   gaatttccat tctctaaccc caatctgcca ccccacttgc aggaaccaga agagaataga 240
   attgtcaggg agctcctgga gacagagcag gcctatgtgt cccgcctcta ccttctggat 300
   caggtcttct ttgaggagct actaaaggag gctcgaaaca gcaaggcttt ctctgaagac 360
   attgtcaagc tcatcttttc taacatttcc tccattcacc aattccactc ccagttcttc 420
   ctcccagagc ttcagaggcg tgtggatgaa tgggttgttg tcccccgcat tggtgatgtg 480
   atccagaaac tggcaccctt tctgaagatg tacagtgaat acgtgaagaa ctttaagcgg 540
   gcagcagagc tgctggtcat ctggacagag aagtgcccac ctttccaaga ggtcatcact 600
   cgaatccaga acagtgacat ctctgacagc ctcaccctgc aacaccacat gctggagccc 660
   gtgcagagaa tccctcgcta tgagctgttg ctgaaggagt acgtccagaa gctgccccct 720
   gatgccccag accgggctga tgcccagaaa gctttggaca tgatcttctc tgcagctcag     780
   cattctaatg cagccatcac tgaaatggag cgtctgcaga acctgtggga tgtgtatcag     840
   cgtcttggcc ttgaagatga tattgtggat ccctctaatg aattattgca ggagggtccc     900
   atccagaaga tctccttcag acatagtagc accatggagc gctacttatt cctgttcaac     960
   aatatgctgc tgtactgtgt gcctaaggtg attcaggttg gggctcagtt ccaggtgagg     1020
   acccgaattg atgtggcggg gatgaaggtc cgggaactga atgatgtgga atttcctcac    1080
   tcgttcttgg tgtcagggaa acagagaacc ctggaactac aagcccagtc tcaggaagaa    1140
   atgaacacct ggatccaggc ctgccaacgg gccatcgatc tgattgggag gcggcacgga    1200
   accttcaagg ctgctgtcca ggggacggag ggagactctg aggagcacca actgaagtct    1260
   gaagagctgg gaatccgagc tccccagtgg gtccgggaca agatggtgac catgtgtatg   1320
   cgttgcaaga cacccttcaa tgccctcacc agaagacgac accactgcag agcctgtagc   1380
   tatgtggtgt gtgctaagtg ctccgattac cgagctaggt tgcaatatga tgacaatcgg    1440
   ctccaccggg tctgcctgca gtgttacgtt ttcctcactg ggaacctact gccagaggac    1500
   aaggaagaga agaagaaagg gatcctggag aaagagtctt cgaaggtatc agaacagagc    1560
   gtgatgtgca gcttcctgca actcctggga gacaagtggg cgaagggcag cagccgaggc    1620
   tggtgtgtca tccccagaga tgaccccctg gcactttacg tctatgctgc cccacaggac    1680
   atgaaagctc acacctccat tccactgttg gggtaccaag tgaccacagg ttccttggcc    1740
   gatccccggg cattccagat acagcagtcg ggtctggtct actcattcag ggcagaatct    1800
   gaggagctaa aggggcaatg gatgaaggcc atagaacgag catccagagg tcagagcttc    1860
   actgggccag aaggagagct gtcagactga                                      1890
<210> 65
   <211> 2467
   <212> DNA
   <213> Fgd2 Pan troglodytes; transcript ENSPTRG00000018116
<400> 65
   acagattcat gggtgattta gcctatctgt cccaggccag ggcggctgag tgtgctggct     60
   ggaggcctct ctctctgctt cgagggtagc tgagatccac cccggaaacc ggcaggatga    120
   agggggcaag tgaggagaag ctggcatctg tgtccaacct ggtcactgtg tttgagaata    180
   gcaggacccc agaagcagca cccagaggcc acaggctaga ggacgtgcat caccgccctg    240
   agtgcaggcc tcccgagtcc ccaggaccac gggagaagac gaatgtcggg gaggccgtgg    300
   ggtctgagcc caggacagtc agcaggaggt acctgaactc cctgaagaac aagctgtcca    360
   gcgaagcctg gaggaaatct tgccagcctg tgaccctctc aggatcgggg acgcaggagc    420
   cagagaagaa gatcgtccag gagctgctgg agacagagca ggcctatgtg gcgcgcctcc    480
   acctgctaga ccaggtgttt ttccaggagc tgctgaagac agcccgcagc agcaaggcct    540
   tcccagagga tgtggtcagg gtcatcttct ccaacatctc ctccatctat cagttccatt 600
   ctcagttctt cctcccagag ctgcagcggc gcctggacga ctggacagct aacccccgca 660
   tcggtgacgt gatccagaag ctggccccct tcctgaagat gtacagtgag tatgtcaaga 720
   actttgagcg agcggctgag ctgctggcca cctggaccga caagtctcca ctcttccagg 780
   aggttctcac tcgcatccag agcagcgagg cttcgggcag cctgaccctg cagcaccaca 840
   tgctggaacc agtgcagaga attccacgtt acgagctgct gctcaaggag tacatccaga 900
   agctgccagc ccaggcccca gaccaggccg atgcccagaa agccctggac atgatcttct 960
   cagctgccca gcactccaat gcagccatca ctgagatgga gcggctgcag gacctgtggg 1020
   aggtgtacca gcgcctgggc ctcgaggacg acatagtaga cccctctaac accctgctcc 1080
   gtgagggccc ggtcctcaag atctccttcc gccgcaacga ccccatggag cgctaccttt 1140
   tcttgttcaa caacatgctg ctctactgtg tgcccagggt gatccaggtg ggcgcccagt 1200
   tccaggtgag gacccgcatc gatgtggccg ggatgaaggt gcgggagctg atggatgctg 1260
   agtttcccca ctccttcctg gtgtccggga agcagcgcac cctggagctg caagcccggt 1320
   cccaggagga aatgatttcc tggatgcagg ccttccaagc agccattgac caaatcgaga 1380
   agcggaatga aaccttcaag gctgcggccc aggggcctga gggagacacc caggagcagg 1440
   agctgcagtc tgaggagctg ggcctccggg caccgcagtg ggtccgggac aagatggtga 1500
   ccatgtgcat gcgctgccag gagcccttca acgctctgac gcgccgtcgc caccactgcc 1560
   gggcctgcgg ctatgtggtg tgtgccaggt gctcccacta ccgggccgaa ctgaaatacg 1620
   acgacaacag gcccaaccga gtctgcctcc actgctacgc attcctcact ggaaatgtgc 1680
   tgcctgaggc caaggaggac aagaggcggg gcatcctgga gaaagggtcc tcagccacgc 1740
   ctgaccagag cctgatgtgc agcttcctgc agctcatcgg ggacaagtgg ggcaagagcg 1800
   gcccccgggg ctggtgtgtg atccctcggg atgaccccct cgtgctctat gtctacgctg 1860
   cccctcagga catgagggct cacacctcca tccccctgct gggctaccag gtgactgttg 1920
   ggccccaggg ggaccctcgg gtcttccagt tacagcagtc aggccagctc tacaccttca 1980
   aggccgagac ggaggagctg aagggccgct gggtgaaggc catggagcgg gcggccagtg 2040
   gctggagccc cagctggccc aacgacgggg acctgtccga ctgagccact gccagccgct 2100
   ctcctgccca cctctcccca ccctgaaccc agctcctgcc acagactgac cctgtggcct 2160
   cagtgaccca ctgccccaag tggtgctttc agagaattga ttcagccatc tgcgcccagg 2220
   ccacgtgtcc caatctggga ttagaaaata tgggtccatt cctttctaga aaggggacaa 2280
   ccaagtgtct cagtttgcct tgtggggagg gggctcctgg gccttgggac ttccagtgct 2340
   aaaactagga aagccccagg taaccccgga ctggtggtca ccatagtgtg gtttttcatt 2400
   tgtatctcct ggggagcttt taaagagtac tggtgaaaaa cacatagtaa attaatttta 2460
   aaaatgt 2467
<210> 66
   <211> 2401
   <212> DNA
   <213> Fgd2 Homo sapiens; transcript OTTHUMG00000014616
<400> 66
   tctttcttca ctctgaagcc aagagccgac cttctgagcc ctcaagaaag atcagaacag 60
   attcatgggt gatttagcct atctgtccca ggccagcgtg gctgagtgtg ctggctggag 120
   gcctctctct ctgcttcgag ggtagctgag atccaccccg gaaaccggca ggatgaaggg 180
   ggcaagtgag gagaagctgg catctgtgtc caacctggtc actgtgtttg agaatagcag 240
   gaccccagaa gcagcaccca gaggccagag gctagaggac gtgcatcacc gccctgagtg 300
   caggcctccc gagtccccag gaccacggga gaagacgaat gtcggggagg ccgtggggtc 360
   tgagcccagg acagtcagca ggaggtacct gaactccctg aagaacaagc tgtccagcga 420
   agcctggagg aaatcttgcc agcctgtgac cctctcagga tcggggacgc aggagccaga 480
   gaagaagatc gtccaggagc tgctggagac agagcaggcc tatgtggcgc gcctccacct 540
   gctagaccag gccatgagtg acctgtcgtg gcggctacag gtgtttttcc aggagctgct 600
   gaagacagcc cgcagcagca aggccttccc agaggatgtg gtcagggtca tcttctccaa 660
   catctcctcc atctatcagt tccattctca gttcttcctc ccagagctgc agcggcgcct 720
   ggacgactgg acagctaacc cccgcatcgg tgacgtgatc cagaagctgg cccccttcct 780
   gaagatgtac agtgagtatg tcaagaactt tgagcgagcg gctgagctgc tggccacctg 840
   gaccgacaag tctccactct tccaggaggt tctcactcgc atccagagca gcgaggcttc 900
   gggcagcctg accctgcagc accacatgct ggaaccagtg cagagaattc cacgttacga 960
   gctgctgctc aaggagtaca tccagaagct gccagcccag gccccagacc aggccgatgc 1020
   ccagaaagcc ctggacatga tcttctcagc tgcccagcac tccaatgcag ccatcactga 1080
   gatggagcgg ctgcaggacc tgtgggaggt gtaccagcgc ctgggcctcg aggacgacat 1140
   agtagacccc tctaacaccc tgctccgtga gggcccggtc ctcaagatct ccttccgccg 1200
   caacgacccc atggagcgct accttttctt gttcaacaac atgctgctct actgtgtgcc 1260
   cagggtgatc caggtgggcg cccagttcca ggtgaggacc cgcatcgatg tggccgggat 1320
   gaagatgcac cggaatttct tcaaccagtc ttctgctgag agacacttag attgttttca 1380
   gttgtcagca gctgcaaatg aggctgcagt gaatagcctg gtgcgggagc tgatggatgc 1440
   tgagtttccc cactccttcc tggtgtccgg gaagcagcgc accctggagc tgcaagcccg 1500
   gtcccaggag gaaatgattt cctggatgca ggccttccaa gcagccattg accaaatcga 1560
   gaagcggaat gaaaccttca aggctgcggc ccaggggcct gagggagaca tccaggagca 1620
   ggagctgcag tctgaggagc tgggcctccg ggcaccgcag tgggtccggg acaagatggt 1680
   gaccatgtgc atgcgctgcc aggagccctt caacgctctg acgcgccgtc gccaccactg 1740
   ccgggcctgc ggctatgtgg tgtgtgccag gtgctccgac taccgggccg aactgaaata 1800
   cgacgacaac aggcccaacc gagtctgcct ccactgctac gcattcctca ctggaaatgt 1860
   gctgcctgag gccaaggagg acaagaggcg gggcatcctg gagaaagggt cctcagccac 1920
   gcctgaccag agcctgatgt gcagcttcct gcagctcatc ggggacaagt ggggcaagag 1980
   cggcccccgg ggctggtgtg tgatccctcg ggatgacccc ctcgtgctct atgtctatgc 2040
   tgcccctcag gacatgaggg ctcacacctc catccccctg ctgggctacc aggtgactgt 2100
   tgggccccag ggggaccctc gggtcttcca gctacagcag tcaggccagc tctacacctt 2160
   caaggccgag acggaggagc tgaagggccg ctgggtgaag gccatggagc gggcggccag 2220
   tggctggagc cccagctggc ccaacgatgg ggacctgtcc gactgagcca ctgccagccg 2280
   ctctcctgcc cacctctccc caccctgaac ccagctcctg ccacagactg accctgtggc 2340
   ctcagtgacc cactgcccca agtggtgctt tcagagaatt gattcagcca tctgcgccca 2400
   g 2401
<210> 67
   <211> 5248
   <212> DNA
   <213> Tiam2 macacca mulatta transcript variant 1; ENSMMUT00000015612
<400> 67
   atgggcaact ccgagagtca gtacaccctt caaggatcta aaaatcatag caatactatc 60
   actggtgcta agcaaattcc ttgctccctg aaaatacgtg ggattcatgc aaaagaggaa 120
   aagtcattgc atggatgggt tcacggaagc agcggagcag gttacaagtc caggtccctg 180
   gcccgaagct gcctttctca ctttaagagt aaccagcctt acgcatccag actcggtggc 240
   tccacgtgca aggtctccag aggcgttgcc tactccacgc acaggacaaa tgccccaggg 300
   aaggatttcc agggcatcag tgctgctttc tcaactgaga acggcttcca ctctgttggc 360
   catgagccgg cagataacca catcacctcc agagactgca atggacacct tctcaactgc 420
   tacgggagga atgagagcgt tgcctccacc ccgccgggcg aagaccgcaa gagccccaga 480
   gtgctcatca aaacgctggg gaagctggat gggtgtttaa gggtcgagtt ccacaatggt 540
   ggcaacccca gcaaagtgcc tgcagaggac tccagtgagc cagtgcagct gctgaggtac 600
   tcacctacct tagcatcaga aacctcccca gtgcctgaag ccaggagggg gtccagcgcc 660
   gattccctgc ccagccatcg cccctctccc acggactctc gcctgcggtc cagcaaaggc 720
   agctccctga gttctgagtc atcctggtac gactcccctt ggggcaacgc tggagagctg 780
   agcgaggctg agggctcctt cctggccccc ggcatgcctg accccagcct ccatgccagc 840
   ttcccacctg gtgatgccaa aaagcctttc aaccaaagct cttccctctc ctcccttcgg 900
   gaactgtaca aagatgccaa cttggggagc ctgtccccct caggtatccg tctttctgat 960
   gaatacatgg gcacgcatgc cagcctgagc aaccgcgtct cttttgcctc tgacattgat 1020
   gtgccctcca gggtggcaca cagggacccc gtccagtaca gttccttcac tctcccctgt 1080
   cggaagccca aagccttagt tgaggatact gcaaagaagg actccctcaa agccaggatg 1140
   cgacggatca gtgactggac gggaagcctc tcaaggaaga aaaggaaact ccaggagccg 1200
   aggtccaagg agggcagtga ctactttgac agtcgctctg atggactgaa tacagaagtg 1260
   caggggccct cccaggcgtc tgcttttctg tggtcagggg gctctgctca aatcctgtct 1320
   cagagaagtg aatccacgca tgcaattggc agcgatcccc tccgacagaa catttatgag 1380
   aatttcatgc gagagttgga aatgagcagg accaacactg agaacataga aatgtctaca 1440
   gaaaccgccg agtccagcag cgagtcgctc agctctctgg aacagctgga tctgctcttt 1500
   gagaaggaac agggggtggt ccggaaagcc gggtggctct tcttcaagcc cctggtcact 1560
   gtgcagaagg aaaggaagct cgagctggtg gcacgaagga aatggaaaca gtactgggta 1620
   acgctgaaag gatgcacgct gctgttttat gagacctatg ggaagaattc catggatcag 1680
   agcagtgccc ctcggtgtgc tctgtttgca gaagacagca tagtgcaggc tgttccagaa 1740
   catcccaaga aagaaaacgt gttctgcctc agcaactcct ttggagacgt ctaccttttc 1800
   caggccacca gccagacaga tctagaaaac tgggtcaccg ccgtacactc tgcttgtgca 1860
   tccctttttg caaagaagca tgggaaagag gacacactgc ggctactgaa gaaccagacc 1920
   aaaaacctgc ttcagaagat agacatggac agcaagatga agaagatggc agagctgcag 1980
   ctgtccattg tgagcgaccc aaagaacagg aaagccatag agaaccagat ccagcaatgg 2040
   gagcagaatc ttgagaaatt tcacatggat ctgttcagga tgcgctgcta tctggccagc 2100
   ctacaaggcg gagagttacc gaaccccaag agtctccttg cagccgccag ccgcccctcc 2160
   aagctggccc tcggcaggct gggcatcttg tctgtttcct ctttccatgc tctggtatgt 2220
   tctagagatg actctgctct ccggaaaagg acactgtcac tgacccagcg agggagaaac 2280
   aagaagggaa tattttcttc gttaaaaggg ctggacacac tggccagaaa agggaaggag 2340
   aagagacctt ctataactca gatatttgat ttaagtggca gccatggatt ttctggaact 2400
   cggctacctc aaaactccaa taactccagt gaggtcgacg aacttctgca tatgtatggt 2460
   tcaacagtag acagtgttcc ccgagacaat gcgtgggaaa tccagactta tgtccacttt 2520
   caggataatc atggagttac tgtagggatc aagccagagc acagagtaga agatattttg 2580
   actctggcat gcaagatgag gcaattggaa cccagccatt atggcctaca acttcgaaaa 2640
   ttagtagatg acaacgttga gtactacatc cctgcaccat atgaatatat gcaagaacag 2700
   gtttatgatg aaatagaagt ctttccacta aatgtttatg atgtgcagct cacgaagact 2760
   gggagtgtgt gtgactttgg gtttgcagtt acagcgcagg tggatgagca tcagcatctc 2820
   agccggatat ttgtaagcga cgttcttccc gatggcctgg cgtatgggga agggctgaga 2880
   aagggcaatg agatcatgac cttaaatggg gaagctgtgt ctgatcttga cctcaagcag 2940
   atggaggctc tattttctga gaagagcgtc ggactcactc tgattgcccg gcctccggac 3000
   acaaaagcca ccctgtgtac atcctggtca gacagtgacc tgttctccag ggaccagaag 3060
   agtctgctac cccctcctaa ccagtctcaa ctgctggagg aattcctgga taactttaaa 3120
   aagaatacag ccaatgattt cagcaacgtc cctgatatca caacaggtct gaaaaggagt 3180
   cagacagatg gcactctgga tcaggtttcc cacagggaga aaatggagca gacattcagg 3240
   agtgctgagc agatcactgc actgtgcagg agttttaacg acactcaggc caacggcatg 3300
   gaaggaccgc gggagagtca ggatcctcct ccgaggcctc tggcccgcca cctctctgat 3360
   gcagaccgcc tccgcaaagt catccaggag cttgtggaca cagagaagtc ctacgtgaag 3420
   gatctgagct gcctctttga gttatacttg gagccacttc agaatgagac ctttcttact 3480
   caagatgaga tggagtcact ttttggaagt ttgccagaga tgcttgagtt tcagaaggtg 3540
   tttctggaga ccctggagga tgggatttca gcatcatctg actttaacac cctagaaacc 3600
   ccctcacagt ttagaaaatt actgttttcc cttggaggct ctttccttta ttacgcggac 3660
   cactttaaac tgtacagtgg attctgtgct aaccatatca aagtacagaa ggttctggag 3720
   cgagctaaaa ctgacaaagc cttcaaggct tttctggacg cccggaaccc caccaagcag 3780
   cattcctcca cgctggagtc ctacctcatc aagccggttc agagagtgct caagtacccg 3840
   ctgctgctca aggagctggt gtccctgacg gaccaggaga gcgaggaaca ttaccacctg 3900
   acggaagcac taaaggcaat ggagaaagta gcgagccaca tcaacgagat gcagaagatc 3960
   tatgaggatt atgggaccgt gtttgaccag ctagtagctg agcagagcgg aaccgagaag 4020
   gaggtaacag aactttcgat gggagagctt ctgatgcact ctacggtttc ctggctgaat 4080
   ccatttctgt ctctaggaaa agctagaaag gaccttgagc tcacagtatt tgtttttaag 4140
   agagccgtca tactggttta taaagaaaac tgcaaactga aaaagaaatt gccctcgaat 4200
   tcccggcctg cacacaactc tgctgacttg gacccattta aattccgctg gttgatcccc 4260
   atctccgcgc ttcaagtcag actggggaat ccagcaggga cagaaaataa ttccatatgg 4320
   gaactgatcc atacgaagtc agaaatagaa ggacggccag aaaccatctt tcagttgtgt 4380
   tgcagtgaca gtgaaagcaa aaccaacatt gttaaggtga ttcgttctat cctgagggaa 4440
   aacttcaggc gtcacataaa gtgtgaatta ccactggaga aaacatgtaa ggatcgcctg 4500
   gtacctctta agaaccgagt tcctgtttca gctaaattag cttcatccag gtctttaaaa 4560
   gtcctgaaga attcctccag caacgagtgg accggtgaga ctggcaaggg aacctcgctg 4620
   gactcagacg agggcagctt gagcagcggc acccagagca gcggctgccc cacggccgag 4680
   ggcaggcagg actccaagac tgcttctccc gggaaatacc cacaccctgg cttggcagat 4740
   tttgcggaca atctcatcaa agagagtgac atcctgagcg atgaagatga tgaccaccat   4800
   caaactctga agcggggcag ccctactaaa gacatcgaaa ttcagttcca gagactgagg   4860
   atttctgagg acccagacgt tcaccccgag gctgagcagc agcctggccc ggaggcgggc   4920
   gagggccaga aaggaggaga gcagcccaaa ctggtccggg ggcacttctg ccccatcaaa   4980
   cgaaaagcca acagcaccaa gagggacaga ggaactttgc tcaaggcaca gctccgtcac   5040
   cagtcccttg acagtcaatc tgaaaatgcc accatcgatc taaattctgt tctagagcga   5100
   gaattcagtg tccagagttt aacatctgtt gtcaatgagg agtgttttta tgaaacagag   5160
   agccatggaa aatcatagta cgattcaatc cagatatggg ttaaattcct cattaaatct   5220
   tttaaactgg tggtaaagtg gaaattgc                                      5248
<210> 68
   <211> 6386
   <212> DNA
   <213> Tiam2 macacca mulatta transcript variant 2; ENSMMUT00000015613
<400> 68
   atgggcaact ccgagagtca gtacaccctt caaggatcta aaaatcatag caatactatc     60
   actggtgcta agcaaattcc ttgctccctg aaaatacgtg ggattcatgc aaaagaggaa    120
   aagtcattgc atggatgggt tcacggaagc agcggagcag gttacaagtc caggtccctg    180
   gcccgaagct gcctttctca ctttaagagt aaccagcctt acgcatccag actcggtggc    240
   tccacgtgca aggtctccag aggcgttgcc tactccacgc acaggacaaa tgccccaggg    300
   aaggatttcc agggcatcag tgctgctttc tcaactgaga acggcttcca ctctgttggc    360
   catgagccgg cagataacca catcacctcc agagactgca atggacacct tctcaactgc    420
   tacgggagga atgagagcgt tgcctccacc ccgccgggcg aagaccgcaa gagccccaga    480
   gtgctcatca aaacgctggg gaagctggat gggtgtttaa gggtcgagtt ccacaatggt    540
   ggcaacccca gcaaagtgcc tgcagaggac tccagtgagc cagtgcagct gctgaggtac    600
   tcacctacct tagcatcaga aacctcccca gtgcctgaag ccaggagggg gtccagcgcc    660
   gattccctgc ccagccatcg cccctctccc acggactctc gcctgcggtc cagcaaaggc    720
   agctccctga gttctgagtc atcctggtac gactcccctt ggggcaacgc tggagagctg    780
   agcgaggctg agggctcctt cctggccccc ggcatgcctg accccagcct ccatgccagc    840
   ttcccacctg gtgatgccaa aaagcctttc aaccaaagct cttccctctc ctcccttcgg    900
   gaactgtaca aagatgccaa cttggggagc ctgtccccct caggtatccg tctttctgat    960
   gaatacatgg gcacgcatgc cagcctgagc aaccgcgtct cttttgcctc tgacattgat   1020
   gtgccctcca gggtggcaca cagggacccc gtccagtaca gttccttcac tctcccctgt   1080
   cggaagccca aagccttagt tgaggatact gcaaagaagg actccctcaa agccaggatg   1140
   cgacggatca gtgactggac gggaagcctc tcaaggaaga aaaggaaact ccaggagccg 1200
   aggtccaagg agggcagtga ctactttgac agtcgctctg atggactgaa tacagaagtg 1260
   caggggccct cccaggcgtc tgcttttctg tggtcagggg gctctgctca aatcctgtct 1320
   cagagaagtg aatccacgca tgcaattggc agcgatcccc tccgacagaa catttatgag 1380
   aatttcatgc gagagttgga aatgagcagg accaacactg agaacataga aatgtctaca 1440
   gaaaccgccg agtccagcag cgagtcgctc agctctctgg aacagctgga tctgctcttt 1500
   gagaaggaac agggggtggt ccggaaagcc gggtggctct tcttcaagcc cctggtcact 1560
   gtgcagaagg aaaggaagct cgagctggtg gcacgaagga aatggaaaca gtactgggta 1620
   acgctgaaag gatgcacgct gctgttttat gagacctatg ggaagaattc catggatcag 1680
   agcagtgccc ctcggtgtgc tctgtttgca gaagacagca tagtgcaggc tgttccagaa 1740
   catcccaaga aagaaaacgt gttctgcctc agcaactcct ttggagacgt ctaccttttc 1800
   caggccacca gccagacaga tctagaaaac tgggtcaccg ccgtacactc tgcttgtgca 1860
   tccctttttg caaagaagca tgggaaagag gacacactgc ggctactgaa gaaccagacc 1920
   aaaaacctgc ttcagaagat agacatggac agcaagatga agaagatggc agagctgcag 1980
   ctgtccattg tgagcgaccc aaagaacagg aaagccatag agaaccagat ccagcaatgg 2040
   gagcagaatc ttgagaaatt tcacatggat ctgttcagga tgcgctgcta tctggccagc 2100
   ctacaaggcg gagagttacc gaaccccaag agtctccttg cagccgccag ccgcccctcc 2160
   aagctggccc tcggcaggct gggcatcttg tctgtttcct ctttccatgc tctggtatgt 2220
   tctagagatg actctgctct ccggaaaagg acactgtcac tgacccagcg agggagaaac 2280
   aagaagggaa tattttcttc gttaaaaggg ctggacacac tggccagaaa agggaaggag 2340
   aagagacctt ctataactca ggtcgacgaa cttctgcata tgtatggttc aacagtagac 2400
   agtgttcccc gagacaatgc gtgggaaatc cagacttatg tccactttca ggataatcat 2460
   ggagttactg tagggatcaa gccagagcac agagtagaag atattttgac tctggcatgc 2520
   aagatgaggc aattggaacc cagccattat ggcctacaac ttcgaaaatt agtagatgac 2580
   aacgttgagt actacatccc tgcaccatat gaatatatgc aagaacaggt ttatgatgaa 2640
   atagaagtct ttccactaaa tgtttatgat gtgcagctca cgaagactgg gagtgtgtgt 2700
   gactttgggt ttgcagttac agcgcaggtg gatgagcatc agcatctcag ccggatattt 2760
   gtaagcgacg ttcttcccga tggcctggcg tatggggaag ggctgagaaa gggcaatgag 2820
   atcatgacct taaatgggga agctgtgtct gatcttgacc tcaagcagat ggaggctcta 2880
   ttttctgaga agagcgtcgg actcactctg attgcccggc ctccggacac aaaagccacc 2940
   ctgtgtacat cctggtcaga cagtgacctg ttctccaggg accagaagag tctgctaccc 3000
   cctcctaacc agtctcaact gctggaggaa ttcctggata actttaaaaa gaatacagcc 3060
   aatgatttca gcaacgtccc tgatatcaca acaggtctga aaaggagtca gacagatggc 3120
   actctggatc aggtttccca cagggagaaa atggagcaga cattcaggag tgctgagcag 3180
   atcactgcac tgtgcaggag ttttaacgac actcaggcca acggcatgga aggaccgcgg 3240
   gagagtcagg atcctcctcc gaggcctctg gcccgccacc tctctgatgc agaccgcctc 3300
   cgcaaagtca tccaggagct tgtggacaca gagaagtcct acgtgaagga tctgagctgc 3360
   ctctttgagt tatacttgga gccacttcag aatgagacct ttcttactca agatgagatg 3420
   gagtcacttt ttggaagttt gccagagatg cttgagtttc agaaggtgtt tctggagacc 3480
   ctggaggatg ggatttcagc atcatctgac tttaacaccc tagaaacccc ctcacagttt 3540
   agaaaattac tgttttccct tggaggctct ttcctttatt acgcggacca ctttaaactg 3600
   tacagtggat tctgtgctaa ccatatcaaa gtacagaagg ttctggagcg agctaaaact 3660
   gacaaagcct tcaaggcttt tctggacgcc cggaacccca ccaagcagca ttcctccacg 3720
   ctggagtcct acctcatcaa gccggttcag agagtgctca agtacccgct gctgctcaag 3780
   gagctggtgt ccctgacgga ccaggagagc gaggaacatt accacctgac ggaagcacta 3840
   aaggcaatgg agaaagtagc gagccacatc aacgagatgc agaagatcta tgaggattat 3900
   gggaccgtgt ttgaccagct agtagctgag cagagcggaa ccgagaagga ggtaacagaa 3960
   ctttcgatgg gagagcttct gatgcactct acggtttcct ggctgaatcc atttctgtct 4020
   ctaggaaaag ctagaaagga ccttgagctc acagtatttg tttttaagag agccgtcata 4080
   ctggtttata aagaaaactg caaactgaaa aagaaattgc cctcgaattc ccggcctgca 4140
   cacaactctg ctgacttgga cccatttaaa ttccgctggt tgatccccat ctccgcgctt 4200
   caagtcagac tggggaatcc agcagggaca gaaaataatt ccatatggga actgatccat 4260
   acgaagtcag aaatagaagg acggccagaa accatctttc agttgtgttg cagtgacagt 4320
   gaaagcaaaa ccaacattgt taaggtgatt cgttctatcc tgagggaaaa cttcaggcgt 4380
   cacataaagt gtgaattacc actggagaaa acatgtaagg atcgcctggt acctcttaag 4440
   aaccgagttc ctgtttcagc taaattagct tcatccaggt ctttaaaagt cctgaagaat 4500
   tcctccagca acgagtggac cggtgagact ggcaagggaa cctcgctgga ctcagacgag 4560
   ggcagcttga gcagcggcac ccagagcagc ggctgcccca cggccgaggg caggcaggac 4620
   tccaagactg cttctcccgg gaaataccca caccctggct tggcagattt tgcggacaat 4680
   ctcatcaaag agagtgacat cctgagcgat gaagatgatg accaccatca aactctgaag 4740
   cggggcagcc ctactaaaga catcgaaatt cagttccaga gactgaggat ttctgaggac 4800
   ccagacgttc accccgaggc tgagcagcag cctggcccgg aggcgggcga gggccagaaa 4860
   ggaggagagc agcccaaact ggtccggggg cacttctgcc ccatcaaacg aaaagccaac 4920
   agcaccaaga gggacagagg aactttgctc aaggcacagc tccgtcacca gtcccttgac 4980
   agtcaatctg aaaatgccac catcgatcta aattctgttc tagagcgaga attcagtgtc 5040
   cagagtttaa catctgttgt caatgaggag tgtttttatg aaacagagag ccatggaaaa 5100
   tcatagtacg attcaatcca gatatgggtt aaattcctca ttaaatcttt taaactggtg 5160
   gtaaagtgga aattgcaaaa aaactattca ttcctgggtt ttgtgcagta tacattttcc 5220
   cacgaaatgg ttgtaaagat ttaagttatt ttaatttatt gtggatcaga aacctagaag 5280
   aaactggtca gaatctgtaa attacttagt ttatatccac tttgagcagg tatcaaatga 5340
   tttagaatcc ttaaaattgc cttctaatta ttttaagtta tgtggaaaaa gtaaggctgg 5400
   ggaagttgtg attaatagtt ttcaaagggt cattttttaa aatcctctgg gcattttctt 5460
   tcagctgtca gtttttgctt tatttaaagc atatttaagt tattttaatg tggtttaggg 5520
   gcaaaatgtg cagatacttc atttttgtaa gattgtaata gatgctgttt atactaaacc 5580
   tgttgtatct atacagtata tattaaaaga aagcttgtac tgtaccttat ttgatgatat 5640
   ttattttctc tgccaagctg tatagtaaaa ggaaagtaag tcacatctgg tcattggcat 5700
   ttttattgtc attctgtaaa gacaaaaggg tacccatata agaagctcca cgcagtgcaa 5760
   atcgacatct ggtaggctgc tcgcccccag gcagcagcta gagtctgtaa ctcttcgcgt 5820
   catcctgttt ttctttcttt ttgatttttc tttgcctgag ttcttctctg aaattatatg 5880
   caaagagttg tgggtcttca tcacacattc ttctgtatac ctcacagagg cttctaaagt 5940
   gtgagatgga gagctggcga ggccgaagag tagggtctat gtctgccaac tctaacagcc 6000
   tgcccgtgtt ttccaagcgc tgcgcttcag ggaataacat tctgagccct cgatggcagt 6060
   atttccttcg gaactgaaat acattctgaa ccactttttc caccagcttg aacggctgct 6120
   ctatcttggg ctgtatcaag ggagtgaagt gcaccacgcc cacgtccacc ttcattgtaa 6180
   gcaaacatat tatcattctg tggcatgata tgtggcatag cgtgatcaat cagctcatcc 6240
   ttgttaaaca ggaggatggg ctgtcaacag cctgttttca taaaccgact cttccatgaa 6300
   cttcagtgtc atctctaggc atggaagatg gtacattctg gatttgcaaa tgacatggag 6360
   aaatcagctg gctgcacctg ttctct 6386
<210> 69
   <211> 5193
   <212> DNA
   <213> Tiam2 Monodelphis domesticus transcript variant 1; ENSMODT00000030824
<400> 69
   gttacgatgg gaaactctga gagtcaatac agtctccaag gatctaaaaa tcatagtagt     60
   tcttctactg gtgctaaaca gaagccatgt cccctgaaaa tccgcagcat ccatgcaaaa    120
   gatgaaaagt cttgctcatt gcatgggtgg ggtcatggaa acagtgggac aaattacaag    180
   tctcgctccc tggccaggag ctgcctttct cactttaaga gtaaccagcc ttattcatcc    240
   agactcgatc cgatggccaa agtctccaaa ggcagtgccc acacaaagca caaagggaat    300
   gtgtcaggaa actatttcca agggaagaat tctgccttct tgtctgaaaa tggctttcat 360
   tatattagtc atgaaccagc agataaccac atcacctcca gagactgcaa tggacacctt 420
   ctgaactgtt atgggaaaaa tgaaagtctt gcatcaacac caccagctga agacagaaag 480
   agccccaagg ttctaattaa aacactaggg aaactggatg gatgtttaag ggttgagttc 540
   cacaatagca gcaacaacaa agtgcctaca gaggactcca ctggaccagt ccagcttttg 600
   agatattccc ctaccttaga gtctgaaact tccaatctaa ctgaaatcag gagaaattct 660
   agcaccgatt attctgccag ccattgcctt tctcctactg attcccggct gagatctagc 720
   aaaggcagct cttttagctc cgagtcttcc tggtatgact ccccttgggg aaatgctgga 780
   gacatcaatg agttggaggg accctacctg accagaagca ctccagatac cagcatccac 840
   tccagttttc catccgatga tgccaaaaag cctttcaacc aaagttcatc tctttcttcc 900
   ctccgggaac tgtacaaaga tgccacctta gaaagtcttc caccctcggg cattagattt 960
   tctgatgaat acattggcac acatgccagt ttaaatagta gagtttcatt tgtctcagac 1020
   attgatgtgc cctcaagagc agagcagaga ggccctgtac agtactgttc ttataccctt 1080
   ccctgtcgga agtccaaacc cttaacagaa gatacttcca aaaaggacac gttaaaaact 1140
   agaatgaggc gaatcagtga ttggacagga agtctctcaa gaaagaaaag gaaactgcag 1200
   gagcccaaat ccaaggatgg atatgaccat ttggaaaacc gaacagatgg tctcagtgca 1260
   gaagtgctgg tcccttctca gctgtctagt ttactgtggc caggtggatc tggccagacc 1320
   ctgcctcaga ggagtgagtc tactaatgca attagcagcg atcccttgcg acagaacatc 1380
   tatgaaaatt tcatgcggga gttggagatg agtaggactc atgtggataa cacagaaaca 1440
   tctacagaca ctgcagattc tagtagtgat tcacttagct ctttagagca actggacctg 1500
   ctctttgaga aggaacaagg agtggtccgg aaagcagggt ggctcttttt caagcctctg 1560
   gtcactcttc agaaagagaa gaagctagag cttgtggcac ggaggaaatg gaaacagtac 1620
   tgggtcactc ttaaaggctg taccctgctg ttctatgaga catatgggag aaattccatg 1680
   gatcaaagta gttcacctag atgtgctctg tttgcggaag atagcgtagt gcaatctgtt 1740
   cctgaacatc ccaagaagga aaatgtattc tgcctcagta actcctttgg agatgtctac 1800
   ctttttcagg ccactagcca gactgacctg gaaaactggg tgactgccat ccattctgct 1860
   tgtgcttctc tttttgcaaa gaagcatggg aaagaagaca cagtccggtt gttgaagaat 1920
   cagaccaaaa atctactcca aaagattgac atggatagca agatgaaaaa aatggctgag 1980
   ttgcagctct ctgtggttag tgaccctaag aacagaaaag ccatagaaaa tcagatccag 2040
   cagtgggagc agaatctaga aaaatttcac atggatctct tcaggatgcg ctgctatttg 2100
   gctagtctac aaggtggaga gttaccaaat cccaagagtc tccttgctgc cacaagtcgt 2160
   ccttctaaac tggcactggg cagattgggc atcttatcag tttcttcctt ccatgctttg 2220
   atatgttcca gagatgaatc agctctgagg aaacgaacat tatcattgac tcagcaaggt 2280
   caaagtaaga agggattgtt ttcatcactg aaaggactgg atactttggc aagaaaaggg 2340
   aaagacaaaa ggccatccat aactcagatt tttgattcaa gtggcaatca tggatttccc 2400
   ggaactcagc tgcctcagag ctccaataat tccagtgagg tggatgaatt tcaacatatt 2460
   tatagttctg catcagacag tggtccccga gaaaacattt gggaaagcca gacttatgtt 2520
   cattttcagg ataatcaagg agttactcta atcatcaaac cagaacatag agtggaagat 2580
   attttgtctc tatcatgcaa gatgaagcaa ctagacccca gctactatgg cctacaactt 2640
   agaagactgg ttgatgacaa ctttgagtat tcaattccag ctctttatga atacatgcaa 2700
   gaacaggcaa ttgtttatga tgaaatagaa atcttccctc tcagtattta tcatgtactt 2760
   cttactaaga ctgggaacat aacagatttt gggtttgcag ttacagctca agttgatgaa 2820
   caccagcacc tcagccgaat atttataagt gatgttctcc ctgatggcct ggcatatggg 2880
   gaagggttga gaacaggcaa tgagatcttg actataaatg gggaagtcat ttctgacctt 2940
   gaccttaggc agatggagtc attattttct gagagaagtg tgaggctcac cctgagaacc 3000
   aattcttcag atacgaagcg aactttgggt acctcctggt cagacagcga cctttccagg 3060
   gaccagaaaa acctgctccc ccctcccaac caatcacagc ttctggaaga gtttttggat 3120
   aatcttaaga agaacacagc aaatgatttc agcaatgttc ctgatgttac agctggtttg 3180
   aaaagaagtc aaactgatgg taccttggac caagttcccc acagggagaa aactgaacat 3240
   acattcagga gtgctgaaca aattgctgct ctgtgtagga gctttaacga ggttcagcca 3300
   agcacagaca gcatggaagg gcccaaagag gcacaggagc cacccccaag gccattggct 3360
   cggcacctct ctgacgcaga tcgactgaga aaagttatcc aggaactcat ggacacggag 3420
   aagtcctatg tcaaggatct gacctgcctt tttgaattat acttagaacc acttcagaat 3480
   gagacatttc ttacccaaga tgaaatggaa tctctttttg gaagtctgcc agaaatgctg 3540
   gagtttcaaa aggtgttttt ggagaccctg gaagatggca tttcttcatc ctctgacttc 3600
   aacatacttg aaactccctc ccagttcaga aaattactct tctcccttgg aggctctttc 3660
   ctttattata cagaccactt taaattgtac agtggatttt gtgccaatca tatcaaagta 3720
   caaaaagttc tagagagagc gaaaacagat aaagcattta aggcgttctt ggatgctcga 3780
   aaccctacca agcagcatgc ttccaccttg gagtcatacc tcatcaagcc tgttcagaga 3840
   gttctgaagt accctctgtt gctcaaagag ttggtgtcac tgacagacaa tgaaagcgag 3900
   gaacattatc acctgacaga agcactaaag gcaatggaaa aggtagcaag tcatatcaat 3960
   gaaatgcaga aaatctatga agattatggc actgtgtttg atcagctggt agcagagcag 4020
   agtgggacag agaaggaggt tacagaactt tccatggggg agctcctgct tcactctaca 4080
   gtttcctggc tgaatccctt tctgtccctt ggaaaagcaa ggaaggatct cgagcttacc 4140
   gtgtttgttt ttaagagagc cgtcatactg gtttataaag aaaactgcaa actgaagaag 4200
   aaattgcctt ccagttcccg acctacacat gttcacggtg acttggaccc cttcaaattc 4260
   cggtggctaa ttcctttatc tgctcttcaa gtcagactgg gaaatgcagc agggacagaa 4320
   aataattcta tctgggagct gatccacacc aagtcggaga tagaagggcg gccggagaca 4380
   acctttcagt tatgttgcag tgactgtgaa agtaaaacca acatcgtgaa ggtgatccgt 4440
   tccatcctga gggagaattt cagacgtcac ataaagagtg aattgccact agagaagacg 4500
   tgtaaagatc gcttgatacc actcaaaaat cgcattcctg tctcagccaa attagcttca 4560
   tcaaggtcct taagggtgct aaagaattct cccagcaacg agtggaacag tgatactgga 4620
   aaaggaaact tactggattc tgatgaatgt agcctgagta gtagcacgca aagcagtggc 4680
   tgtcacacta ctgaaagtcg gcaggaatcc aaggattcgt cacctgagaa gtacccacag 4740
   acatgttcat ctgatttttc agacagtctt attaaagagt ctgatatttt gagtgatgaa 4800
   gatgatgatt ttcatcaaac tctgaaaaaa ggcagcccta caaaagacat tgaaattcag 4860
   ttccaaaggc tgaagatctc cgaggactct gagggtgaca gcactgccga tcaacagccc 4920
   agaacagagg ttggaaacaa tcttaacagt gtagcgcatc caaagctggt gcgtggacat 4980
   ttctgtccca ttaagcggaa agcaaacagt accaaaaggg atagggggac tctgttaaca 5040
   ctgcaagcac gccatcagtc tcttgacagt aaatctgaaa atgctaacat tgatttaaat 5100
   tccatactag agagggaatt cagtgtccag agtttaacat ctgtcgttaa tgaggagtgc 5160
   ttttatgaga cagacagcca tggaaaatca tag 5193
<210> 70
   <211> 5121
   <212> DNA
   <213> Tiam2 Monodelphis domesticus transcript variant 2; ENSMODT00000010456
<400> 70
   gttacgatgg gaaactctga gagtcaatac agtctccaag gatctaaaaa tcatagtagt 60
   tcttctactg gtgctaaaca gaagccatgt cccctgaaaa tccgcagcat ccatgcaaaa 120
   gatgaaaagt cttgctcatt gcatgggtgg ggtcatggaa acagtgggac aaattacaag 180
   tctcgctccc tggccaggag ctgcctttct cactttaaga gtaaccagcc ttattcatcc 240
   agactcgatc cgatggccaa agtctccaaa ggcagtgccc acacaaagca caaagggaat 300
   gtgtcaggaa actatttcca agggaagaat tctgccttct tgtctgaaaa tggctttcat 360
   tatattagtc atgaaccagc agataaccac atcacctcca gagactgcaa tggacacctt 420
   ctgaactgtt atgggaaaaa tgaaagtctt gcatcaacac caccagctga agacagaaag 480
   agccccaagg ttctaattaa aacactaggg aaactggatg gatgtttaag ggttgagttc 540
   cacaatagca gcaacaacaa agtgcctaca gaggactcca ctggaccagt ccagcttttg 600
   agatattccc ctaccttaga gtctgaaact tccaatctaa ctgaaatcag gagaaattct 660
   agcaccgatt attctgccag ccattgcctt tctcctactg attcccggct gagatctagc 720
   aaaggcagct cttttagctc cgagtcttcc tggtatgact ccccttgggg aaatgctgga 780
   gacatcaatg agttggaggg accctacctg accagaagca ctccagatac cagcatccac 840
   tccagttttc catccgatga tgccaaaaag cctttcaacc aaagttcatc tctttcttcc 900
   ctccgggaac tgtacaaaga tgccacctta gaaagtcttc caccctcggg cattagattt 960
   tctgatgaat acattggcac acatgccagt ttaaatagta gagtttcatt tgtctcagac 1020
   attgatgtgc cctcaagagc agagcagaga ggccctgtac agtactgttc ttataccctt 1080
   ccctgtcgga agtccaaacc cttaacagaa gatacttcca aaaaggacac gttaaaaact 1140
   agaatgaggc gaatcagtga ttggacagga agtctctcaa gaaagaaaag gaaactgcag 1200
   gagcccaaat ccaaggatgg atatgaccat ttggaaaacc gaacagatgg tctcagtgca 1260
   gaagtgctgg tcccttctca gctgtctagt ttactgtggc caggtggatc tggccagacc 1320
   ctgcctcaga ggagtgagtc tactaatgca attagcagcg atcccttgcg acagaacatc 1380
   tatgaaaatt tcatgcggga gttggagatg agtaggactc atgtggataa cacagaaaca 1440
   tctacagaca ctgcagattc tagtagtgat tcacttagct ctttagagca actggacctg 1500
   ctctttgaga aggaacaagg agtggtccgg aaagcagggt ggctcttttt caagcctctg 1560
   gtcactcttc agaaagagaa gaagctagag cttgtggcac ggaggaaatg gaaacagtac 1620
   tgggtcactc ttaaaggctg taccctgctg ttctatgaga catatgggag aaattccatg 1680
   gatcaaagta gttcacctag atgtgctctg tttgcggaag atagcgtagt gcaatctgtt 1740
   gctgaacatc ccaagaagga aaatgtattc tgcctcagta actcctttgg agatgtctac 1800
   ctttttcagg ccactagcca gactgacctg gaaaactggg tgactgccat ccattctgct 1860
   tgtgcttctc tttttgcaaa gaagcatggg aaagaagaca cagtccggtt gttgaagaat 1920
   cagaccaaaa atctactcca aaagattgac atggatagca agatgaaaaa aatggctgag 1980
   ttgcagctct ctgtggttag tgaccctaag aacagaaaag ccatagaaaa tcagatccag 2040
   cagtgggagc agaatctaga aaaatttcac atggatctct tcaggatgcg ctgctatttg 2100
   gctagtctac aaggtggaga gttaccaaat cccaagagtc tccttgctgc cacaagtcgt 2160
   ccttctaaac tggcactggg cagattgggc atcttatcag tttcttcctt ccatgctttg 2220
   atatgttcca gagatgaatc agctctgagg aaacgaacat tatcattgac tcagcaaggt 2280
   caaagtaaga agggattgtt ttcatcactg aaaggactgg atactttggc aagaaaaggg 2340
   aaagacaaaa ggccatccat aactcaggtg gatgaatttc aacatattta tagttctgca 2400
   tcagacagtg gtccccgaga aaacatttgg gaaagccaga cttatgttca ttttcaggat 2460
   aatcaaggag ttactctaat catcaaacca gaacatagag tggaagatat tttgtctcta 2520
   tcatgcaaga tgaagcaact agaccccagc tactatggcc tacaacttag aagactggtt 2580
   gatgacaact ttgagtattc aattccagct ctttatgaat acatgcaaga acaggcaatt 2640
   gtttatgatg aaatagaaat cttccctctc agtatttatc atgtacttct tactaagact 2700
   gggaacataa cagattttgg gtttgcagtt acagctcaag ttgatgaaca ccagcacctc 2760
   agccgaatat ttataagtga tgttctccct gatggcctgg catatgggga agggttgaga 2820
   acaggcaatg agatcttgac tataaatggg gaagtcattt ctgaccttga ccttaggcag 2880
   atggagtcat tattttctga gagaagtgtg aggctcaccc tgagaaccaa ttcttcagat 2940
   acgaagcgaa ctttgggtac ctcctggtca gacagcgacc tttccaggga ccagaaaaac 3000
   ctgctccccc ctcccaacca atcacagctt ctggaagagt ttttggataa tcttaagaag 3060
   aacacagcaa atgatttcag caatgttcct gatgttacag ctggtttgaa aagaagtcaa 3120
   actgatggta ccttggacca agttccccac agggagaaaa ctgaacatac attcaggagt 3180
   gctgaacaaa ttgctgctct gtgtaggagc tttaacgagg ttcagccaag cacagacagc 3240
   atggaagggc ccaaagaggc acaggagcca cccccaaggc cattggctcg gcacctctct 3300
   gacgcagatc gactgagaaa agttatccag gaactcatgg acacggagaa gtcctatgtc 3360
   aaggatctga cctgcctttt tgaattatac ttagaaccac ttcagaatga gacatttctt 3420
   acccaagatg aaatggaatc tctttttgga agtctgccag aaatgctgga gtttcaaaag 3480
   gtgtttttgg agaccctgga agatggcatt tcttcatcct ctgacttcaa catacttgaa 3540
   actccctccc agttcagaaa attactcttc tcccttggag gctctttcct ttattataca 3600
   gaccacttta aattgtacag tggattttgt gccaatcata tcaaagtaca aaaagttcta 3660
   gagagagcga aaacagataa agcatttaag gcgttcttgg atgctcgaaa ccctaccaag 3720
   cagcatgctt ccaccttgga gtcatacctc atcaagcctg ttcagagagt tctgaagtac 3780
   cctctgttgc tcaaagagtt ggtgtcactg acagacaatg aaagcgagga acattatcac 3840
   ctgacagaag cactaaaggc aatggaaaag gtagcaagtc atatcaatga aatgcagaaa 3900
   atctatgaag attatggcac tgtgtttgat cagctggtag cagagcagag tgggacagag 3960
   aaggaggtta cagaactttc catgggggag ctcctgcttc actctacagt ttcctggctg 4020
   aatccctttc tgtcccttgg aaaagcaagg aaggatctcg agcttaccgt gtttgttttt 4080
   aagagagccg tcatactggt ttataaagaa aactgcaaac tgaagaagaa attgccttcc 4140
   agttcccgac ctacacatgt tcacggtgac ttggacccct tcaaattccg gtggctaatt 4200
   cctttatctg ctcttcaagt cagactggga aatgcagcag ggacagaaaa taattctatc 4260
   tgggagctga tccacaccaa gtcggagata gaagggcggc cggagacaac ctttcagtta 4320
   tgttgcagtg actgtgaaag taaaaccaac atcgtgaagg tgatccgttc catcctgagg 4380
   gagaatttca gacgtcacat aaagagtgaa ttgccactag agaagacgtg taaagatcgc 4440
   ttgataccac tcaaaaatcg cattcctgtc tcagccaaat tagcttcatc aaggtcctta   4500
   agggtgctaa agaattctcc cagcaacgag tggaacagtg atactggaaa aggaaactta   4560
   ctggattctg atgaatgtag cctgagtagt agcacgcaaa gcagtggctg tcacactact   4620
   gaaagtcggc aggaatccaa ggattcgtca cctgagaagt acccacagac atgttcatct   4680
   gatttttcag acagtcttat taaagagtct gatattttga gtgatgaaga tgatgatttt   4740
   catcaaactc tgaaaaaagg cagccctaca aaagacattg aaattcagtt ccaaaggctg   4800
   aagatctccg aggactctga gggtgacagc actgccgatc aacagcccag aacagaggtt   4860
   ggaaacaatc ttaacagtgt agcgcatcca aagctggtgc gtggacattt ctgtcccatt   4920
   aagcggaaag caaacagtac caaaagggat agggggactc tgttaacact gcaagcacgc   4980
   catcagtctc ttgacagtaa atctgaaaat gctaacattg atttaaattc catactagag   5040
   agggaattca gtgtccagag tttaacatct gtcgttaatg aggagtgctt ttatgagaca   5100
   gacagccatg gaaaatcata g                                               5121
<210> 71
   <211> 5193
   <212> DNA
   <213> Tiam2 Monodelphis domesticus transcript variant 3; ENSMODT00000010453
<400> 71
   gttacgatgg gaaactctga gagtcaatac agtctccaag gatctaaaaa tcatagtagt     60
   tcttctactg gtgctaaaca gaagccatgt cccctgaaaa tccgcagcat ccatgcaaaa    120
   gatgaaaagt cttgctcatt gcatgggtgg ggtcatggaa acagtgggac aaattacaag    180
   tctcgctccc tggccaggag ctgcctttct cactttaaga gtaaccagcc ttattcatcc    240
   agactcgatc cgatggccaa agtctccaaa ggcagtgccc acacaaagca caaagggaat    300
   gtgtcaggaa actatttcca agggaagaat tctgccttct tgtctgaaaa tggctttcat    360
   tatattagtc atgaaccagc agataaccac atcacctcca gagactgcaa tggacacctt    420
   ctgaactgtt atgggaaaaa tgaaagtctt gcatcaacac caccagctga agacagaaag    480
   agccccaagg ttctaattaa aacactaggg aaactggatg gatgtttaag ggttgagttc    540
   cacaatagca gcaacaacaa agtgcctaca gaggactcca ctggaccagt ccagcttttg    600
   agatattccc ctaccttaga gtctgaaact tccaatctaa ctgaaatcag gagaaattct    660
   agcaccgatt attctgccag ccattgcctt tctcctactg attcccggct gagatctagc    720
   aaaggcagct cttttagctc cgagtcttcc tggtatgact ccccttgggg aaatgctgga    780
   gacatcaatg agttggaggg accctacctg accagaagca ctccagatac cagcatccac    840
   tccagttttc catccgatga tgccaaaaag cctttcaacc aaagttcatc tctttcttcc    900
   ctccgggaac tgtacaaaga tgccacctta gaaagtcttc caccctcggg cattagattt    960
   tctgatgaat acattggcac acatgccagt ttaaatagta gagtttcatt tgtctcagac   1020
   attgatgtgc cctcaagagc agagcagaga ggccctgtac agtactgttc ttataccctt 1080
   ccctgtcgga agtccaaacc cttaacagaa gatacttcca aaaaggacac gttaaaaact 1140
   agaatgaggc gaatcagtga ttggacagga agtctctcaa gaaagaaaag gaaactgcag 1200
   gagcccaaat ccaaggatgg atatgaccat ttggaaaacc gaacagatgg tctcagtgca 1260
   gaagtgctgg tcccttctca gctgtctagt ttactgtggc caggtggatc tggccagacc 1320
   ctgcctcaga ggagtgagtc tactaatgca attagcagcg atcccttgcg acagaacatc 1380
   tatgaaaatt tcatgcggga gttggagatg agtaggactc atgtggataa cacagaaaca 1440
   tctacagaca ctgcagattc tagtagtgat tcacttagct ctttagagca actggacctg 1500
   ctctttgaga aggaacaagg agtggtccgg aaagcagggt ggctcttttt caagcctctg 1560
   gtcactcttc agaaagagaa gaagctagag cttgtggcac ggaggaaatg gaaacagtac 1620
   tgggtcactc ttaaaggctg taccctgctg ttctatgaga catatgggag aaattccatg 1680
   gatcaaagta gttcacctag atgtgctctg tttgcggaag atagcgtagt gcaatctgtt 1740
   cctgaacatc ccaagaagga aaatgtattc tgcctcagta actcctttgg agatgtctac 1800
   ctttttcagg ccactagcca gactgacctg gaaaactggg tgactgccat ccattctgct 1860
   tgtgcttctc tttttgcaaa gaagcatggg aaagaagaca cagtccggtt gttgaagaat 1920
   cagaccaaaa atctactcca aaagattgac atggatagca agatgaaaaa aatggctgag 1980
   ttgcagctct ctgtggttag tgaccctaag aacagaaaag ccatagaaaa tcagatccag 2040
   cagtgggagc agaatctaga aaaatttcac atggatctct tcaggatgcg ctgctatttg 2100
   gctagtctac aaggtggaga gttaccaaat cccaagagtc tccttgctgc cacaagtcgt 2160
   ccttctaaac tggcactggg cagattgggc atcttatcag tttcttcctt ccatgctttg 2220
   atatgttcca gagatgaatc agctctgagg aaacgaacat tatcattgac tcagcaaggt 2280
   caaagtaaga agggattgtt ttcatcactg aaaggactgg atactttggc aagaaaaggg 2340
   aaagacaaaa ggccatccat aactcagatt tttgattcaa gtggcaatca tggatttccc 2400
   ggaactcagc tgcctcagag ctccaataat tccagtgagg tggatgaatt tcaacatatt 2460
   tatagttctg catcagacag tggtccccga gaaaacattt gggaaagcca gacttatgtt 2520
   cattttcagg ataatcaagg agttactcta atcatcaaac cagaacatag agtggaagat 2580
   attttgtctc tatcatgcaa gatgaagcaa ctagacccca gctactatgg cctacaactt 2640
   agaagactgg ttgatgacaa ctttgagtat tcaattccag ctctttatga atacatgcaa 2700
   gaacaggcaa aagtttatga tgaaatagaa atcttccctc tcagtattta tcatgtactt 2760
   cttactaaga ctgggaacat aacagatttt gggtttgcag ttacagctca agttgatgaa 2820
   caccagcacc tcagccgaat atttataagt gatgttctcc ctgatggcct ggcatatggg 2880
   gaagggttga gaacaggcaa tgagatcttg actataaatg gggaagtcat ttctgacctt 2940
   gaccttaggc agatggagtc attattttct gagagaagtg tgaggctcac cctgagaacc 3000
   aattcttcag atacgaagcg aactttgggt acctcctggt cagacagcga cctttccagg 3060
   gaccagaaaa acctgctccc ccctcccaac caatcacagc ttctggaaga gtttttggat 3120
   aatcttaaga agaacacagc aaatgatttc agcaatgttc ctgatgttac agctggtttg 3180
   aaaagaagtc aaactgatgg taccttggac caagttcccc acagggagaa aactgaacat 3240
   acattcagga gtgctgaaca aattgctgct ctgtgtagga gctttaacga ggttcagcca 3300
   agcacagaca gcatggaagg gcccaaagag gcacaggagc cacccccaag gccattggct 3360
   cggcacctct ctgacgcaga tcgactgaga aaagttatcc aggaactcat ggacacggag 3420
   aagtcctatg tcaaggatct gacctgcctt tttgaattat acttagaacc acttcagaat 3480
   gagacatttc ttacccaaga tgaaatggaa tctctttttg gaagtctgcc agaaatgctg 3540
   gagtttcaaa aggtgttttt ggagaccctg gaagatggca tttcttcatc ctctgacttc 3600
   aacatacttg aaactccctc ccagttcaga aaattactct tctcccttgg aggctctttc 3660
   ctttattata cagaccactt taaattgtac agtggatttt gtgccaatca tatcaaagta 3720
   caaaaagttc tagagagagc gaaaacagat aaagcattta aggcgttctt ggatgctcga 3780
   aaccctacca agcagcatgc ttccaccttg gagtcatacc tcatcaagcc tgttcagaga 3840
   gttctgaagt accctctgtt gctcaaagag ttggtgtcac tgacagacaa tgaaagcgag 3900
   gaacattatc acctgacaga agcactaaag gcaatggaaa aggtagcaag tcatatcaat 3960
   gaaatgcaga aaatctatga agattatggc actgtgtttg atcagctggt agcagagcag 4020
   agtgggacag agaaggaggt tacagaactt tccatggggg agctcctgct tcactctaca 4080
   gtttcctggc tgaatccctt tctgtccctt ggaaaagcaa ggaaggatct cgagcttacc 4140
   gtgtttgttt ttaagagagc cgtcatactg gtttataaag aaaactgcaa actgaagaag 4200
   aaattgcctt ccagttcccg acctacacat gttcacggtg acttggaccc cttcaaattc 4260
   cggtggctaa ttcctttatc tgctcttcaa gtcagactgg gaaatgcagc agggacagaa 4320
   aataattcta tctgggagct gatccacacc aagtcggaga tagaagggcg gccggagaca 4380
   acctttcagt tatgttgcag tgactgtgaa agtaaaacca acatcgtgaa ggtgatccgt 4440
   tccatcctga gggagaattt cagacgtcac ataaagagtg aattgccact agagaagacg 4500
   tgtaaagatc gcttgatacc actcaaaaat cgcattcctg tctcagccaa attagcttca 4560
   tcaaggtcct taagggtgct aaagaattct cccagcaacg agtggaacag tgatactgga 4620
   aaaggaaact tactggattc tgatgaatgt agcctgagta gtagcacgca aagcagtggc 4680
   tgtcacacta ctgaaagtcg gcaggaatcc aaggattcgt cacctgagaa gtacccacag 4740
   acatgttcat ctgatttttc agacagtctt attaaagagt ctgatatttt gagtgatgaa 4800
   gatgatgatt ttcatcaaac tctgaaaaaa ggcagcccta caaaagacat tgaaattcag 4860
   ttccaaaggc tgaagatctc cgaggactct gagggtgaca gcactgccga tcaacagccc   4920
   agaacagagg ttggaaacaa tcttaacagt gtagcgcatc caaagctggt gcgtggacat   4980
   ttctgtccca ttaagcggaa agcaaacagt accaaaaggg atagggggac tctgttaaca   5040
   ctgcaagcac gccatcagtc tcttgacagt aaatctgaaa atgctaacat tgatttaaat   5100
   tccatactag agagggaatt cagtgtccag agtttaacat ctgtcgttaa tgaggagtgc   5160
   ttttatgaga cagacagcca tggaaaatca tag                                5193
<210> 72
   <211> 3251
   <212> DNA
   <213> Tiam2 Pan troglodytes fragment; Transcript ENSPTRT00000043097
<400> 72
   agtgctgagc agatcactgc actgtgcagg agttttaacg acagtcaggc caacggcatg     60
   gaaggaccgc gggagagtca ggatcctcct ccgaggcctc tggcccgcca cctgtctgat    120
   gcagaccgcc tccgcaaagt catccaggag cttgtggaca cagagaagtc ctacgtgaag    180
   gatttgagct gcctctttga attatacttg gagccacttc agaatgagac ctttcttacc    240
   caagatgaga tggagtcact ttttggaagt ttgccagaga tgcttgagtt tcagaaggtg    300
   tttctggaga ccctggagga tgggatttca gcatcatctg actttaacac cctagaaacc    360
   ccctcacagt ttagaaaatt actgttttcc cttggaggct ctttccttta ttacgcggac    420
   cactttaaac tgtacagtgg attctgtgct aaccatatca aagtacagaa ggttctggag    480
   cgagctaaaa ctgacaaagc cttcaaggct tttctggaca cccggaaccc caccaagcag    540
   cattcctcca cgctggagtc ctacctcatc aagccggttc agagagtgct caagtaccca    600
   ctgctgctca aggagctggt gtccctgacg gaccaggaga gcgaggagca ctaccacctg    660
   acggaagcac taaaggcaat ggagaaagta gcgagccaca tcaatgagat gcagaagatc    720
   tatgaggatt atgggaccgt gtttgaccag ctagtagctg agcagagcgg aacagagaag    780
   gaggtaacag aactttcgat gggagagctt ctgatgcact ctacggtttc ctggttgaat    840
   ccatttctgt ctctaggaaa agctagaaag gaccttgagc tcacagtatt tgtttttaag    900
   agagccgtca tactggttta taaagaaaac tgcaaactga aaaagaaatt ggcctcgaat    960
   tcccggcctg cacacaactc tactgacttg gacccattta aattccgctg gttgatcccc   1020
   atctccgcgc ttcaagtcag actggggaat ccagcaggga cagaaaataa ttccatatgg   1080
   gaactgatcc atacgaagtc agaaatagaa ggacggccag aaaccatctt tcagttgtgt   1140
   tgtagtgaca gtgaaagcaa aaccaacatt gttaaggtga ttcgttctat tctgagggaa   1200
   aacttcaggc gtcacataaa gtgtgaatta ccactggaga aaacgtgtaa ggatcgcctg   1260
   gtacctctta agaaccgagt tcctgtttcg gccaaattag cttcatccag gtctttaaaa   1320
   gtcctgaaga attcctccag caacgagtgg accggtgaga ctggcaaggg aaccttgctg 1380
   gactctgacg agggcagctt gagcagcagc acccagagca gcggctgccc cacggctgag 1440
   ggcaggcagg actccaagag cacttctccc gggaaatacc cacaccccgg cttggcagat 1500
   tttgctgaca atctcatcaa agagagtgac atcctgagcg atgaagatga tgaccaccgt 1560
   cagactgtga agcagggcag ccctactaaa gacatcgaaa ttcagttcca gagactgagg 1620
   atttccgagg acccagacgt tcaccccgag gctgagcagc agcctggccc ggagtcgggt 1680
   gagggtcaga aaggaggaga gcagcccaaa ctggtccggg ggcacttctg ccccattaaa 1740
   cgaaaagcca acagcaccaa gagggacaga ggaactttgc tcaaggcgca gatccgtcac 1800
   cagtcccttg acagtcagtc tgaaaatgcc accatcgacc taaattctgt tctagagcga 1860
   gaattcagtg tccagagttt aacatctgtt gtcagtgagg agtgttttta tgaaacagag 1920
   agccacggaa aatcatagta tgattcaatc cagatatggg ttaaattcct cattttactt 1980
   ttaaactggt ggtaaagtgg aaattgcaaa aaaaaccaaa aaacaaaaaa ctgttcattc 2040
   ctgggttttg tgcagtatac attttcccac ataatggttg taaagattta agttatttta 2100
   atttattgtg gatcagaaac ctagatgaaa ctggtcagaa tctgtaaatt acttagttta 2160
   tatccacttt gagcaggtat caaatgattt aggatcctta aaattacatt ctaataatta 2220
   ttttaagtta tgtggaaaaa gtaaggctgg ggaagttgtg attaatagtt ttcaaagggc 2280
   cattttttta aatcctctgg gcattttctt tcagctgtta gtttttgctt tatttaaagc 2340
   atatttaagt tattttaatg tggtttaggg gcaaaatgtg cagatacttc atttttgtaa 2400
   gattgtaata gatgctgttt atactaaaca tgtcatatct atacagtata tattaaaaga 2460
   aagcttgtac tgtatctatc ttatttgatg atatttattt tctctgccaa gctgtatagt 2520
   aaaaggaaaa taagtaagtc acatctggtc attggcattt gtatcgtcat tctgtaaaga 2580
   aagacaaaag agtacctata taagaagctc cacgtagtgc aaatcgacat ctggtaggct 2640
   gctcgccccc aggcagcagc tagagtctgt aattctctgc gtcatcctct tctttttctt 2700
   catttttgct ttttcttcgc ttgagttctt ctctgaaatt atatgcaaag agttgtgggt 2760
   cttcatcaca catttttctg tatacctcac agaggctctt aaagtgtgag atggagagct 2820
   ggcggggccg aagagtaggg tctatgtctg ccaactctaa cagcctgccc gtgctttcca 2880
   agcgctgcgc ttcagggaat aacattctga gccctcgatg gcagtatttc cttcggaact 2940
   gaaatacatt ctgaaccact ttttccacca gcttgaatgg ctgctctatc ttgggctgta 3000
   tcaagggagt gaagtgcacc acgcccacgt ccaccttcgt tgtaagcaaa catattatca 3060
   ttctgtggca tgatatgtgg catagtgtga tcaatcaact catccttgta aaacaggagg 3120
   atgggctgtc aacagcctgt tttcataaac agacctttcc acgtactttg gtttcatctc 3180
   taggcatgga agatggtaca ttctggattt gcaaatgaca tggagaaatc agctggctgc 3240
   acctgttctc t 3251
<210> 73
   <211> 655
   <212> PRT
   <213> Fgd2 mouse transcript variant 1a t6/tw5
<400> 73
   Met Glu Arg Ala Cys Glu Lys Gln Asp Ser Val Cys Asn Leu Val Ala
   1               5                   10                  15
   Val Phe Glu Asn Asn Arg Thr Pro Gly Glu Ala Pro Gly Ser His Ser
               20                  25                  30
   Leu Glu Asp Gln Leu His Ser Pro Glu His Gln Leu Ser Leu Ser Pro
           35                  40                  45
   Glu Pro Trp Glu Ala Pro Pro Val Lys Glu Ala Leu Lys Ser Glu Phe
       50                  55                  60
   Arg Pro Val Ser Arg Thr Tyr Leu Ser Ser Leu Lys Asn Lys Leu Ser
   65                  70                  75                  80
   Ser Gly Ala Trp Arg Arg Ser Cys Gln Pro Gly Val Ser Pro Gly Pro
                   85                  90                  95
   Glu Thr Gln Glu Pro Glu Glu Lys Arg Val Val Arg Glu Leu Leu Glu
               100                 105                 110
   Thr Glu Gln Ala Tyr Val Ala Arg Leu His Leu Leu Asp Gln Val Phe
           115                 120                 125
   Phe Gln Glu Leu Leu Arg Glu Ala Gly Arg Ser Lys Ala Phe Pro Glu
       130                 135                 140
   Asp Val Val Lys Leu Ile Phe Ser Asn Ile Ser Ser Ile Tyr Arg Phe
   145                 150                 155                 160
   His Ala Gln Phe Phe Leu Pro Glu Leu Gln Arg Arg Val Asp Asp Trp
                   165                 170                 175
   Ala Ala Thr Pro Arg Ile Gly Asp Val Ile Gln Lys Leu Ala Pro Phe
               180                 185                 190
   Leu Lys Met Tyr Ser Glu Tyr Val Lys Asn Phe Glu Arg Ala Ala Glu
           195                 200                 205
   Leu Leu Ala Thr Trp Met Asp Lys Ser Gln Pro Phe Gln Glu Val Val
       210                 215                 220
   Thr Arg Ile Gln Cys Ser Glu Ala Ser Gly Ser Leu Thr Leu Gln His
   225 230 235 240
   His Met Leu Glu Pro Val Gln Arg Ile Pro Arg Tyr Glu Leu Leu Leu
   245 250 255
   Lys Glu Tyr Val Gln Lys Leu Pro Ala Gln Ala Pro Asp Leu Glu Asp
   260 265 270
   Ala Gln Arg Ala Leu Asp Met Ile Phe Ser Ala Ala Gln His Ser Asn
   275 280 285
   Ala Ala Ile Ala Glu Met Glu Arg Leu Gln Gly Leu Trp Asp Val Tyr
   290 295 300
   Gln Arg Leu Gly Leu Glu Asp Asp Ile Val Asp Pro Ser Asn Thr Leu
   305 310 315 320
   Leu Arg Glu Gly Pro Val Leu Lys Ile Ser Phe Arg Arg Ser Asp Pro
   325 330 335
   Met Glu Arg Tyr Leu Val Leu Phe Asn Asn Met Leu Leu Tyr Cys Val
   340 345 350
   Pro Arg Val Leu Gln Val Gly Ala Gln Phe Gln Val Arg Thr Arg Ile
   355 360 365
   Asp Val Ala Gly Met Lys Val Arg Glu Leu Thr Asp Ala Glu Phe Pro
   370 375 380
   His Ser Phe Leu Val Ser Gly Lys Gln Arg Thr Leu Glu Leu Gln Ala
   385 390 395 400
   Arg Ser Arg Asp Glu Met Val Ser Trp Met Gln Ala Cys Gln Ala Ala
   405 410 415
   Ile Asp Gln Val Glu Lys Arg Ser Glu Thr Phe Lys Ala Ala Val Gln
   420 425 430
   Gly Pro Gln Gly Asp Thr Gln Glu Pro Lys Pro Gln Val Glu Glu Leu
   435 440 445
   Gly Leu Arg Ala Pro Gln Trp Val Arg Asp Lys Met Val Thr Met Cys
   450 455 460
   Met Arg Cys Gln Glu Pro Phe Asn Ala Leu Thr Arg Arg Arg His His
   465 470 475 480
   Cys Arg Ala Cys Gly Tyr Val Val Cys Ala Lys Cys Ser Asp Tyr Arg
   485 490 495
   Ala Glu Leu Lys Tyr Asp Ser Asn Arg Pro Asn Arg Val Cys Leu Thr
   500 505 510
   Cys Tyr Thr Phe Leu Thr Gly Asn Val Leu Pro Gln Gly Lys Glu Asp
   515 520 525
   Lys Arg Arg Gly Ile Leu Glu Lys Glu Ala Ser Ala Ala Pro Glu Gln
   530 535 540
   Ser Leu Val Cys Ser Phe Leu Gln Leu Ile Gly Asp Lys Cys Ser Arg
   545 550 555 560
   Ser Leu Pro Arg Ser Trp Cys Val Ile Pro Arg Asp Asp Pro Leu Val
   565 570 575
   Leu Tyr Val Tyr Ala Ala Pro Gln Asp Thr Lys Ala His Thr Ser Ile
   580 585 590
   Pro Leu Leu Gly Tyr Gln Val Ile Ser Gly Pro Gln Gly Asp Pro Arg
   595 600 605
   Val Phe Gln Leu Gln Gln Ser Gly Gln Gln Tyr Thr Phe Lys Ala Glu
   610 615 620
   Ser Val Glu Leu Gln Gly Arg Trp Val Thr Ala Ile Lys Arg Ala Ala
   625 630 635 640
   Ser Gly Arg Thr Pro Glu Gly Pro Asp Glu Glu Asp Val Ser Asp
   645 650 655
<210> 74
   <211> 687
   <212> PRT
   <213> Tagap1 Danio rerio; deduced from Ensembl Transcript ENSDART00000063826
<400> 74
   Met Lys Val Leu Ser Gly Asn Thr Thr Asn Lys Thr Leu Ser Gly Asp
   1 5 10 15
   Gly Met Asp Ser Asp Leu Leu Val Asp Ser Asn Gly Lys Asn Ser Ala
   20 25 30
   Leu Ser Lys Glu Leu His Asn Gln Glu Ala His Ala Gln Gln Pro Ile
   35 40 45
   Gly Glu Asn Gly Gly Lys Lys Ser Phe Lys Ile Pro Phe Lys Leu Lys
   50 55 60
   Arg Ser Ser Thr Ser Pro Ser Val Thr Ala His Pro Glu Ser Ser Thr
   65 70 75 80
   Lys Asn Leu Leu Phe Gly Arg Asn Leu Gln Asp Asp Thr Ala Leu Pro
   85 90 95
   Lys Pro Ile Ala Glu Ile Leu Leu Leu Leu Phe Arg Lys Gly Pro Val
   100 105 110
   Thr Glu Gly Val Phe Arg Val Ser Cys Asn Ser Lys Asn Leu His Thr
   115 120 125
   Leu Arg Asn Gln Leu Asn Ser Gly Ala Glu Val Asp Met Asp Ala Leu
   130 135 140
   Pro Val Thr Leu Leu Val Gly Leu Leu Lys Ile Phe Leu Arg Glu Leu
   145 150 155 160
   Pro Gly Gly Leu Leu Met Ser Glu His Tyr Glu Ser Trp Ile Gly Ala
   165 170 175
   Leu Glu Lys Glu Arg Thr Glu Glu Val Gln Ser Glu Leu Arg Gly Met
   180 185 190
   Ala Glu Lys Leu Pro Lys Ala Asn Ser Leu Leu Leu Gln His Leu Leu
   195 200 205
   Cys Leu Phe His His Ile Ser Gln Arg Ser Glu Thr Asn Lys Met Asp
   210 215 220
   Ala Lys Asn Leu Ala Val Cys Ile Ala Pro Thr Leu Leu His Arg Asp
   225 230 235 240
   Ser Gln Pro Leu Asp Val Asp Val Val Glu Lys Val Thr Lys Leu Ile
   245 250 255
   Gln Phe Leu Thr Glu Asn Cys Cys Glu Ile Phe Gly His Asp Ile Leu
   260 265 270
   Thr Leu Phe Gly Asp Leu Glu Asp Arg Lys Ala Thr Asp Lys Ser Asp
   275 280 285
   Ser Ala Ser Leu Met Ser Pro Asp Ile Ser Phe Glu Val His Gln His
   290 295 300
   Asp Ser Ala Tyr Asp Ser Thr Asp Pro Asp Ala Asp Cys Asp Cys Val
   305 310 315 320
   Glu Ala Glu Ser Tyr Glu Glu Gly Met Met Ala His Gly Ser Leu Gly
   325 330 335
   Leu Ser Lys Lys Gly Arg Ser Asp Ile Gln Ser Cys Ser Ser Asp Ala
   340 345 350
   Ile Phe Asp Thr Phe Thr Lys Thr Phe Ser Arg Arg Ser Ser Glu Pro
   355 360 365
   Ser Ile Leu Ser Thr Leu Pro Ile Thr Gly Leu Arg Glu Leu Ala Arg
   370 375 380
   Ser His Asp Asp Phe Ser Thr Glu Lys Glu His Phe Asp Asn Gln Pro
   385 390 395 400
   Leu Lys Lys Gln Asn Ser Asp Asp Ser Phe Leu Leu Pro Asn Arg Ser
   405 410 415
   Glu Asn Arg Arg Ser Leu Lys Lys Leu Ala Gly Ser Phe Asn Met Asp
   420 425 430
   Leu Pro Ile Ile Val Ser Ser Pro Thr Ser Lys Ile Gly Ser Cys Pro
   435 440 445
   Ser Phe Cys Ser Ser Asp Ser Ser Ser Ser Asn His Ser Glu Gln Ser
   450 455 460
   Lys Thr Pro Ser Pro Leu Pro Ser Pro Ala Asn Pro Arg Lys Thr Gln
   465 470 475 480
   Ser Thr Arg His Ala Ser Phe Met Ile Lys Ser Arg His Asn His Ala
   485 490 495
   Gln Gly Asp Gln Glu Val Thr Arg Arg Ser Leu Ser Met Arg Ala Lys
   500 505 510
   Ser Leu Gly Asn Phe Thr Phe Asn Arg Ser Ser Leu Lys Lys Gly Asp
   515 520 525
   Ser Gln Lys Glu Val Val Phe Pro Cys Glu Thr Leu Gln Glu Asp Ser
   530 535 540
   Gln Asn Glu Thr Glu Asn Pro Asp Glu Leu Val Arg Arg Arg Arg Pro
   545 550 555 560
   Leu Ser Ala Ile Glu Val Phe Gln Gln Val Asp Ser Arg Met Pro Cys
   565 570 575
   Ser Pro Pro Ser Tyr Glu Gln Ala Leu Gln Thr Gly Val His Gln Ala
   580 585 590
   Pro Pro Gln Tyr Arg Glu Met Thr Val Gln His Ala Arg Glu Leu Gly
   595 600 605
   Lys Lys Ser Arg Pro Ile Ser Met Asn Asp Asn Leu Leu Asp Ile Tyr
   610 615 620
   Lys Val Asn Glu Ser Thr Glu Cys Leu Glu Thr Phe Thr Glu Ser Ile
   625 630 635 640
   Gln Leu Asp Lys Pro Gln Leu Val Thr Phe Arg Gln Arg Ala Met Ser
   645 650 655
   Glu Ser Val Ser Gln Leu Lys His Glu Lys Val Ser Arg Arg Cys Ser
   660 665 670
   Gln Pro Val Phe Glu Glu Phe Ser Tyr Ala Lys Glu Ser Tyr Val
   675 680 685
<210> 75
   <211> 731
   <212> PRT
   <213> Tagap1 Macaca mulatta; deduced fromEnsembl Transcript ENSMMUT00000016796
<400> 75
   Met Lys Leu Arg Ser Ser His Asn Ala Ser Lys Thr Leu Asn Ala Ser
   1 5 10 15
   Asn Met Glu Thr Leu Ile Glu Cys Gln Ser Glu Gly Asp Ile Lys Glu
   20 25 30
   His Pro Leu Leu Ala Ser Cys Glu Ser Glu Asp Ser Ile Cys Gln Leu
   35 40 45
   Ile Glu Val Lys Lys Arg Lys Lys Val Leu Ser Trp Pro Phe Leu Met
   50 55 60
   Arg Arg Leu Ser Pro Ala Ser Asp Phe Ser Gly Ala Leu Glu Thr Asp
   65 70 75 80
   Leu Lys Ala Ser Leu Phe Asp Gln Pro Leu Ser Ile Ile Cys Gly Gly
   85 90 95
   Ser Asp Thr Leu Pro Arg Pro Ile Gln Asp Ile Leu Thr Ile Leu Cys
   100 105 110
   Leu Lys Gly Pro Ser Thr Glu Gly Ile Phe Arg Arg Ala Ala Asn Glu
   115 120 125
   Lys Ala Arg Lys Glu Leu Lys Glu Glu Leu Asn Ser Gly Asp Ala Val
   130 135 140
   Asp Leu Glu Ser Leu Pro Val His Leu Leu Ala Val Val Phe Lys Asp
   145 150 155 160
   Phe Leu Arg Ser Ile Pro Arg Lys Leu Leu Ser Ser Asp Leu Phe Glu
   165 170 175
   Glu Trp Met Gly Thr Leu Glu Met Gln Asp Glu Glu Asp Arg Ile Glu
   180 185 190
   Ala Leu Lys Gln Val Ala Asp Lys Leu Pro Gln Pro Asn Leu Leu Leu
   195 200 205
   Leu Lys His Leu Val Tyr Val Leu His Leu Ile Ser Lys Asn Ser Glu
   210 215 220
   Val Asn Arg Met Asp Ser Ser Asn Leu Ala Ile Cys Ile Gly Pro Asn
   225 230 235 240
   Met Leu Thr Leu Glu Asn Asp Gln Cys Leu Ser Phe Glu Ala Gln Lys
   245 250 255
   Asp Leu Asn Asn Lys Val Lys Thr Leu Val Glu Phe Leu Ile Asp Asn
   260 265 270
   Cys Phe Glu Ile Phe Gly Glu Asn Ile Pro Val His Ser Ser Ile Thr
   275 280 285
   Ser Val Asp Ser Leu Glu His Thr Asp Ser Ser Asp Val Ser Thr Leu
   290 295 300
   Gln Asn Asp Ser Ala Tyr Asp Ser Asn Asp Pro Asp Val Glu Ser Asn
   305 310 315 320
   Ser Ser Ser Gly Ile Ser Ser Pro Ser Arg Gln Pro Gln Val Pro Met
   325 330 335
   Ala Thr Ala Ala Gly Phe Asp Ser Gly Gly Pro Gln Asp Ala Arg Glu
   340 345 350
   Val Ser Pro Glu Pro Ile Val Ser Thr Val Ala Arg Leu Lys Ser Ser
   355 360 365
   Leu Ala Gln Pro Asp Arg Arg Tyr Ser Glu Pro Ser Met Pro Ser Ser
   370 375 380
   Gln Glu Cys Leu Glu Ser Arg Val Thr Asn Gln Thr Leu Thr Lys Ser
   385 390 395 400
   Glu Gly Asp Phe Pro Val Pro Arg Val Gly Ser Arg Leu Glu Arg Glu
   405 410 415
   Glu Ala Glu Asp Pro Phe Pro Glu Glu Val Phe Pro Ala Val Gln Gly
   420 425 430
   Lys Thr Lys Arg Pro Val Asp Leu Lys Ile Lys Asn Leu Thr Pro Gly
   435 440 445
   Ser Val Leu Pro Arg Ala Leu Val Pro Lys Ala Phe Ser Ser Ser Ser
   450 455 460
   Leu Asp Ala Ser Ser Asp Ser Ser Pro Val Ala Ser Pro Ser Ser Pro
   465 470 475 480
   Lys Arg Asn Phe Phe Ser Arg His Gln Ser Phe Thr Thr Lys Thr Glu
   485 490 495
   Lys Gly Lys Pro Ser Arg Glu Ile Lys Lys His Ser Met Ser Phe Ser
   500 505 510
   Phe Ala Pro His Lys Lys Val Leu Thr Lys Asn Leu Ser Ala Gly Ser
   515 520 525
   Gly Lys Ser Gln Asp Phe Thr Arg Asp His Val Pro Arg Gly Val Arg
   530 535 540
   Lys Glu Ser Gln Leu Ala Gly Arg Ile Val Gln Glu Asn Gly Ser Glu
   545 550 555 560
   Thr His Asn Gln Thr Thr Arg Ala Phe Cys Leu Arg Pro His Ala Leu
   565 570 575
   Ser Val Asp Asp Val Phe Gln Gly Ala Asp Trp Glu Arg Pro Gly Ser
   580 585 590
   Pro Pro Ser Tyr Glu Glu Ala Met Gln Gly Pro Ala Ala Arg Leu Ala
   595 600 605
   Ala Tyr Gly Ser Gln Thr Val Gly Ser Met Thr Val Gly Ser Met Arg
       610                 615                 620
   Ala Arg Met Leu Glu Val Asp Ser Leu Leu Pro Pro Leu Pro Pro Ala
   625                 630                 635                 640
   His His Thr Gly Asp Ser Arg His Arg Asp Ser Lys Glu Pro Leu Pro
                   645                 650                 655
   Gly His Gly Pro Ser Pro Leu Pro Glu Arg Trp Ile Gln Ser Arg Thr
               660                 665                 670
   Val His Ala Ser Trp Asp Ser Val Gly His Val Ser Gly Pro Gly Arg
           675                 680                 685
   Pro Glu Leu Leu Arg Leu Arg Thr Val Ser Glu Ser Val Gln Arg Asn
       690                 695                 700
   Lys Arg Asp Cys Leu Met Arg Arg Cys Ser Gln Pro Val Phe Glu Ala
   705                 710                 715                 720
   Asp Gln Phe Gln His Ala Lys Glu Ser Tyr Ile
                   725                 730
<210> 76
   <211> 724
   <212> PRT
   <213> Tagap1 Monodelphis domestica; deduced from Ensembl Transcript ENSMODT00000009606
<400> 76
   Met Lys Val Leu Ser Gly Cys Asn Ala Ser Lys Thr Leu Asn Ala Ser
   1               5                   10                  15
   Asp Met Glu Thr Leu Ile Glu Cys Gln Pro Glu Gly Asn Val Lys Gln
               20                  25                  30
   His His Leu Leu Ala Gln Cys Asp Ser Glu Asp Gly Ile Cys His Leu
           35                  40                  45
   Ile Glu Asn Lys Lys Arg Lys Val Ile Ala Trp Pro Phe Pro Met Arg
       50                  55                  60
   Arg Thr Ala Ser Ser Ser Asp Ser Ser Gly Ser Leu Thr Pro Glu Trp
   65                  70                  75                  80
   Lys Ala Ser Leu Phe Asp Gln Pro Leu Ser Ala Ile Cys Thr Asp Glu
                   85                  90                  95
   Asp Ile Leu Pro Gln Pro Ile Gln Glu Ile Leu Asn Ile Leu Gln Asn
   100 105 110
   Lys Gly Pro Ser Thr Glu Gly Ile Phe Arg Lys Ala Ala Asn Glu Lys
   115 120 125
   Ala Arg Lys Glu Leu Lys Glu Glu Leu Asn Ser Gly Gly Met Val Asn
   130 135 140
   Leu Glu Thr Lys Ser Val His Leu Leu Ala Ala Val Leu Lys Asp Phe
   145 150 155 160
   Leu Arg Ser Ile Pro Leu Lys Leu Leu Ser Ser Asp Leu Phe Glu Glu
   165 170 175
   Trp Met Thr Ala Leu Glu Arg Pro Asn Glu Asp Asp Lys Ile Glu Ser
   180 185 190
   Leu Lys Gln Val Val Lys Lys Leu Pro Arg Thr Asn Ile Leu Leu Leu
   195 200 205
   Lys His Leu Val Tyr Val Leu Tyr Asn Ile Ser Lys Asn Ser Asp Val
   210 215 220
   Ser Lys Met Asp Ser Ser Asn Leu Ala Ile Cys Ile Gly Pro Asn Met
   225 230 235 240
   Leu Ser Leu Asn Thr Asp Gln Ser Leu Ser Phe Asp Ala Gln Lys Glu
   245 250 255
   Leu Asn Asn Lys Ile Lys Thr Leu Val Glu Phe Phe Ile Asp Asn Cys
   260 265 270
   Phe Glu Ile Phe Glu Glu Asp Ile Pro Gly His Ser Pro Arg Phe Asn
   275 280 285
   Ser Asp Asp Ser Leu Glu His Ile Asn Asn Ser Asp Met Ser Thr Leu
   290 295 300
   Gln Asn Asp Ser Ala Tyr Glu Ser Thr Asp Ala Asp Thr Glu Cys Ser
   305 310 315 320
   Asn Ser Phe Gly Ser Gln Asn Lys Pro Ser Leu Asp Thr Ala Gly Leu
   325 330 335
   Ser Ser Asp Leu Asp Asn Arg Lys His Leu Tyr Glu Ser Arg Pro Gly
   340 345 350
   Ser Val Val Ser Tyr Thr Asn Leu Leu Lys Ser Ser Leu Ser Thr Gln
   355 360 365
   Glu Arg Arg Tyr Ser Glu Pro Ser Thr Pro Ser Thr Lys Asp Cys Leu
   370 375 380
   Glu Ser Arg Ile Thr Ser His Lys Leu Thr Lys Ser Glu Asp Ser Phe
   385 390 395 400
   Ala Val Pro Gln Ala Ser Ser Cys Phe Gly Gly Gln Glu Ile Lys Glu
   405 410 415
   Ser Phe Pro Glu Glu Cys Phe Pro Ser Leu Gln His Arg Lys Gln Lys
   420 425 430
   Ser Leu Gly Leu Gln Ile Lys Glu Gly Thr Leu Cys Thr Glu Ser Leu
   435 440 445
   Val Glu Pro Ser Pro Lys Thr Ser Ser Cys Gly Ser Leu Asp Ser Ser
   450 455 460
   Ser Asp Ser Ser Val Phe Ala Asn Ser Pro Val Val Ser Pro Ser Ser
   465 470 475 480
   Pro Lys Arg Asn Phe Phe Thr Arg His Gln Ser Phe Thr Thr Lys Thr
   485 490 495
   Ala Gly Ala Asp Ser Lys Leu Thr Arg Glu Arg Lys Lys His Ser Met
   500 505 510
   Ser Phe Ser Phe Ala Thr His Lys Lys Val Pro Asn Lys Thr Ala Asn
   515 520 525
   Trp Arg Phe Pro Arg Asp Gln Gly Lys Lys Asp Leu Lys Lys Glu Ser
   530 535 540
   Gln Leu Thr Gly Arg Ile Val Gln Glu Val Cys Val Gly Ala Ile Asp
   545 550 555 560
   Asp Tyr Gln Pro Thr Pro Glu Cys Gly Ser Ser Arg Ser His Leu Leu
   565 570 575
   Ser Val Glu Glu Val Phe Gln Leu Val Asp Gln Lys Asn Pro Gly Ser
   580 585 590
   Pro Pro Ser Tyr Glu Glu Ala Val Gln Tyr Cys Arg Leu Ser Lys Ile
   595 600 605
   Pro Pro Tyr Glu Ser Arg Thr Val Gln Ser Met Arg Asp Thr Met Gln
   610 615 620
   Ser Gln Asn Ser Arg Leu Pro Ser Leu Ser Leu Leu Asp Tyr Gly Glu
   625 630 635 640
   His Ala Lys Asn Thr His Gly Lys Glu Thr Leu Asn Arg Asp Ser Ala
   645 650 655
   Ser Ser Val Gly Glu Thr Trp Val Gln Asn Gly Ala Ser Asn Val Ala
   660 665 670
   Met Glu Arg Lys Gly Gln Ile Pro Lys Ser Glu Val Arg Arg Leu Arg
   675 680 685
   Val Leu Ser Glu Ser Leu Leu Lys Asn Lys Gln Asp Cys Val Ile Arg
   690 695 700
   Arg Cys Ser Gln Pro Ile Phe Glu Val Asp Gln Ile Gln Tyr Ala Lys
   705 710 715 720
   Glu Ser Tyr Val
<210> 77
   <211> 702
   <212> PRT
   <213> Tagap1 Xenopus tropicalis; deduced from Ensembl Transcript ENSXETT00000021546
<210> 78
   <211> 731
   <212> PRT
   <213> Tagap1 Pan troglodytes; deduced from Ensembl Transcript ENSPTRT00000034625
<400> 78
   Met Lys Leu Arg Ser Ser His Asn Ala Ser Lys Thr Leu Asn Ala Asn
   1 5 10 15
   Asn Met Glu Thr Leu Ile Glu Cys Gln Ser Glu Gly Asp Ile Lys Glu
   20 25 30
   His Pro Leu Leu Ala Ser Cys Glu Ser Glu Asp Ser Ile Cys Gln Leu
   35 40 45
   Ile Glu Val Lys Lys Arg Lys Lys Val Leu Ser Trp Pro Phe Leu Met
   50 55 60
   Arg Arg Leu Ser Pro Ala Ser Asp Phe Ser Gly Ala Leu Glu Thr Asp
   65 70 75 80
   Leu Lys Ala Ser Leu Phe Asp Gln Pro Leu Ser Ile Ile Cys Gly Asp
   85 90 95
   Ser Asp Thr Leu Pro Arg Pro Ile Gln Asp Ile Leu Thr Ile Leu Cys
   100 105 110
   Leu Lys Gly Pro Ser Thr Glu Gly Ile Phe Arg Arg Ala Ala Asn Glu
   115 120 125
   Lys Ala Arg Lys Glu Leu Lys Glu Glu Leu Asn Ser Gly Asp Ala Val
   130 135 140
   Asp Leu Glu Arg Leu Pro Val His Leu Leu Ala Val Val Phe Lys Asp
   145 150 155 160
   Phe Leu Arg Ser Ile Pro Arg Lys Leu Leu Ser Ser Asp Leu Phe Glu
   165 170 175
   Glu Trp Met Gly Ala Leu Glu Met Gln Asp Glu Glu Asp Arg Ile Glu
   180 185 190
   Ala Leu Lys Gln Val Ala Asp Lys Leu Pro Gln Pro Asn Leu Leu Leu
   195 200 205
   Leu Lys His Leu Val Tyr Val Leu His Leu Ile Ser Lys Asn Ser Glu
   210 215 220
   Val Asn Arg Met Asp Ser Ser Asn Leu Ala Ile Cys Ile Gly Pro Asn
   225 230 235 240
   Met Leu Thr Leu Glu Asn Asp Gln Ser Leu Ser Phe Glu Ala Gln Lys
   245 250 255
   Asp Leu Asn Asn Lys Val Lys Thr Leu Val Glu Phe Leu Ile Asp Asn
   260 265 270
   Cys Phe Glu Ile Phe Gly Glu Asn Ile Pro Val His Ser Ser Ile Thr
   275 280 285
   Ser Asp Asp Ser Leu Glu His Thr Asp Ser Ser Asp Val Ser Thr Leu
   290 295 300
   Gln Asn Asp Ser Ala Tyr Asp Ser Asn Asp Pro Asp Val Glu Ser Asn
   305 310 315 320
   Ser Ser Ser Gly Ile Ser Ser Pro Ser Arg Gln Pro Gln Val Pro Met
   325 330 335
   Ala Thr Ala Ala Gly Leu Asp Ser Ala Gly Pro Gln Asp Ala Arg Glu
   340 345 350
   Val Ser Pro Glu Pro Ile Val Ser Thr Val Ala Arg Leu Lys Ser Ser
   355 360 365
   Leu Ala Gln Pro Asp Arg Arg Tyr Ser Glu Pro Ser Met Pro Ser Ser
   370 375 380
   Gln Glu Cys Leu Glu Ser Arg Val Thr Asn Gln Thr Leu Thr Lys Ser
   385 390 395 400
   Glu Gly Asp Phe Pro Val Pro Arg Val Gly Ser Arg Leu Glu Ser Glu
   405 410 415
   Glu Ala Glu Asp Pro Phe Pro Glu Glu Val Phe Pro Ala Val Gln Gly
   420 425 430
   Lys Thr Lys Arg Pro Val Asp Leu Lys Ile Lys Asn Leu Ala Pro Gly
   435 440 445
   Ser Val Leu Pro Arg Ala Leu Val Leu Lys Ala Phe Ser Ser Ser Ser
   450 455 460
   Leu Asp Ala Ser Ser Asp Ser Ser Pro Val Ala Ser Pro Ser Ser Pro
   465 470 475 480
   Lys Arg Asn Phe Phe Ser Arg His Gln Ser Phe Thr Thr Lys Thr Glu
   485 490 495
   Lys Gly Lys Pro Ser Arg Glu Ile Lys Lys His Ser Met Ser Phe Ser
   500 505 510
   Phe Ala Pro His Lys Lys Val Leu Thr Lys Asn Leu Ser Ala Gly Ser
   515 520 525
   Gly Lys Ser Gln Asp Phe Thr Arg Asp His Val Pro Arg Gly Val Arg
   530 535 540
   Lys Glu Ser Gln Leu Ala Gly Arg Ile Val Gln Glu Asn Gly Cys Glu
   545 550 555 560
   Thr His Asn Gln Thr Ala Arg Gly Phe Cys Leu Arg Pro His Ala Leu
   565 570 575
   Ser Val Asp Asp Val Phe Gln Gly Ala Asp Trp Glu Arg Pro Gly Ser
   580 585 590
   Pro Pro Ser Tyr Glu Glu Ala Met Gln Gly Pro Ala Ala Arg Leu Val
   595 600 605
   Ala Tyr Glu Ser Gln Thr Val Gly Ser Met Thr Val Gly Ser Met Arg
   610 615 620
   Ala Arg Met Leu Glu Ala His Cys Leu Leu Pro Pro Leu Pro Pro Ala
   625 630 635 640
   His His Val Glu Asp Ser Arg His Arg Gly Ser Lys Glu Pro Leu Pro
   645 650 655
   Gly His Gly Leu Ser Pro Leu Pro Glu Arg Trp Lys Gln Ser Arg Thr
   660 665 670
   Val His Ala Ser Gly Asp Ser Leu Gly His Val Ser Gly Pro Gly Arg
   675 680 685
   Pro Glu Leu Leu Pro Leu Arg Thr Val Ser Glu Ser Met Gln Arg Asn
   690 695 700
   Lys Arg Asp Cys Leu Val Arg Arg Cys Ser Gln Pro Val Phe Glu Ala
   705 710 715 720
   Asp Gln Phe Gln Tyr Ala Lys Glu Ser Tyr Ile
   725 730
<210> 79
   <211> 657
   <212> PRT
   <213> Fgd2 Macacca mulatta transcript variant 1; deduced from ENSMMUP00000033771
<400> 79
   Met Lys Glu Ala Ser Glu Glu Lys Leu Ala Ser Val Ser Asn Leu Val
   1 5 10 15
   Thr Val Phe Glu Asn Ser Arg Thr Pro Glu Ala Ala Pro Arg Gly His
   20 25 30
   Arg Leu Glu Asp Ala His His His Pro Glu Cys Arg Pro Pro Arg Ser
   35 40 45
   Pro Gly Pro Trp Glu Lys Leu Asn Val Gly Glu Ala Met Gly Ser Glu
   50 55 60
   Pro Arg Thr Val Ser Gly Arg Tyr Leu Asn Ser Leu Lys Asn Lys Leu
   65 70 75 80
   Ser Ser Gly Ala Trp Arg Lys Ser Cys Gln Pro Val Thr Leu Ser Gly
   85 90 95
   Ser Gly Met Gln Glu Pro Glu Lys Lys Ile Val Gln Glu Leu Leu Glu
   100 105 110
   Thr Glu Gln Ala Tyr Val Ala Arg Leu His Leu Leu Asp Gln Ala Met
   115 120 125
   Val Phe Phe Gln Glu Leu Leu Arg Ala Ala Arg Arg Ser Lys Ala Phe
       130                 135                 140
   Pro Glu Asp Val Val Arg Val Ile Phe Ser Asn Ile Ser Ser Ile Tyr
   145                 150                 155                 160
   Gln Phe His Ser Gln Phe Phe Leu Pro Glu Leu Gln Arg Arg Leu Asp
                   165                 170                 175
   Asp Trp Thr Ala Asn Pro Arg Ile Gly Asp Val Ile Gln Lys Leu Ala
               180                 185                 190
   Pro Phe Leu Lys Met Tyr Gly Glu Tyr Val Lys Asn Phe Glu Arg Ala
           195                 200                 205
   Ala Glu Leu Leu Ala Thr Trp Thr Glu Lys Ser Pro Leu Phe Gln Glu
       210                 215                 220
   Val Leu Thr Arg Ile Gln Ser Gly Glu Ala Ser Gly Ser Leu Thr Leu
   225                 230                 235                 240
   Gln His His Met Leu Glu Pro Val Gln Arg Ile Pro Arg Tyr Glu Leu
                   245                 250                 255
   Leu Leu Lys Glu Tyr Val Gln Lys Leu Pro Ala Gln Ala Pro Asp Arg
               260                 265                 270
   Ala Asp Ala Gln Lys Ala Leu Asp Met Ile Phe Ser Ala Ala Gln His
           275                 280                 285
   Ser Asn Ala Ala Ile Thr Glu Met Glu Arg Leu Gln Asp Leu Trp Glu
       290                 295                 300
   Val Tyr Gln Arg Leu Gly Leu Glu Asp Asp Ile Val Asp Pro Ser Asn
   305                 310                 315                 320
   Thr Leu Leu Arg Glu Gly Pro Val Phe Lys Ile Ser Phe Arg Arg Asn
                   325                 330                 335
   Asp Pro Met Glu Arg Tyr Leu Phe Leu Phe Asn Asn Met Leu Leu Tyr
               340                 345                 350
   Cys Val Pro Arg Val Ile Gln Val Gly Ala Gln Phe Gln Val Arg Thr
           355                 360                 365
   Arg Ile Asp Val Ala Gly Met Lys Val Arg Glu Leu Met Asp Ala Glu
       370                 375                 380
   Phe Pro His Ser Phe Leu Val Ser Gly Lys Gln Arg Thr Leu Glu Leu
   385 390 395 400
   Gln Ala Arg Ser Gln Glu Glu Lys Ile Ser Trp Met Gln Ala Phe Gln
   405 410 415
   Ala Ala Val Asp Gln Ile Glu Lys Arg Asn Glu Thr Phe Lys Ala Ala
   420 425 430
   Ala Gln Gly Pro Glu Gly Asp Thr Gln Glu Gln Glu Leu Gln Ser Glu
   435 440 445
   Glu Leu Gly Leu Arg Ala Pro Gln Trp Val Arg Asp Lys Met Val Thr
   450 455 460
   Met Cys Met Arg Cys Gln Glu Pro Phe Asn Ala Leu Thr Arg Arg Arg
   465 470 475 480
   His His Cys Arg Ala Cys Gly Tyr Val Val Cys Ala Arg Cys Ser Asp
   485 490 495
   Tyr Arg Ala Glu Leu Lys Tyr Asn Asp Asn Arg Gln Asn Arg Val Cys
   500 505 510
   Leu His Cys Tyr Thr Phe Leu Thr Gly Asn Val Leu Pro Glu Ala Lys
   515 520 525
   Glu Asp Lys Arg Arg Gly Ile Leu Glu Lys Gly Ser Ser Ala Thr Pro
   530 535 540
   Asp Gln Ser Leu Met Cys Ser Phe Leu Gln Leu Ile Gly Asp Lys Trp
   545 550 555 560
   Gly Lys Ser Gly Pro Arg Gly Trp Cys Val Ile Pro Arg Asp Asp Pro
   565 570 575
   Leu Val Leu Tyr Val Tyr Ala Ala Pro Gln Asp Met Arg Ala His Thr
   580 585 590
   Ser Ile Pro Leu Leu Gly Tyr Gln Val Thr Val Gly Pro Gln Gly Asp
   595 600 605
   Pro Arg Val Phe Gln Leu Gln Gln Ser Gly Gln Leu Tyr Thr Phe Lys
   610 615 620
   Ala Glu Thr Glu Glu Leu Lys Gly Arg Trp Val Lys Ala Met Glu Arg
   625 630 635 640
   Ala Ala Ser Gly Trp Ser Pro Ser Trp Pro Asn Asp Gly Asp Leu Ser
   645 650 655
   Asp
<210> 80
   <211> 657
   <212> PRT
   <213> Fgd2 Macaca mulatta transcript variant 3; deduced from ENSMMUP00000033770
<400> 80
   Met Lys Glu Ala Ser Glu Glu Lys Leu Ala Ser Val Ser Asn Leu Val
   1               5                   10                  15
   Thr Val Phe Glu Asn Ser Arg Thr Pro Glu Ala Ala Pro Arg Gly His
               20                  25                  30
   Arg Leu Glu Asp Ala His His His Pro Glu Cys Arg Pro Pro Arg Ser
           35                  40                  45
   Pro Gly Pro Trp Glu Lys Leu Asn Val Gly Glu Ala Met Gly Ser Glu
       50                  55                  60
   Pro Arg Thr Val Ser Gly Arg Tyr Leu Asn Ser Leu Lys Asn Lys Leu
   65                  70                  75                  80
   Ser Ser Gly Ala Trp Arg Lys Ser Cys Gln Pro Val Thr Leu Ser Gly
                   85                  90                  95
   Ser Gly Met Gln Glu Pro Glu Lys Lys Ile Val Gln Glu Leu Leu Glu
               100                 105                 110
   Thr Glu Gln Ala Tyr Val Ala Arg Leu His Leu Leu Asp Gln Ala Met
           115                 120                 125
   Val Phe Phe Gln Glu Leu Leu Arg Ala Ala Arg Arg Ser Lys Ala Phe
       130                 135                 140
   Pro Glu Asp Val Val Arg Val Ile Phe Ser Asn Ile Ser Ser Ile Tyr
   145                 150                 155                 160
   Gln Phe His Ser Gln Phe Phe Leu Pro Glu Leu Gln Arg Arg Leu Asp
                   165                 170                 175
   Asp Trp Thr Ala Asn Pro Arg Ile Gly Asp Val Ile Gln Lys Leu Ala
               180                 185                 190
   Pro Phe Leu Lys Met Tyr Gly Glu Tyr Val Lys Asn Phe Glu Arg Ala
   195 200 205
   Ala Glu Leu Leu Ala Thr Trp Thr Glu Lys Ser Pro Leu Phe Gln Glu
   210 215 220
   Val Leu Thr Arg Ile Gln Ser Gly Glu Ala Ser Gly Ser Leu Thr Leu
   225 230 235 240
   Gln His His Met Leu Glu Pro Val Gln Arg Ile Pro Arg Tyr Glu Leu
   245 250 255
   Leu Leu Lys Glu Tyr Val Gln Lys Leu Pro Ala Gln Ala Pro Asp Arg
   260 265 270
   Ala Asp Ala Gln Lys Ala Leu Asp Met Ile Phe Ser Ala Ala Gln His
   275 280 285
   Ser Asn Ala Ala Ile Thr Glu Met Glu Arg Leu Gln Asp Leu Trp Glu
   290 295 300
   Val Tyr Gln Arg Leu Gly Leu Glu Asp Asp Ile Val Asp Pro Ser Asn
   305 310 315 320
   Thr Leu Leu Arg Glu Gly Pro Val Phe Lys Ile Ser Phe Arg Arg Asn
   325 330 335
   Asp Pro Met Glu Arg Tyr Leu Phe Leu Phe Asn Asn Met Leu Leu Tyr
   340 345 350
   Cys Val Pro Arg Val Ile Gln Val Gly Ala Gln Phe Gln Val Arg Thr
   355 360 365
   Arg Ile Asp Val Ala Gly Met Lys Val Arg Glu Leu Met Asp Ala Glu
   370 375 380
   Phe Pro His Ser Phe Leu Val Ser Gly Lys Gln Arg Thr Leu Glu Leu
   385 390 395 400
   Gln Ala Arg Ser Gln Glu Glu Lys Ile Ser Trp Met Gln Ala Phe Gln
   405 410 415
   Ala Ala Val Asp Gln Ile Glu Lys Arg Asn Glu Thr Phe Lys Ala Ala
   420 425 430
   Ala Gln Gly Pro Glu Gly Asp Thr Gln Glu Gln Glu Leu Gln Ser Glu
   435 440 445
   Glu Leu Gly Leu Arg Ala Pro Gln Trp Val Arg Asp Lys Met Val Thr
   450 455 460
   Met Cys Met Arg Cys Gln Glu Pro Phe Asn Ala Leu Thr Arg Arg Arg
   465 470 475 480
   His His Cys Arg Ala Cys Gly Tyr Val Val Cys Ala Arg Cys Ser Asp
   485 490 495
   Tyr Arg Ala Glu Leu Lys Tyr Asn Asp Asn Arg Gln Asn Arg Val Cys
   500 505 510
   Leu His Cys Tyr Thr Phe Leu Thr Gly Asn Val Leu Pro Glu Ala Lys
   515 520 525
   Glu Asp Lys Arg Arg Gly Ile Leu Glu Lys Gly Ser Ser Ala Thr Pro
   530 535 540
   Asp Gln Ser Leu Met Cys Ser Phe Leu Gln Leu Ile Gly Asp Lys Trp
   545 550 555 560
   Gly Lys Ser Gly Pro Arg Gly Trp Cys Val Ile Pro Arg Asp Asp Pro
   565 570 575
   Leu Val Leu Tyr Val Tyr Ala Ala Pro Gln Asp Met Arg Ala His Thr
   580 585 590
   Ser Ile Pro Leu Leu Gly Tyr Gln Val Thr Val Gly Pro Gln Gly Asp
   595 600 605
   Pro Arg Val Phe Gln Leu Gln Gln Ser Gly Gln Leu Tyr Thr Phe Lys
   610 615 620
   Ala Glu Thr Glu Glu Leu Lys Gly Arg Trp Val Lys Ala Met Glu Arg
   625 630 635 640
   Ala Ala Ser Gly Trp Ser Pro Ser Trp Pro Asn Asp Gly Asp Leu Ser
   645 650 655
   Asp

<210> 81
   <211> 680
   <212> PRT
   <213> Fgd2 Macaca mulatta transcript variant 3; deduced from ENSMMUP00000029213
<400> 81
   Met Phe Pro Lys Lys Ala Arg His Pro Gly Phe Pro Ala Leu Glu Ile
   1 5 10 15
   Cys Thr Arg Gln Pro Ser Thr Pro Gly Thr Cys Ser Cys Phe Pro Cys
   20 25 30
   Ser Pro Gly Arg Lys Pro Ser Gly Leu Ser Leu Leu Leu Arg Thr Pro
   35 40 45
   Glu Ala Ala Pro Arg Gly His Arg Leu Glu Asp Ala His His His Pro
   50 55 60
   Glu Cys Arg Pro Pro Arg Ser Pro Gly Pro Trp Glu Lys Leu Asn Val
   65 70 75 80
   Gly Glu Ala Met Gly Ser Glu Pro Arg Thr Val Ser Gly Arg Tyr Leu
   85 90 95
   Asn Ser Leu Lys Asn Lys Leu Ser Ser Gly Ala Trp Arg Lys Ser Cys
   100 105 110
   Gln Pro Val Thr Leu Ser Gly Ser Gly Met Gln Glu Pro Glu Lys Lys
   115 120 125
   Ile Val Gln Glu Leu Leu Glu Thr Glu Gln Ala Tyr Val Ala Arg Leu
   130 135 140
   His Leu Leu Asp Gln Ala Met Val Phe Phe Gln Glu Leu Leu Arg Ala
   145 150 155 160
   Ala Arg Arg Ser Lys Ala Phe Pro Glu Asp Val Val Arg Val Ile Phe
   165 170 175
   Ser Asn Ile Ser Ser Ile Tyr Gln Phe His Ser Gln Phe Phe Leu Pro
   180 185 190
   Glu Leu Gln Arg Arg Leu Asp Asp Trp Thr Ala Asn Pro Arg Ile Gly
   195 200 205
   Asp Val Ile Gln Lys Leu Ala Pro Phe Leu Lys Met Tyr Gly Glu Tyr
   210 215 220
   Val Lys Asn Phe Glu Arg Ala Ala Glu Leu Leu Ala Thr Trp Thr Glu
   225 230 235 240
   Lys Ser Pro Leu Phe Gln Glu Val Leu Thr Arg Ile Gln Ser Gly Glu
   245 250 255
   Ala Ser Gly Ser Leu Thr Leu Gln His His Met Leu Glu Pro Val Gln
   260 265 270
   Arg Ile Pro Arg Tyr Glu Leu Leu Leu Lys Glu Tyr Val Gln Lys Leu
   275 280 285
   Pro Ala Gln Ala Pro Asp Arg Ala Asp Ala Gln Lys Ala Leu Asp Met
   290 295 300
   Ile Phe Ser Ala Ala Gln His Ser Asn Ala Ala Ile Thr Glu Met Glu
   305 310 315 320
   Arg Leu Gln Asp Leu Trp Glu Val Tyr Gln Arg Leu Gly Leu Glu Asp
   325 330 335
   Asp Ile Val Asp Pro Ser Asn Thr Leu Leu Arg Glu Gly Pro Val Phe
   340 345 350
   Lys Ile Ser Phe Arg Arg Asn Asp Pro Met Glu Arg Tyr Leu Phe Leu
   355 360 365
   Phe Asn Asn Met Leu Leu Tyr Cys Val Pro Arg Val Ile Gln Val Gly
   370 375 380
   Ala Gln Phe Gln Val Arg Thr Arg Ile Asp Val Ala Gly Met Lys Val
   385 390 395 400
   Arg Glu Leu Met Asp Ala Glu Phe Pro His Ser Phe Leu Val Ser Gly
   405 410 415
   Lys Gln Arg Thr Leu Glu Leu Gln Ala Arg Ser Gln Glu Glu Lys Ile
   420 425 430
   Ser Trp Met Gln Ala Phe Gln Ala Ala Val Asp Gln Ile Glu Lys Arg
   435 440 445
   Asn Glu Thr Phe Lys Ala Ala Ala Gln Gly Pro Glu Gly Asp Thr Gln
   450 455 460
   Glu Gln Glu Leu Gln Ser Glu Glu Leu Gly Leu Arg Ala Pro Gln Trp
   465 470 475 480
   Val Arg Asp Lys Met Val Thr Met Cys Met Arg Cys Gln Glu Pro Phe
   485 490 495
   Asn Ala Leu Thr Arg Arg Arg His His Cys Arg Ala Cys Gly Tyr Val
   500 505 510
   Val Cys Ala Arg Cys Ser Asp Tyr Arg Ala Glu Leu Lys Tyr Asn Asp
   515 520 525
   Asn Arg Gln Asn Arg Val Cys Leu His Cys Tyr Thr Phe Leu Thr Gly
   530 535 540
   Asn Val Leu Pro Glu Ala Lys Glu Asp Lys Arg Arg Gly Ile Leu Glu
   545 550 555 560
   Lys Gly Ser Ser Ala Thr Pro Asp Gln Ser Leu Met Cys Ser Phe Leu
   565 570 575
   Gln Leu Ile Gly Asp Lys Trp Gly Lys Ser Gly Pro Arg Gly Trp Cys
   580 585 590
   Val Ile Pro Arg Asp Asp Pro Leu Val Leu Tyr Val Tyr Ala Ala Pro
   595 600 605
   Gln Asp Met Arg Ala His Thr Ser Ile Pro Leu Leu Gly Tyr Gln Val
   610 615 620
   Thr Val Gly Pro Gln Gly Asp Pro Arg Val Phe Gln Leu Gln Gln Ser
   625 630 635 640
   Gly Gln Leu Tyr Thr Phe Lys Ala Glu Thr Glu Glu Leu Lys Gly Arg
   645 650 655
   Trp Val Lys Ala Met Glu Arg Ala Ala Ser Gly Trp Ser Pro Ser Trp
   660 665 670
   Pro Asn Asp Gly Asp Leu Ser Asp
   675 680
<210> 82
   <211> 629
   <212> PRT
   <213> Fgd2 Monodelphis domestica; deduced from ENSMODP00000016834
<400> 82
   Ser Asn Ser Ile Ser Gln Met Pro Gln Thr Leu Met Glu Pro His Phe
   1 5 10 15
   Asp Arg Thr Pro Gly Pro Trp Glu Arg Asp Ser Leu Glu Asp Arg Pro
   20 25 30
   Gly Pro Gly His Trp Ala Val Ser Thr Arg Leu Leu Ser Ser Leu Arg
   35 40 45
   Asn Lys Ile Ser Asn Ser Gly Trp Arg Lys Ser Cys Glu Phe Pro Phe
   50 55 60
   Ser Asn Pro Asn Leu Pro Pro His Leu Gln Glu Pro Glu Glu Asn Arg
   65 70 75 80
   Ile Val Arg Glu Leu Leu Glu Thr Glu Gln Ala Tyr Val Ser Arg Leu
   85 90 95
   Tyr Leu Leu Asp Gln Val Phe Phe Glu Glu Leu Leu Lys Glu Ala Arg
   100 105 110
   Asn Ser Lys Ala Phe Ser Glu Asp Ile Val Lys Leu Ile Phe Ser Asn
   115 120 125
   Ile Ser Ser Ile His Gln Phe His Ser Gln Phe Phe Leu Pro Glu Leu
   130 135 140
   Gln Arg Arg Val Asp Glu Trp Val Val Val Pro Arg Ile Gly Asp Val
   145 150 155 160
   Ile Gln Lys Leu Ala Pro Phe Leu Lys Met Tyr Ser Glu Tyr Val Lys
   165 170 175
   Asn Phe Lys Arg Ala Ala Glu Leu Leu Val Ile Trp Thr Glu Lys Cys
   180 185 190
   Pro Pro Phe Gln Glu Val Ile Thr Arg Ile Gln Asn Ser Asp Ile Ser
   195 200 205
   Asp Ser Leu Thr Leu Gln His His Met Leu Glu Pro Val Gln Arg Ile
   210 215 220
   Pro Arg Tyr Glu Leu Leu Leu Lys Glu Tyr Val Gln Lys Leu Pro Pro
   225 230 235 240
   Asp Ala Pro Asp Arg Ala Asp Ala Gln Lys Ala Leu Asp Met Ile Phe
   245 250 255
   Ser Ala Ala Gln His Ser Asn Ala Ala Ile Thr Glu Met Glu Arg Leu
   260 265 270
   Gln Asn Leu Trp Asp Val Tyr Gln Arg Leu Gly Leu Glu Asp Asp Ile
   275 280 285
   Val Asp Pro Ser Asn Glu Leu Leu Gln Glu Gly Pro Ile Gln Lys Ile
   290 295 300
   Ser Phe Arg His Ser Ser Thr Met Glu Arg Tyr Leu Phe Leu Phe Asn
   305 310 315 320
   Asn Met Leu Leu Tyr Cys Val Pro Lys Val Ile Gln Val Gly Ala Gln
   325 330 335
   Phe Gln Val Arg Thr Arg Ile Asp Val Ala Gly Met Lys Val Arg Glu
   340 345 350
   Leu Asn Asp Val Glu Phe Pro His Ser Phe Leu Val Ser Gly Lys Gln
   355 360 365
   Arg Thr Leu Glu Leu Gln Ala Gln Ser Gln Glu Glu Met Asn Thr Trp
   370 375 380
   Ile Gln Ala Cys Gln Arg Ala Ile Asp Leu Ile Gly Arg Arg His Gly
   385 390 395 400
   Thr Phe Lys Ala Ala Val Gln Gly Thr Glu Gly Asp Ser Glu Glu His
   405 410 415
   Gln Leu Lys Ser Glu Glu Leu Gly Ile Arg Ala Pro Gln Trp Val Arg
   420 425 430
   Asp Lys Met Val Thr Met Cys Met Arg Cys Lys Thr Pro Phe Asn Ala
   435 440 445
   Leu Thr Arg Arg Arg His His Cys Arg Ala Cys Ser Tyr Val Val Cys
   450 455 460
   Ala Lys Cys Ser Asp Tyr Arg Ala Arg Leu Gln Tyr Asp Asp Asn Arg
   465 470 475 480
   Leu His Arg Val Cys Leu Gln Cys Tyr Val Phe Leu Thr Gly Asn Leu
   485 490 495
   Leu Pro Glu Asp Lys Glu Glu Lys Lys Lys Gly Ile Leu Glu Lys Glu
   500 505 510
   Ser Ser Lys Val Ser Glu Gln Ser Val Met Cys Ser Phe Leu Gln Leu
   515 520 525
   Leu Gly Asp Lys Trp Ala Lys Gly Ser Ser Arg Gly Trp Cys Val Ile
   530 535 540
   Pro Arg Asp Asp Pro Leu Ala Leu Tyr Val Tyr Ala Ala Pro Gln Asp
   545 550 555 560
   Met Lys Ala His Thr Ser Ile Pro Leu Leu Gly Tyr Gln Val Thr Thr
   565 570 575
   Gly Ser Leu Ala Asp Pro Arg Ala Phe Gln Ile Gln Gln Ser Gly Leu
   580 585 590
   Val Tyr Ser Phe Arg Ala Glu Ser Glu Glu Leu Lys Gly Gln Trp Met
   595 600 605
   Lys Ala Ile Glu Arg Ala Ser Arg Gly Gln Ser Phe Thr Gly Pro Glu
   610 615 620
   Gly Glu Leu Ser Asp
   625
<210> 83
   <211> 655
   <212> PRT
   <213> Fgd2 Pan troglodytes; deduced from ENSPTRG00000018116
<400> 83
   Met Lys Gly Ala Ser Glu Glu Lys Leu Ala Ser Val Ser Asn Leu Val
   1 5 10 15
   Thr Val Phe Glu Asn Ser Arg Thr Pro Glu Ala Ala Pro Arg Gly His
   20 25 30
   Arg Leu Glu Asp Val His His Arg Pro Glu Cys Arg Pro Pro Glu Ser
   35 40 45
   Pro Gly Pro Arg Glu Lys Thr Asn Val Gly Glu Ala Val Gly Ser Glu
   50 55 60
   Pro Arg Thr Val Ser Arg Arg Tyr Leu Asn Ser Leu Lys Asn Lys Leu
   65 70 75 80
   Ser Ser Glu Ala Trp Arg Lys Ser Cys Gln Pro Val Thr Leu Ser Gly
   85 90 95
   Ser Gly Thr Gln Glu Pro Glu Lys Lys Ile Val Gln Glu Leu Leu Glu
   100 105 110
   Thr Glu Gln Ala Tyr Val Ala Arg Leu His Leu Leu Asp Gln Val Phe
   115 120 125
   Phe Gln Glu Leu Leu Lys Thr Ala Arg Ser Ser Lys Ala Phe Pro Glu
   130 135 140
   Asp Val Val Arg Val Ile Phe Ser Asn Ile Ser Ser Ile Tyr Gln Phe
   145 150 155 160
   His Ser Gln Phe Phe Leu Pro Glu Leu Gln Arg Arg Leu Asp Asp Trp
                   165                 170                 175
   Thr Ala Asn Pro Arg Ile Gly Asp Val Ile Gln Lys Leu Ala Pro Phe
               180                 185                 190
   Leu Lys Met Tyr Ser Glu Tyr Val Lys Asn Phe Glu Arg Ala Ala Glu
           195                 200                 205
   Leu Leu Ala Thr Trp Thr Asp Lys Ser Pro Leu Phe Gln Glu Val Leu
       210                 215                 220
   Thr Arg Ile Gln Ser Ser Glu Ala Ser Gly Ser Leu Thr Leu Gln His
   225                 230                 235                 240
   His Met Leu Glu Pro Val Gln Arg Ile Pro Arg Tyr Glu Leu Leu Leu
                   245                 250                 255
   Lys Glu Tyr Ile Gln Lys Leu Pro Ala Gln Ala Pro Asp Gln Ala Asp
               260                 265                 270
   Ala Gln Lys Ala Leu Asp Met Ile Phe Ser Ala Ala Gln His Ser Asn
           275                 280                 285
   Ala Ala Ile Thr Glu Met Glu Arg Leu Gln Asp Leu Trp Glu Val Tyr
       290                 295                 300
   Gln Arg Leu Gly Leu Glu Asp Asp Ile Val Asp Pro Ser Asn Thr Leu
   305                 310                 315                 320
   Leu Arg Glu Gly Pro Val Leu Lys Ile Ser Phe Arg Arg Asn Asp Pro
                   325                 330                 335
   Met Glu Arg Tyr Leu Phe Leu Phe Asn Asn Met Leu Leu Tyr Cys Val
               340                 345                 350
   Pro Arg Val Ile Gln Val Gly Ala Gln Phe Gln Val Arg Thr Arg Ile
           355                 360                 365
   Asp Val Ala Gly Met Lys Val Arg Glu Leu Met Asp Ala Glu Phe Pro
       370                 375                 380
   His Ser Phe Leu Val Ser Gly Lys Gln Arg Thr Leu Glu Leu Gln Ala
   385                 390                 395                 400
   Arg Ser Gln Glu Glu Met Ile Ser Trp Met Gln Ala Phe Gln Ala Ala
                   405                 410                 415
   Ile Asp Gln Ile Glu Lys Arg Asn Glu Thr Phe Lys Ala Ala Ala Gln
   420 425 430
   Gly Pro Glu Gly Asp Thr Gln Glu Gln Glu Leu Gln Ser Glu Glu Leu
   435 440 445
   Gly Leu Arg Ala Pro Gln Trp Val Arg Asp Lys Met Val Thr Met Cys
   450 455 460
   Met Arg Cys Gln Glu Pro Phe Asn Ala Leu Thr Arg Arg Arg His His
   465 470 475 480
   Cys Arg Ala Cys Gly Tyr Val Val Cys Ala Arg Cys Ser His Tyr Arg
   485 490 495
   Ala Glu Leu Lys Tyr Asp Asp Asn Arg Pro Asn Arg Val Cys Leu His
   500 505 510
   Cys Tyr Ala Phe Leu Thr Gly Asn Val Leu Pro Glu Ala Lys Glu Asp
   515 520 525
   Lys Arg Arg Gly Ile Leu Glu Lys Gly Ser Ser Ala Thr Pro Asp Gln
   530 535 540
   Ser Leu Met Cys Ser Phe Leu Gln Leu Ile Gly Asp Lys Trp Gly Lys
   545 550 555 560
   Ser Gly Pro Arg Gly Trp Cys Val Ile Pro Arg Asp Asp Pro Leu Val
   565 570 575
   Leu Tyr Val Tyr Ala Ala Pro Gln Asp Met Arg Ala His Thr Ser Ile
   580 585 590
   Pro Leu Leu Gly Tyr Gln Val Thr Val Gly Pro Gln Gly Asp Pro Arg
   595 600 605
   Val Phe Gln Leu Gln Gln Ser Gly Gln Leu Tyr Thr Phe Lys Ala Glu
   610 615 620
   Thr Glu Glu Leu Lys Gly Arg Trp Val Lys Ala Met Glu Arg Ala Ala
   625 630 635 640
   Ser Gly Trp Ser Pro Ser Trp Pro Asn Asp Gly Asp Leu Ser Asp
   645 650 655
<210> 84
   <211> 697
   <212> PRT
<210> 85
   <211> 1725.
   <212> PRT
   <213> Tiam2 macacca mulatta transcript variant 1; deduced from ENSMMUP00000014621
<400> 85
   Met Gly Asn Ser Glu Ser Gln Tyr Thr Leu Gln Gly Ser Lys Asn His
   1 5 10 15
   Ser Asn Thr Ile Thr Gly Ala Lys Gln Ile Pro Cys Ser Leu Lys Ile
   20 25 30
   Arg Gly Ile His Ala Lys Glu Glu Lys Ser Leu His Gly Trp Val His
   35 40 45
   Gly Ser Ser Gly Ala Gly Tyr Lys Ser Arg Ser Leu Ala Arg Ser Cys
   50 55 60
   Leu Ser His Phe Lys Ser Asn Gln Pro Tyr Ala Ser Arg Leu Gly Gly
   65 70 75 80
   Ser Thr Cys Lys Val Ser Arg Gly Val Ala Tyr Ser Thr His Arg Thr
   85 90 95
   Asn Ala Pro Gly Lys Asp Phe Gln Gly Ile Ser Ala Ala Phe Ser Thr
   100 105 110
   Glu Asn Gly Phe His Ser Val Gly His Glu Pro Ala Asp Asn His Ile
   115 120 125
   Thr Ser Arg Asp Cys Asn Gly His Leu Leu Asn Cys Tyr Gly Arg Asn
   130 135 140
   Glu Ser Val Ala Ser Thr Pro Pro Gly Glu Asp Arg Lys Ser Pro Arg
   145 150 155 160
   Val Leu Ile Lys Thr Leu Gly Lys Leu Asp Gly Cys Leu Arg Val Glu
   165 170 175
   Phe His Asn Gly Gly Asn Pro Ser Lys Val Pro Ala Glu Asp Ser Ser
   180 185 190
   Glu Pro Val Gln Leu Leu Arg Tyr Ser Pro Thr Leu Ala Ser Glu Thr
   195 200 205
   Ser Pro Val Pro Glu Ala Arg Arg Gly Ser Ser Ala Asp Ser Leu Pro
   210 215 220
   Ser His Arg Pro Ser Pro Thr Asp Ser Arg Leu Arg Ser Ser Lys Gly
   225 230 235 240
   Ser Ser Leu Ser Ser Glu Ser Ser Trp Tyr Asp Ser Pro Trp Gly Asn
   245 250 255
   Ala Gly Glu Leu Ser Glu Ala Glu Gly Ser Phe Leu Ala Pro Gly Met
   260 265 270
   Pro Asp Pro Ser Leu His Ala Ser Phe Pro Pro Gly Asp Ala Lys Lys
           275                 280                 285
   Pro Phe Asn Gln Ser Ser Ser Leu Ser Ser Leu Arg Glu Leu Tyr Lys
       290                 295                 300
   Asp Ala Asn Leu Gly Ser Leu Ser Pro Ser Gly Ile Arg Leu Ser Asp
   305                 310                 315                 320
   Glu Tyr Met Gly Thr His Ala Ser Leu Ser Asn Arg Val Ser Phe Ala
                   325                 330                 335
   Ser Asp Ile Asp Val Pro Ser Arg Val Ala His Arg Asp Pro Val Gln
               340                 345                 350
   Tyr Ser Ser Phe Thr Leu Pro Cys Arg Lys Pro Lys Ala Leu Val Glu
           355                 360                 365
   Asp Thr Ala Lys Lys Asp Ser Leu Lys Ala Arg Met Arg Arg Ile Ser
       370                 375                 380
   Asp Trp Thr Gly Ser Leu Ser Arg Lys Lys Arg Lys Leu Gln Glu Pro
   385                 390                 395                 400
   Arg Ser Lys Glu Gly Ser Asp Tyr Phe Asp Ser Arg Ser Asp Gly Leu
                   405                 410                 415
   Asn Thr Glu Val Gln Gly Pro Ser Gln Ala Ser Ala Phe Leu Trp Ser
               420                 425                 430
   Gly Gly Ser Ala Gln Ile Leu Ser Gln Arg Ser Glu Ser Thr His Ala
           435                 440                 445
   Ile Gly Ser Asp Pro Leu Arg Gln Asn Ile Tyr Glu Asn Phe Met Arg
       450                 455                 460
   Glu Leu Glu Met Ser Arg Thr Asn Thr Glu Asn Ile Glu Met Ser Thr
   465                 470                 475                 480
   Glu Thr Ala Glu Ser Ser Ser Glu Ser Leu Ser Ser Leu Glu Gln Leu
                   485                 490                 495
   Asp Leu Leu Phe Glu Lys Glu Gln Gly Val Val Arg Lys Ala Gly Trp
               500                 505                 510
   Leu Phe Phe Lys Pro Leu Val Thr Val Gln Lys Glu Arg Lys Leu Glu
           515                 520                 525
   Leu Val Ala Arg Arg Lys Trp Lys Gln Tyr Trp Val Thr Leu Lys Gly
   530 535 540
   Cys Thr Leu Leu Phe Tyr Glu Thr Tyr Gly Lys Asn Ser Met Asp Gln
   545 550 555 560
   Ser Ser Ala Pro Arg Cys Ala Leu Phe Ala Glu Asp Ser Ile Val Gln
   565 570 575
   Ala Val Pro Glu His Pro Lys Lys Glu Asn Val Phe Cys Leu Ser Asn
   580 585 590
   Ser Phe Gly Asp Val Tyr Leu Phe Gln Ala Thr Ser Gln Thr Asp Leu
   595 600 605
   Glu Asn Trp Val Thr Ala Val His Ser Ala Cys Ala Ser Leu Phe Ala
   610 615 620
   Lys Lys His Gly Lys Glu Asp Thr Leu Arg Leu Leu Lys Asn Gln Thr
   625 630 635 640
   Lys Asn Leu Leu Gln Lys Ile Asp Met Asp Ser Lys Met Lys Lys Met
   645 650 655
   Ala Glu Leu Gln Leu Ser Ile Val Ser Asp Pro Lys Asn Arg Lys Ala
   660 665 670
   Ile Glu Asn Gln Ile Gln Gln Trp Glu Gln Asn Leu Glu Lys Phe His
   675 680 685
   Met Asp Leu Phe Arg Met Arg Cys Tyr Leu Ala Ser Leu Gln Gly Gly
   690 695 700
   Glu Leu Pro Asn Pro Lys Ser Leu Leu Ala Ala Ala Ser Arg Pro Ser
   705 710 715 720
   Lys Leu Ala Leu Gly Arg Leu Gly Ile Leu Ser Val Ser Ser Phe His
   725 730 735
   Ala Leu Val Cys Ser Arg Asp Asp Ser Ala Leu Arg Lys Arg Thr Leu
   740 745 750
   Ser Leu Thr Gln Arg Gly Arg Asn Lys Lys Gly Ile Phe Ser Ser Leu
   755 760 765
   Lys Gly Leu Asp Thr Leu Ala Arg Lys Gly Lys Glu Lys Arg Pro Ser
   770 775 780
   Ile Thr Gln Ile Phe Asp Leu Ser Gly Ser His Gly Phe Ser Gly Thr
   785                 790                 795                 800
   Arg Leu Pro Gln Asn Ser Asn Asn Ser Ser Glu Val Asp Glu Leu Leu
                   805                 810                 815
   His Met Tyr Gly Ser Thr Val Asp Ser Val Pro Arg Asp Asn Ala Trp
               820                 825                 830
   Glu Ile Gln Thr Tyr Val His Phe Gln Asp Asn His Gly Val Thr Val
           835                 840                 845
   Gly Ile Lys Pro Glu His Arg Val Glu Asp Ile Leu Thr Leu Ala Cys
       850                 855                 860
   Lys Met Arg Gln Leu Glu Pro Ser His Tyr Gly Leu Gln Leu Arg Lys
   865                 870                 875                 880
   Leu Val Asp Asp Asn Val Glu Tyr Tyr Ile Pro Ala Pro Tyr Glu Tyr
                   885                 890                 895
   Met Gln Glu Gln Val Tyr Asp Glu Ile Glu Val Phe Pro Leu Asn Val
               900                 905                 910
   Tyr Asp Val Gln Leu Thr Lys Thr Gly Ser Val Cys Asp Phe Gly Phe
           915                 920                 925
   Ala Val Thr Ala Gln Val Asp Glu His Gln His Leu Ser Arg Ile Phe
       930                 935                 940
   Val Ser Asp Val Leu Pro Asp Gly Leu Ala Tyr Gly Glu Gly Leu Arg
   945                 950                 955                 960
   Lys Gly Asn Glu Ile Met Thr Leu Asn Gly Glu Ala Val Ser Asp Leu
                   965                 970                 975
   Asp Leu Lys Gln Met Glu Ala Leu Phe Ser Glu Lys Ser Val Gly Leu
               980                 985                 990
   Thr Leu Ile Ala Arg Pro Pro Asp  Thr Lys Ala Thr Leu  Cys Thr Ser
           995                 1000                1005
   Trp Ser Asp Ser Asp Leu Phe  Ser Arg Asp Gln Lys  Ser Leu Leu
       1010                1015                1020
   Pro Pro  Pro Asn Gln Ser Gln  Leu Leu Glu Glu Phe  Leu Asp Asn
       1025                1030                1035
   Phe Lys Lys Asn Thr Ala Asn Asp Phe Ser Asn Val Pro Asp Ile
   1040 1045 1050
   Thr Thr Gly Leu Lys Arg Ser Gln Thr Asp Gly Thr Leu Asp Gln
   1055 1060 1065
   Val Ser His Arg Glu Lys Met Glu Gln Thr Phe Arg Ser Ala Glu
   1070 1075 1080
   Gln Ile Thr Ala Leu Cys Arg Ser Phe Asn Asp Thr Gln Ala Asn
   1085 1090 1095
   Gly Met Glu Gly Pro Arg Glu Ser Gln Asp Pro Pro Pro Arg Pro
   1100 1105 1110
   Leu Ala Arg His Leu Ser Asp Ala Asp Arg Leu Arg Lys Val Ile
   1115 1120 1125
   Gln Glu Leu Val Asp Thr Glu Lys Ser Tyr Val Lys Asp Leu Ser
   1130 1135 1140
   Cys Leu Phe Glu Leu Tyr Leu Glu Pro Leu Gln Asn Glu Thr Phe
   1145 1150 1155
   Leu Thr Gln Asp Glu Met Glu Ser Leu Phe Gly Ser Leu Pro Glu
   1160 1165 1170
   Met Leu Glu Phe Gln Lys Val Phe Leu Glu Thr Leu Glu Asp Gly
   1175 1180 1185
   Ile Ser Ala Ser Ser Asp Phe Asn Thr Leu Glu Thr Pro Ser Gln
   1190 1195 1200
   Phe Arg Lys Leu Leu Phe Ser Leu Gly Gly Ser Phe Leu Tyr Tyr
   1205 1210 1215
   Ala Asp His Phe Lys Leu Tyr Ser Gly Phe Cys Ala Asn His Ile
   1220 1225 1230
   Lys Val Gln Lys Val Leu Glu Arg Ala Lys Thr Asp Lys Ala Phe
   1235 1240 1245
   Lys Ala Phe Leu Asp Ala Arg Asn Pro Thr Lys Gln His Ser Ser
   1250 1255 1260
   Thr Leu Glu Ser Tyr Leu Ile Lys Pro Val Gln Arg Val Leu Lys
   1265 1270 1275
   Tyr Pro  Leu Leu Leu Lys Glu  Leu Val Ser Leu Thr  Asp Gln Glu
       1280                 1285                 1290
   Ser Glu  Glu His Tyr His Leu  Thr Glu Ala Leu Lys  Ala Met Glu
       1295                 1300                 1305
   Lys Val  Ala Ser His Ile Asn  Glu Met Gln Lys Ile  Tyr Glu Asp
       1310                 1315                 1320
   Tyr Gly  Thr Val Phe Asp Gln  Leu Val Ala Glu Gln  Ser Gly Thr
       1325                 1330                 1335
   Glu Lys  Glu Val Thr Glu Leu  Ser Met Gly Glu Leu  Leu Met His
       1340                 1345                 1350
   Ser Thr  Val Ser Trp Leu Asn  Pro Phe Leu Ser Leu  Gly Lys Ala
       1355                 1360                 1365
   Arg Lys  Asp Leu Glu Leu Thr  Val Phe Val Phe Lys  Arg Ala Val
       1370                 1375                 1380
   Ile Leu  Val Tyr Lys Glu Asn  Cys Lys Leu Lys Lys  Lys Leu Pro
       1385                 1390                 1395
   Ser Asn  Ser Arg Pro Ala His  Asn Ser Ala Asp Leu  Asp Pro Phe
       1400                 1405                 1410
   Lys Phe  Arg Trp Leu Ile Pro  Ile Ser Ala Leu Gln  Val Arg Leu
       1415                 1420                 1425
   Gly Asn  Pro Ala Gly Thr Glu  Asn Asn Ser Ile Trp  Glu Leu Ile
       1430                 1435                 1440
   His Thr  Lys Ser Glu Ile Glu  Gly Arg Pro Glu Thr  Ile Phe Gln
       1445                 1450                 1455
   Leu Cys  Cys Ser Asp Ser Glu  Ser Lys Thr Asn Ile  Val Lys Val
       1460                 1465                 1470
   Ile Arg  Ser Ile Leu Arg Glu  Asn Phe Arg Arg His  Ile Lys Cys
       1475                 1480                 1485
   Glu Leu  Pro Leu Glu Lys Thr  Cys Lys Asp Arg Leu  Val Pro Leu
       1490                 1495                 1500
   Lys Asn  Arg Val Pro Val Ser  Ala Lys Leu Ala Ser  Ser Arg Ser
       1505                 1510                 1515
   Leu Lys Val Leu Lys Asn Ser Ser Ser Asn Glu Trp Thr Gly Glu
   1520 1525 1530
   Thr Gly Lys Gly Thr Ser Leu Asp Ser Asp Glu Gly Ser Leu Ser
   1535 1540 1545
   Ser Gly Thr Gln Ser Ser Gly Cys Pro Thr Ala Glu Gly Arg Gln
   1550 1555 1560
   Asp Ser Lys Thr Ala Ser Pro Gly Lys Tyr Pro His Pro Gly Leu
   1565 1570 1575
   Ala Asp Phe Ala Asp Asn Leu Ile Lys Glu Ser Asp Ile Leu Ser
   1580 1585 1590
   Asp Glu Asp Asp Asp His His Gln Thr Leu Lys Arg Gly Ser Pro
   1595 1600 1605
   Thr Lys Asp Ile Glu Ile Gln Phe Gln Arg Leu Arg Ile Ser Glu
   1610 1615 1620
   Asp Pro Asp Val His Pro Glu Ala Glu Gln Gln Pro Gly Pro Glu
   1625 1630 1635
   Ala Gly Glu Gly Gln Lys Gly Gly Glu Gln Pro Lys Leu Val Arg
   1640 1645 1650
   Gly His Phe Cys Pro Ile Lys Arg Lys Ala Asn Ser Thr Lys Arg
   1655 1660 1665
   Asp Arg Gly Thr Leu Leu Lys Ala Gln Leu Arg His Gln Ser Leu
   1670 1675 1680
   Asp Ser Gln Ser Glu Asn Ala Thr Ile Asp Leu Asn Ser Val Leu
   1685 1690 1695
   Glu Arg Glu Phe Ser Val Gln Ser Leu Thr Ser Val Val Asn Glu
   1700 1705 1710
   Glu Cys Phe Tyr Glu Thr Glu Ser His Gly Lys Ser
   1715 1720 1725
<210> 86
   <211> 1701
   <212> PRT
   <213> Tiam2 macacca mulatta transcript variant 2; deduced from ENSMMUP00000014622
<400> 86
   Met Gly Asn Ser Glu Ser Gln Tyr Thr Leu Gln Gly Ser Lys Asn His
   1 5 10 15
   Ser Asn Thr Ile Thr Gly Ala Lys Gln Ile Pro Cys Ser Leu Lys Ile
   20 25 30
   Arg Gly Ile His Ala Lys Glu Glu Lys Ser Leu His Gly Trp Val His
   35 40 45
   Gly Ser Ser Gly Ala Gly Tyr Lys Ser Arg Ser Leu Ala Arg Ser Cys
   50 55 60
   Leu Ser His Phe Lys Ser Asn Gln Pro Tyr Ala Ser Arg Leu Gly Gly
   65 70 75 80
   Ser Thr Cys Lys Val Ser Arg Gly Val Ala Tyr Ser Thr His Arg Thr
   85 90 95
   Asn Ala Pro Gly Lys Asp Phe Gln Gly Ile Ser Ala Ala Phe Ser Thr
   100 105 110
   Glu Asn Gly Phe His Ser Val Gly His Glu Pro Ala Asp Asn His Ile
   115 120 125
   Thr Ser Arg Asp Cys Asn Gly His Leu Leu Asn Cys Tyr Gly Arg Asn
   130 135 140
   Glu Ser Val Ala Ser Thr Pro Pro Gly Glu Asp Arg Lys Ser Pro Arg
   145 150 155 160
   Val Leu Ile Lys Thr Leu Gly Lys Leu Asp Gly Cys Leu Arg Val Glu
   165 170 175
   Phe His Asn Gly Gly Asn Pro Ser Lys Val Pro Ala Glu Asp Ser Ser
   180 185 190
   Glu Pro Val Gln Leu Leu Arg Tyr Ser Pro Thr Leu Ala Ser Glu Thr
   195 200 205
   Ser Pro Val Pro Glu Ala Arg Arg Gly Ser Ser Ala Asp Ser Leu Pro
   210 215 220
   Ser His Arg Pro Ser Pro Thr Asp Ser Arg Leu Arg Ser Ser Lys Gly
   225 230 235 240
   Ser Ser Leu Ser Ser Glu Ser Ser Trp Tyr Asp Ser Pro Trp Gly Asn
   245 250 255
   Ala Gly Glu Leu Ser Glu Ala Glu Gly Ser Phe Leu Ala Pro Gly Met
               260                 265                 270
   Pro Asp Pro Ser Leu His Ala Ser Phe Pro Pro Gly Asp Ala Lys Lys
           275                 280                 285
   Pro Phe Asn Gln Ser Ser Ser Leu Ser Ser Leu Arg Glu Leu Tyr Lys
       290                 295                 300
   Asp Ala Asn Leu Gly Ser Leu Ser Pro Ser Gly Ile Arg Leu Ser Asp
   305                 310                 315                 320
   Glu Tyr Met Gly Thr His Ala Ser Leu Ser Asn Arg Val Ser Phe Ala
                   325                 330                 335
   Ser Asp Ile Asp Val Pro Ser Arg Val Ala His Arg Asp Pro Val Gln
               340                 345                 350
   Tyr Ser Ser Phe Thr Leu Pro Cys Arg Lys Pro Lys Ala Leu Val Glu
           355                 360                 365
   Asp Thr Ala Lys Lys Asp Ser Leu Lys Ala Arg Met Arg Arg Ile Ser
       370                 375                 380
   Asp Trp Thr Gly Ser Leu Ser Arg Lys Lys Arg Lys Leu Gln Glu Pro
   385                 390                 395                 400
   Arg Ser Lys Glu Gly Ser Asp Tyr Phe Asp Ser Arg Ser Asp Gly Leu
                   405                 410                 415
   Asn Thr Glu Val Gln Gly Pro Ser Gln Ala Ser Ala Phe Leu Trp Ser
               420                 425                 430
   Gly Gly Ser Ala Gln Ile Leu Ser Gln Arg Ser Glu Ser Thr His Ala
           435                 440                 445
   Ile Gly Ser Asp Pro Leu Arg Gln Asn Ile Tyr Glu Asn Phe Met Arg
       450                 455                 460
   Glu Leu Glu Met Ser Arg Thr Asn Thr Glu Asn Ile Glu Met Ser Thr
   465                 470                 475                 480
   Glu Thr Ala Glu Ser Ser Ser Glu Ser Leu Ser Ser Leu Glu Gln Leu
                   485                 490                 495
   Asp Leu Leu Phe Glu Lys Glu Gln Gly Val Val Arg Lys Ala Gly Trp
               500                 505                 510
   Leu Phe Phe Lys Pro Leu Val Thr Val Gln Lys Glu Arg Lys Leu Glu
   515 520 525
   Leu Val Ala Arg Arg Lys Trp Lys Gln Tyr Trp Val Thr Leu Lys Gly
   530 535 540
   Cys Thr Leu Leu Phe Tyr Glu Thr Tyr Gly Lys Asn Ser Met Asp Gln
   545 550 555 560
   Ser Ser Ala Pro Arg Cys Ala Leu Phe Ala Glu Asp Ser Ile Val Gln
   565 570 575
   Ala Val Pro Glu His Pro Lys Lys Glu Asn Val Phe Cys Leu Ser Asn
   580 585 590
   Ser Phe Gly Asp Val Tyr Leu Phe Gln Ala Thr Ser Gln Thr Asp Leu
   595 600 605
   Glu Asn Trp Val Thr Ala Val His Ser Ala Cys Ala Ser Leu Phe Ala
   610 615 620
   Lys Lys His Gly Lys Glu Asp Thr Leu Arg Leu Leu Lys Asn Gln Thr
   625 630 635 640
   Lys Asn Leu Leu Gln Lys Ile Asp Met Asp Ser Lys Met Lys Lys Met
   645 650 655
   Ala Glu Leu Gln Leu Ser Ile Val Ser Asp Pro Lys Asn Arg Lys Ala
   660 665 670
   Ile Glu Asn Gln Ile Gln Gln Trp Glu Gln Asn Leu Glu Lys Phe His
   675 680 685
   Met Asp Leu Phe Arg Met Arg Cys Tyr Leu Ala Ser Leu Gln Gly Gly
   690 695 700
   Glu Leu Pro Asn Pro Lys Ser Leu Leu Ala Ala Ala Ser Arg Pro Ser
   705 710 715 720
   Lys Leu Ala Leu Gly Arg Leu Gly Ile Leu Ser Val Ser Ser Phe His
   725 730 735
   Ala Leu Val Cys Ser Arg Asp Asp Ser Ala Leu Arg Lys Arg Thr Leu
   740 745 750
   Ser Leu Thr Gln Arg Gly Arg Asn Lys Lys Gly Ile Phe Ser Ser Leu
   755 760 765
   Lys Gly Leu Asp Thr Leu Ala Arg Lys Gly Lys Glu Lys Arg Pro Ser
       770                 775                 780
   Ile Thr Gln Val Asp Glu Leu Leu His Met Tyr Gly Ser Thr Val Asp
   785                 790                 795                 800
   Ser Val Pro Arg Asp Asn Ala Trp Glu Ile Gln Thr Tyr Val His Phe
                   805                 810                 815
   Gln Asp Asn His Gly Val Thr Val Gly Ile Lys Pro Glu His Arg Val
               820                 825                 830
   Glu Asp Ile Leu Thr Leu Ala Cys Lys Met Arg Gln Leu Glu Pro Ser
           835                 840                 845
   His Tyr Gly Leu Gln Leu Arg Lys Leu Val Asp Asp Asn Val Glu Tyr
       850                 855                 860
   Tyr Ile Pro Ala Pro Tyr Glu Tyr Met Gln Glu Gln Val Tyr Asp Glu
   865                 870                 875                 880
   Ile Glu Val Phe Pro Leu Asn Val Tyr Asp Val Gln Leu Thr Lys Thr
                   885                 890                 895
   Gly Ser Val Cys Asp Phe Gly Phe Ala Val Thr Ala Gln Val Asp Glu
               900                 905                 910
   His Gln His Leu Ser Arg Ile Phe Val Ser Asp Val Leu Pro Asp Gly
           915                 920                 925
   Leu Ala Tyr Gly Glu Gly Leu Arg Lys Gly Asn Glu Ile Met Thr Leu
       930                 935                 940
   Asn Gly Glu Ala Val Ser Asp Leu Asp Leu Lys Gln Met Glu Ala Leu
   945                 950                 955                 960
   Phe Ser Glu Lys Ser Val Gly Leu Thr Leu Ile Ala Arg Pro Pro Asp
                   965                 970                 975
   Thr Lys Ala Thr Leu Cys Thr Ser Trp Ser Asp Ser Asp Leu Phe Ser
               980                 985                 990
   Arg Asp Gln Lys Ser Leu Leu Pro  Pro Pro Asn Gln Ser  Gln Leu Leu
           995                 1000                 1005
   Glu Glu  Phe Leu Asp Asn Phe  Lys Lys Asn Thr Ala  Asn Asp Phe
       1010                 1015                 1020
   Ser Asn Val Pro Asp Ile Thr Thr Gly Leu Lys Arg Ser Gln Thr
   1025 1030 1035
   Asp Gly Thr Leu Asp Gln Val Ser His Arg Glu Lys Met Glu Gln
   1040 1045 1050
   Thr Phe Arg Ser Ala Glu Gln Ile Thr Ala Leu Cys Arg Ser Phe
   1055 1060 1065
   Asn Asp Thr Gln Ala Asn Gly Met Glu Gly Pro Arg Glu Ser Gln
   1070 1075 1080
   Asp Pro Pro Pro Arg Pro Leu Ala Arg His Leu Ser Asp Ala Asp
   1085 1090 1095
   Arg Leu Arg Lys Val Ile Gln Glu Leu Val Asp Thr Glu Lys Ser
   1100 1105 1110
   Tyr Val Lys Asp Leu Ser Cys Leu Phe Glu Leu Tyr Leu Glu Pro
   1115 1120 1125
   Leu Gln Asn Glu Thr Phe Leu Thr Gln Asp Glu Met Glu Ser Leu
   1130 1135 1140
   Phe Gly Ser Leu Pro Glu Met Leu Glu Phe Gln Lys Val Phe Leu
   1145 1150 1155
   Glu Thr Leu Glu Asp Gly Ile Ser Ala Ser Ser Asp Phe Asn Thr
   1160 1165 1170
   Leu Glu Thr Pro Ser Gln Phe Arg Lys Leu Leu Phe Ser Leu Gly
   1175 1180 1185
   Gly Ser Phe Leu Tyr Tyr Ala Asp His Phe Lys Leu Tyr Ser Gly
   1190 1195 1200
   Phe Cys Ala Asn His Ile Lys Val Gln Lys Val Leu Glu Arg Ala
   1205 1210 1215
   Lys Thr Asp Lys Ala Phe Lys Ala Phe Leu Asp Ala Arg Asn Pro
   1220 1225 1230
   Thr Lys Gln His Ser Ser Thr Leu Glu Ser Tyr Leu Ile Lys Pro
   1235 1240 1245
   Val Gln Arg Val Leu Lys Tyr Pro Leu Leu Leu Lys Glu Leu Val
   1250 1255 1260
   Ser Leu Thr Asp Gln Glu Ser Glu Glu His Tyr His Leu Thr Glu
   1265 1270 1275
   Ala Leu Lys Ala Met Glu Lys Val Ala Ser His Ile Asn Glu Met
   1280 1285 1290
   Gln Lys Ile Tyr Glu Asp Tyr Gly Thr Val Phe Asp Gln Leu Val
   1295 1300 1305
   Ala Glu Gln Ser Gly Thr Glu Lys Glu Val Thr Glu Leu Ser Met
   1310 1315 1320
   Gly Glu Leu Leu Met His Ser Thr Val Ser Trp Leu Asn Pro Phe
   1325 1330 1335
   Leu Ser Leu Gly Lys Ala Arg Lys Asp Leu Glu Leu Thr Val Phe
   1340 1345 1350
   Val Phe Lys Arg Ala Val Ile Leu Val Tyr Lys Glu Asn Cys Lys
   1355 1360 1365
   Leu Lys Lys Lys Leu Pro Ser Asn Ser Arg Pro Ala His Asn Ser
   1370 1375 1380
   Ala Asp Leu Asp Pro Phe Lys Phe Arg Trp Leu Ile Pro Ile Ser
   1385 1390 1395
   Ala Leu Gln Val Arg Leu Gly Asn Pro Ala Gly Thr Glu Asn Asn
   1400 1405 1410
   Ser Ile Trp Glu Leu Ile His Thr Lys Ser Glu Ile Glu Gly Arg
   1415 1420 1425
   Pro Glu Thr Ile Phe Gln Leu Cys Cys Ser Asp Ser Glu Ser Lys
   1430 1435 1440
   Thr Asn Ile Val Lys Val Ile Arg Ser Ile Leu Arg Glu Asn Phe
   1445 1450 1455
   Arg Arg His Ile Lys Cys Glu Leu Pro Leu Glu Lys Thr Cys Lys
   1460 1465 1470
   Asp Arg Leu Val Pro Leu Lys Asn Arg Val Pro Val Ser Ala Lys
   1475 1480 1485
   Leu Ala Ser Ser Arg Ser Leu Lys Val Leu Lys Asn Ser Ser Ser
   1490 1495 1500
   Asn Glu Trp Thr Gly Glu Thr Gly Lys Gly Thr Ser Leu Asp Ser
   1505 1510 1515
   Asp Glu Gly Ser Leu Ser Ser Gly Thr Gln Ser Ser Gly Cys Pro
   1520 1525 1530
   Thr Ala Glu Gly Arg Gln Asp Ser Lys Thr Ala Ser Pro Gly Lys
   1535 1540 1545
   Tyr Pro His Pro Gly Leu Ala Asp Phe Ala Asp Asn Leu Ile Lys
   1550 1555 1560
   Glu Ser Asp Ile Leu Ser Asp Glu Asp Asp Asp His His Gln Thr
   1565 1570 1575
   Leu Lys Arg Gly Ser Pro Thr Lys Asp Ile Glu Ile Gln Phe Gln
   1580 1585 1590
   Arg Leu Arg Ile Ser Glu Asp Pro Asp Val His Pro Glu Ala Glu
   1595 1600 1605
   Gln Gln Pro Gly Pro Glu Ala Gly Glu Gly Gln Lys Gly Gly Glu
   1610 1615 1620
   Gln Pro Lys Leu Val Arg Gly His Phe Cys Pro Ile Lys Arg Lys
   1625 1630 1635
   Ala Asn Ser Thr Lys Arg Asp Arg Gly Thr Leu Leu Lys Ala Gln
   1640 1645 1650
   Leu Arg His Gln Ser Leu Asp Ser Gln Ser Glu Asn Ala Thr Ile
   1655 1660 1665
   Asp Leu Asn Ser Val Leu Glu Arg Glu Phe Ser Val Gln Ser Leu
   1670 1675 1680
   Thr Ser Val Val Asn Glu Glu Cys Phe Tyr Glu Thr Glu Ser His
   1685 1690 1695
   Gly Lys Ser
   1700
<210> 87
   <211> 1730
   <212> PRT
   <213> Tiam2 Monodelphis domesticus transcript variant 1; deduced from ENSMODP00000029251
<400> 87
   Val Thr Met Gly Asn Ser Glu Ser Gln Tyr Ser Leu Gln Gly Ser Lys
   1 5 10 15
   Asn His Ser Ser Ser Ser Thr Gly Ala Lys Gln Lys Pro Cys Pro Leu
   20 25 30
   Lys Ile Arg Ser Ile His Ala Lys Asp Glu Lys Ser Cys Ser Leu His
   35 40 45
   Gly Trp Gly His Gly Asn Ser Gly Thr Asn Tyr Lys Ser Arg Ser Leu
   50 55 60
   Ala Arg Ser Cys Leu Ser His Phe Lys Ser Asn Gln Pro Tyr Ser Ser
   65 70 75 80
   Arg Leu Asp Pro Met Ala Lys Val Ser Lys Gly Ser Ala His Thr Lys
   85 90 95
   His Lys Gly Asn Val Ser Gly Asn Tyr Phe Gln Gly Lys Asn Ser Ala
   100 105 110
   Phe Leu Ser Glu Asn Gly Phe His Tyr Ile Ser His Glu Pro Ala Asp
   115 120 125
   Asn His Ile Thr Ser Arg Asp Cys Asn Gly His Leu Leu Asn Cys Tyr
   130 135 140
   Gly Lys Asn Glu Ser Leu Ala Ser Thr Pro Pro Ala Glu Asp Arg Lys
   145 150 155 160
   Ser Pro Lys Val Leu Ile Lys Thr Leu Gly Lys Leu Asp Gly Cys Leu
   165 170 175
   Arg Val Glu Phe His Asn Ser Ser Asn Asn Lys Val Pro Thr Glu Asp
   180 185 190
   Ser Thr Gly Pro Val Gln Leu Leu Arg Tyr Ser Pro Thr Leu Glu Ser
   195 200 205
   Glu Thr Ser Asn Leu Thr Glu Ile Arg Arg Asn Ser Ser Thr Asp Tyr
   210 215 220
   Ser Ala Ser His Cys Leu Ser Pro Thr Asp Ser Arg Leu Arg Ser Ser
   225 230 235 240
   Lys Gly Ser Ser Phe Ser Ser Glu Ser Ser Trp Tyr Asp Ser Pro Trp
   245 250 255
   Gly Asn Ala Gly Asp Ile Asn Glu Leu Glu Gly Pro Tyr Leu Thr Arg
   260 265 270
   Ser Thr Pro Asp Thr Ser Ile His Ser Ser Phe Pro Ser Asp Asp Ala
   275 280 285
   Lys Lys Pro Phe Asn Gln Ser Ser Ser Leu Ser Ser Leu Arg Glu Leu
   290 295 300
   Tyr Lys Asp Ala Thr Leu Glu Ser Leu Pro Pro Ser Gly Ile Arg Phe
   305 310 315 320
   Ser Asp Glu Tyr Ile Gly Thr His Ala Ser Leu Asn Ser Arg Val Ser
   325 330 335
   Phe Val Ser Asp Ile Asp Val Pro Ser Arg Ala Glu Gln Arg Gly Pro
   340 345 350
   Val Gln Tyr Cys Ser Tyr Thr Leu Pro Cys Arg Lys Ser Lys Pro Leu
   355 360 365
   Thr Glu Asp Thr Ser Lys Lys Asp Thr Leu Lys Thr Arg Met Arg Arg
   370 375 380
   Ile Ser Asp Trp Thr Gly Ser Leu Ser Arg Lys Lys Arg Lys Leu Gln
   385 390 395 400
   Glu Pro Lys Ser Lys Asp Gly Tyr Asp His Leu Glu Asn Arg Thr Asp
   405 410 415
   Gly Leu Ser Ala Glu Val Leu Val Pro Ser Gln Leu Ser Ser Leu Leu
   420 425 430
   Trp Pro Gly Gly Ser Gly Gln Thr Leu Pro Gln Arg Ser Glu Ser Thr
   435 440 445
   Asn Ala Ile Ser Ser Asp Pro Leu Arg Gln Asn Ile Tyr Glu Asn Phe
   450 455 460
   Met Arg Glu Leu Glu Met Ser Arg Thr His Val Asp Asn Thr Glu Thr
   465 470 475 480
   Ser Thr Asp Thr Ala Asp Ser Ser Ser Asp Ser Leu Ser Ser Leu Glu
   485 490 495
   Gln Leu Asp Leu Leu Phe Glu Lys Glu Gln Gly Val Val Arg Lys Ala
   500 505 510
   Gly Trp Leu Phe Phe Lys Pro Leu Val Thr Leu Gln Lys Glu Lys Lys
   515 520 525
   Leu Glu Leu Val Ala Arg Arg Lys Trp Lys Gln Tyr Trp Val Thr Leu
   530 535 540
   Lys Gly Cys Thr Leu Leu Phe Tyr Glu Thr Tyr Gly Arg Asn Ser Met
   545 550 555 560
   Asp Gln Ser Ser Ser Pro Arg Cys Ala Leu Phe Ala Glu Asp Ser Val
   565 570 575
   Val Gln Ser Val Pro Glu His Pro Lys Lys Glu Asn Val Phe Cys Leu
   580 585 590
   Ser Asn Ser Phe Gly Asp Val Tyr Leu Phe Gln Ala Thr Ser Gln Thr
   595 600 605
   Asp Leu Glu Asn Trp Val Thr Ala Ile His Ser Ala Cys Ala Ser Leu
   610 615 620
   Phe Ala Lys Lys His Gly Lys Glu Asp Thr Val Arg Leu Leu Lys Asn
   625 630 635 640
   Gln Thr Lys Asn Leu Leu Gln Lys Ile Asp Met Asp Ser Lys Met Lys
   645 650 655
   Lys Met Ala Glu Leu Gln Leu Ser Val Val Ser Asp Pro Lys Asn Arg
   660 665 670
   Lys Ala Ile Glu Asn Gln Ile Gln Gln Trp Glu Gln Asn Leu Glu Lys
   675 680 685
   Phe His Met Asp Leu Phe Arg Met Arg Cys Tyr Leu Ala Ser Leu Gln
   690 695 700
   Gly Gly Glu Leu Pro Asn Pro Lys Ser Leu Leu Ala Ala Thr Ser Arg
   705 710 715 720
   Pro Ser Lys Leu Ala Leu Gly Arg Leu Gly Ile Leu Ser Val Ser Ser
   725 730 735
   Phe His Ala Leu Ile Cys Ser Arg Asp Glu Ser Ala Leu Arg Lys Arg
   740 745 750
   Thr Leu Ser Leu Thr Gln Gln Gly Gln Ser Lys Lys Gly Leu Phe Ser
   755 760 765
   Ser Leu Lys Gly Leu Asp Thr Leu Ala Arg Lys Gly Lys Asp Lys Arg
       770                 775                 780
   Pro Ser Ile Thr Gln Ile Phe Asp Ser Ser Gly Asn His Gly Phe Pro
   785                 790                 795                 800
   Gly Thr Gln Leu Pro Gln Ser Ser Asn Asn Ser Ser Glu Val Asp Glu
                   805                 810                 815
   Phe Gln His Ile Tyr Ser Ser Ala Ser Asp Ser Gly Pro Arg Glu Asn
               820                 825                 830
   Ile Trp Glu Ser Gln Thr Tyr Val His Phe Gln Asp Asn Gln Gly Val
           835                 840                 845
   Thr Leu Ile Ile Lys Pro Glu His Arg Val Glu Asp Ile Leu Ser Leu
       850                 855                 860
   Ser Cys Lys Met Lys Gln Leu Asp Pro Ser Tyr Tyr Gly Leu Gln Leu
   865                 870                 875                 880
   Arg Arg Leu Val Asp Asp Asn Phe Glu Tyr Ser Ile Pro Ala Leu Tyr
                   885                 890                 895
   Glu Tyr Met Gln Glu Gln Ala Ile Val Tyr Asp Glu Ile Glu Ile Phe
               900                 905                 910
   Pro Leu Ser Ile Tyr His Val Leu Leu Thr Lys Thr Gly Asn Ile Thr
           915                 920                 925
   Asp Phe Gly Phe Ala Val Thr Ala Gln Val Asp Glu His Gln His Leu
       930                 935                 940
   Ser Arg Ile Phe Ile Ser Asp Val Leu Pro Asp Gly Leu Ala Tyr Gly
   945                 950                 955                 960
   Glu Gly Leu Arg Thr Gly Asn Glu Ile Leu Thr Ile Asn Gly Glu Val
                   965                 970                 975
   Ile Ser Asp Leu Asp Leu Arg Gln Met Glu Ser Leu Phe Ser Glu Arg
               980                 985                 990
   Ser Val Arg Leu Thr Leu Arg Thr  Asn Ser Ser Asp Thr  Lys Arg Thr
           995                 1000                 1005
   Leu Gly  Thr Ser Trp Ser Asp  Ser Asp Leu Ser Arg  Asp Gln Lys
       1010                 1015                 1020
   Asn Leu Leu Pro Pro Pro Asn Gln Ser Gln Leu Leu Glu Glu Phe
   1025 1030 1035
   Leu Asp Asn Leu Lys Lys Asn Thr Ala Asn Asp Phe Ser Asn Val
   1040 1045 1050
   Pro Asp Val Thr Ala Gly Leu Lys Arg Ser Gln Thr Asp Gly Thr
   1055 1060 1065
   Leu Asp Gln Val Pro His Arg Glu Lys Thr Glu His Thr Phe Arg
   1070 1075 1080
   Ser Ala Glu Gln Ile Ala Ala Leu Cys Arg Ser Phe Asn Glu Val
   1085 1090 1095
   Gln Pro Ser Thr Asp Ser Met Glu Gly Pro Lys Glu Ala Gln Glu
   1100 1105 1110
   Pro Pro Pro Arg Pro Leu Ala Arg His Leu Ser Asp Ala Asp Arg
   1115 1120 1125
   Leu Arg Lys Val Ile Gln Glu Leu Met Asp Thr Glu Lys Ser Tyr
   1130 1135 1140
   Val Lys Asp Leu Thr Cys Leu Phe Glu Leu Tyr Leu Glu Pro Leu
   1145 1150 1155
   Gln Asn Glu Thr Phe Leu Thr Gln Asp Glu Met Glu Ser Leu Phe
   1160 1165 1170
   Gly Ser Leu Pro Glu Met Leu Glu Phe Gln Lys Val Phe Leu Glu
   1175 1180 1185
   Thr Leu Glu Asp Gly Ile Ser Ser Ser Ser Asp Phe Asn Ile Leu
   1190 1195 1200
   Glu Thr Pro Ser Gln Phe Arg Lys Leu Leu Phe Ser Leu Gly Gly
   1205 1210 1215
   Ser Phe Leu Tyr Tyr Thr Asp His Phe Lys Leu Tyr Ser Gly Phe
   1220 1225 1230
   Cys Ala Asn His Ile Lys Val Gln Lys Val Leu Glu Arg Ala Lys
   1235 1240 1245
   Thr Asp Lys Ala Phe Lys Ala Phe Leu Asp Ala Arg Asn Pro Thr
   1250 1255 1260
   Lys Gln His Ala Ser Thr Leu Glu Ser Tyr Leu Ile Lys Pro Val
   1265 1270 1275
   Gln Arg Val Leu Lys Tyr Pro Leu Leu Leu Lys Glu Leu Val Ser
   1280 1285 1290
   Leu Thr Asp Asn Glu Ser Glu Glu His Tyr His Leu Thr Glu Ala
   1295 1300 1305
   Leu Lys Ala Met Glu Lys Val Ala Ser His Ile Asn Glu Met Gln
   1310 1315 1320
   Lys Ile Tyr Glu Asp Tyr Gly Thr Val Phe Asp Gln Leu Val Ala
   1325 1330 1335
   Glu Gln Ser Gly Thr Glu Lys Glu Val Thr Glu Leu Ser Met Gly
   1340 1345 1350
   Glu Leu Leu Leu His Ser Thr Val Ser Trp Leu Asn Pro Phe Leu
   1355 1360 1365
   Ser Leu Gly Lys Ala Arg Lys Asp Leu Glu Leu Thr Val Phe Val
   1370 1375 1380
   Phe Lys Arg Ala Val Ile Leu Val Tyr Lys Glu Asn Cys Lys Leu
   1385 1390 1395
   Lys Lys Lys Leu Pro Ser Ser Ser Arg Pro Thr His Val His Gly
   1400 1405 1410
   Asp Leu Asp Pro Phe Lys Phe Arg Trp Leu Ile Pro Leu Ser Ala
   1415 1420 1425
   Leu Gln Val Arg Leu Gly Asn Ala Ala Gly Thr Glu Asn Asn Ser
   1430 1435 1440
   Ile Trp Glu Leu Ile His Thr Lys Ser Glu Ile Glu Gly Arg Pro
   1445 1450 1455
   Glu Thr Thr Phe Gln Leu Cys Cys Ser Asp Cys Glu Ser Lys Thr
   1460 1465 1470
   Asn Ile Val Lys Val Ile Arg Ser Ile Leu Arg Glu Asn Phe Arg
   1475 1480 1485
   Arg His Ile Lys Ser Glu Leu Pro Leu Glu Lys Thr Cys Lys Asp
   1490 1495 1500
   Arg Leu Ile Pro Leu Lys Asn Arg Ile Pro Val Ser Ala Lys Leu
   1505 1510 1515
   Ala Ser Ser Arg Ser Leu Arg Val Leu Lys Asn Ser Pro Ser Asn
   1520 1525 1530
   Glu Trp Asn Ser Asp Thr Gly Lys Gly Asn Leu Leu Asp Ser Asp
   1535 1540 1545
   Glu Cys Ser Leu Ser Ser Ser Thr Gln Ser Ser Gly Cys His Thr
   1550 1555 1560
   Thr Glu Ser Arg Gln Glu Ser Lys Asp Ser Ser Pro Glu Lys Tyr
   1565 1570 1575
   Pro Gln Thr Cys Ser Ser Asp Phe Ser Asp Ser Leu Ile Lys Glu
   1580 1585 1590
   Ser Asp Ile Leu Ser Asp Glu Asp Asp Asp Phe His Gln Thr Leu
   1595 1600 1605
   Lys Lys Gly Ser Pro Thr Lys Asp Ile Glu Ile Gln Phe Gln Arg
   1610 1615 1620
   Leu Lys Ile Ser Glu Asp Ser Glu Gly Asp Ser Thr Ala Asp Gln
   1625 1630 1635
   Gln Pro Arg Thr Glu Val Gly Asn Asn Leu Asn Ser Val Ala His
   1640 1645 1650
   Pro Lys Leu Val Arg Gly His Phe Cys Pro Ile Lys Arg Lys Ala
   1655 1660 1665
   Asn Ser Thr Lys Arg Asp Arg Gly Thr Leu Leu Thr Leu Gln Ala
   1670 1675 1680
   Arg His Gln Ser Leu Asp Ser Lys Ser Glu Asn Ala Asn Ile Asp
   1685 1690 1695
   Leu Asn Ser Ile Leu Glu Arg Glu Phe Ser Val Gln Ser Leu Thr
   1700 1705 1710
   Ser Val Val Asn Glu Glu Cys Phe Tyr Glu Thr Asp Ser His Gly
   1715 1720 1725
   Lys Ser
   1730
<210> 88
   <211> 1706
   <212> PRT
   <213> Tiam2 Monodelphis domestica transcript variant 2; deduced from ENSMODP00000010258
<400> 88
   Val Thr Met Gly Asn Ser Glu Ser Gln Tyr Ser Leu Gln Gly Ser Lys
   1               5                   10                  15
   Asn His Ser Ser Ser Ser Thr Gly Ala Lys Gln Lys Pro Cys Pro Leu
               20                  25                  30
   Lys Ile Arg Ser Ile His Ala Lys Asp Glu Lys Ser Cys Ser Leu His
           35                  40                  45
   Gly Trp Gly His Gly Asn Ser Gly Thr Asn Tyr Lys Ser Arg Ser Leu
       50                  55                  60
   Ala Arg Ser Cys Leu Ser His Phe Lys Ser Asn Gln Pro Tyr Ser Ser
   65                  70                  75                  80
   Arg Leu Asp Pro Met Ala Lys Val Ser Lys Gly Ser Ala His Thr Lys
                   85                  90                  95
   His Lys Gly Asn Val Ser Gly Asn Tyr Phe Gln Gly Lys Asn Ser Ala
               100                 105                 110
   Phe Leu Ser Glu Asn Gly Phe His Tyr Ile Ser His Glu Pro Ala Asp
           115                 120                 125
   Asn His Ile Thr Ser Arg Asp Cys Asn Gly His Leu Leu Asn Cys Tyr
       130                 135                 140
   Gly Lys Asn Glu Ser Leu Ala Ser Thr Pro Pro Ala Glu Asp Arg Lys
   145                 150                 155                 160
   Ser Pro Lys Val Leu Ile Lys Thr Leu Gly Lys Leu Asp Gly Cys Leu
                   165                 170                 175
   Arg Val Glu Phe His Asn Ser Ser Asn Asn Lys Val Pro Thr Glu Asp
               180                 185                 190
   Ser Thr Gly Pro Val Gln Leu Leu Arg Tyr Ser Pro Thr Leu Glu Ser
           195                 200                 205
   Glu Thr Ser Asn Leu Thr Glu Ile Arg Arg Asn Ser Ser Thr Asp Tyr
       210                 215                 220
   Ser Ala Ser His Cys Leu Ser Pro Thr Asp Ser Arg Leu Arg Ser Ser
   225 230 235 240
   Lys Gly Ser Ser Phe Ser Ser Glu Ser Ser Trp Tyr Asp Ser Pro Trp
   245 250 255
   Gly Asn Ala Gly Asp Ile Asn Glu Leu Glu Gly Pro Tyr Leu Thr Arg
   260 265 270
   Ser Thr Pro Asp Thr Ser Ile His Ser Ser Phe Pro Ser Asp Asp Ala
   275 280 285
   Lys Lys Pro Phe Asn Gln Ser Ser Ser Leu Ser Ser Leu Arg Glu Leu
   290 295 300
   Tyr Lys Asp Ala Thr Leu Glu Ser Leu Pro Pro Ser Gly Ile Arg Phe
   305 310 315 320
   Ser Asp Glu Tyr Ile Gly Thr His Ala Ser Leu Asn Ser Arg Val Ser
   325 330 335
   Phe Val Ser Asp Ile Asp Val Pro Ser Arg Ala Glu Gln Arg Gly Pro
   340 345 350
   Val Gln Tyr Cys Ser Tyr Thr Leu Pro Cys Arg Lys Ser Lys Pro Leu
   355 360 365
   Thr Glu Asp Thr Ser Lys Lys Asp Thr Leu Lys Thr Arg Met Arg Arg
   370 375 380
   Ile Ser Asp Trp Thr Gly Ser Leu Ser Arg Lys Lys Arg Lys Leu Gln
   385 390 395 400
   Glu Pro Lys Ser Lys Asp Gly Tyr Asp His Leu Glu Asn Arg Thr Asp
   405 410 415
   Gly Leu Ser Ala Glu Val Leu Val Pro Ser Gln Leu Ser Ser Leu Leu
   420 425 430
   Trp Pro Gly Gly Ser Gly Gln Thr Leu Pro Gln Arg Ser Glu Ser Thr
   435 440 445
   Asn Ala Ile Ser Ser Asp Pro Leu Arg Gln Asn Ile Tyr Glu Asn Phe
   450 455 460
   Met Arg Glu Leu Glu Met Ser Arg Thr His Val Asp Asn Thr Glu Thr
   465 470 475 480
   Ser Thr Asp Thr Ala Asp Ser Ser Ser Asp Ser Leu Ser Ser Leu Glu
   485 490 495
   Gln Leu Asp Leu Leu Phe Glu Lys Glu Gln Gly Val Val Arg Lys Ala
   500 505 510
   Gly Trp Leu Phe Phe Lys Pro Leu Val Thr Leu Gln Lys Glu Lys Lys
   515 520 525
   Leu Glu Leu Val Ala Arg Arg Lys Trp Lys Gln Tyr Trp Val Thr Leu
   530 535 540
   Lys Gly Cys Thr Leu Leu Phe Tyr Glu Thr Tyr Gly Arg Asn Ser Met
   545 550 555 560
   Asp Gln Ser Ser Ser Pro Arg Cys Ala Leu Phe Ala Glu Asp Ser Val
   565 570 575
   Val Gln Ser Val Pro Glu His Pro Lys Lys Glu Asn Val Phe Cys Leu
   580 585 590
   Ser Asn Ser Phe Gly Asp Val Tyr Leu Phe Gln Ala Thr Ser Gln Thr
   595 600 605
   Asp Leu Glu Asn Trp Val Thr Ala Ile His Ser Ala Cys Ala Ser Leu
   610 615 620
   Phe Ala Lys Lys His Gly Lys Glu Asp Thr Val Arg Leu Leu Lys Asn
   625 630 635 640
   Gln Thr Lys Asn Leu Leu Gln Lys Ile Asp Met Asp Ser Lys Met Lys
   645 650 655
   Lys Met Ala Glu Leu Gln Leu Ser Val Val Ser Asp Pro Lys Asn Arg
   660 665 670
   Lys Ala Ile Glu Asn Gln Ile Gln Gln Trp Glu Gln Asn Leu Glu Lys
   675 680 685
   Phe His Met Asp Leu Phe Arg Met Arg Cys Tyr Leu Ala Ser Leu Gln
   690 695 700
   Gly Gly Glu Leu Pro Asn Pro Lys Ser Leu Leu Ala Ala Thr Ser Arg
   705 710 715 720
   Pro Ser Lys Leu Ala Leu Gly Arg Leu Gly Ile Leu Ser Val Ser Ser
   725 730 735
   Phe His Ala Leu Ile Cys Ser Arg Asp Glu Ser Ala Leu Arg Lys Arg
   740 745 750
   Thr Leu Ser Leu Thr Gln Gln Gly Gln Ser Lys Lys Gly Leu Phe Ser
   755 760 765
   Ser Leu Lys Gly Leu Asp Thr Leu Ala Arg Lys Gly Lys Asp Lys Arg
   770 775 780
   Pro Ser Ile Thr Gln Val Asp Glu Phe Gln His Ile Tyr Ser Ser Ala
   785 790 795 800
   Ser Asp Ser Gly Pro Arg Glu Asn Ile Trp Glu Ser Gln Thr Tyr Val
   805 810 815
   His Phe Gln Asp Asn Gln Gly Val Thr Leu Ile Ile Lys Pro Glu His
   820 825 830
   Arg Val Glu Asp Ile Leu Ser Leu Ser Cys Lys Met Lys Gln Leu Asp
   835 840 845
   Pro Ser Tyr Tyr Gly Leu Gln Leu Arg Arg Leu Val Asp Asp Asn Phe
   850 855 860
   Glu Tyr Ser Ile Pro Ala Leu Tyr Glu Tyr Met Gln Glu Gln Ala Ile
   865 870 875 880
   Val Tyr Asp Glu Ile Glu Ile Phe Pro Leu Ser Ile Tyr His Val Leu
   885 890 895
   Leu Thr Lys Thr Gly Asn Ile Thr Asp Phe Gly Phe Ala Val Thr Ala
   900 905 910
   Gln Val Asp Glu His Gln His Leu Ser Arg Ile Phe Ile Ser Asp Val
   915 920 925
   Leu Pro Asp Gly Leu Ala Tyr Gly Glu Gly Leu Arg Thr Gly Asn Glu
   930 935 940
   Ile Leu Thr Ile Asn Gly Glu Val Ile Ser Asp Leu Asp Leu Arg Gln
   945 950 955 960
   Met Glu Ser Leu Phe Ser Glu Arg Ser Val Arg Leu Thr Leu Arg Thr
   965 970 975
   Asn Ser Ser Asp Thr Lys Arg Thr Leu Gly Thr Ser Trp Ser Asp Ser
   980 985 990
   Asp Leu Ser Arg Asp Gln Lys Asn Leu Leu Pro Pro Pro Asn Gln Ser
   995 1000 1005
   Gln Leu Leu Glu Glu Phe Leu Asp Asn Leu Lys Lys Asn Thr Ala
   1010 1015 1020
   Asn Asp Phe Ser Asn Val Pro Asp Val Thr Ala Gly Leu Lys Arg
   1025 1030 1035
   Ser Gln Thr Asp Gly Thr Leu Asp Gln Val Pro His Arg Glu Lys
   1040 1045 1050
   Thr Glu His Thr Phe Arg Ser Ala Glu Gln Ile Ala Ala Leu Cys
   1055 1060 1065
   Arg Ser Phe Asn Glu Val Gln Pro Ser Thr Asp Ser Met Glu Gly
   1070 1075 1080
   Pro Lys Glu Ala Gln Glu Pro Pro Pro Arg Pro Leu Ala Arg His
   1085 1090 1095
   Leu Ser Asp Ala Asp Arg Leu Arg Lys Val Ile Gln Glu Leu Met
   1100 1105 1110
   Asp Thr Glu Lys Ser Tyr Val Lys Asp Leu Thr Cys Leu Phe Glu
   1115 1120 1125
   Leu Tyr Leu Glu Pro Leu Gln Asn Glu Thr Phe Leu Thr Gln Asp
   1130 1135 1140
   Glu Met Glu Ser Leu Phe Gly Ser Leu Pro Glu Met Leu Glu Phe
   1145 1150 1155
   Gln Lys Val Phe Leu Glu Thr Leu Glu Asp Gly Ile Ser Ser Ser
   1160 1165 1170
   Ser Asp Phe Asn Ile Leu Glu Thr Pro Ser Gln Phe Arg Lys Leu
   1175 1180 1185
   Leu Phe Ser Leu Gly Gly Ser Phe Leu Tyr Tyr Thr Asp His Phe
   1190 1195 1200
   Lys Leu Tyr Ser Gly Phe Cys Ala Asn His Ile Lys Val Gln Lys
   1205 1210 1215
   Val Leu Glu Arg Ala Lys Thr Asp Lys Ala Phe Lys Ala Phe Leu
   1220 1225 1230
   Asp Ala Arg Asn Pro Thr Lys Gln His Ala Ser Thr Leu Glu Ser
   1235 1240 1245
   Tyr Leu Ile Lys Pro Val Gln Arg Val Leu Lys Tyr Pro Leu Leu
   1250 1255 1260
   Leu Lys Glu Leu Val Ser Leu Thr Asp Asn Glu Ser Glu Glu His
   1265 1270 1275
   Tyr His Leu Thr Glu Ala Leu Lys Ala Met Glu Lys Val Ala Ser
   1280 1285 1290
   His Ile Asn Glu Met Gln Lys Ile Tyr Glu Asp Tyr Gly Thr Val
   1295 1300 1305
   Phe Asp Gln Leu Val Ala Glu Gln Ser Gly Thr Glu Lys Glu Val
   1310 1315 1320
   Thr Glu Leu Ser Met Gly Glu Leu Leu Leu His Ser Thr Val Ser
   1325 1330 1335
   Trp Leu Asn Pro Phe Leu Ser Leu Gly Lys Ala Arg Lys Asp Leu
   1340 1345 1350
   Glu Leu Thr Val Phe Val Phe Lys Arg Ala Val Ile Leu Val Tyr
   1355 1360 1365
   Lys Glu Asn Cys Lys Leu Lys Lys Lys Leu Pro Ser Ser Ser Arg
   1370 1375 1380
   Pro Thr His Val His Gly Asp Leu Asp Pro Phe Lys Phe Arg Trp
   1385 1390 1395
   Leu Ile Pro Leu Ser Ala Leu Gln Val Arg Leu Gly Asn Ala Ala
   1400 1405 1410
   Gly Thr Glu Asn Asn Ser Ile Trp Glu Leu Ile His Thr Lys Ser
   1415 1420 1425
   Glu Ile Glu Gly Arg Pro Glu Thr Thr Phe Gln Leu Cys Cys Ser
   1430 1435 1440
   Asp Cys Glu Ser Lys Thr Asn Ile Val Lys Val Ile Arg Ser Ile
   1445 1450 1455
   Leu Arg Glu Asn Phe Arg Arg His Ile Lys Ser Glu Leu Pro Leu
   1460 1465 1470
   Glu Lys Thr Cys Lys Asp Arg Leu Ile Pro Leu Lys Asn Arg Ile
   1475 1480 1485
   Pro Val Ser Ala Lys Leu Ala Ser Ser Arg Ser Leu Arg Val Leu
   1490 1495 1500
   Lys Asn Ser Pro Ser Asn Glu Trp Asn Ser Asp Thr Gly Lys Gly
   1505 1510 1515
   Asn Leu Leu Asp Ser Asp Glu Cys Ser Leu Ser Ser Ser Thr Gln
   1520 1525 1530
   Ser Ser Gly Cys His Thr Thr Glu Ser Arg Gln Glu Ser Lys Asp
   1535 1540 1545
   Ser Ser Pro Glu Lys Tyr Pro Gln Thr Cys Ser Ser Asp Phe Ser
   1550 1555 1560
   Asp Ser Leu Ile Lys Glu Ser Asp Ile Leu Ser Asp Glu Asp Asp
   1565 1570 1575
   Asp Phe His Gln Thr Leu Lys Lys Gly Ser Pro Thr Lys Asp Ile
   1580 1585 1590
   Glu Ile Gln Phe Gln Arg Leu Lys Ile Ser Glu Asp Ser Glu Gly
   1595 1600 1605
   Asp Ser Thr Ala Asp Gln Gln Pro Arg Thr Glu Val Gly Asn Asn
   1610 1615 1620
   Leu Asn Ser Val Ala His Pro Lys Leu Val Arg Gly His Phe Cys
   1625 1630 1635
   Pro Ile Lys Arg Lys Ala Asn Ser Thr Lys Arg Asp Arg Gly Thr
   1640 1645 1650
   Leu Leu Thr Leu Gln Ala Arg His Gln Ser Leu Asp Ser Lys Ser
   1655 1660 1665
   Glu Asn Ala Asn Ile Asp Leu Asn Ser Ile Leu Glu Arg Glu Phe
   1670 1675 1680
   Ser Val Gln Ser Leu Thr Ser Val Val Asn Glu Glu Cys Phe Tyr
   1685 1690 1695
   Glu Thr Asp Ser His Gly Lys Ser
   1700 1705
<210> 89
   <211> 1730
   <212> PRT
   <213> Tiam2 Monodelphis domestica transcript variant 3; deduced from ENSMODP00000010258
<400> 89
   Val Thr Met Gly Asn Ser Glu Ser Gln Tyr Ser Leu Gln Gly Ser Lys
   1               5                   10                  15
   Asn His Ser Ser Ser Ser Thr Gly Ala Lys Gln Lys Pro Cys Pro Leu
               20                  25                  30
   Lys Ile Arg Ser Ile His Ala Lys Asp Glu Lys Ser Cys Ser Leu His
           35                  40                  45
   Gly Trp Gly His Gly Asn Ser Gly Thr Asn Tyr Lys Ser Arg Ser Leu
       50                  55                  60
   Ala Arg Ser Cys Leu Ser His Phe Lys Ser Asn Gln Pro Tyr Ser Ser
   65                  70                  75                  80
   Arg Leu Asp Pro Met Ala Lys Val Ser Lys Gly Ser Ala His Thr Lys
                   85                  90                  95
   His Lys Gly Asn Val Ser Gly Asn Tyr Phe Gln Gly Lys Asn Ser Ala
               100                 105                 110
   Phe Leu Ser Glu Asn Gly Phe His Tyr Ile Ser His Glu Pro Ala Asp
           115                 120                 125
   Asn His Ile Thr Ser Arg Asp Cys Asn Gly His Leu Leu Asn Cys Tyr
       130                 135                 140
   Gly Lys Asn Glu Ser Leu Ala Ser Thr Pro Pro Ala Glu Asp Arg Lys
   145                 150                 155                 160
   Ser Pro Lys Val Leu Ile Lys Thr Leu Gly Lys Leu Asp Gly Cys Leu
                   165                 170                 175
   Arg Val Glu Phe His Asn Ser Ser Asn Asn Lys Val Pro Thr Glu Asp
               180                 185                 190
   Ser Thr Gly Pro Val Gln Leu Leu Arg Tyr Ser Pro Thr Leu Glu Ser
           195                 200                 205
   Glu Thr Ser Asn Leu Thr Glu Ile Arg Arg Asn Ser Ser Thr Asp Tyr
       210                 215                 220
   Ser Ala Ser His Cys Leu Ser Pro Thr Asp Ser Arg Leu Arg Ser Ser
   225 230 235 240
   Lys Gly Ser Ser Phe Ser Ser Glu Ser Ser Trp Tyr Asp Ser Pro Trp
   245 250 255
   Gly Asn Ala Gly Asp Ile Asn Glu Leu Glu Gly Pro Tyr Leu Thr Arg
   260 265 270
   Ser Thr Pro Asp Thr Ser Ile His Ser Ser Phe Pro Ser Asp Asp Ala
   275 280 285
   Lys Lys Pro Phe Asn Gln Ser Ser Ser Leu Ser Ser Leu Arg Glu Leu
   290 295 300
   Tyr Lys Asp Ala Thr Leu Glu Ser Leu Pro Pro Ser Gly Ile Arg Phe
   305 310 315 320
   Ser Asp Glu Tyr Ile Gly Thr His Ala Ser Leu Asn Ser Arg Val Ser
   325 330 335
   Phe Val Ser Asp Ile Asp Val Pro Ser Arg Ala Glu Gln Arg Gly Pro
   340 345 350
   Val Gln Tyr Cys Ser Tyr Thr Leu Pro Cys Arg Lys Ser Lys Pro Leu
   355 360 365
   Thr Glu Asp Thr Ser Lys Lys Asp Thr Leu Lys Thr Arg Met Arg Arg
   370 375 380
   Ile Ser Asp Trp Thr Gly Ser Leu Ser Arg Lys Lys Arg Lys Leu Gln
   385 390 395 400
   Glu Pro Lys Ser Lys Asp Gly Tyr Asp His Leu Glu Asn Arg Thr Asp
   405 410 415
   Gly Leu Ser Ala Glu Val Leu Val Pro Ser Gln Leu Ser Ser Leu Leu
   420 425 430
   Trp Pro Gly Gly Ser Gly Gln Thr Leu Pro Gln Arg Ser Glu Ser Thr
   435 440 445
   Asn Ala Ile Ser Ser Asp Pro Leu Arg Gln Asn Ile Tyr Glu Asn Phe
   450 455 460
   Met Arg Glu Leu Glu Met Ser Arg Thr His Val Asp Asn Thr Glu Thr
   465 470 475 480
   Ser Thr Asp Thr Ala Asp Ser Ser Ser Asp Ser Leu Ser Ser Leu Glu
   485 490 495
   Gln Leu Asp Leu Leu Phe Glu Lys Glu Gln Gly Val Val Arg Lys Ala
   500 505 510
   Gly Trp Leu Phe Phe Lys Pro Leu Val Thr Leu Gln Lys Glu Lys Lys
   515 520 525
   Leu Glu Leu Val Ala Arg Arg Lys Trp Lys Gln Tyr Trp Val Thr Leu
   530 535 540
   Lys Gly Cys Thr Leu Leu Phe Tyr Glu Thr Tyr Gly Arg Asn Ser Met
   545 550 555 560
   Asp Gln Ser Ser Ser Pro Arg Cys Ala Leu Phe Ala Glu Asp Ser Val
   565 570 575
   Val Gln Ser Val Pro Glu His Pro Lys Lys Glu Asn Val Phe Cys Leu
   580 585 590
   Ser Asn Ser Phe Gly Asp Val Tyr Leu Phe Gln Ala Thr Ser Gln Thr
   595 600 605
   Asp Leu Glu Asn Trp Val Thr Ala Ile His Ser Ala Cys Ala Ser Leu
   610 615 620
   Phe Ala Lys Lys His Gly Lys Glu Asp Thr Val Arg Leu Leu Lys Asn
   625 630 635 640
   Gln Thr Lys Asn Leu Leu Gln Lys Ile Asp Met Asp Ser Lys Met Lys
   645 650 655
   Lys Met Ala Glu Leu Gln Leu Ser Val Val Ser Asp Pro Lys Asn Arg
   660 665 670
   Lys Ala Ile Glu Asn Gln Ile Gln Gln Trp Glu Gln Asn Leu Glu Lys
   675 680 685
   Phe His Met Asp Leu Phe Arg Met Arg Cys Tyr Leu Ala Ser Leu Gln
   690 695 700
   Gly Gly Glu Leu Pro Asn Pro Lys Ser Leu Leu Ala Ala Thr Ser Arg
   705 710 715 720
   Pro Ser Lys Leu Ala Leu Gly Arg Leu Gly Ile Leu Ser Val Ser Ser
   725 730 735
   Phe His Ala Leu Ile Cys Ser Arg Asp Glu Ser Ala Leu Arg Lys Arg
               740                 745                 750
   Thr Leu Ser Leu Thr Gln Gln Gly Gln Ser Lys Lys Gly Leu Phe Ser
           755                 760                 765
   Ser Leu Lys Gly Leu Asp Thr Leu Ala Arg Lys Gly Lys Asp Lys Arg
       770                 775                 780
   Pro Ser Ile Thr Gln Ile Phe Asp Ser Ser Gly Asn His Gly Phe Pro
   785                 790                 795                 800
   Gly Thr Gln Leu Pro Gln Ser Ser Asn Asn Ser Ser Glu Val Asp Glu
                   805                 810                 815
   Phe Gln His Ile Tyr Ser Ser Ala Ser Asp Ser Gly Pro Arg Glu Asn
               820                 825                 830
   Ile Trp Glu Ser Gln Thr Tyr Val His Phe Gln Asp Asn Gln Gly Val
           835                 840                 845
   Thr Leu Ile Ile Lys Pro Glu His Arg Val Glu Asp Ile Leu Ser Leu
       850                 855                 860
   Ser Cys Lys Met Lys Gln Leu Asp Pro Ser Tyr Tyr Gly Leu Gln Leu
   865                 870                 875                 880
   Arg Arg Leu Val Asp Asp Asn Phe Glu Tyr Ser Ile Pro Ala Leu Tyr
                   885                 890                 895
   Glu Tyr Met Gln Glu Gln Ala Lys Val Tyr Asp Glu Ile Glu Ile Phe
               900                 905                 910
   Pro Leu Ser Ile Tyr His Val Leu Leu Thr Lys Thr Gly Asn Ile Thr
           915                 920                 925
   Asp Phe Gly Phe Ala Val Thr Ala Gln Val Asp Glu His Gln His Leu
       930                 935                 940
   Ser Arg Ile Phe Ile Ser Asp Val Leu Pro Asp Gly Leu Ala Tyr Gly
   945                 950                 955                 960
   Glu Gly Leu Arg Thr Gly Asn Glu Ile Leu Thr Ile Asn Gly Glu Val
                   965                 970                 975
   Ile Ser Asp Leu Asp Leu Arg Gln Met Glu Ser Leu Phe Ser Glu Arg
               980                 985                 990
   Ser Val Arg Leu Thr Leu Arg Thr Asn Ser Ser Asp Thr Lys Arg Thr
   995 1000 1005
   Leu Gly Thr Ser Trp Ser Asp Ser Asp Leu Ser Arg Asp Gln Lys
   1010 1015 1020
   Asn Leu Leu Pro Pro Pro Asn Gln Ser Gln Leu Leu Glu Glu Phe
   1025 1030 1035
   Leu Asp Asn Leu Lys Lys Asn Thr Ala Asn Asp Phe Ser Asn Val
   1040 1045 1050
   Pro Asp Val Thr Ala Gly Leu Lys Arg Ser Gln Thr Asp Gly Thr
   1055 1060 1065
   Leu Asp Gln Val Pro His Arg Glu Lys Thr Glu His Thr Phe Arg
   1070 1075 1080
   Ser Ala Glu Gln Ile Ala Ala Leu Cys Arg Ser Phe Asn Glu Val
   1085 1090 1095
   Gln Pro Ser Thr Asp Ser Met Glu Gly Pro Lys Glu Ala Gln Glu
   1100 1105 1110
   Pro Pro Pro Arg Pro Leu Ala Arg His Leu Ser Asp Ala Asp Arg
   1115 1120 1125
   Leu Arg Lys Val Ile Gln Glu Leu Met Asp Thr Glu Lys Ser Tyr
   1130 1135 1140
   Val Lys Asp Leu Thr Cys Leu Phe Glu Leu Tyr Leu Glu Pro Leu
   1145 1150 1155
   Gln Asn Glu Thr Phe Leu Thr Gln Asp Glu Met Glu Ser Leu Phe
   1160 1165 1170
   Gly Ser Leu Pro Glu Met Leu Glu Phe Gln Lys Val Phe Leu Glu
   1175 1180 1185
   Thr Leu Glu Asp Gly Ile Ser Ser Ser Ser Asp Phe Asn Ile Leu
   1190 1195 1200
   Glu Thr Pro Ser Gln Phe Arg Lys Leu Leu Phe Ser Leu Gly Gly
   1205 1210 1215
   Ser Phe Leu Tyr Tyr Thr Asp His Phe Lys Leu Tyr Ser Gly Phe
   1220 1225 1230
   Cys Ala Asn His Ile Lys Val Gln Lys Val Leu Glu Arg Ala Lys
   1235 1240 1245
   Thr Asp Lys Ala Phe Lys Ala Phe Leu Asp Ala Arg Asn Pro Thr
   1250 1255 1260
   Lys Gln His Ala Ser Thr Leu Glu Ser Tyr Leu Ile Lys Pro Val
   1265 1270 1275
   Gln Arg Val Leu Lys Tyr Pro Leu Leu Leu Lys Glu Leu Val Ser
   1280 1285 1290
   Leu Thr Asp Asn Glu Ser Glu Glu His Tyr His Leu Thr Glu Ala
   1295 1300 1305
   Leu Lys Ala Met Glu Lys Val Ala Ser His Ile Asn Glu Met Gln
   1310 1315 1320
   Lys Ile Tyr Glu Asp Tyr Gly Thr Val Phe Asp Gln Leu Val Ala
   1325 1330 1335
   Glu Gln Ser Gly Thr Glu Lys Glu Val Thr Glu Leu Ser Met Gly
   1340 1345 1350
   Glu Leu Leu Leu His Ser Thr Val Ser Trp Leu Asn Pro Phe Leu
   1355 1360 1365
   Ser Leu Gly Lys Ala Arg Lys Asp Leu Glu Leu Thr Val Phe Val
   1370 1375 1380
   Phe Lys Arg Ala Val Ile Leu Val Tyr Lys Glu Asn Cys Lys Leu
   1385 1390 1395
   Lys Lys Lys Leu Pro Ser Ser Ser Arg Pro Thr His Val His Gly
   1400 1405 1410
   Asp Leu Asp Pro Phe Lys Phe Arg Trp Leu Ile Pro Leu Ser Ala
   1415 1420 1425
   Leu Gln Val Arg Leu Gly Asn Ala Ala Gly Thr Glu Asn Asn Ser
   1430 1435 1440
   Ile Trp Glu Leu Ile His Thr Lys Ser Glu Ile Glu Gly Arg Pro
   1445 1450 1455
   Glu Thr Thr Phe Gln Leu Cys Cys Ser Asp Cys Glu Ser Lys Thr
   1460 1465 1470
   Asn Ile Val Lys Val Ile Arg Ser Ile Leu Arg Glu Asn Phe Arg
   1475 1480 1485
   Arg His Ile Lys Ser Glu Leu Pro Leu Glu Lys Thr Cys Lys Asp
   1490 1495 1500
   Arg Leu Ile Pro Leu Lys Asn Arg Ile Pro Val Ser Ala Lys Leu
   1505 1510 1515
   Ala Ser Ser Arg Ser Leu Arg Val Leu Lys Asn Ser Pro Ser Asn
   1520 1525 1530
   Glu Trp Asn Ser Asp Thr Gly Lys Gly Asn Leu Leu Asp Ser Asp
   1535 1540 1545
   Glu Cys Ser Leu Ser Ser Ser Thr Gln Ser Ser Gly Cys His Thr
   1550 1555 1560
   Thr Glu Ser Arg Gln Glu Ser Lys Asp Ser Ser Pro Glu Lys Tyr
   1565 1570 1575
   Pro Gln Thr Cys Ser Ser Asp Phe Ser Asp Ser Leu Ile Lys Glu
   1580 1585 1590
   Ser Asp Ile Leu Ser Asp Glu Asp Asp Asp Phe His Gln Thr Leu
   1595 1600 1605
   Lys Lys Gly Ser Pro Thr Lys Asp Ile Glu Ile Gln Phe Gln Arg
   1610 1615 1620
   Leu Lys Ile Ser Glu Asp Ser Glu Gly Asp Ser Thr Ala Asp Gln
   1625 1630 1635
   Gln Pro Arg Thr Glu Val Gly Asn Asn Leu Asn Ser Val Ala His
   1640 1645 1650
   Pro Lys Leu Val Arg Gly His Phe Cys Pro Ile Lys Arg Lys Ala
   1655 1660 1665
   Asn Ser Thr Lys Arg Asp Arg Gly Thr Leu Leu Thr Leu Gln Ala
   1670 1675 1680
   Arg His Gln Ser Leu Asp Ser Lys Ser Glu Asn Ala Asn Ile Asp
   1685 1690 1695
   Leu Asn Ser Ile Leu Glu Arg Glu Phe Ser Val Gln Ser Leu Thr
   1700 1705 1710
   Ser Val Val Asn Glu Glu Cys Phe Tyr Glu Thr Asp Ser His Gly
   1715 1720 1725
   Lys Ser
   1730
<210> 90
   <211> 644
   <212> PRT
   <213> Tiam2 Pan troglodytes fragment; deduced from ENSPTRP00000044120
<400> 90
   Ser Ala Glu Gln Ile Thr Ala Leu Cys Arg Ser Phe Asn Asp Ser Gln
   1 5 10 15
   Ala Asn Gly Met Glu Gly Pro Arg Glu Ser Gln Asp Pro Pro Pro Arg
   20 25 30
   Pro Leu Ala Arg His Leu Ser Asp Ala Asp Arg Leu Arg Lys Val Ile
   35 40 45
   Gln Glu Leu Val Asp Thr Glu Lys Ser Tyr Val Lys Asp Leu Ser Cys
   50 55 60
   Leu Phe Glu Leu Tyr Leu Glu Pro Leu Gln Asn Glu Thr Phe Leu Thr
   65 70 75 80
   Gln Asp Glu Met Glu Ser Leu Phe Gly Ser Leu Pro Glu Met Leu Glu
   85 90 95
   Phe Gln Lys Val Phe Leu Glu Thr Leu Glu Asp Gly Ile Ser Ala Ser
   100 105 110
   Ser Asp Phe Asn Thr Leu Glu Thr Pro Ser Gln Phe Arg Lys Leu Leu
   115 120 125
   Phe Ser Leu Gly Gly Ser Phe Leu Tyr Tyr Ala Asp His Phe Lys Leu
   130 135 140
   Tyr Ser Gly Phe Cys Ala Asn His Ile Lys Val Gln Lys Val Leu Glu
   145 150 155 160
   Arg Ala Lys Thr Asp Lys Ala Phe Lys Ala Phe Leu Asp Thr Arg Asn
   165 170 175
   Pro Thr Lys Gln His Ser Ser Thr Leu Glu Ser Tyr Leu Ile Lys Pro
   180 185 190
   Val Gln Arg Val Leu Lys Tyr Pro Leu Leu Leu Lys Glu Leu Val Ser
   195 200 205
   Leu Thr Asp Gln Glu Ser Glu Glu His Tyr His Leu Thr Glu Ala Leu
   210 215 220
   Lys Ala Met Glu Lys Val Ala Ser His Ile Asn Glu Met Gln Lys Ile
   225 230 235 240
   Tyr Glu Asp Tyr Gly Thr Val Phe Asp Gln Leu Val Ala Glu Gln Ser
   245 250 255
   Gly Thr Glu Lys Glu Val Thr Glu Leu Ser Met Gly Glu Leu Leu Met
   260 265 270
   His Ser Thr Val Ser Trp Leu Asn Pro Phe Leu Ser Leu Gly Lys Ala
   275 280 285
   Arg Lys Asp Leu Glu Leu Thr Val Phe Val Phe Lys Arg Ala Val Ile
   290 295 300
   Leu Val Tyr Lys Glu Asn Cys Lys Leu Lys Lys Lys Leu Ala Ser Asn
   305 310 315 320
   Ser Arg Pro Ala His Asn Ser Thr Asp Leu Asp Pro Phe Lys Phe Arg
   325 330 335
   Trp Leu Ile Pro Ile Ser Ala Leu Gln Val Arg Leu Gly Asn Pro Ala
   340 345 350
   Gly Thr Glu Asn Asn Ser Ile Trp Glu Leu Ile His Thr Lys Ser Glu
   355 360 365
   Ile Glu Gly Arg Pro Glu Thr Ile Phe Gln Leu Cys Cys Ser Asp Ser
   370 375 380
   Glu Ser Lys Thr Asn Ile Val Lys Val Ile Arg Ser Ile Leu Arg Glu
   385 390 395 400
   Asn Phe Arg Arg His Ile Lys Cys Glu Leu Pro Leu Glu Lys Thr Cys
   405 410 415
   Lys Asp Arg Leu Val Pro Leu Lys Asn Arg Val Pro Val Ser Ala Lys
   420 425 430
   Leu Ala Ser Ser Arg Ser Leu Lys Val Leu Lys Asn Ser Ser Ser Asn
   435 440 445
   Glu Trp Thr Gly Glu Thr Gly Lys Gly Thr Leu Leu Asp Ser Asp Glu
           450                 455                 460
   Gly Ser Leu Ser Ser Ser Thr Gln Ser Ser Gly Cys Pro Thr Ala Gly
   465                 470                 475                 480
   Arg Gln Asp Ser Lys Ser Thr Ser Pro Gly Lys Tyr Pro His Pro Gly
                   485                 490                 495
   Leu Ala Asp Phe Ala Asp Asn Leu Ile Lys Glu Ser Asp Ile Leu Ser
               500                 505                 510
   Asp Glu Asp Asp Asp His Arg Gln Thr Val Lys Gln Gly Ser Pro Thr
           515                 520                 525
   Lys Asp Ile Glu Ile Gln Phe Gln Arg Leu Arg Ile Ser Glu Asp Pro
       530                 535                 540
   Asp Val His Pro Glu Ala Glu Gln Gln Pro Gly Pro Glu Ser Gly Glu
   545                 550                 555                 560
   Gly Gln Lys Gly Gly Glu Gln Pro Lys Leu Val Arg Gly His Phe Cys
                   565                 570                 575
   Pro Ile Lys Arg Lys Ala Asn Ser Thr Lys Arg Asp Arg Gly Thr Leu
               580                 585                 590
   Leu Lys Ala Gln Ile Arg His Gln Ser Leu Asp Ser Gln Ser Glu Asn
           595                 600                 605
   Ala Thr Ile Asp Leu Asn Ser Val Leu Glu Arg Glu Phe Ser Val Gln
       610                 615                 620
   Ser Leu Thr Ser Val Val Ser Glu Glu Cys Phe Tyr Glu Thr Glu Ser
   625                 630                 635                 640
   His Gly Lys Ser